# EUROPEAN PATENT APPLICATION

(11) **EP 4 357 348 A1**
(43) Date of publication of application: **24.04.2024**
(21) Application number: 22824264.0
(22) Date of filing: 16.06.2022
(51) Int. Cl.: C07D 491/22, C07K 16/28, A61K 39/395, A61P 35/00

(54) **ANTITUMOR COMPOUND AND USE THEREOF**

(30) Priority: 17.06.2021 CN 202110673571
(71) Applicant: Minghui Pharmaceutical (Hangzhou) Ltd., Hangzhou, Zhejiang 310018 (CN); Minghui Pharmaceutical (Shanghai) Ltd., Shanghai 201203 (CN)
(72) Inventor: LI, Ao, Hangzhou, Zhejiang 310018 (CN); CHEN, Yile, Hangzhou, Zhejiang 310018 (CN); CAO, Guoqing, Hangzhou, Zhejiang 310018 (CN)
(74) Representative: Mewburn Ellis LLP
(86) International application number: PCT/CN2022/099053
(87) International publication number: WO 2022/262789

(57) **Abstract**

The present application relates to an anti-tumor compound and use thereof, specifically, the present application relates to a ligand conjugate or a tautomer, a mesomer, a racemate, an enantiomer and a diastereomer thereof, or a mixture thereof, or a pharmaceutically acceptable salt thereof. The present application further relates to a method for preparing the ligand conjugate and the use thereof.

## Description

### Technical field

The present application relates to biomedical field, particularly relates to anti-tumor compounds and their applications.

### Background technique

Antibody-drug conjugate (ADC) links monoclonal antibodies or antibody fragments to biologically active cytotoxic agents *via* stable chemical linkers, which takes both the advantages of the specificity of antibody binding to antigens on the surface of normal and tumor cells and the efficiency of cytotoxic substances with anti-tumor effects. The application of the camptothecin derivative exatecan to antibody-coupled drugs (ADCs) has been reported in the literature, but further development of ADCs with better efficacy and safety is still needed in this field.

### Summary of the invention

In one aspect, the present application provides a ligand conjugate, or a tautomer, a mesomer, a racemate, an enantiomer, a diastereomer, or a mixture thereof, or a pharmaceutically acceptable salt or hydrate thereof, wherein the ligand conjugate comprises the structure shown in formula (I):

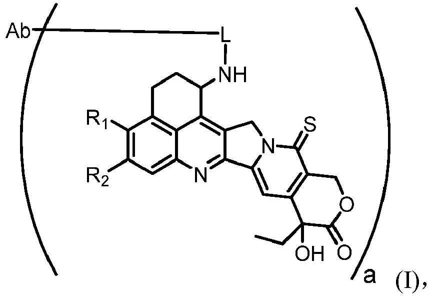

In one embodiment, said structure shown in formula (I) comprises the structure shown in (I-a):

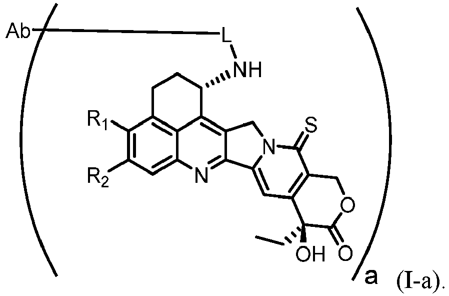

In one embodiment, said structure shown in formula (I) comprises the structure shown in (I-b): 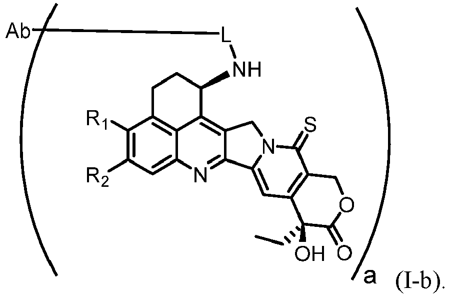
wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
L is an optionally substituted linker,
Ab is a ligand, "a" is a number greater than 0, and "a" is a decimal or integer.

In the structure shown in formula (I), said L is a linker -L₁- L₂-L₃-L₄-L₅-.

In the structure shown in formula (I), said L₁ is optionally substituted 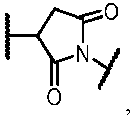 and L₁ is directly linked to Ab.

In the structure shown in formula (I), said L₂ is selected from the group consisting of optionally substituted -(CH₂)ₘ₁-X₁-(CH₂)ₘ₂-C(O)-, optionally substituted -(CH₂CH₂O)ₙ-C(O)-, and optionally substituted -(CH₂)ₚ-C(O)-,
X₁ is selected from the group consisting of -O-, optionally substituted -C(O)-NH-, optionally substituted aryl, optionally substituted heteroaryl, optionally substituted aliphatic heterocyclyl, and optionally substituted aliphatic cyclyl,
wherein, m1, m2, and n are each independently selected from integers of at least 0, and p is an integer selected from 2 to 8.

In the structure shown in formula (I), said L₂ is optionally substituted -(CH₂CH₂O)ₙ-C(O)-.

In the structure shown in formula (I), wherein n is 2.

In the structure shown in formula (I), said L₂ is optionally substituted -(CH₂)ₘ₁-X₁-(CH₂)ₘ₂-C(O)-.

In the structure shown in formula (I), X₁ is selected from the group consisting of -O- and optionally substituted -C(O)-NH-.

In the structure shown in formula (I), wherein, m1 is 2.

In the structure shown in formula (I), wherein, m2 is 2.

In the structure shown in formula (I), X₁ is selected from the group consisting of optionally substituted aryl and optionally substituted heteroaryl.

In the structure shown in formula (I), X₁ is optionally substituted phenyl.

In the structure shown in formula (I), X₁ is optionally substituted pyridinyl.

In the structure shown in formula (I), wherein, m1 is 0.

In the structure shown in formula (I), wherein, m2 is 1.

In the structure shown in formula (I), X₁ is optionally substituted

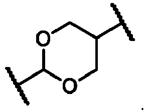

In the structure shown in formula (I), X₁ is optionally substituted cyclohexyl.

In the structure shown in formula (I), wherein, m1 is 1.

In the structure shown in formula (I), wherein, m2 is 0.

In the structure shown in formula (I), said L₂ is optionally substituted -(CH₂)ₚ-C(O)-.

In the structure shown in formula (I), wherein, p is 5.

In the structure shown in formula (I), said L₂ is a structure selected from the group consisting of optionally substituted 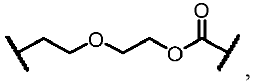 optionally substituted 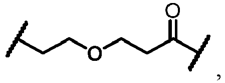 optionally substituted 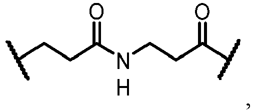 optionally substituted 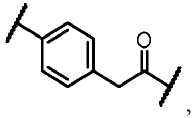 optionally substituted 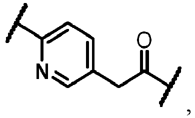 optionally substituted 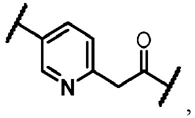 optionally substituted 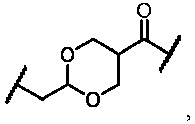 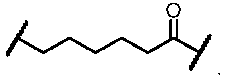 optionally substituted 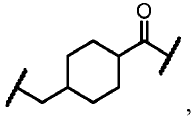 and optionally substituted

In the structure shown in formula (I), said L₃ is a peptide residue.

In the structure shown in formula (I),Said L₃ is a peptide residue is composed of amino acids selected from the group consisting of phenylalanine, isoleucine, leucine, tryptophan, valine, methionine, tyrosine, alanine, threonine, histidine, serine, glutamine, arginine, lysine, asparagine, glutamate, proline, citrulline, aspartate, and glycine.

In the structure shown in formula (I), said L₃ is a peptide residue is composed of amino acids selected from the group consisting of glycine, phenylalanine, valine, and citrulline.

In the structure shown in formula (I), said L₃ is a peptide residue selected from the group consisting of -glycine-phenylalanine-glycine- (-Gly-Phe-Gly-), -glycine-glycine-phenylalanine-glycine- (-Gly-Gly-Phe-Gly-), and -valine-citrulline- (-Val-Cit-).

In the structure shown in formula (I), said L₃ is a structure selected from the group consisting of 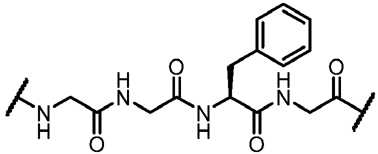 and

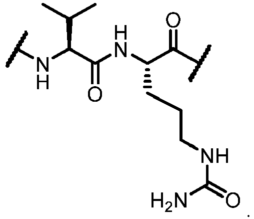

In the structure shown in formula (I), said L₄ is optionally substituted -L₄ₐ-NR₄-CH₂-L_{4b}-,
L₄ₐ is absent or L₄ₐ is optionally substituted 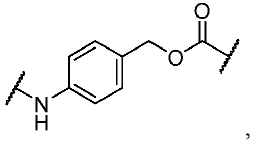
L_{4b} is absent or L_{4b} is optionally substituted 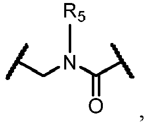
R₄ and R₅ are each independently selected from the group consisting of hydrogen and optionally substituted alkyl.

In the structure shown in formula (I), said L₄ is optionally substituted

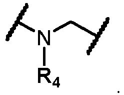

In the structure shown in formula (I), said L₄ is optionally substituted

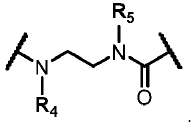

In the structure shown in formula (I), said L₄ is optionally substituted

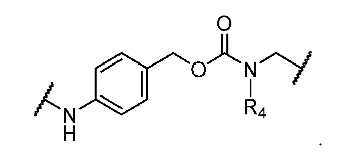

In the structure shown in formula (I), said L₄ is optionally substituted

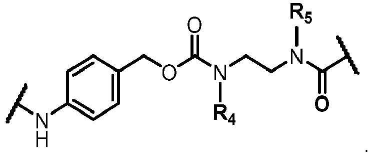

In the structure shown in formula (I), said R₄ is selected from the group consisting of hydrogen and optionally substituted methyl.

In the structure shown in formula (I), said R₅ is selected from the group consisting of hydrogen and optionally substituted methyl.

In the structure shown in formula (I), said L₄ is a structure selected from the group consisting of optionally substituted 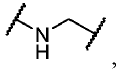 optionally substituted 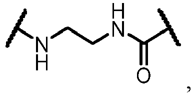 optionally substituted 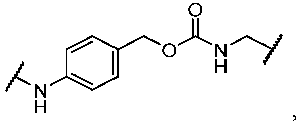 optionally substituted 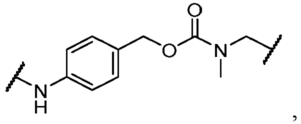 and optionally substituted

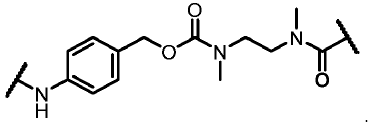

In the structure shown in formula (I), said L₅ is optionally substituted is directly linked to the following structure: 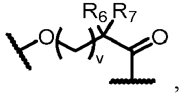 and L₅ 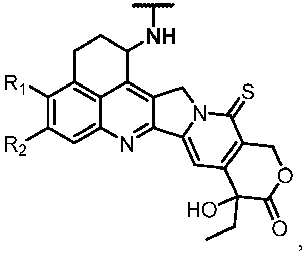
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl, or R₆ and R ₇ together with the atom to which they are attached form an optionally substituted cycloalkyl,
wherein, v is a number that is at least 0.

In the structure shown in formula (I), said L₅ is optionally substituted is directly linked to the following structure: 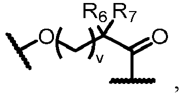 and L₅ 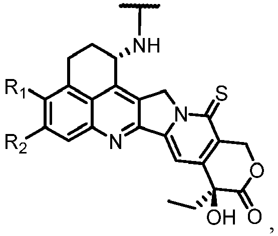
R₆ and R₇ are each independently selected from the group consisting of hydrogen, deuterium, optionally substituted alkyl, optionally substituted haloalkyl, optionally substituted cycloalkyl, and optionally substituted cycloalkyl-alkyl, or R₆ and R₇ together with the atom to which they are attached form an optionally substituted cycloalkyl,
wherein, v is a number that is at least 0.

In the structure shown in formula (I), said L₅ is optionally substituted is directly linked to the following structure: 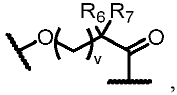 and L₅ 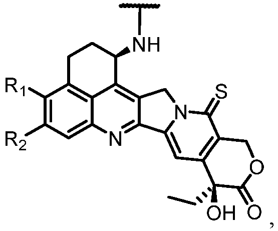
R₆ and R₇ are each independently selected from the group consisting of hydrogen, deuterium, optionally substituted alkyl, optionally substituted haloalkyl, optionally substituted cycloalkyl, and optionally substituted cycloalkyl-alkyl, or R₆ and R ₇ together with the atom to which they are attached form an optionally substituted cycloalkyl,
wherein, v is a number that is at least 0.

In the structure shown in formula (I), said R₆ is hydrogen.

In the structure shown in formula (I), said R₆ is optionally substituted methyl.

In the structure shown in formula (I), said R₆ is fluorine substituted methyl.

In the structure shown in formula (I), said R₆ is trifluoromethyl.

In the structure shown in formula (I), said R₆ is optionally substituted cycloalkyl.

In the structure shown in formula (I), said R₆ is optionally substituted cyclopropyl.

In the structure shown in formula (I), said R₇ is hydrogen.

In the structure shown in formula (I), R₆ and R₇ together with the atom to which they are attached form an optionally substituted cyclopropyl.

In the structure shown in formula (I), R₆ and R₇ together with the atom to which they are attached form an optionally substituted cyclobutyl.

In the structure shown in formula (I), wherein, v is 0.

In the structure shown in formula (I), wherein, v is 1.

In the structure shown in formula (I), said L₅ is a structure selected from the group consisting of optionally substituted 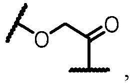 optionally substituted 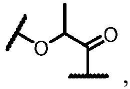 optionally substituted 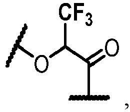 optionally substituted 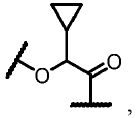 optionally substituted 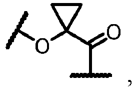 optionally substituted 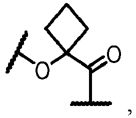 optionally substituted 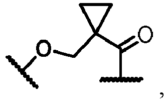 and optionally substituted

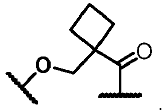

The ligand conjugate described in this application, wherein, said Ab is an antibody or antigen-binding fragment thereof.

The ligand conjugate described in this application, wherein, said antibody is selected from the group consisting of murine antibodies, chimeric antibodies, humanized antibodies, and fully human antibodies.

The ligand conjugate described in this application, wherein, said antibody is a monoclonal antibody.

The ligand conjugate described in this application, wherein, said the antigen-binding fragment is selected from the group consisting of Fab, Fab', Fv fragment, F(ab')₂, F(ab)₂, scFv, di-scFv, VHH and dAb.

The ligand conjugate described in this application, wherein, said Ab comprises HER2 antibodies and/or TROP2 antibodies.

The ligand conjugate described in this application, wherein, said Ab comprises HCDR1, HCDR2, and HCDR3 of heavy chain variable regions, the amino acid sequence of HCDR1 is shown in SEQ ID NO: 1, the amino acid sequence of HCDR2 is shown in SEQ ID NO: 2, and the amino acid sequence of HCDR3 is shown in SEQ ID NO: 3.

The ligand conjugate described in this application, wherein, said Ab comprises LCDR1, LCDR2, and LCDR3 of light chain variable regions, the amino acid sequence of LCDR1 is shown in SEQ ID NO: 4, the amino acid sequence of LCDR2 is shown in SEQ ID NO: 5, and the amino acid sequence of LCDR3 is shown in SEQ ID NO: 6.

The ligand conjugate described in this application, wherein, said Ab comprises a heavy chain variable region, and the amino acid sequence of the heavy chain variable region is shown in SEQ ID NO: 7.

The ligand conjugate described in this application, wherein, said Ab comprises a light chain variable region, and the amino acid sequence of the light chain variable region is shown in SEQ ID NO: 8.

The ligand conjugate described in this application, wherein, said Ab comprises a heavy chain, and the amino acid sequence of the heavy chain is shown in SEQ ID NO: 9.

The ligand conjugate described in this application, wherein, said Ab comprises a light chain, and the amino acid sequence of the light chain is shown in SEQ ID NO: 10.

The ligand conjugate described in this application, wherein, said Ab comprises HCDR1, HCDR2, and HCDR3 of heavy chain variable region, the amino acid sequence of HCDR1 is shown in SEQ ID NO: 11, the amino acid sequence of HCDR2 is shown in SEQ ID NO: 12, and the amino acid sequence of HCDR3 is shown in SEQ ID NO: 13.

The ligand conjugate described in this application, wherein, said Ab comprises LCDR1, LCDR2, and LCDR3 of light chain variable region, the amino acid sequence of LCDR1 is shown in SEQ ID NO: 14, the amino acid sequence of LCDR2 is shown in SEQ ID NO: 15, and the amino acid sequence of LCDR3 is shown in SEQ ID NO: 16.

The ligand conjugate described in this application, wherein, said Ab comprises a heavy chain variable region, and the amino acid sequence of the heavy chain variable region is shown in SEQ ID NO: 17.

The ligand conjugate described in this application, wherein, said Ab comprises a light chain variable region, and the amino acid sequence of the light chain variable region is shown in SEQ ID NO: 18.

The ligand conjugate described in this application, wherein, said Ab comprises a heavy chain, and the amino acid sequence of the heavy chain is shown in SEQ ID NO: 19.

The ligand conjugate described in this application, wherein, said Ab comprises a light chain, and the amino acid sequence of the light chain is shown in SEQ ID NO: 20.

In another aspect, the present application provides a ligand conjugate, or a tautomer, a mesomer, a racemate, an enantiomer, a diastereomer, or a mixture thereof, or a pharmaceutically acceptable salt or hydrate thereof, wherein the ligand conjugate is a structure selected from the group consisting of 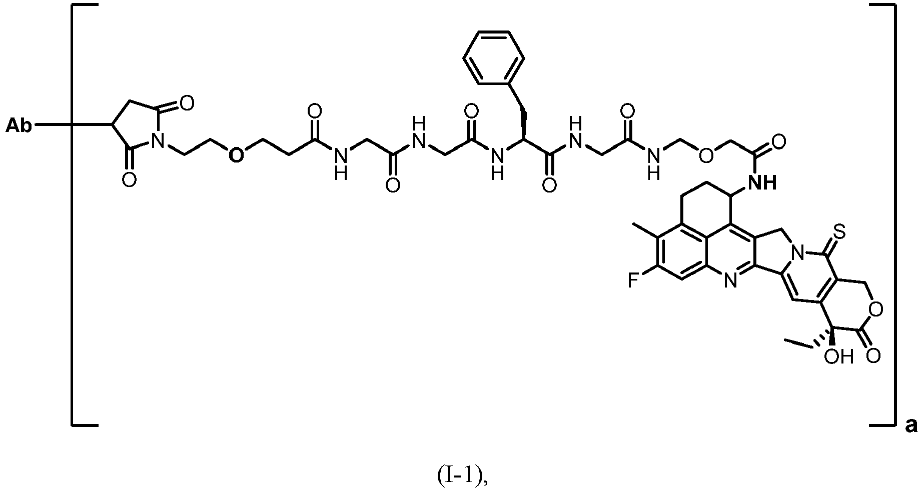 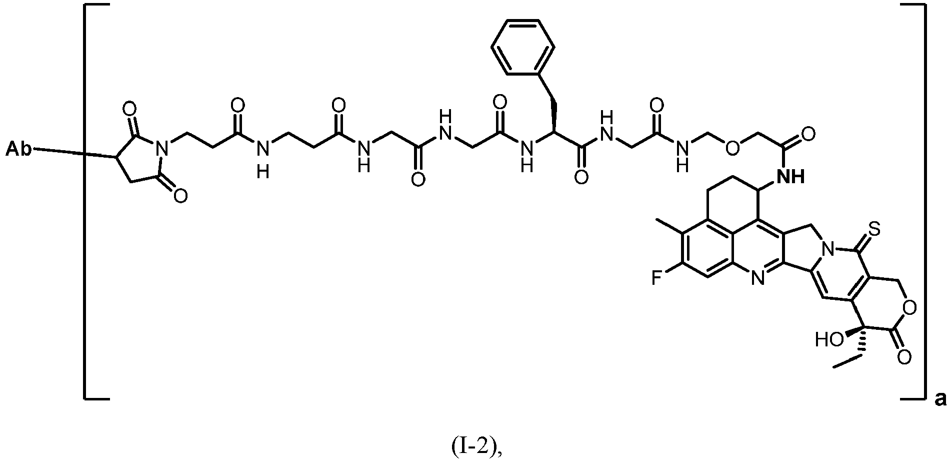 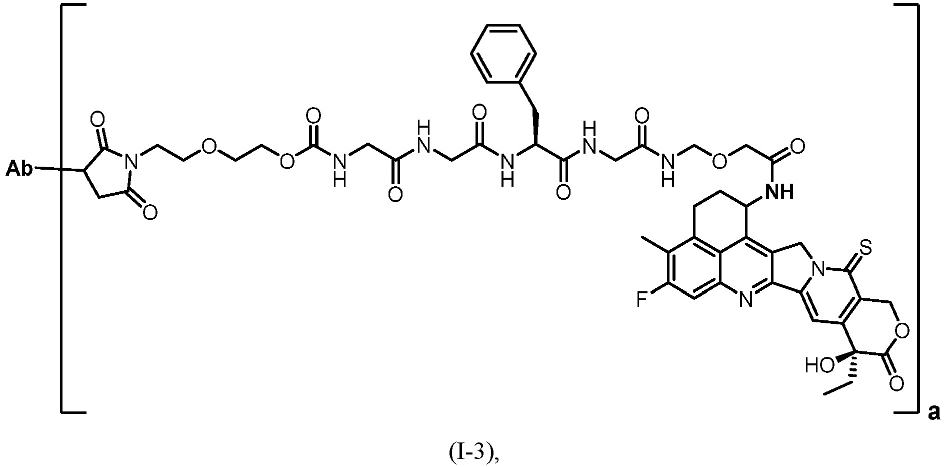 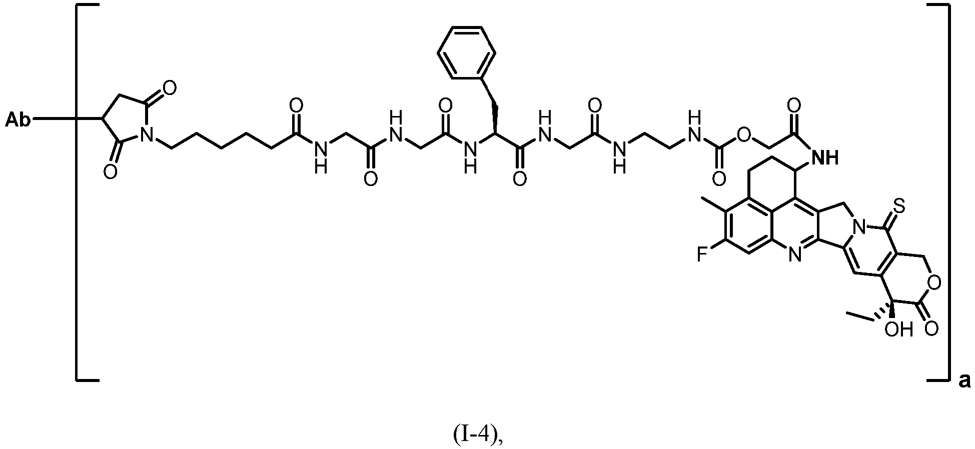 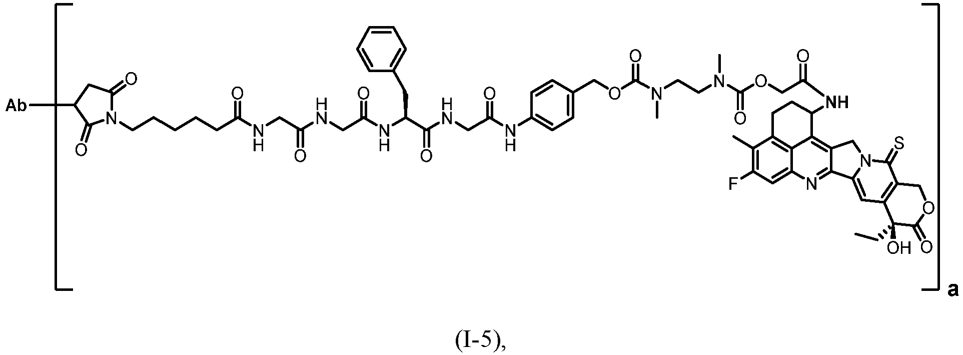 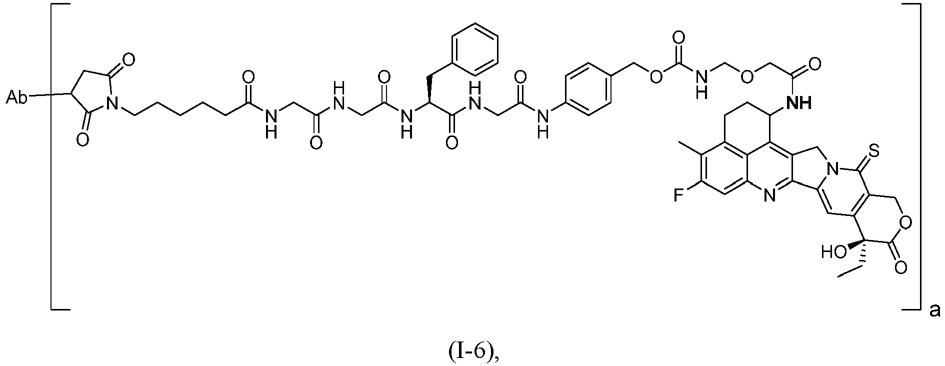 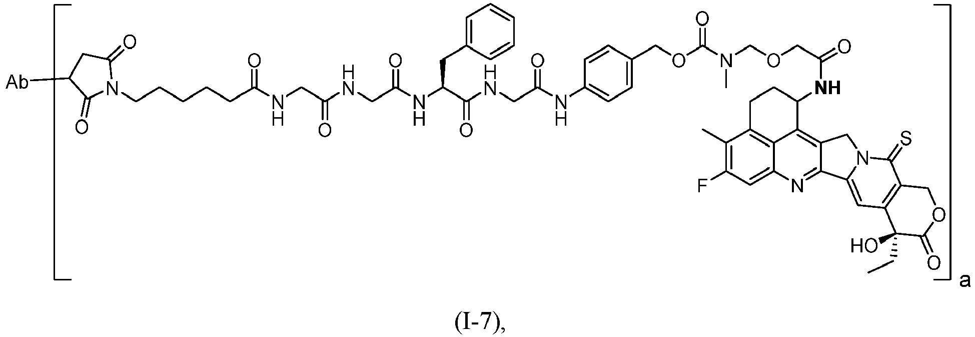 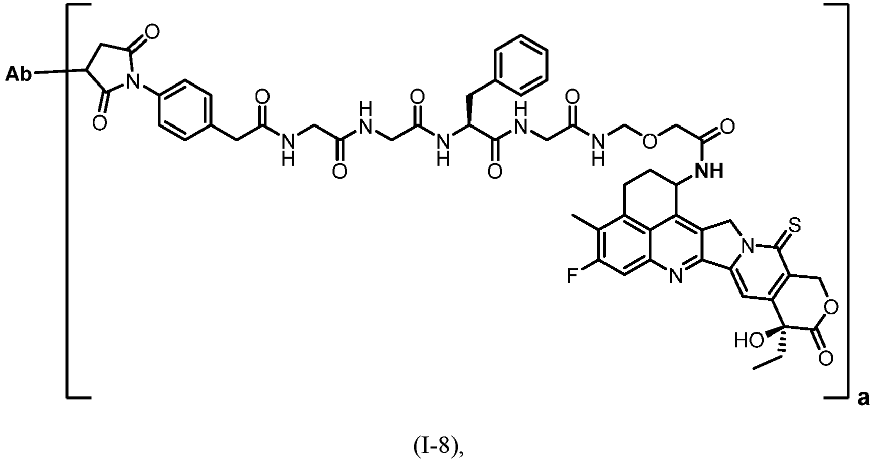 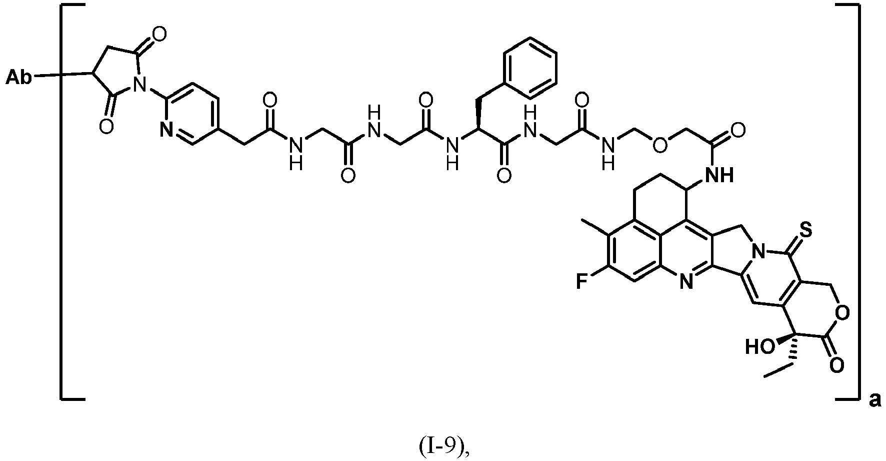 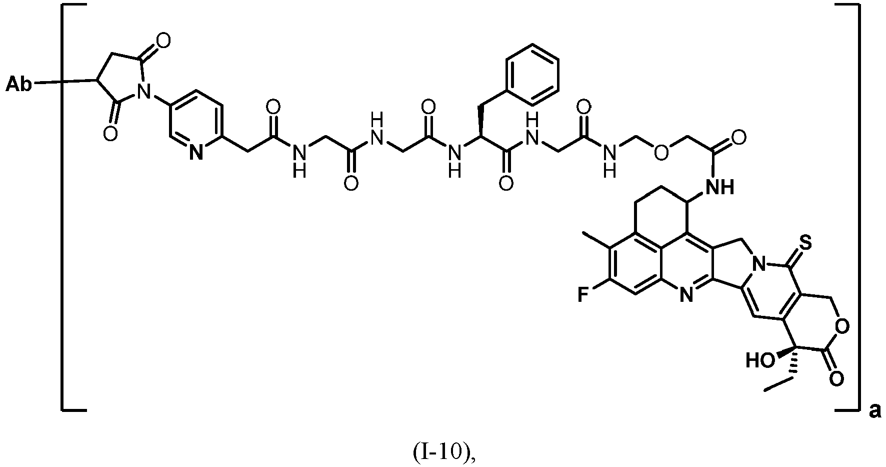 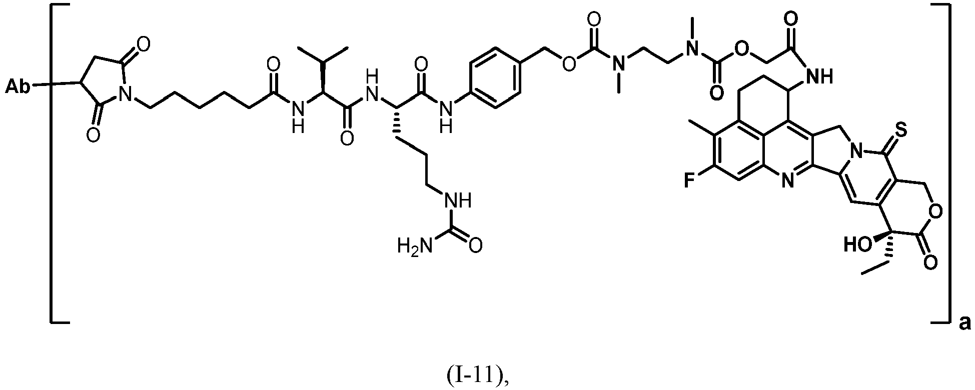 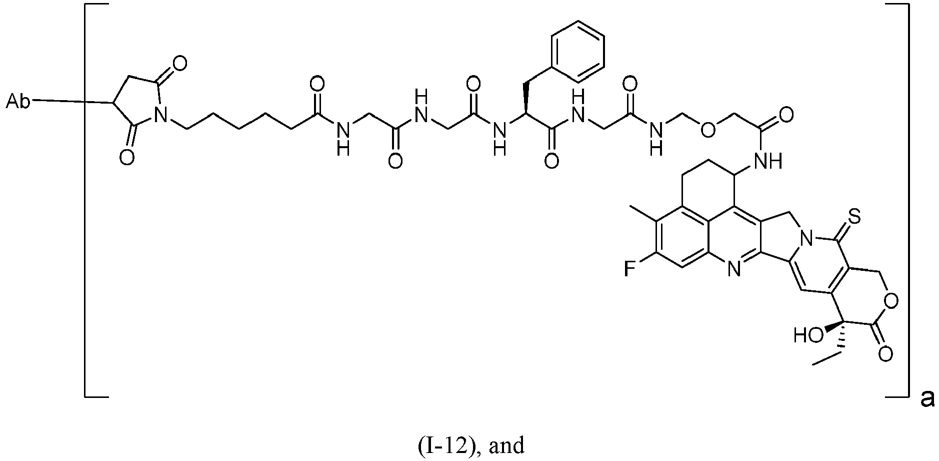 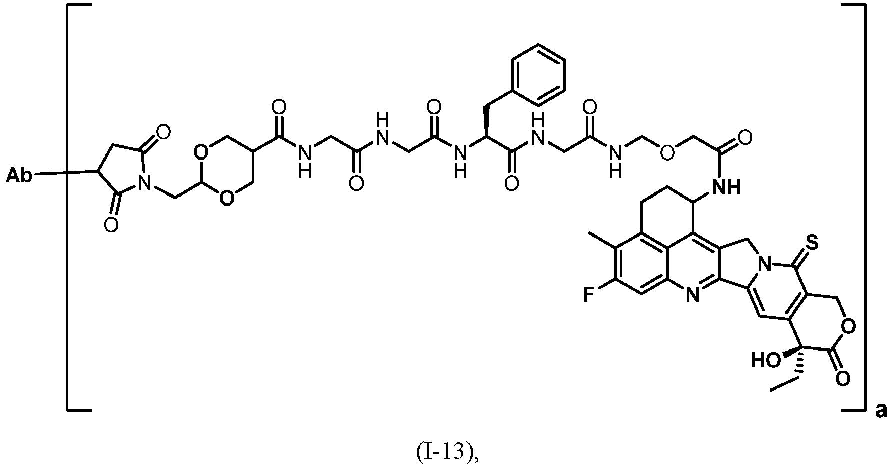 wherein, Ab is a ligand, "a" is a number greater than 0, and "a" is a decimal or integer.

In another aspect, the present application provides a compound, or a tautomer, a mesomer, a racemate, an enantiomer, a diastereomer, or a mixture thereof, or a pharmaceutically acceptable salt or hydrate thereof, wherein the compound comprises the structure shown in formula (II): 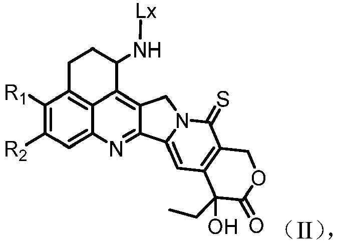 wherein,
R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
Lx is a linking group, and said Lx is L₁ₓ-L₂-L₃-L₄-L₅-, said L₁ₓ may be directly or indirectly linked to a ligand.

In one embodiment, said the structure shown in formula (II) comprises the structure shown in formula (II-a):

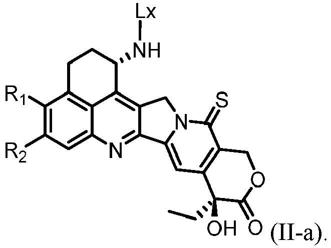

In one embodiment, said the structure shown in formula (II) comprises the structure shown in formula (II-b):

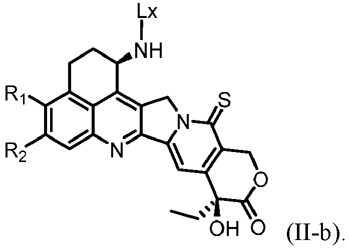

In the structure shown in formula (II), said L₁ₓ is capable of being linked directly to the thiol group of the ligand.

In the structure shown in formula (II), said L₁ₓ is optionally substituted

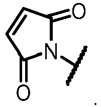

In the structure shown in formula (II), said L₂ is selected from the group consisting of optionally substituted -(CH₂)ₘ₁-X₁-(CH₂)ₘ₂-C(O)-, optionally substituted -(CH₂CH₂O)ₙ-C(O)-, and optionally substituted -(CH₂)ₚ-C(O)-,
X₁ is selected from the group consisting of -O-, optionally substituted -C(O)-NH-, optionally substituted aryl, optionally substituted heteroaryl, optionally substituted aliphatic heterocyclyl, and optionally substituted aliphatic cyclyl,
wherein, m1, m2, and n are each independently selected from integers at least 0, and p is an integer selected from 2 to 8.

In the structure shown in formula (II), said L₂ comprises optionally substituted -(CH₂CH₂O)ₙ-C(O)-.

In the structure shown in formula (II), wherein n is 2.

In the structure shown in formula (II), said L₂ is optionally substituted -(CH₂)ₘ₁-X₁-(CH₂)ₘ₂-C(O)-.

In the structure shown in formula (II), X₁ is selected from the group consisting of -O- and optionally substituted -C(O)-NH-.

In the structure shown in formula (II), wherein m1 is 2.

In the structure shown in formula (II), wherein m2 is 2.

In the structure shown in formula (II), X₁ is selected from the group consisting of optionally substituted aryl and optionally substituted heteroaryl.

In the structure shown in formula (II), X₁ is optionally substituted phenyl.

In the structure shown in formula (II), X₁ is optionally substituted pyridinyl.

In the structure shown in formula (II), wherein m1 is 0.

In the structure shown in formula (II), wherein m2 is 1.

In the structure shown in formula (II), X₁ is optionally substituted

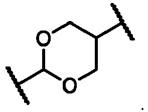

In the structure shown in formula (II), X₁ is optionally substituted cyclohexyl.

In the structure shown in formula (II), wherein m1 is 1.

In the structure shown in formula (II), wherein m2 is 0.

In the structure shown in formula (II), said L₂ is optionally substituted -(CH₂)ₚ-C(O)-.

In the structure shown in formula (II), wherein, p is 5.

In the structure shown in formula (II), said L₂ is a structure selected from the group consisting of optionally substituted 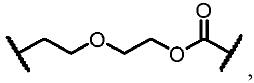 optionally substituted 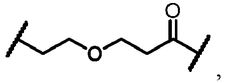 optionally substituted 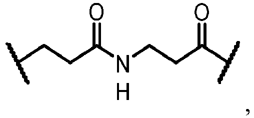 optionally substituted 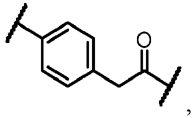 optionally substituted 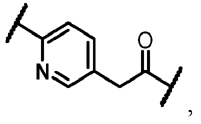 optionally substituted 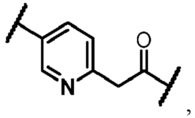 optionally substituted 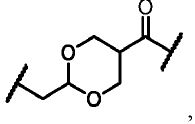 optionally substituted 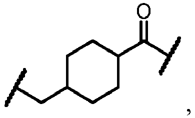 and optionally substituted

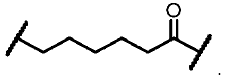

In the structure shown in formula (II), said L₃ is a peptide residue.

In the structure shown in formula (II), said L₃ is a peptide residue is composed of amino acids selected from the group consisting of phenylalanine, isoleucine, leucine, tryptophan, valine, methionine, tyrosine, alanine, threonine, histidine, serine, glutamine, arginine, lysine, asparagine, glutamate, proline, citrulline, aspartate, and glycine.

In the structure shown in formula (II), said L₃ is a peptide residue is composed of amino acids selected from the group consisting of glycine, phenylalanine, valine, and citrulline.

In the structure shown in formula (II), said L₃ is a peptide residue selected from the group consisting of -glycine-phenylalanine-glycine- (-Gly-Phe-Gly-), -glycine-glycine-phenylalanine-glycine- (-Gly-Gly-Phe-Gly-), and -valine-citrulline- (-Val-Cit-).

In the structure shown in formula (II), said L₃ comprises a structure selected from the group consisting of 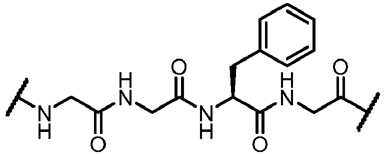 and

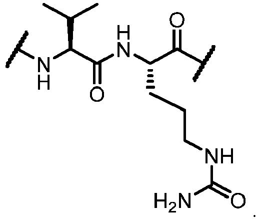

In the structure shown in formula (II), said L₄ is optionally substituted -L₄ₐ-NR₄-CH₂-L_{4b}-,
L₄ₐ is absent or L₄ₐ is optionally substituted 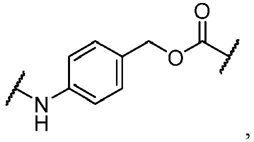
L_{4b} is absent or L_{4b} is optionally substituted 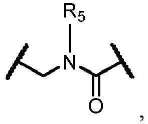
R₄ and R₅ are each independently selected from the group consisting of hydrogen and optionally substituted alkyl.

In the structure shown in formula (II), said L₄ is optionally substituted

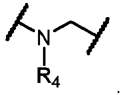

In the structure shown in formula (II), said L₄ is optionally substituted

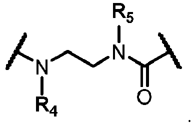

In the structure shown in formula (II), said L₄ is optionally substituted

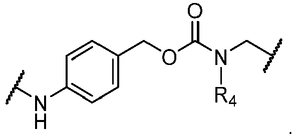

In the structure shown in formula (II), said L₄ is optionally substituted

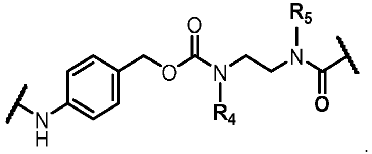

In the structure shown in formula (II), said R₄ is selected from the group consisting of hydrogen and optionally substituted methyl.

In the structure shown in formula (II), said R₅ is selected from the group consisting of hydrogen and optionally substituted methyl.

In the structure shown in formula (II), said L₄ is a structure selected from the group consisting of optionally substituted 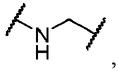 optionally substituted 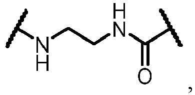 optionally substituted 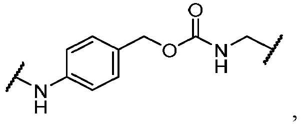 optionally substituted 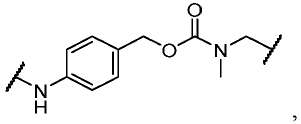 and optionally substituted

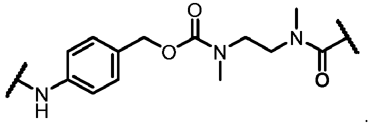

In the structure shown in formula (II), said L₅ is optionally substituted L₅ is directly linked to the following structure: 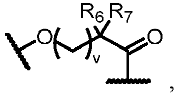 and 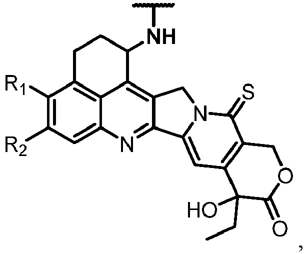
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl, or R₆ and R ₇ together with the atom to which they are attached form an optionally substituted cycloalkyl,
wherein, v is a number that is at least 0.

In the structure shown in formula (II), said L₅ is optionally substituted 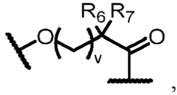 and L₅ is directly linked to the following structure: 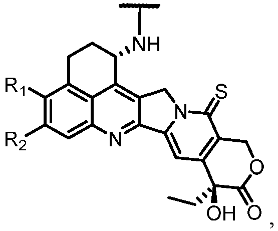
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl, or R₆ and R₇ together with the atom to which they are attached form an optionally substituted cycloalkyl,
wherein, v is a number that is at least 0.

In the structure shown in formula (II), said L₅ is optionally substituted L₅ is directly linked to the following structure: 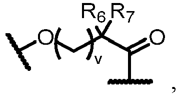 and 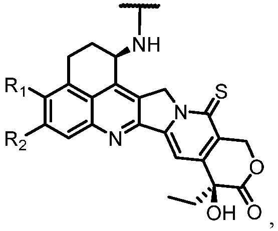
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl, or R₆ and R ₇ together with the atom to which they are attached form an optionally substituted cycloalkyl,
wherein, v is a number that is at least 0.

In the structure shown in formula (II), said R₆ is hydrogen.

In the structure shown in formula (II), said R₆ is optionally substituted methyl.

In the structure shown in formula (II), wherein R₆ is fluorine substituted methyl.

In the structure shown in formula (II), said R₆ is trifluoromethyl.

In the structure shown in formula (II), said R₆ is optionally substituted cycloalkyl.

In the structure shown in formula (II), said R₆ is optionally substituted cyclopropyl.

In the structure shown in formula (II), said R₇ is hydrogen.

In the structure shown in formula (II), R₆ and R₇ together with the atom to which they are attached form an optionally substituted cyclopropyl.

In the structure shown in formula (II), R₆ and R₇ together with the atom to which they are attached form an optionally substituted cyclobutyl.

In the structure shown in formula (II), wherein, v is 0.

In the structure shown in formula (II), wherein, v is 1.

In the structure shown in formula (II), said L₅ is a structure selected from the group consisting of optionally substituted 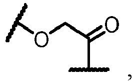 optionally substituted 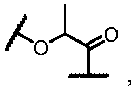 optionally substituted 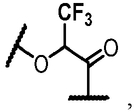 optionally substituted 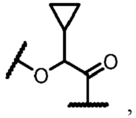 optionally substituted 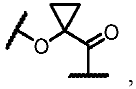 optionally substituted 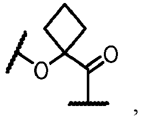 optionally substituted 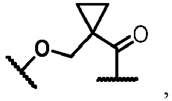 and optionally substituted

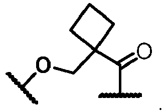

In another aspect, the present application provides a compound, or a tautomer, a mesomer, a racemate, an enantiomer, a diastereomer, or a mixture thereof, or a pharmaceutically acceptable salt or hydrate thereof, wherein the said compound is selected from the group consisting of

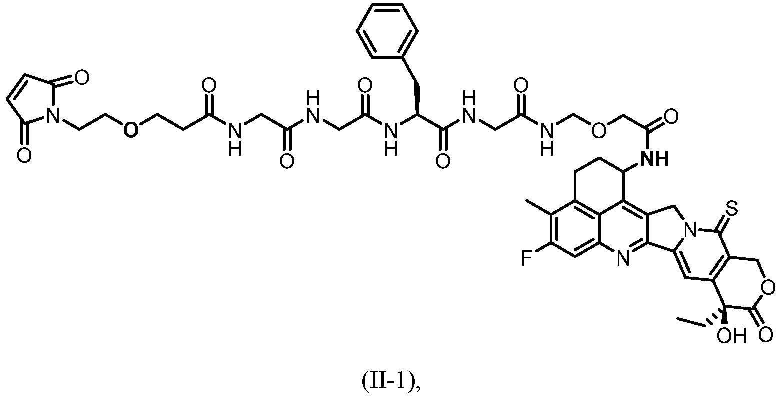

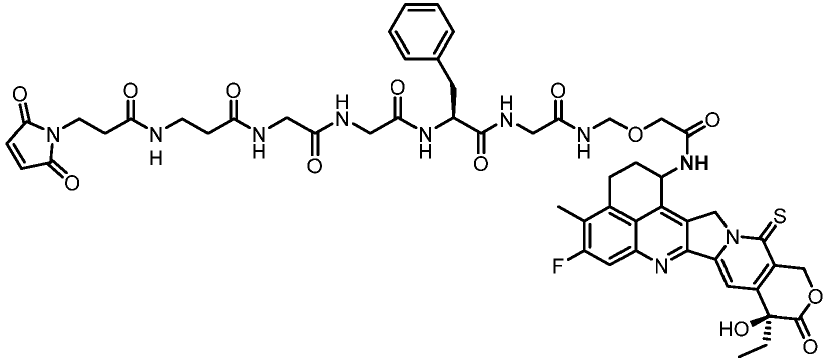

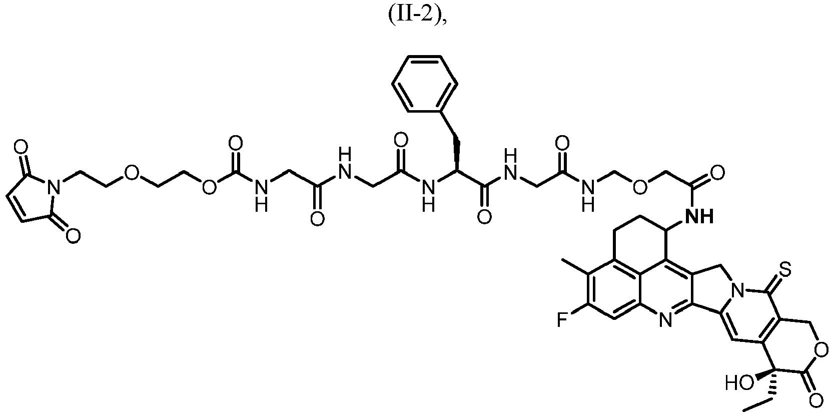

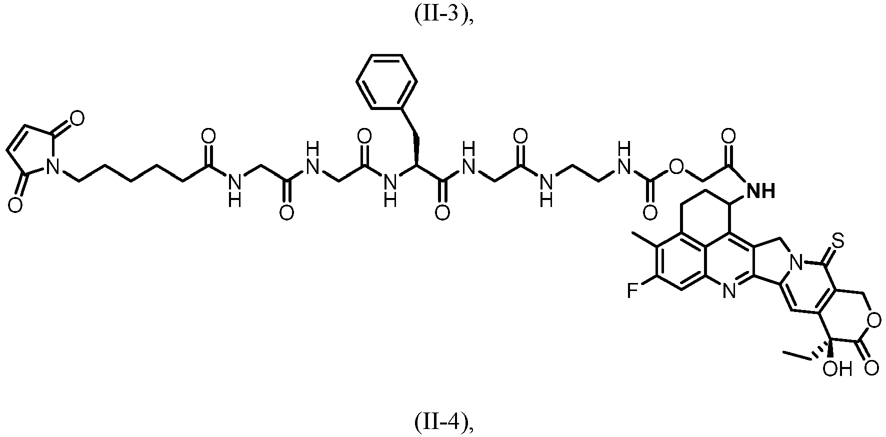

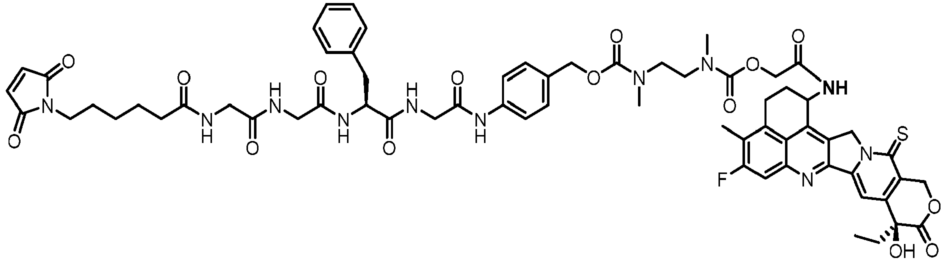

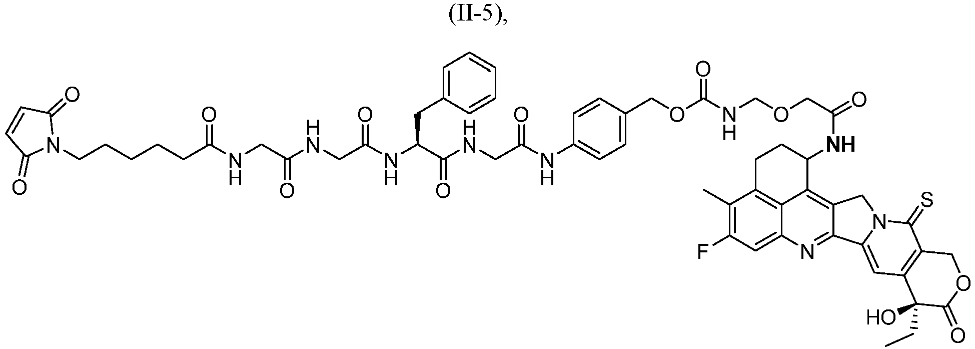

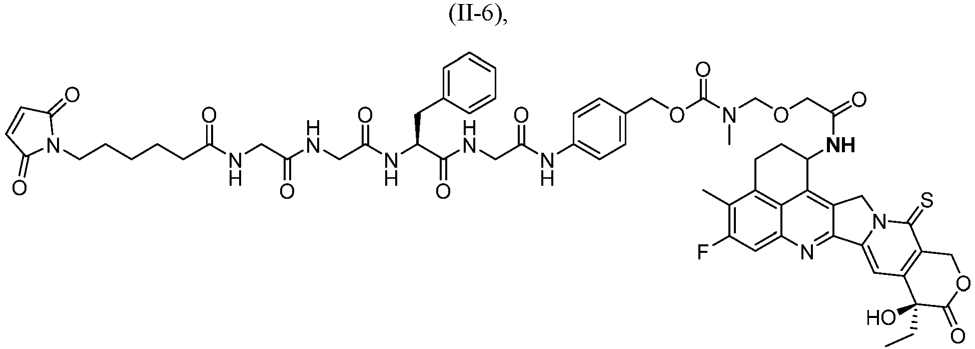

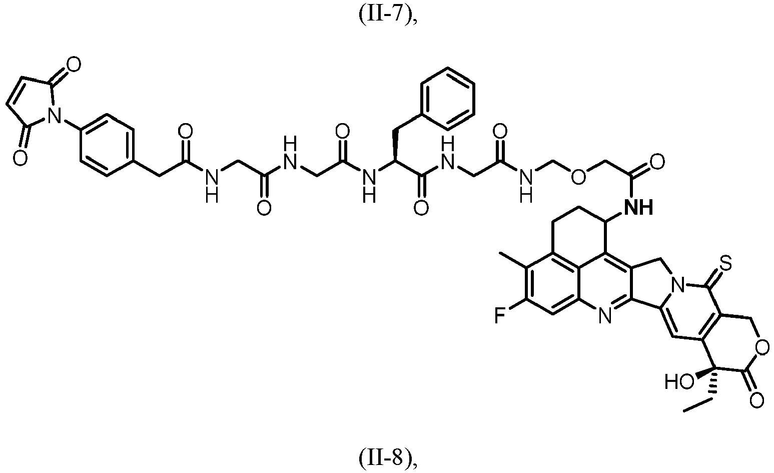

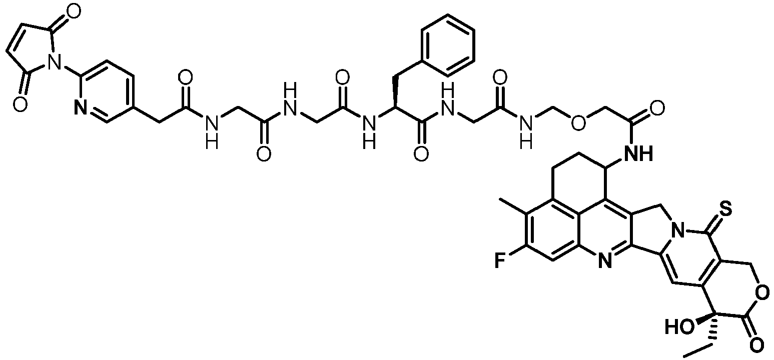

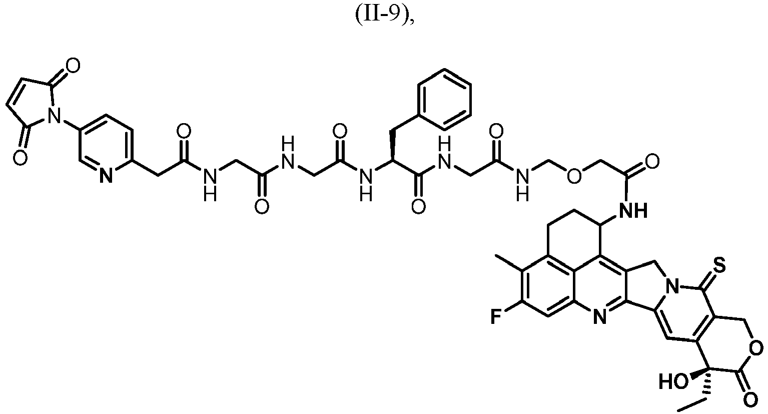

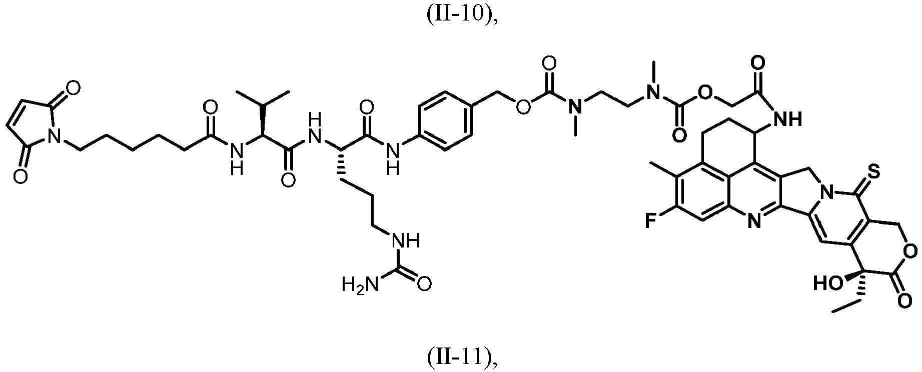

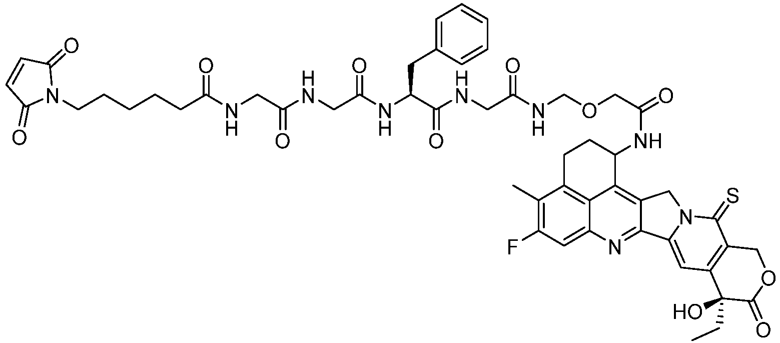

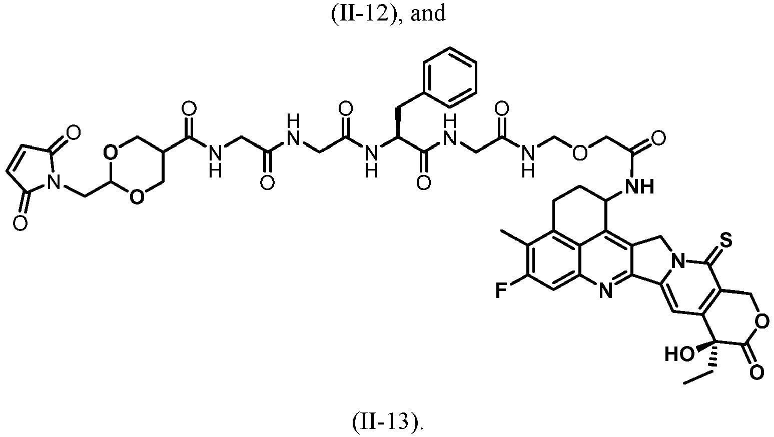

In another aspect, the present application provides a linker as represented in formula (L-1), for obtaining a ligand-drug conjugate formed by linking a drug unit to a ligand *via* the said linker:

-L₁-L₂-L₃-L₄-L₅- (L-1),

wherein L₁ is optionally substituted 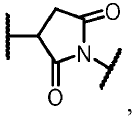
said L₂ is selected from the group consisting of optionally substituted -(CH₂)ₘ₁-X₁-(CH₂)ₘ₂-C(O)-, and optionally substituted -(CH₂CH₂O)ₙ-C(O)-,
X₁ is selected from the group consisting of -O-, optionally substituted -C(O)-NH-, optionally substituted aryl, optionally substituted heteroaryl, optionally substituted aliphatic heterocyclyl, and optionally substituted aliphatic cyclyl,
wherein, m1, m2, and n are each independently selected from numbers that is at least 0.

In the structure shown in formula (L-1), wherein, n is 2.

In the structure shown in formula (L-1), wherein X₁ is selected from the group consisting of -O- and optionally substituted -C(O)-NH-.

In the structure shown in formula (L-1), wherein, m1 is 2.

In the structure shown in formula (L-1), wherein, m2 is 2.

In the structure shown in formula (L-1), wherein X₁ is selected from the group consisting of optionally substituted aryl and optionally substituted heteroaryl.

In the structure shown in formula (L-1), wherein X₁ is optionally substituted phenyl.

In the structure shown in formula (L-1), wherein X₁ is optionally substituted pyridinyl.

In the structure shown in formula (L-1), wherein, m1 is 0.

In the structure shown in formula (L-1), wherein, m2 is 1.

In the structure shown in formula (L-1), wherein X₁ is optionally substituted

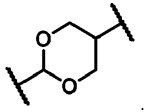

In the structure shown in formula (L-1), wherein X₁ is optionally substituted cyclohexyl.

In the structure shown in formula (L-1), wherein, m1 is 1.

In the structure shown in formula (L-1), wherein, m2 is 0.

In the structure shown in formula (L-1), said L₂ is a structure selected from the group consisting of optionally substituted 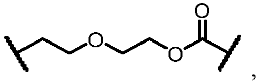 optionally substituted 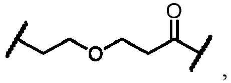 optionally substituted 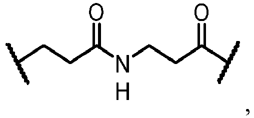 optionally substituted 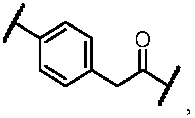 optionally substituted 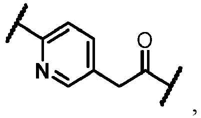 optionally substituted 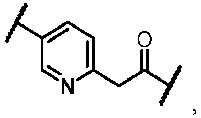 optionally substituted 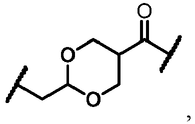 and optionally substituted

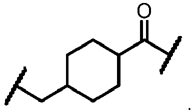

In the structure shown in formula (L-1), said L₃ is a peptide residue selected from the group consisting of -glycine-phenylalanine-glycine- (-Gly-Phe-Gly-), -glycine-glycine-phenylalanine-glycine- (-Gly-Gly-Phe-Gly-), and -valine-citrulline- (-Val-Cit-).

In the structure shown in formula (L-1), said L₃ is a structure selected from the group consisting of 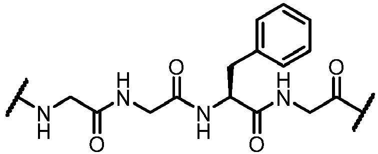 and

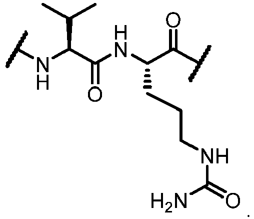

In the structure shown in formula (L-1), said L₄ is optionally substituted -L₄ₐ-NR₄-CH₂-L_{4b}-,
L₄ₐ is absent or L₄ₐ is optionally substituted 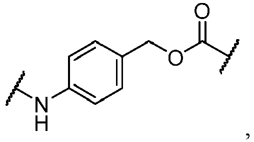
L_{4b} is absent or L_{4b} is optionally substituted 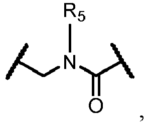
R₄ and R₅ are each independently selected from the group consisting of hydrogen and optionally substituted alkyl.

In the structure shown in formula (L-1), said L₄ is optionally substituted

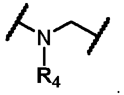

In the structure shown in formula (L-1), said L₄ is a structure selected from the group consisting of optionally substituted 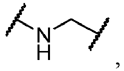 optionally substituted 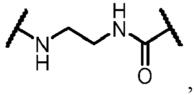 optionally substituted 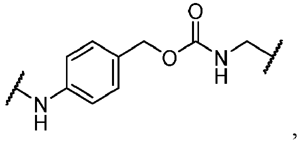 optionally substituted 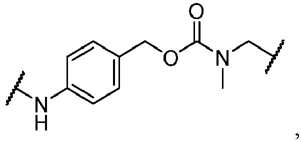 and optionally substituted

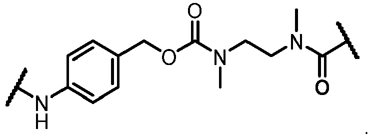

In the structure shown in formula (L-1), said L₅ is optionally substituted said L₅ is directly linked to the following structure: 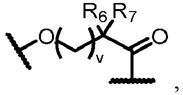 and 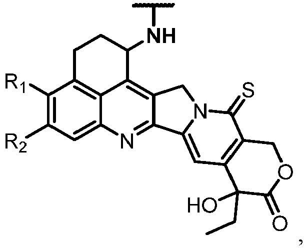
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl, or R₆ and R₇ together with the atom to which they are attached form an optionally substituted cycloalkyl,
wherein, v is a number that is at least 0.

In the structure shown in formula (L-1), said L₅ is optionally substituted 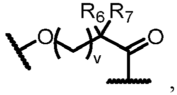 and said L₅ is directly linked to the following structure: 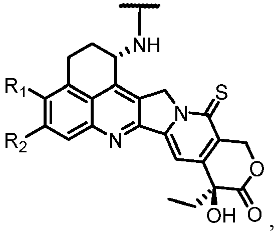
R₆ and R₇ are each independently selected from the group consisting of hydrogen, deuterium, optionally substituted alkyl, optionally substituted haloalkyl, optionally substituted cycloalkyl, and optionally substituted cycloalkyl-alkyl, or R₆ and R₇ together with the atom to which they are attached form an optionally substituted cycloalkyl,
wherein, v is a number that is at least 0.

In the structure shown in formula (L-1), wherein L₅ is optionally substituted and L₅ is directly linked to the following structure: 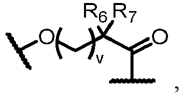 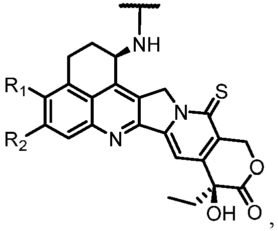
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl, or R₆ and R₇ together with the atom to which they are attached form an optionally substituted cycloalkyl,
wherein, v is a number that is at least 0.

In the structure shown in formula (L-1), said R₆ is hydrogen.

In the structure shown in formula (L-1), said R₆ is optionally substituted methyl.

In the structure shown in formula (L-1), said R₆ is fluorine substituted methyl.

In the structure shown in formula (L-1), said R₆ is trifluoromethyl.

In the structure shown in formula (L-1), said R₆ is optionally substituted cycloalkyl.

In the structure shown in formula (L-1), said R₇ is hydrogen.

In the structure shown in formula (L-1), R₆ and R₇ together with the atom to which they are attached form an optionally substituted cyclopropyl.

In the structure shown in formula (L-1), R₆ and R₇ together with the atom to which they are attached form an optionally substituted cyclobutyl.

In the structure shown in formula (L-1), wherein v is 0.

In the structure shown in formula (L-1), wherein v is 1.

In the structure shown in formula (L-1), said L₅ a structure selected from the group consisting of optionally substituted 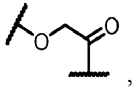 optionally substituted 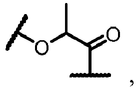 optionally substituted 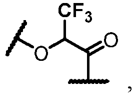 optionally substituted 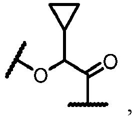 optionally substituted 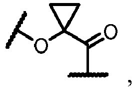 optionally substituted 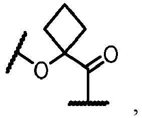 optionally substituted 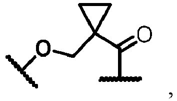 and optionally substituted

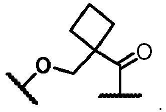

In another aspect, the present application provides a linker, or a tautomer, a mesomer, a racemate, an enantiomer, a diastereomer, or a mixture thereof, or a pharmaceutically acceptable salt or hydrate thereof, wherein said the linker comprises structures selected from the group consisting of

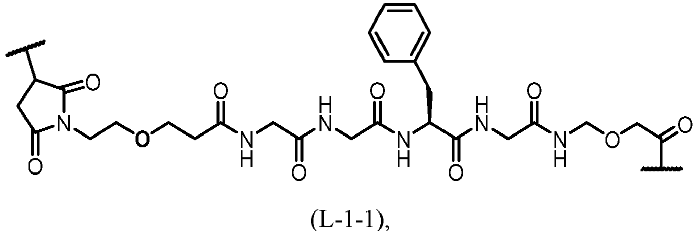

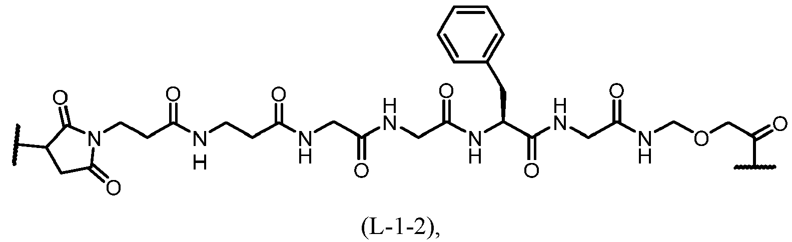

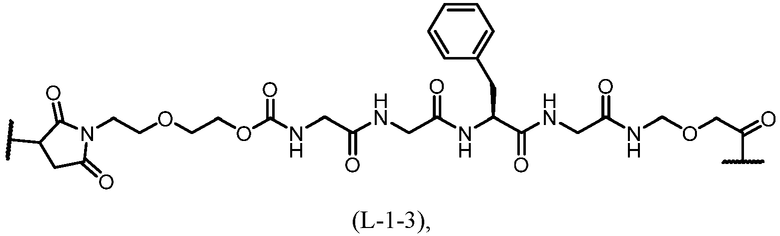

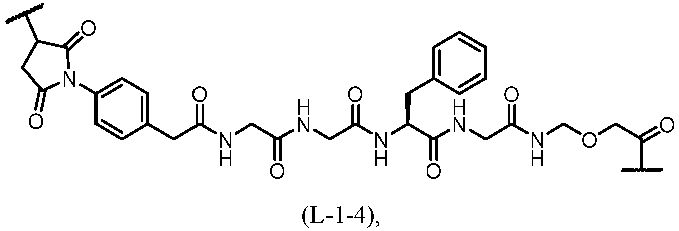

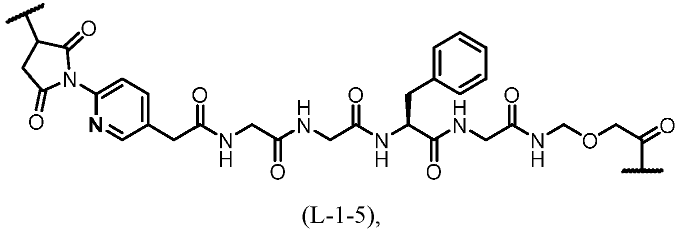

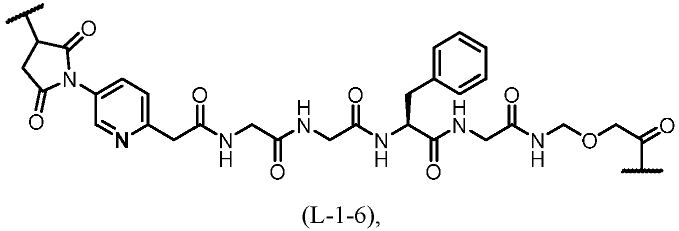

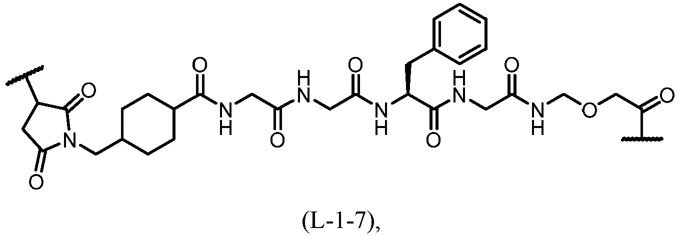

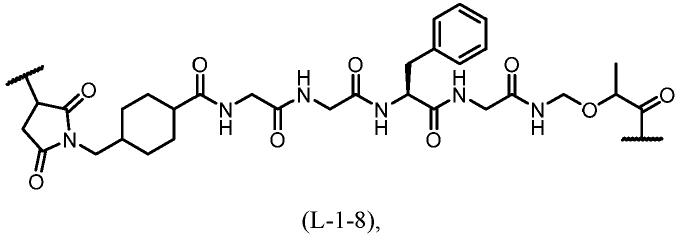

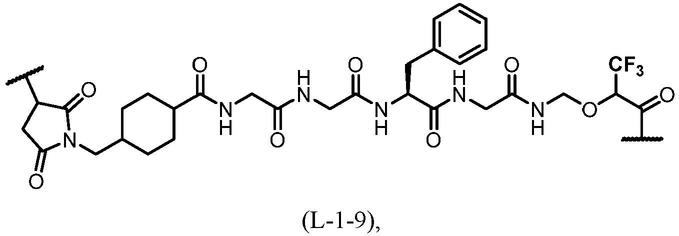

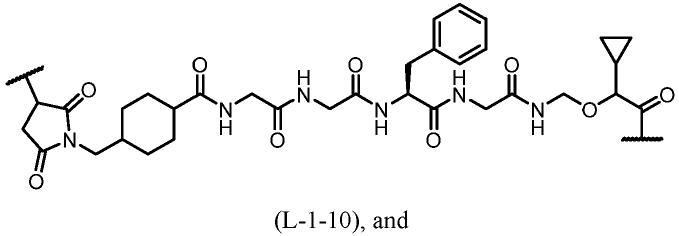

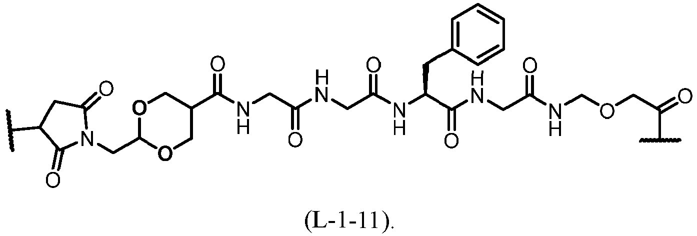

In another aspect, the present application provides a linker as represented in formula (L-2), for obtaining a ligand-drug conjugate formed by linking a drug unit to a ligand *via* the said linker:

-L₁-L₂-L₃-L₄-L₅- (L-2),

wherein L₁ is optionally substituted 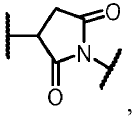
L₂ is a structure selected from the group consisting of optionally substituted 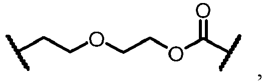 optionally substituted 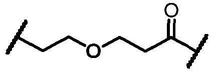 , optionally substituted 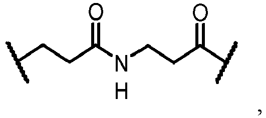 optionally substituted 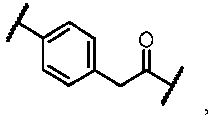 optionally substituted 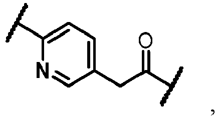 optionally substituted 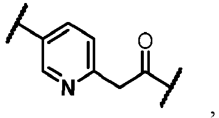 optionally substituted 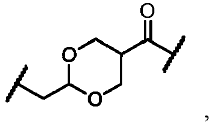 optionally substituted 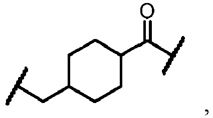 and optionally substituted 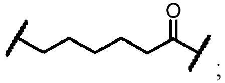
L₃ is a structure selected from the group consisting of 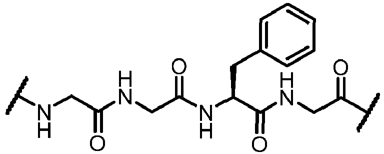 and 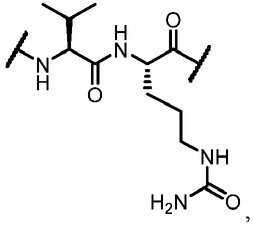
L₄ is optionally substituted -L₄ₐ-NR₄-CH₂-L_{4b}-,
L₄ₐ is absent or L₄ₐ is optionally substituted 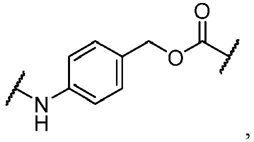
L_{4b} is absent or L_{4b} is optionally substituted 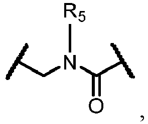 L₄ₐ and L_{4b} are not absent at the same time,
R₄ and R₅ are each independently selected from the group consisting of hydrogen and optionally substituted alkyl,
Said L₅ is a structure selected from the group consisting of optionally substituted 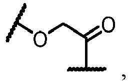 optionally substituted 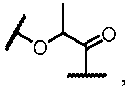 optionally substituted 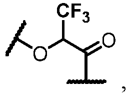 optionally substituted 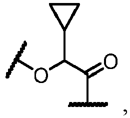 optionally substituted 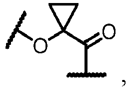 optionally substituted 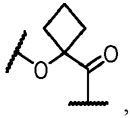 optionally substituted 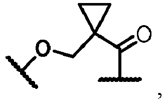 and optionally substituted

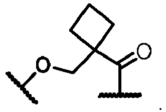

In the structure shown in formula (L-2), said L₄ is optionally substituted

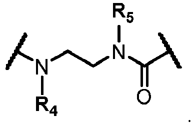

In the structure shown in formula (L-2), said L₄ is optionally substituted

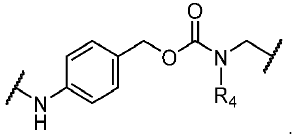

In the structure shown in formula (L-2), said L₄ is optionally substituted

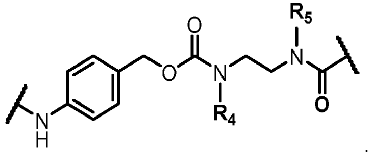

In the structure shown in formula (L-2), said L₄ is a structure selected from the group consisting of optionally substituted 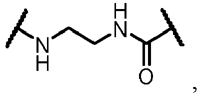 optionally substituted 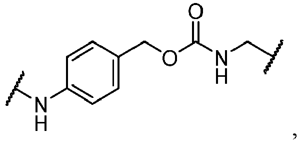 optionally substituted 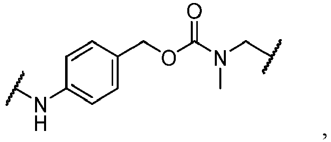 and optionally substituted

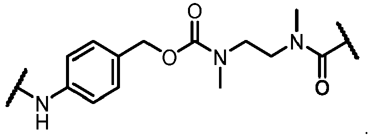

In another aspect, the present application provides a linker, or a tautomer, a mesomer, a racemate, an enantiomer, a diastereomer, or a mixture thereof, or a pharmaceutically acceptable salt or hydrate thereof, wherein said the linker comprises structures selected from the group consisting of

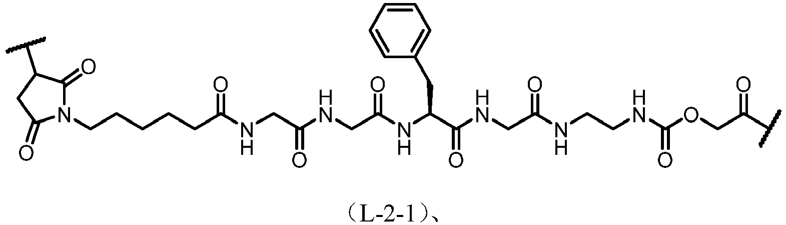

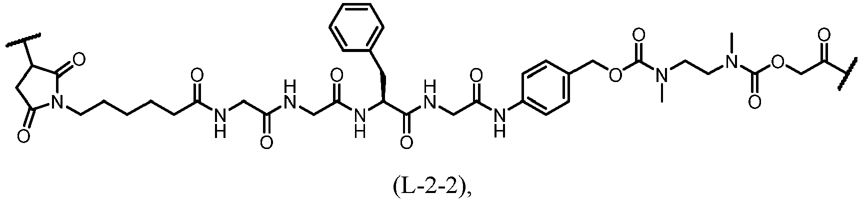

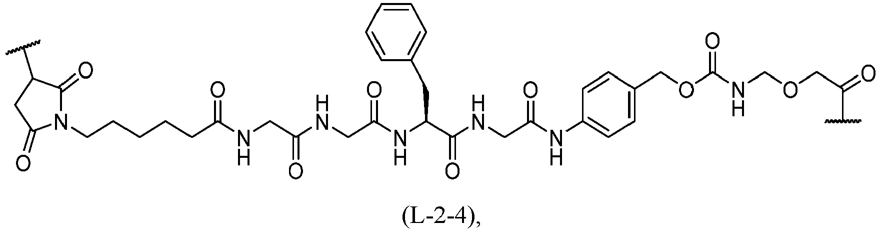

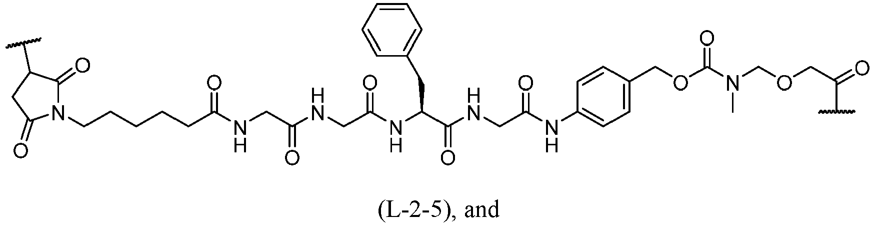

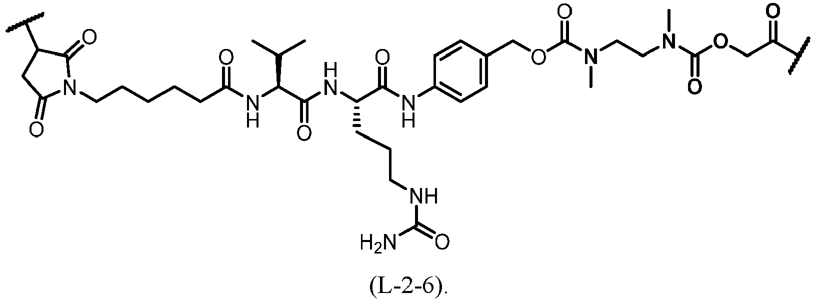

In another aspect, the present application provides a pharmaceutical composition comprising the ligand conjugate as described in the present application, or a tautomer, a mesomer, a racemate, an enantiomer, a diastereomer, or a mixture thereof, or a pharmaceutically acceptable salt, a prodrug or a solvate thereof, and/or a compound as described in the present application, and optionally pharmaceutically acceptable carriers.

In another aspect, the present application also provides the use of the ligand conjugate of the present application, or a tautomer, a mesomer, a racemate, an enantiomer, a diastereomer, or a mixture thereof, or a pharmaceutically acceptable salt, a prodrug or a solvate thereof, the compounds of the present application, and/or of the pharmaceutical compositions of the present application, in the preparation a drug for the treatment and/or prevention of tumors.

In one embodiment, said the tumor is selected from tumors associated with the expression of the target consisting of HER2 and TROP2.

In one embodiment, said the tumor associated with the target expression comprises a tumor with high target expression and/or a tumor with positive target expression.

In one embodiment, said the tumor comprises solid tumors and/or hematological tumors.

In one embodiment, said the tumour is selected from the group consisting of breast cancer, gastric cancer, ovarian cancer, bladder cancer, colorectal cancer, endometrial cancer, kidney cancer, lung cancer, pancreatic cancer, prostate cancer and thyroid cancer.

In one embodiment, said the tumour is selected from the group consisting of breast cancer, ovarian cancer, cervical cancer, endometrial cancer, urothelial cancer, lung cancer, prostate cancer, colorectal cancer, gastric cancer, esophageal cancer, bladder cancer, kidney cancer, pancreatic cancer, thyroid cancer and head and neck cancer.

Those skilled in the art will be able to readily gain insight into other aspects and advantages of the present application from the detailed description below. Only illustrative embodiments of the present application are shown and described in the detailed description below. As will be recognised by those skilled in the art, the contents of the present application enable those skilled in the art to make changes to the specific embodiments disclosed without departing from the spirit and scope of the application to which the present application relates. Accordingly, the descriptions in the specification of this application are merely illustrative and not intended to be limitative.

### DETAILED DESCRIPTION OF EMBODIMENTS

The following are specific embodiments to illustrate the implementation methods of the present application. Those familiar with this technology can easily understand the other advantages and effects of the present application from the disclosure of this specification.

### Definitions of Terms

In the present application, the term "ligand" usually refers to a macromolecular compound capable of recognizing and binding to an antigen or receptor associated with a target cell. The role of ligands can be to delivery drugs to target cells population to which the ligand binds, and these ligands includes but are not limited to protein hormones, lectins, growth factors, antibodies, or other molecules capable of binding to a cell, receptors, and/or antigens. In the present application, the ligand can be represented in Ab, and the ligand forms a linking bond with the linking unit *via* a heteroatom on the ligand, which can be an antibody or antigen-binding fragment thereof. The antibody can be selected from a chimeric antibody, a humanized antibody, a fully human antibody, or a murine antibody. The antibody can be a monoclonal antibody. For example, the antibody can be an antibody or antigen-binding fragment targeting targets selected from HER2 and TROP2.

In the present application, the terms "Trop2" and "TROP2" generally refer to single-pass Type I transmembrane proteins. In the present application, the term "Trop2" may also cover homologues, variants, and isoforms of Trop2, including splicing isoforms. The term "Trop" also includes proteins having one or more sequences of Trop 2 homologues, variants, and isoforms, as well as fragments of the sequence, as long as it is the variant protein (including isoforms). Trop2 may be a human Trop2. For example, Uniprot Accession Number P09758 provides a description of Trop2 and sequence thereof.

In the present application, the term "HER2" generally refers to human epidermal growth factor receptor 2 (HER2). For example, the term "HER2" refers to any natural HER2 of any human source. This term also covers "full length" and unprocessed HER2, as well as any form of HER2 derived from processing in cells (such as mature proteins). This term also covers the naturally occurring variants and isoforms of HER2, such as splicing variants or allelic variants. For example, Uniprot login number P04626 provides a description of HER2 and sequence thereof.

In the present application, the term "trastuzumab" generally refers to an antibody targeting HER2. For example, Trastuzumab may be recorded in US20060275305A1. In this application, trastuzumab may refer to an antibody or antigen-binding fragment containing the heavy chain variable region CDR1-3 and the light chain variable region CDR1-3 of trastuzumab. As used herein, trastuzumab may refer to an antibody or antigen-binding fragment containing the heavy chain variable region and light chain variable region of trastuzumab.

In the present application, the term "peptide residue" usually refers to a residue composed of one or more amino acid residues linked together. For example, one or more amino acids in peptide residues can be optionally substituted. For example, the peptide residue of the present application may comprise glycine-glycine-phenylalanine-glycine (Gly-Gly-Phe-Gly).

In the present application, the term "drug unit" usually refers to the chemical moiety that was directly or indirectly conjugated to antibodies or antigen-binding fragments to form immunoconjugates. For example, "drug unit" includes but is not limited to compounds with anti-tumor activity described in the present application. For example, drug units include topoisomerase inhibitors.

In the present application, the term "compound with anti-tumor activity" generally refers to compounds with the ability to reduce the proliferation rate, viability, or metastatic ability of tumor cells. For example, anti-tumor activity may be demonstrated by a decrease in the growth rate of abnormal cells or a stable or reduced tumor size during treatment, or by a longer survival period compared to untreated controls. The anti-tumor activity may be evaluated using recognized *in vitro* or *in vivo* tumor models, such as xenograft models.

In the present application, the term "topoisomerase inhibitor" generally refers to compounds includes topoisomerase I inhibitors and topoisomerase II inhibitors or derivative thereof. Examples of topoisomerase I inhibitor include but are not limited to camptothecin and its analogues. For example, topoisomerase may refer to an enzyme that corrects the number of DNA linkages by cutting the phosphodiester bonds in one or two strands of DNA, then rewinding and sealing them.

In the present application, the term "camptothecin derivatives" usually refers to compounds with a structure similar to or derived from camptothecin. For example, the structure of camptothecin may be recorded in CAS registry number 7689-03-4. For example, camptothecin derivatives may refer to Exatecan (CAS registry number 171335-80-1).

In the present application, the term "tumor" usually refers to any new pathological tissue hyperplasia. For this application, angiogenesis is a part of tumor characteristics. The tumor may be benign or malignant. The term "tumor" is generally used to refer to benign or malignant tumors, while the term "cancer" is generally used to refer to malignant tumors, which can be either metastatic or non-metastatic. When used for research, these tumor tissues can be isolated from readily available resources using methods familiar to those skilled in the art.

In the present application, the term "alkyl" usually refers to a residue derived from the removal of one hydrogen atom from alkane. Alkyl may be substituted or unsubstituted, replaced or unreplaced. The term "alkyl" usually refers to saturated linear or branched aliphatic hydrocarbon group, which have a residue derived from the removal of hydrogen atom from the same carbon atom or two different carbon atoms of the parent alkane. The said alkyl may be a linear or branched group containing 1 to 20 carbon atoms, such as a chain alkyl containing 1 to 12 carbon atoms, such as a chain alkyl containing 1 to 6 carbon atoms. Non limiting examples of alkyl include but are not limited to methyl, ethyl, propyl, propyl, butyl, etc. Alkyl may be substituted or unsubstituted, replaced or unreplaced, for example, when substituted, the substituent may be substituted at any available connection point, and the said substituents may be independently and optionally selected from one or more the group consisting of alkyl, alkenyl, alkynyl, alkoxy, alkylthio, alkylamino, halogen, thiol, hydroxyl, nitro, cyano, cycloalkyl, heterocyclic, aryl, heteroaryl, cycloalkoxy, heterocycloalkoxy, cycloalkylthio, heterocyclic alkylthio, and oxo, such as hydrogen, protium, deuterium, tritium, halogen, - NO₂, -CN, -OH, -SH, -NH₂, - C(O)H, - CO₂H, - C(O)C(O)H, -C(O)CH₂C(O)H, -S(O)H,-S(O)₂H, -C(O)NH₂, - SO₂NH₂, - OC(O)H, -N(H)SO₂H, or C₁₋₆ aliphatic groups.

In the present application, the term "alkylene" generally refers to a saturated linear or branched aliphatic hydrocarbon group having two residues derived from the removal of two hydrogens from the same carbon atom or two different carbon atoms of the parent alkane, which may be a linear or branched group containing 1 to 20 carbon atoms. For example, the term "methylene" may refer to a residue derived from the removal of two hydrogen atoms from a group with 1 carbon atom. Methylene can be substituted or unsubstituted, replaced or unreplaced; For example, alkylene with 1 to 12 carbon atoms, such as with 1 to 6 carbon atoms. Non limiting examples of alkylene groups include, but are not limited to, methylene (-CH₂-), 1,1-ethylene (-CH(CH₃)-), 1,2-ethylene (-CH₂CH₂)-, 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and 1,5-butylene (-CH₂CH₂CH₂CH₂CH₂-), etc. Alkylene can be substituted or unsubstituted, replaced or unreplaced. For example, when it is substituted, substitution with a substituent may be performed at any available linking point, and the substituent may be independently and optionally one or more of the substituents selected from alkyl, alkenyl, alkynyl, alkoxy, alkylthio, alkylamino, halogen, thiol, hydroxyl, nitro, cyano, cycloalkyl, heterocyclic, aryl, heteroaryl, cycloalkoxy, heterocycloalkoxy, cycloalkylthio, heterocyclic alkylthio, and oxo, which may be, for example, hydrogen, protium, deuterium, tritium, halogen, - NO₂, -CN, -OH, -SH, -NH₂, -C(O)H, -CO₂H, -C(O)C(O)H, -C(O)CH₂C(O)H, -S(O)H, -S(O)₂H, -C(O)NH₂, -SO₂NH₂, -OC(O)H, -N(H)SO₂H, or C₁₋₆ aliphatic groups. The methylene or alkylene may be substituted or unsubstituted.

In the present application, the term "alkenyl" refers to a linear or branched hydrocarbon group containing one or more double bonds. Illustrative examples of alkenyl include allyl, homoallyl, vinyl, crotyl, butenyl, pentenyl, and hexenyl, etc. Illustrative examples of C₂₋₆ alkenyl chains with more than one double bond include butadienyl, pentadienyl, hexadienyl, and hexatrienyl, as well as branched forms thereof. The position of an unsaturated bond (double bond) may be at any position in the carbon chain. The alkenyl may be substituted or unsubstituted.

In the present application, the term "alkenylene" generally refers to a residue derived from the removal of two hydrogen atoms from the carbon atom of an alkene. For example, it can be allylidene, ethenylidene, butenylidene, pentenylidene, and hexadienylidene etc. The alkenylene group may be substituted or unsubstituted.

In the present application, the term "alkynyl" usually refers to a unsaturated linear or branched alkynyl group, such as ethynyl, 1-propynyl, propargyl, butynyl, etc. The alkynyl group may be substituted or unsubstituted.

In the present application, the term "alkynylene" generally refers to a residue derived from the removal of two hydrogen atoms from the carbon atom of an alkyne. For example, it can be ethynylene, propylene, propargylene, butynylene, etc. alkynylene may be substituted or unsubstituted.

In the present application, the term "aryl" generally refers to a residue derived from the removal of one hydrogen atom from the carbon atom of aromatic ring. The term "aromatic ring" may refer to a 6 to 14 membered all carbon mono-cyclic ring or fused poly-cyclic ring (i.e. a ring that shares adjacent pairs of carbon atoms) with a conjugated π electron system, which may be 6- to 10-membered, such as benzene and naphthalene. The aromatic ring may be fused to a heteroaryl, heterocyclic, or cycloalkyl ring, wherein the ring attached to the parent structure is an aromatic ring. The aryl may be substituted or unsubstituted, and when substituted, the substituent may be one or more of the following groups independently selected from the group consisting of alkyl, alkenyl, alkynyl, alkoxy, alkylthio, alkylamino, halogen, thiol, hydroxyl, nitro, cyano, cycloalkyl, heterocycloalkyl, aryl, heteroaryl, cycloalkoxy, heterocycloalkoxy, cycloalkylthio, and heterocycloalkylthio. The aryl may be substituted or unsubstituted.

In the present application, the term "arylene" generally refers to a residue derived from the removal of two hydrogen atoms from the carbon atom of an aromatic ring. For example, it can be phenylene and naphthylene. Arylene may be substituted or unsubstituted.

In the present application, the term "heteroaryl" generally refers to a residue derived from the removal of one hydrogen atom from the carbon atom of a heteroaromatic ring. The term "heteroaromatic ring" refers to a heteroaromatic system consisting of 1 to 4 heteroatoms and 5 to 14 ring atoms, wherein heteroatoms may be selected from the group consisting of oxygen, sulfur, and nitrogen. Heteroaryl may be 5 to 10 membered, and may be 5 or 6 membered, such as furanyl, thiophenyl, pyridinyl, pyrrolyl, N-alkyl pyrrolyl, pyrimidinyl, pyrazinyl, imidazolyl, tetrazolyl, etc. Heteroaryl may be fused to an aromatic, heterocyclic, or cycloalkyl ring, wherein the ring attached to the parent structure is a heteroaryl ring. Heteroaryl may be optionally substituted or unsubstituted, and when substituted, the substituent can be one or more substituents independently selected from the group consisting of alkyl, alkenyl, alkynyl, alkoxy, alkylthio, alkylamino, halogen, thiol, hydroxyl, nitro, cyano, cycloalkyl, heterocyclic, aryl, heteroaryl, cycloalkoxy, heterocycloalkoxy, cycloalkylthio, and heterocycloalkylthio. Heteroaryl may be substituted or unsubstituted.

In the present application, the term "heteroarylene" usually refers to a residue derived from the removal of two hydrogen atoms from the carbon atoms of heteroaromatic ring. For example, it can be furanylene, thienylene, pyridylene, pyrrolylene, pyrimidinylene, pyrazinylene, imidazolylene, tetrazolylene, etc. Heteroarylene may be substituted or unsubstituted.

In the present application, the term "aliphatic cyclyl" generally refers to a residue derived from the removal of hydrogen atoms from the same carbon atom or multiple different carbon atoms of the aliphatic ring. The term "cycloalkane" usually refers to saturated or partially unsaturated mono- or poly-cyclic hydrocarbons, wherein the carbon ring contains 3 to 20 carbon atoms, 3 to 12 carbon atoms, 3 to 10 carbon atoms, or 3 to 8 carbon atoms. Non limiting examples of aliphatic cyclyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Poly-cyclic carbon ring may include spiro, fused and bridged carbocycles. Alicyclic group may be substituted or unsubstituted. As used herein, the term "carbocyclic group" generally refers to a residue derived from the removal of one hydrogen from the carbon atom of carbon ring. The term "carbon ring" usually refers to a saturated or partially unsaturated mono- or poly-cyclic hydrocarbons, which contain 3 to 20 carbon atoms, 3 to 12 carbon atoms, 3 to 10 carbon atoms, or 3 to 8 carbon atoms. Non limiting examples of monocyclic carbon ring include cyclopropane, cyclobutane, cyclopentane, cyclopentene, cyclohexane, cyclohexene, cyclohexdiene, cycloheptane, cycloheptatriene, cyclooctane, etc. Poly-cyclic carbon ring may include spiro, fused and bridged carbocycles. Carbocyclic group may be substituted or unsubstituted. In some cases, aliphatic rings and carbocyclic rings may be used interchangeably.

In the present application, the term "partially unsaturated" generally refers to the presence of at least one double or triple bond between ring atoms in a cyclic structure. The term "partially unsaturated" encompasses cyclic structures with multiple unsaturated sites, but is not intended to include aromatic or heteroaromatic rings as defined in this application. The term "unsaturated" indicates partially having one or more degrees of unsaturation.

In the present application, the term "aliphatic cyclylene" usually refers to a residue derived from the removal of two hydrogen atoms from the carbon atoms of aliphatic ring. Such as cyclopropylene, cyclobutylene, cyclopentanylene, cyclopentenylene, cyclohexanylene, cyclohexadienylene, cycloheptanylene, cycloheptatrienylene, cyclooctanylene, etc.; and the poly-cyclic carbon ring may include spiro, fused, and bridged carbocycles. aliphatic cyclylene may be substituted or unsubstituted.

In the present application, the term "aliphatic heterocyclyl" usually refers to a stable and nonaromatic 3-7-membered mono-cyclic carbon ring structure, a fused 7-10 membered bicyclic heterocyclic structure, or a bridged 6-10 membered bicyclic heterocyclic structure. These ring structures may be either saturated or partially saturated, and in addition to carbon atoms, these ring structures also contain one or more heteroatoms, wherein the heteroatom may be selected from the group consisting of oxygen, sulfur, and nitrogen. For example, it contains 1-4 heteroatoms as defined above. When used to represent atoms on the cyclic structure of the aliphatic heterocyclic group, the term "nitrogen" may include nitrogen that has undergone a substitution reaction. For example, aliphatic heterocyclyl may contain "heterocycloalkyl", which may refer to stable non aromatic 3- to 7-membered monocyclic alkyl structures, fused 7- to 10-membered bicyclic heterocyclic structures, or bridged 6- to 10-membered bicyclic heterocyclic structures. In addition to carbon atoms, these cyclic structures may also contain one or more heteroatoms, wherein the heteroatoms may be selected from the group consisting of oxygen, sulfur, and nitrogen. For example, it contains 1-4 heteroatoms as defined above. Heterocycloalkyl may be substituted or unsubstituted. Aliphatic heterocyclyl may be substituted or unsubstituted.

In the present application, the term "aliphatic heterocyclylene" generally refers to a residue derived from the removal of two hydrogen atoms from the carbon atoms of an aliphatic heterocyclic ring. Aliphatic heterocyclylene group may be substituted or unsubstituted.

In the present application, the term "optional" or "optionally" usually refers to the subsequent described event or circumstance that may but does not necessarily occur, and that the description includes instances where the event or circumstance occurs or does not occur. For example, "heterocyclyl group optionally substituted with alkyl" means that alkyl may be, but not necessarily, exist, and that this description may include instances where the heterocyclyl group is or is not substituted with alkyl.

In the present application, the term "substituted" generally refers to one or more hydrogen atoms in a group, for example, up to 5, or 1-3 hydrogen atoms, are each independently substituted with a corresponding number of substituents. The substituent exists only in their possible chemical positions, and those skilled in the art can determine (either experimentally or theoretically) possible or impossible substitutions without undue effort. For example, the connection of amino or hydroxyl groups with free hydrogen to carbon atoms with unsaturated (such as olefinic) bonds may be unstable. For example, the substituents for optionally substituted are optionally selected from hydrogen, protium, deuterium, tritium, alkyl, alkenyl, alkynyl, alkoxy, alkylthio, alkylamino, halogen, thiol, hydroxyl, nitro, cyano, cycloalkyl, heterocyclyl, aryl, heteroaryl, cycloalkoxy, heterocycloalkoxy, cycloalkylthio, heterocyclic alkylthio, and -oxo, which may be, for example, hydrogen, protium, deuterium, tritium, halogen, -NO₂, -CN, -OH, -SH, -NH₂, -C(O)H, -CO₂H, -C(O)C(O)H,-C(O)CH₂C(O)H, -S(O)H, -S(O)₂H, -C(O)NH₂, -SO₂NH₂, -OC(O)H, -N(H)SO₂H, or C₁₋₆ aliphatic group.

In the present application, as known to those skilled in the art, the terms such as "alkyl", "alkenyl", "cycloalkyl" may be prefixed with a label to the name indicating the number of atoms present in the group under specific circumstances, such as C₁-C₄ alkyl, C₃-C₇ cycloalkoxy, C₁-C₄ alkycarbonylamino, etc. The subscript number following "C" indicates the number of carbon atoms present in the group. For example, C₃ alkyl refers to an alkyl with three carbon atoms (such as n-propyl, isopropyl). In C₁₋₁₀, the members of a group can have any number of carbon atoms falling within the range of 1-10.

One or more hydrogen atoms in a group, e.g. up to 5, e.g. 1 to 3 hydrogen atoms are independently substituted by a corresponding number of substituents. The substituents are only in their possible chemical positions, and those skilled in this field can determine (experimentally or theoretically) possible or impossible substitutions without undue effort. For example, the connection of amino or hydroxyl groups with free hydrogen to carbon atoms with unsaturated (such as olefinic) bonds may be unstable.

In the present application, the term "compound" generally refers to a substance with two or more different elements. For example, the compound of the present application may be an organic compound, for example, the compound of the present application may be a compound with a molecular weight of less than 500, a compound with a molecular weight of less than 1000, a compound with a molecular weight of more than 1000, or a compound with a molecular weight of more than 10000 or 100000. In the present application, the compound may also refer to a compound that involves linking by a chemical bond, for example, a compound in which one or more molecules with a molecular weight of less than 1000 are linked to a biological macromolecule via chemical bonds, wherein the biological macromolecule may be polysaccharides, proteins, nucleic acids, peptides, etc. For example, the compound of the present application may include a compound where a protein is linked to one or more molecules with a molecular weight of less than 1000, may include a compound where a protein is linked to one or more molecules with a molecular weight of less than 10000, and may include a compound where a protein is linked to one or more molecules with a molecular weight of less than 100000.

In the present application, the term "include", "comprise" or "contain" usually refers to the inclusion of explicitly specified features, but not excluding other elements. The term " more than " and " less than " usually refer to situations that include this number.

In the present application, the term "about" generally refers to variation in the range of 0.5% to 10% above or below the specified value, for example, variation in the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

In the present application, the compound of the present application, or tautomers, mesomers, racemates, enantiomers, and/or a diastereomers. As used herein, the term "diastereomers" generally refers to stereoisomers with two or more chiral centers and the molecules are not stereoisomers that mirror each other. Diastereomers may have different physical properties, such as melting point, boiling point, spectral properties, and reactivity. In the present application, the terms "tautomers" or "tautomeric forms" can be used interchangeably and typically refer to structural isomers of different energies that can be converted to each other through low energy barriers. For example, proton tautomers (also known as prototropic tautomers) include mutual transformations through proton migration, such as keto-enol isomerization and imine-enamine isomerization. Valence tautomers include mutual transformations through the recombination of some bonding electrons. As used herein, the term "mesomers" usually refers to a molecule that contains asymmetric atoms but has a total intramolecular rotation of zero due to symmetry factors. The term "racemates" or "racemic mixtures" refers to a composition composed of two enantiomers of equal molar amounts.

In the present application, certain atoms of the compound of the present application may appear in the form of more than one isotope. For example, hydrogen may exist in the form of protium (¹H), deuterium (²H), and tritium (³H), and carbon may naturally exist in three different isotopes (¹²C, ¹³C, and ¹⁴C). Examples of isotopes that can be incorporated into the compound of the present application include but are not limited to ¹⁵N, ¹⁸O, ¹⁷O, ¹⁸F, ³²P, ³³P, ¹²⁹I, ¹³¹I, ¹²³I, ¹²⁴I, ¹²⁵I, or similar isotopes. Therefore, compared to the natural abundance of these isotopes, the compounds of the present application can be enriched in one or more of these isotopes. As known to those skilled in the art, such isotopes enriched compound may be used for various purposes. For example, the replacement of heavy isotopes such as deuterium (²H) may provide certain therapeutic advantages, which may be due to higher metabolic stability. For example, the natural abundance of deuterium (²H) is approximately 0.015%. Therefore, for every 6500 hydrogen atoms in nature, there is approximately one deuterium atom. Therefore, the deuterium abundance of the deuterium-containing compound in the present application is greater than 0.015% at one or more positions (depending on the situation). Unless otherwise specified, the structure as described in the present application may also include compounds that differ only in the presence or absence of one or more isotope enriched atoms. For example, except for hydrogen replaced by deuterium or tritium, or carbon replaced by carbon-13 or carbon-14, compounds with the rest of the structure being consistent with the compound of the present application are all within the scope of the present application.

In the present application, the term "pharmaceutical composition" usually refers to a mixture containing one or more of the compounds as described in present application or a physiologically/pharmaceutically acceptable salts or prodrugs thereof and other chemical components, or other components such as physiologically/pharmaceutically acceptable carriers and excipients. The pharmaceutical compositions may facilitate the administration to organism and facilitate the absorption of active ingredient and thus exert its biological activity. The preparation of conventional pharmaceutical compositions can be referred to in commonly used techniques in this field.

In the present application, the terms "pharmaceutically acceptable salt" or "medicinal salt" generally refer to the salt of the compound or ligand-drug conjugate of the present application, or the salt of the compound described in this application, which can be safe and/or effective when used in mammals, and may have the expected biological activity. The antibody-antibody drug conjugate in this application can form a salt with an acid, the non limiting examples of the pharmaceutically acceptable salts include: hydrochloride, hydrobromide, hydriodate, sulfate, bisulfate, citrate, acetate, succinate, ascorbate, oxalate, nitrate, sorbate, hydrophosphate, dihydrogen phosphate, salicylate, hydrogen citrate, tartrate, maleate, fumarate, formate, benzoate, methanesulfonate, ethanesulfonate, benzenesulfonate orp-toluenesulfonate.

In the present application, the term "conjugate" generally refers to a compound prepared by one or more chemical reactions of the compound in the present application, or by linking the compounds of this application to each other through one or more linking structures such as bridges, spacers, or linking moieties, etc.

In the present application, the term "pharmaceutically acceptable carrier" generally refers to a carrier for administering therapeutic agents, such as antibodies or peptides, genes, and other therapeutic agents. The term refers to any drug carrier that does not itself induce the production of any antibodie that is harmful to the individual receiving the composition and can be administered without producing undue toxicity. For example, pharmaceutically acceptable carriers may be distinguished from nucleic acid vectors used in genetic engineering to contain target genes. Suitable carriers may be large, slowly metabolized macromolecules, such as proteins, polysaccharides, polylactic acids, polyglycolic acid, polyamino acids, amino acid copolymers, lipid aggregates, and inactivated virus particles. those skilled in the field are familiar with these carriers. Pharmaceutically acceptable carriers in therapeutic compositions may include liquids such as water, saline, glycerin, and ethanol. These carriers may also contain auxiliary substances, such as wetting agents or emulsifying agents, pH buffering substances, etc.

In the present application, "antibody" may generally cover monoclonal antibodies, polyclonal antibodies, dimers, polymers, polyspecific antibodies (such as bispecific antibodies), and antibody fragments, as long as they exhibit the desired biological activity. Antibodies may be murine-derived, human-derived, humanized, chimeric antibodies, or derived from other species. Antibodies are proteins produced by the immune system that recognize and bind to specific antigens. Target antigen generally have a large number of binding sites recognized by CDRs of various antibodies, also known as epitopes. Antibodies that specifically bind to different epitopes have different structures. Therefore, an antigen may have more than one corresponding antibody. Antibody includes full-length immunoglobulin molecules or the immunoactive part of full-length immunoglobulin molecules, that is, molecules containing antigens or its part that specifically bind to the target of interest. These targets include, but are not limited to, cancer cells or cells that produce autoimmune antibodies related to autoimmune diseases. The immunoglobulin described in this application can have any type (such as IgG, IgE, IgM, IgD, and IgA), class (such as IgG1, IgG2, IgG3, IgG4, IgA1, and IgA2), or subclass of immunoglobulin molecules. Immunoglobulin may derive from any species. However, in one aspect, immunoglobulins originate from humans, murine, or rabbits. "Antibody fragment" may contain a part of a full-length antibody, typically its antigen-binding region or variable region. Examples of antibody fragment include Fab, Fab', F(ab')2, and Fv fragments; diabody; linear antibody; minibody; fragments prepared by fab expression library; anti-idiotypic (anti-Id) antibody; CDR (Complementary Determining Region); any of the above-mentioned epitope-binding fragment that binds to cancer cell antigens, viral antigens, or microbial antigens in an immunospecific manner; single chain antibody molecules; and multi-specific antibodies formed by antibody fragments. The antibody constituting the antibody drug conjugate in the application may maintain their antigen binding ability in their original wild state. Therefore, the antibody of the present application may, for example, specifically bind to antigens. The antigens involved include, for example, tumor associated antigens (TAAs), cell surface receptor proteins and other cell surface molecules, cell survival regulators, cell proliferation regulators, molecules related to tissue growth and differentiation (such as those known or predicted to be functional), lymphokines, cytokines, molecules involved in cell cycle regulation, and molecules involved in angiogenesis, and molecules related to angiogenesis (such as antigens that bind to known antibodies can be one or a subset of the aforementioned classifications, while other subsets contain other molecules/antigens with special properties (compared to the target antigen). Antibodies applied in antibody drug conjugates include, but are not limited to, antibodies targeting cell surface receptors and tumor-related antigens. Such tumor-related antigens are well-known in the art and can be prepared through antibody preparation methods and information well known in the art. These targets can be specifically expressed on the surface of one or more cancer cells, with little or no expression on the surface of one or more non-cancer cells. Usually, compared to the surface of non-cancer cells, such tumor related peptides can be especially overexpressed on the surface of cancer cells.

In the present application, the term "chimeric antibody" usually refers to an antibody formed by the fusion of the variable region of murine antibodies with the constant region of human antibodies, which can alleviate the immune response induced by murine antibodies. For establishing a chimeric antibody, a hybridoma that secrete murine-derived antigen-specific monoclonal antibody can be established, and then clone variable region genes from murine hybridoma cells. Human antibody constant region genes can be cloned as needed, and the murine variable region genes can be connected to human constant region genes to form chimeric genes and then insert into expression vectors. Chimeric antibody molecules can be expressed in eukaryotic or prokaryotic systems.

In the present application, the term "humanized antibody", also known as CDR grafted antibody, generally refers to the transplantation of a murine CDR sequence into a human antibody variable region frame, which is an antibody produced in different types of human lineage antibody frame sequences. It can overcome the heterologous reactions induced by chimeric antibodies due to carrying a large amount of murine protein components. This type of frame sequence can be obtained from a public DNA database or publicly available references that include germline antibody gene sequences. For example, the lineage DNA sequences of human heavy and light chain variable region genes can be found in the "VBase" human lineage sequence database.

In the present application, the terms "fully humanized antibody", "fully human antibody", or "fully human derived antibody", also known as "fully human monoclonal antibody", may be of human origin in both the variable region and the constant region of the antibody, removing immunogenicity and toxic side effects. The development of monoclonal antibodies has gone through four stages, namely: murine monoclonal antibodies, chimeric monoclonal antibodies, humanized monoclonal antibodies, and fully human derived monoclonal antibodies. The antibodies or ligands described in this application may be fully human monoclonal antibodies. The relevant technologies for the preparation of fully human antibodies can include: human hybridoma technology, EBV transformation technology of B lymphocyte, phage display technology, transgenic mouse antibody preparation technology, and single B cell antibody preparation technology, etc.

In the present application, the term "CDR" generally refers to one of the six highly variable regions within the variable domain of the antibody that primarily facilitate antigen binding. One of the most commonly used definitions of the six CDRs mentioned was provided by Kabat E.A. et al., Chothia et al., and MacCallum et al., As used herein, the Kabat definition of CDR can be CDR1, CDR2, and CDR3 (CDR L1, CDR L2, CDR L3 or L1, L2, L3) of light chain variable domains, as well as CDR1, CDR2, and CDR3 (CDR H1, CDR H2, CDR H3 or H1, H2, H3) of heavy chain variable domains.

In the present application, the term "groups capable of coupling with thiol groups" generally refers to compound A having a thiol group, compound B having a group capable of coupling with a thiol group, and compound B reacting with the thiol of compound A through the group capable of coupling with the thiol group, thereby achieving the connection between compound A and compound B.

In the present application, the term "linker" generally refers to a chemical structure fragment or bond that is attached to one group at one end and to another group at the other end, and may also be attached to other linkers before being attached to the drugs and/or ligands. By directly or indirectly attached ligand may mean that said group is directly attached to the ligand *via* a covalent bond or may be attached to the ligand *via* a linker. For example, the linker may be the structure shown for the linker described in this application. For example, a chemical structure fragment containing an acid-labile linker structure (e.g., hydrazone), a protease-sensitive (e.g., peptidase-sensitive) linker structure, a light-labile linker structure, a dimethyl linker structure, or a disulfide linker structure may be used as a linker.

In the present application, the term "linking group" generally refers to a group capable of linking to another group. For example, for a compound with a linking group, the attachment between the compound and another group can be achieved through a coupling reaction between the linking group and the another group. For example, the maleimide group can serve as a linking group.

In the present application, the term disease "associated with the expression" of a target generally refers to the occurrence and/or progression of the disease being associated with the expression level of the target. For example, when the expression level of a certain target in cells from a disease area, such as within a particular tissue or organ of a patient, is increased relative to the expression level in normal cells from a tissue or an organ, that is, the target is highly expressed. Alternatively, for example, when the expression level of a certain target in cells from a disease area, such as within a particular tissue or organ of a patient, is increased relative to the expression level in normal cells from a tissue or an organ, that is, the target is low expressed. Alternatively, for example, when cells from diseased areas, such as within a particular tissue or organ of a patient, express a specific target, i.e., the cells is positive. Alternatively, for example, when cells from diseased areas, such as within a particular tissue or organ of a patient, do not express a specific target, i.e., the cells is negative. For example, the features of target expression may be determined by standard assays known in the art.

In the present application, the term "effective amount" generally refers to the amount of a therapeutic agent used to treat, alleviate, or prevent the target disease or condition, or the amount that exhibits measurable therapeutic or prophylactic effects. The precise effective amount for a certain subject depends on its body size and health condition, the nature and extent of the disease, as well as the therapeutic agents and/or combination of therapeutic agents chosen for administration. Therefore, it is useless to pre-specify the exact effective amount. However, for a given condition, routine experiments can be used to determine the effective amount, which can be judged by clinicians.

Unless otherwise specified, all compounds present in this application are intended to include all possible optical isomers, such as single chiral compounds or mixtures of various chiral compounds (i.e., racemates). In all the compounds of this application, each chiral carbon atom may optionally be R or S configuration, or a mixture of R and S configurations.

In the present application, the term "compound of the present application" usually refers to the compound of the present application. The term also includes various crystalline forms, pharmaceutically acceptable salts, hydrates or solvates of the compound of present application.

When a trade name is used herein, it is intended to include the formulation of the trade name product, its corresponding generic drug, and the active pharmaceutical ingredients of the trade name product.

### Detailed description of the invention

In one aspect, the present application provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (I): 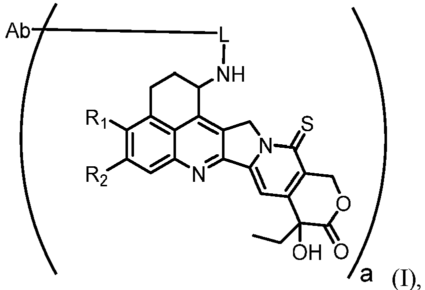 For example, it can be 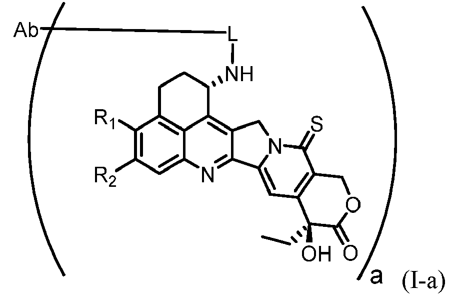 and/or 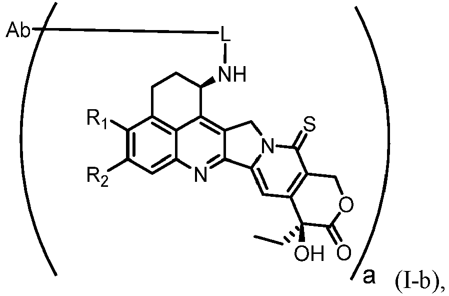
wherein, R₁ and R₂ may each independently be selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl,
L may be an optionally substituted linker,
Ab may be a ligand, "a" can a number greater than 0.

For example, in the structure shown in formula (I), said L may comprise a linker L₁, said L₁ may be directly or indirectly linked to a ligand. For example, said L₁ may comprise optionally substituted 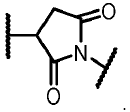 For example, L₁ in the structure shown in formula (I) can exist in a ring-opened form as shown in the following equation.

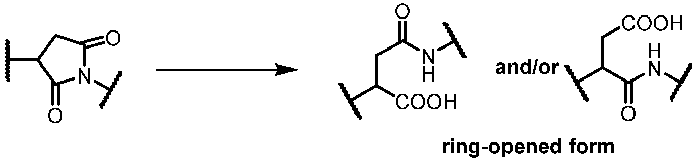

For example, in the structure shown in formula (I), said L may comprise a linker L₂. For example, said L₂ may be directly or indirectly linked to L₁. For example, said L₂ may comprise a group selected from the group consisting of optionally substituted -(CH₂)ₘ₁-X₁-(CH₂)ₘ₂-C(O)-, optionally substituted -(CH₂CH₂O)ₙ-C(O)-, and optionally substituted -(CH₂)ₚ-C(O)-, X₁ may be selected from the group consisting of -O-, optionally substituted -C(O)-NH-, optionally substituted aryl, optionally substituted heteroaryl, optionally substituted aliphatic heterocyclyl, and optionally substituted aliphatic cyclyl, wherein, m1, m2, and n are each independently selected from numbers of at least 0.

For example, in the structure shown in formula (I), said L₂ may comprise optionally substituted -(CH₂CH₂O)ₙ-C(O)-. For example, wherein, n can be 2.

For example, in the structure shown in formula (I), said L₂ may comprise optionally substituted -(CH₂)ₘ₁-X₁-(CH₂)ₘ₂-C(O)-. For example, X₁ may be selected from the group consisting of -O-, optionally substituted -C(O)-NH-. For example, m1 can be 2. For example, m2 can be 2.

For example, in the structure shown in formula (I), X₁ may comprise optionally substituted aryl and optionally substituted heteroaryl. For example, X₁ may comprise optionally substituted phenyl. For example, X₁ may comprise optionally substituted pyridyl. For example, m1 can be 0. For example, m2 can be 1.

For example, in the structure shown in formula (I), X₁ may comprise an optionally substituted aliphatic heterocyclyl including two oxygen atoms. For example, X₁ may comprise optionally substituted 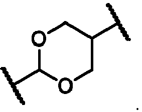 For example, X₁ may comprise an optionally substituted cyclohexyl group. For example, m1 can be 1. For example, m2 can be 0.

For example, in the structure shown in formula (I), said L₂ may comprise optionally substituted -(CH₂)ₚ-C(O)-. For example, p can be 5.

For example, in the structure shown in formula (I), said L₂ may comprise a structure selected from the group consisting of optionally substituted 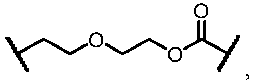 optionally substituted 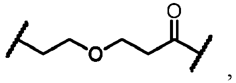 optionally substituted 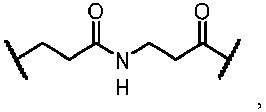 optionally substituted 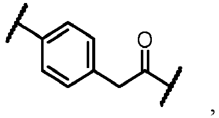 optionally substituted 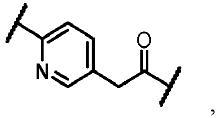 optionally substituted 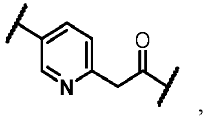 optionally substituted 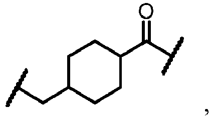 and optionally substituted

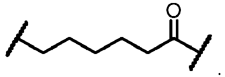

For example, in the structure shown in formula (I), said L may also comprise a linker L₃. For example, said L₃ may be directly or indirectly linked to said L₂. For example, said L₃ may comprise a peptide residue.

For example, said L₃ may comprise peptide residues composed of amino acids selected from the group consisting of phenylalanine, isoleucine, leucine, tryptophan, valine, methionine, tyrosine, alanine, threonine, histidine, serine, glutamine, arginine, lysine, asparagine, glutamate, proline, citrulline, aspartate, and glycine. For example, said L₃ may comprise peptide residues composed of amino acids selected from the group consisting of glycine, phenylalanine, valine, and citrulline. For example, said L₃ may comprise peptide residues selected from the group consisting of -glycine-phenylalanine-glycine- (-Gly-Phe-Gly-), -glycine-glycine-phenylalanine-glycine- (-Gly-Gly-Phe-Gly-), and -valine-citrulline- (-Val-Cit-).

For example, in the structure shown in formula (I), said L₃ may comprise a structure selected from the group consisting of 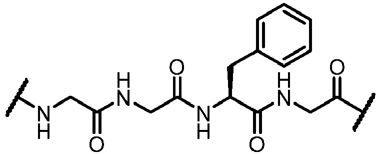 and

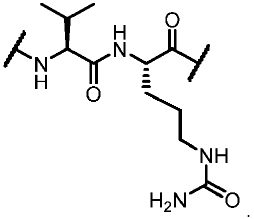

For example, in the structure shown in formula (I), said L may also comprise a linker L₄. For example, said L₄ may be directly or indirectly linked to said L₃.

For example, in the structure shown in formula (I), said L₄ may comprise optionally substituted -L₄ₐ-NR₄-CH₂-L_{4b}-,
L₄ₐ may be absent or L₄ₐ may comprise optionally substituted 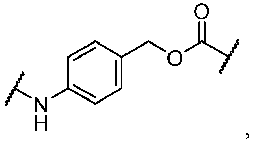
L_{4b} may be absent or L_{4b} may comprise optionally substituted 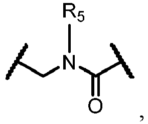
R₄ and R₅ may each independently be selected from the group consisting of hydrogen and optionally substituted alkyl.

For example, in the structure shown in formula (I), said L₄ may comprise optionally substituted 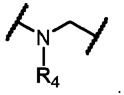 For example, said L₄ may comprise optionally substituted 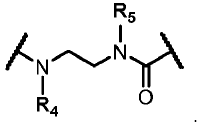 For example, said L₄ may comprise optionally substituted 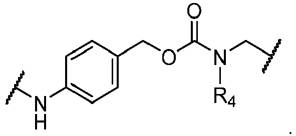 For example, said L₄ may comprise optionally substituted 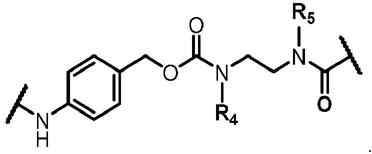 For example, said R₄ may be selected from the group consisting of hydrogen and optionally substituted methyl. For example, said R₅ may be selected from the group consisting of hydrogen and optionally substituted methyl.

For example, in the structure shown in formula (I), said L₄ may comprise a structure selected from the group consisting of optionally substituted 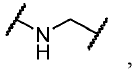 optionally substituted 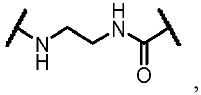 optionally substituted 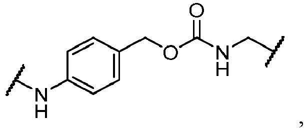 optionally substituted 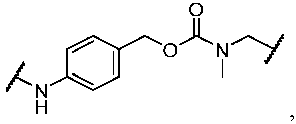 optionally substituted

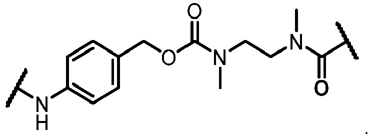

For example, in the structure shown in formula (I), said L may also comprise a linker L₅. For example, said L₅ may be directly or indirectly linked to said L₄.

For example, in the structure shown in formula (I), said L₅ may comprise optionally substituted 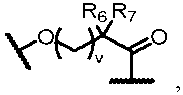 R₆ and R₇ may each be independently selected from the group consisting of hydrogen, deuterium, optionally substituted alkyl, optionally substituted haloalkyl, optionally substituted cycloalkyl, and optionally substituted cycloalkylalkyl, or R₆ and R₇ may be joined with the atom to which they attached to form an optionally substituted cycloalkyl, wherein v may be selected from a number at least 0.

For example, in the structure shown in formula (I), said R₆ may be hydrogen. For example, said R₆ may be optionally substituted methyl. For example, said R₆ may be optionally substituted halomethyl. For example, said R₆ may be fluorine substituted methyl. For example, said R₆ may be trifluoromethyl. For example, said R₆ may be optionally substituted cycloalkyl. For example, said R₆ may be optionally substituted cyclopropyl. For example, in the structure shown in formula (I), said R₇ may be hydrogen.

For example, in the structure shown in formula (I), R₆ and R₇ may be joined with the atom to which they attached to form an optionally substituted cyclopropyl. For example, R₆ and R₇ may be joined with the atom to which they attached to form an optionally substituted cyclobutyl. For example, v can be 0. For example, v can be 1.

For example, in the structure shown in formula (I), said L₅ may comprise a structure selected from the group consisting of optionally substituted 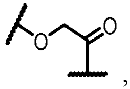 optionally substituted 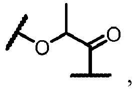 optionally substituted 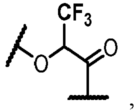 optionally substituted 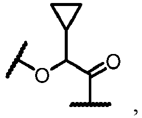 optionally substituted 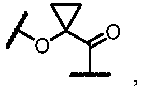 optionally substituted 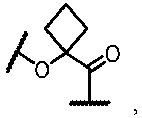 optionally substituted 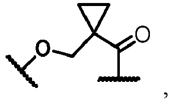 and optionally substituted

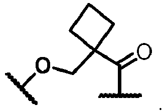

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (I): 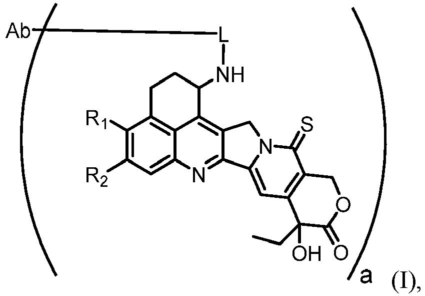 For example, it can be 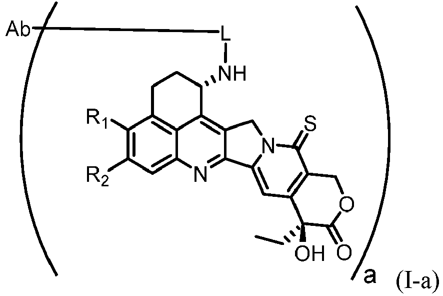 and/or 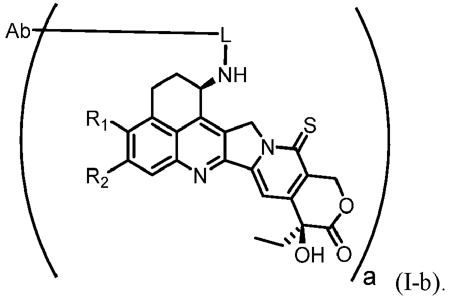
wherein R₁ and R₂ may each be independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached may form a structure selected from the group consisting of optionally substituted aliphatic cyclyl, optionally substituted aliphatic heterocyclyl, optionally substituted aryl, and optionally substituted heteroaryl;
L may be an optionally substituted linker, said L may comprise -L₁-L₂-L₃-L₄-L₅-.
Ab may be a ligand and "a" may be a number at least 0.

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (I): 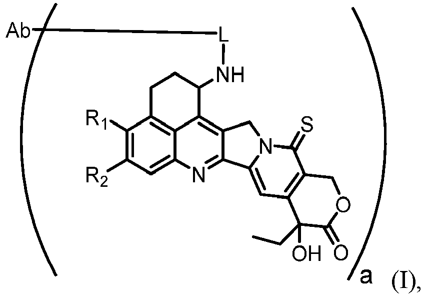 For example, it can be 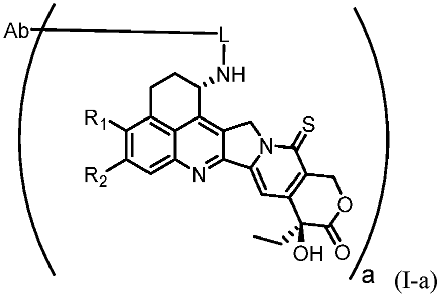 and/or 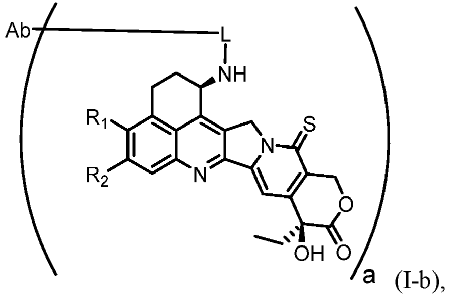
wherein, R₁ and R₂ may each be independently selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl,
   L may be an optionally substituted linker, said L may comprise -L₁-L₂-L₃-L₄-L₅-,
   Ab may be a ligand and "a" may be a number at least 0,
   wherein, said L₁ may comprise a linker after attachment to a thiol group,
said L₂ may comprise a group selected from the group consisting of optionally substituted -(CH₂)ₘ₁-X₁-(CH₂)ₘ₂-C(O)-, optionally substituted -(CH₂CH₂O)ₙ-C(O)-, and optionally substituted -(CH₂)ₚ-C(O)-, X₁ may be selected from the group consisting of -O-, optionally substituted -C(O)-NH-, optionally substituted phenyl, optionally substituted pyridyl, optionally substituted aliphatic heterocyclyl containing two oxygen atoms and four carbon atoms, and optionally substituted cyclohexyl, wherein m1, m2 and n may each be independently selected from a number of at least 0,
said L₃ may comprise peptide residues.
said L₄ may comprise optionally substituted 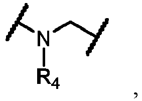 optionally substituted 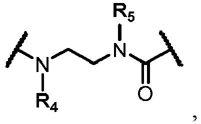 optionally substituted 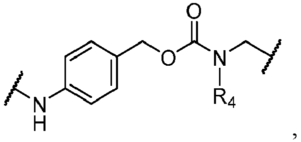 or optionally substituted 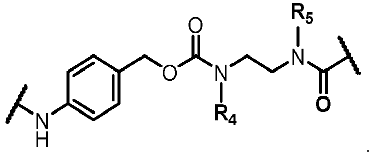 For example, said R₄ may be selected from the group consisting of hydrogen and optionally substituted C₁-C₆ alkyl. For example, said R₅ may be selected from the group consisting of hydrogen and optionally substituted C₁-C₆ alkyl.
said L₅ may comprise optionally substituted 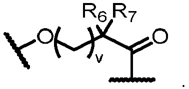 R₆ and R₇ may each be independently selected from the group consisting of hydrogen, deuterium, optionally substituted alkyl, optionally substituted haloalkyl, optionally substituted cycloalkyl, and optionally substituted cycloalkylalkyl, or R₆ and R₇ may be joined with the atom to which they attached to form an optionally substituted cycloalkyl, wherein v may be selected from a number at least 0. For example, cycloalkylalkyl in the present application may refer to an alkyl group substituted with cycloalkyl, wherein the cycloalkylalkyl group may also be optionally substituted with an additional group.

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (I): 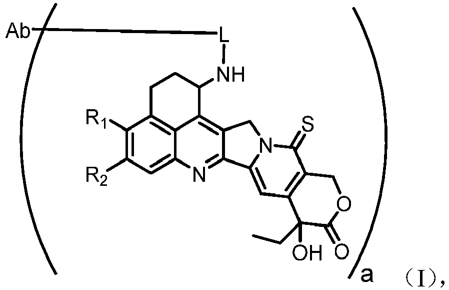 for example, it can be 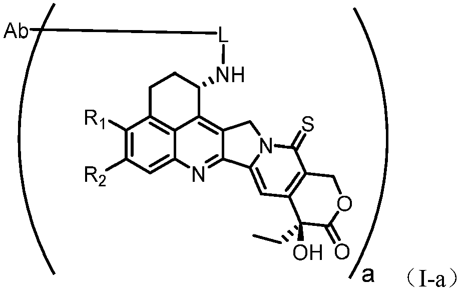 and/or 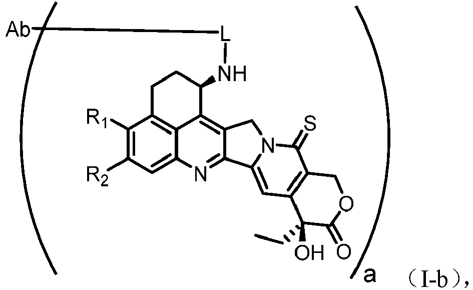
wherein R₁ and R₂ may each be independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
L may be an optionally substituted linker, said L may comprise -L₁-L₂-L₃-L₄-L₅-.
Ab may be a ligand, "a" may be a number at least 0.
wherein, said L₁ may comprise optionally substituted 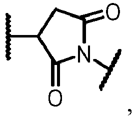
said L₂ may comprise a structure selected from the group consisting of optionally substituted 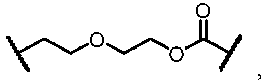 optionally substituted 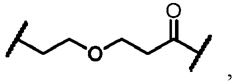 optionally substituted 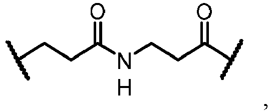 optionally substituted 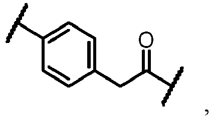 optionally substituted 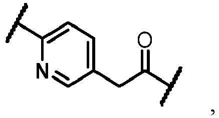 optionally substituted 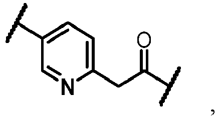 optionally substituted 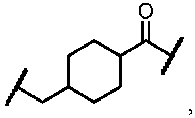 and optionally substituted 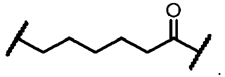
said L₃ may comprise a structure selected from the group consisting of 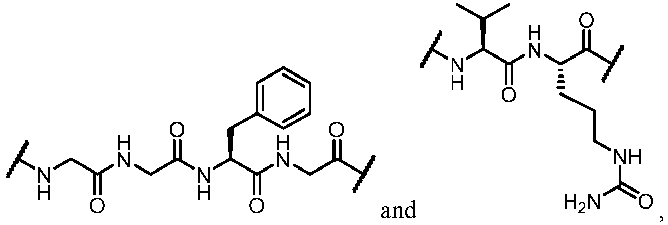
said L₄ may comprise a structure selected from the group consisting of optionally substituted 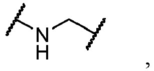 optionally substituted 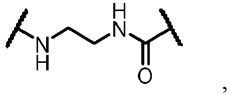 optionally substituted 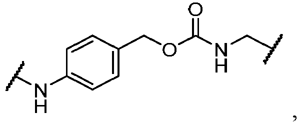 optionally substituted 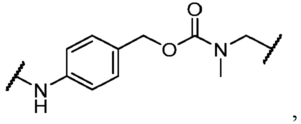 and optionally substituted 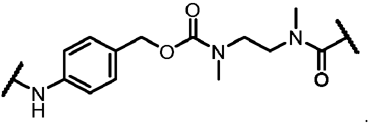
said L₅ may comprise a structure selected from the group consisting of optionally substituted 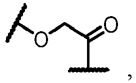 optionally substituted 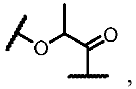 optionally substituted 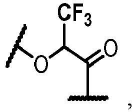 optionally substituted 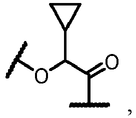 optionally substituted 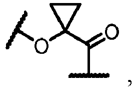 optionally substituted 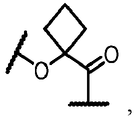 optionally substituted 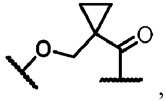 and optionally substituted

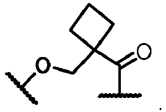

For example, the Ab of the present application may comprise an antibody or an antigen-binding fragment thereof.

For example, wherein said antibody may be selected from the group consisting of murine antibody, chimeric antibody, humanized antibody and full human antibody. For example, wherein said antibody may comprise a monoclonal antibody. For example, wherein said antibody may comprise a bispecific antibody. For example, wherein said antigen-binding fragment may be selected from the group consisting of Fab, Fab', Fv fragment, F(ab')₂, F(ab)₂, scFv, di-scFv, VHH and dAb.

For example, wherein said Ab may be selected from the group consisting of HER2 antibodies and TROP2 antibodies.

Said ligands described in the present application may comprise at least one CDR in the antibody heavy chain variable region VH. Said CDR may be defined according to Kabat.

In the present application, said ligand may comprise an HCDR1 and said HCDR1 may comprise the amino acid sequence shown in any one of SEQ ID NO: 1 and 11. Said CDR may be defined according to Kabat.

In the present application, said ligand may comprise HCDR2, and said HCDR2 may comprise the amino acid sequence shown in any one of SEQ ID NO: 2 and 12. Said CDR may be defined according to Kabat.

In the present application, said ligand may comprise HCDR3, and said HCDR3 may comprise the amino acid sequence shown in any one of SEQ ID NO: 3 and 13. Said CDR may be defined according to Kabat.

In the present application, said isolated ligand may comprise HCDR1-3, wherein said HCDR1 comprises the amino acid sequence shown in any one of SEQ ID NO: 1 and 11; said HCDR2 comprises the amino acid sequence shown in any one of SEQ ID NO: 2 and 12; and said HCDR3 comprises the amino acid sequence shown in any one of SEQ ID NO: 3 and 13. Said CDR may be defined according to Kabat.

For example, a ligand described in the present application may comprise HCDR1-3, wherein said HCDR1 may comprise the amino acid sequence shown in SEQ ID NO: 1; said HCDR2 may comprise the amino acid sequence shown in SEQ ID NO: 2; and said HCDR3 may comprise the amino acid sequence shown in SEQ ID NO: 3. For example, a ligand described in the present application may have HER2 binding capacity. Said CDR may be defined according to Kabat.

For example, a ligand described in the present application may comprise HCDR1-3, wherein said HCDR1 may comprise the amino acid sequence shown in SEQ ID NO: 11; said HCDR2 may comprise the amino acid sequence shown in SEQ ID NO: 12; and said HCDR3 may comprise the amino acid sequence shown in SEQ ID NO: 13. For example, a ligand described in the present application may have TROP2 binding capacity. Said CDR may be defined according to Kabat.

In the present application, said ligand may comprise at least one CDR in the antibody light chain variable region VL. Said CDR of the present application may be defined according to Kabat; it may also be defined according to Chothia, and CDR sequences defined in various ways are included in the scope of protection of the present application.

In the present application, said ligand may comprise LCDR1 and said LCDR1 may comprise the amino acid sequence shown in any one of SEQ ID NO: 4 and 14. Said CDR may be defined according to Kabat.

In the present application, said ligand may comprise an LCDR2, and said LCDR2 may comprise the amino acid sequence shown in any one of SEQ ID NO: 5 and 15. Said CDR may be defined according to Kabat.

In the present application, said ligand may comprise an LCDR3, and said LCDR3 may comprise the amino acid sequence shown in any one of SEQ ID NO: 6 and 16. Said CDR may be defined according to Kabat.

In the present application, said isolated ligand may comprise LCDRs 1-3, wherein said LCDR1 comprises the amino acid sequence shown in any one of SEQ ID NO: 4 and 14; said LCDR2 comprises the amino acid sequence shown in any one of SEQ ID NO: 5 and 15; and said LCDR3 comprises the amino acid sequence shown in any one of SEQ ID NO: 6 and 16. Said CDR may be defined according to Kabat.

For example, a ligand described in the present application may comprise LCDR1-3, wherein said LCDR1 may comprise the amino acid sequence shown in SEQ ID NO: 4; said LCDR2 may comprise the amino acid sequence shown in SEQ ID NO: 5; and said LCDR3 may comprise the amino acid sequence shown in SEQ ID NO: 6. For example, a ligand described in the present application may have HER2 binding capacity. Said CDR may be defined according to Kabat.

For example, a ligand described in the present application may comprise with LCDR1-3, wherein said LCDR1 may comprise the amino acid sequence shown in SEQ ID NO: 14; said LCDR2 may comprise the amino acid sequence shown in SEQ ID NO: 15; and said LCDR3 may comprise the amino acid sequence shown in SEQ ID NO: 16. For example, a ligand described in the present application may have a TROP2 binding capacity. Said CDR may be defined according to Kabat.

In the present application, said ligand may comprise LCDR1-3 and HCDR1-3, wherein said HCDR1 comprises the amino acid sequence shown in any one of SEQ ID NO: 1 and 11; said HCDR2 comprises the amino acid sequence shown in any one of SEQ ID NO: 2 and 12; and said HCDR3 comprises the amino acid sequence shown in any one of SEQ ID NO: 3 and 13 amino acid sequence, said LCDR1 comprising the amino acid sequence shown in either of SEQ ID NO: 4 and 14; said LCDR2 comprising the amino acid sequence shown in either of SEQ ID NO: 5 and 15; and said LCDR3 comprising the amino acid sequence shown in either of SEQ ID NO: 6 and 16. Said CDR may be defined according to Kabat.

For example, the ligands described in the present application may comprise LCDR1-3 and HCDR1-3, wherein said HCDR1 comprises the amino acid sequence shown in SEQ ID NO: 1; said HCDR2 comprises the amino acid sequence shown in SEQ ID NO: 2; and said HCDR3 comprises the amino acid sequence shown in SEQ ID NO: 3. Said LCDR1 comprises the amino acid sequence shown in SEQ ID NO: 4; said LCDR2 comprises the amino acid sequence shown in SEQ ID NO: 5; and said LCDR3 comprises the amino acid sequence shown in SEQ ID NO: 6. Said CDR may be defined according to Kabat.

For example, the ligands described in the present application may comprise LCDR1-3 and HCDR1-3, wherein said HCDR1 comprises the amino acid sequence shown in SEQ ID NO: 11; said HCDR2 comprises the amino acid sequence shown in SEQ ID NO: 12; and said HCDR3 comprises the amino acid sequence shown in SEQ ID NO: 13. Said LCDR1 comprises the amino acid sequence shown in SEQ ID NO: 14; said LCDR2 comprises the amino acid sequence shown in SEQ ID NO: 15; and said LCDR3 comprises the amino acid sequence shown in SEQ ID NO: 16. Said CDR may be defined according to Kabat.

In the present application, said ligand may comprise a heavy chain variable region VH, and said VH may comprise the amino acid sequence shown in any one of SEQ ID NO: 7 and 17.

In the present application, said ligand may comprise a light chain variable region VL, and said VL may comprise the amino acid sequence shown in any one of SEQ ID NO: 8 and 18.

In the present application, said ligand may comprise a light chain variable region VL and a heavy chain variable region VH. wherein said VH may comprise the amino acid sequence shown in any one of SEQ ID NO: 7 and 17, and said VL may comprise the amino acid sequence shown in any one of SEQ ID NO: 8 and 18.

For example, a ligand described in the present application may comprise a light chain variable region VL and a heavy chain variable region VH, wherein said VH may comprise the amino acid sequence shown in SEQ ID NO: 7 and said VL may comprise the amino acid sequence shown in SEQ ID NO: 8. For example, the ligands described in the present application may have HER2 binding capacity.

For example, a ligand described in the present application may comprise a light chain variable region VL and a heavy chain variable region VH, wherein said VH may comprise the amino acid sequence shown in SEQ ID NO: 17 and said VL may comprise the amino acid sequence shown in SEQ ID NO: 18. For example, the ligands described in the present application may have TROP2 binding capacity.

In the present application, said ligand may comprise HCDR1-3 in the heavy chain variable region VH, and said VH may comprise the amino acid sequence shown in any one of SEQ ID NO: 7 and 17.

In the present application, said ligand may comprise LCDR1-3 in the light chain variable region VL, and said VL may comprise the amino acid sequence shown in any one of SEQ ID NO: 8 and 18.

In the present application, said ligand may comprise LCDR1-3 in the light chain variable region VL and HCDR1-3 in the heavy chain variable region VH, wherein said VH may comprise the amino acid sequence shown in any one of SEQ ID NO: 7 and 17, and said VL may comprise the amino acid sequence shown in any one of SEQ ID NO: 8 and 18.

For example, a ligand described in the present application may comprise LCDR1-3 in the light chain variable region VL and HCDR1-3 in the heavy chain variable region VH, wherein said VH may comprise the amino acid sequence shown in SEQ ID NO: 7 and said VL may comprise the amino acid sequence shown in SEQ ID NO: 8. For example, the ligands described in this application may have HER2 binding capacity.

For example, a ligand described in the present application may comprise LCDR1-3 in the light chain variable region VL and HCDR1-3 in the heavy chain variable region VH, wherein said VH may comprise the amino acid sequence shown in SEQ ID NO: 17 and said VL may comprise the amino acid sequence shown in SEQ ID NO: 18. For example, the ligands described in the present application may have TROP2 binding capacity.

In the present application, said ligand may comprise a heavy chain, and said heavy chain may comprise an amino acid sequence shown in any one of SEQ ID NO: 9 and 19.

In the present application, said ligand may comprise a light chain, and said light chain may comprise an amino acid sequence shown in any one of SEQ ID NO: 10 and 20.

In the present application, said ligand may comprise an antibody light chain and an antibody heavy chain, wherein said heavy chain may comprise the amino acid sequence shown in any one of SEQ ID NO: 9 and 19, and said light chain may comprise the amino acid sequence shown in any one of SEQ ID NO: 10 and 20.

In the present application, said ligand may comprise an antibody light chain and an antibody heavy chain, wherein said heavy chain may comprise the amino acid sequence shown in SEQ ID NO: 9, and said light chain may comprise the amino acid sequence shown in SEQ ID NO: 10.

In the present application, said ligand may comprise an antibody light chain and an antibody heavy chain, wherein said heavy chain may comprise the amino acid sequence shown in SEQ ID NO: 19, and said light chain may comprise the amino acid sequence shown in SEQ ID NO: 20.

The antibodies of the present application may be prepared using techniques well known in the art, such as hybridoma methods, recombinant DNA techniques, phage display techniques, synthetic techniques or combinations of such techniques, or other techniques known in the art. Variants may refer to mutants of the amino acid sequence of an antibody, as well as covalent derivatives of a natural polypeptide, provided that biological activity equivalent to that of the natural polypeptide is retained. Amino acid sequence mutants differ from the natural amino acid sequence generally in that a live plurality of amino acids in the natural amino acid sequence are substituted or one or more amino acids are missing and/or inserted in the polypeptide sequence. Deletion mutants include fragments of the natural polypeptide and N-terminal and/or C-terminal truncation mutants. Generally the amino acid sequence mutants have at least 70%, 75%, 80%, 85%, 90%, 95%, 98% or 99% homology compared to the natural sequence.

For example, wherein said "a" may be taken as an average of the molar ratio of drug molecules to antibody molecules in the antibody-drug conjugate obtained by coupling a single antibody molecule to drug molecules, and wherein "a" may be an integer or decimal number from 1 to 8, for example, "a" may be from about 1 to about 2, from about 1 to about 3, from about 1 to about 4, from about 1 to about 5, from about 1 to about 6, from about 1 to about 7, or from about 1 to about 8; for example, "a" may be from about 2 to about 8 , about 3 to about 8, about 4 to about 8, about 5 to about 8, about 6 to about 8, about 7 to about 8, or about 1, about 2, about 3, about 4, about 5, about 6, about 7 or about 8. For example, wherein said "a" can be determined by a method selected from the group consisting of hydrophobic chromatography, sodium dodecyl sulphate polyacrylamide gel electrophoresis, and liquid mass spectrometry.

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise a structure selected from the group consisting of 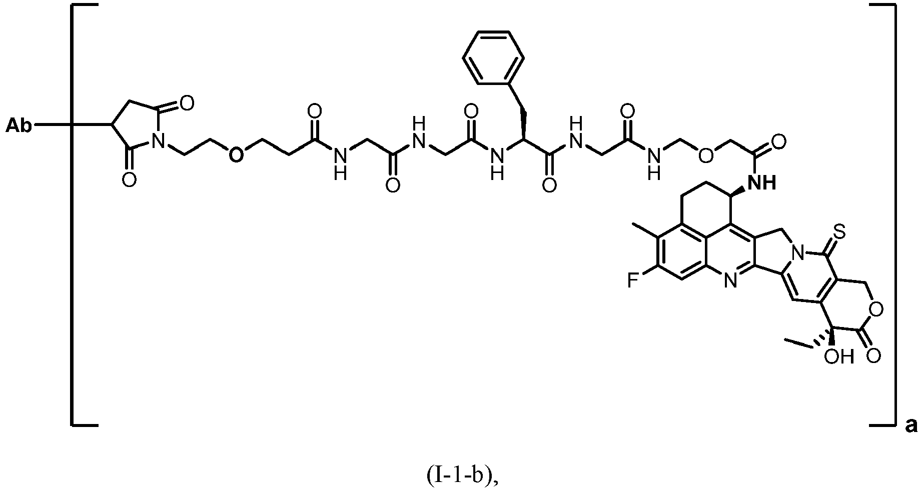 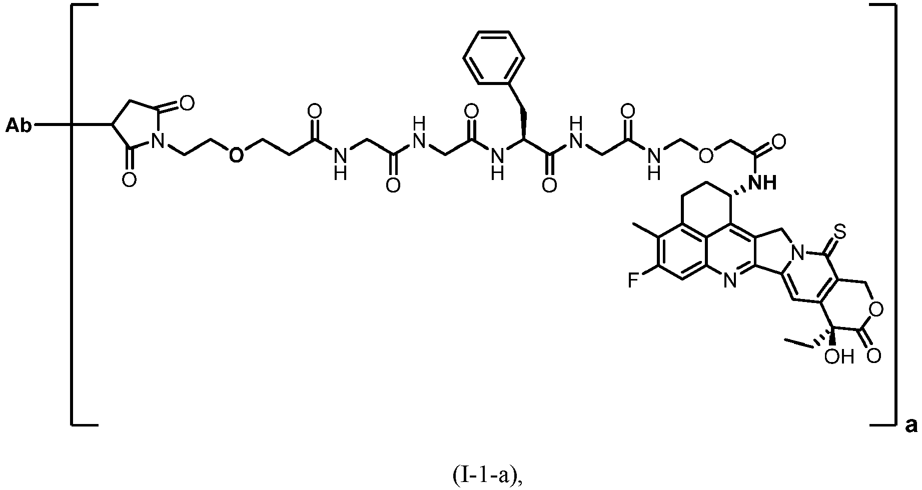 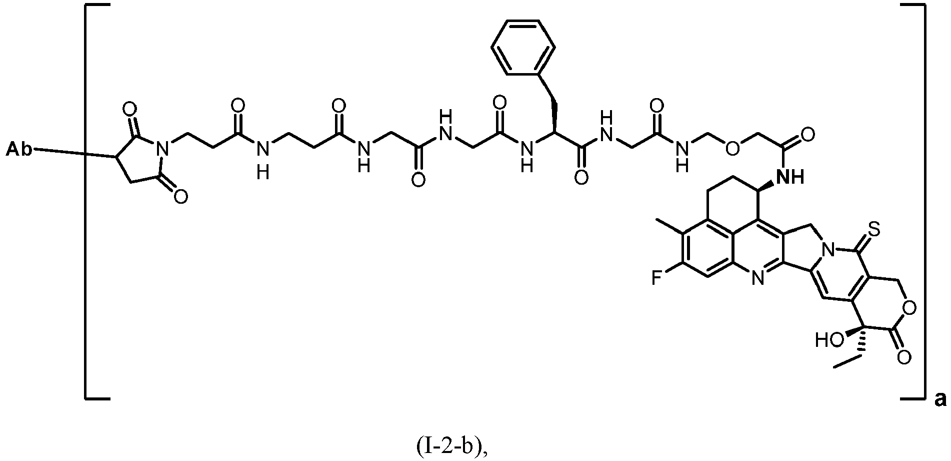 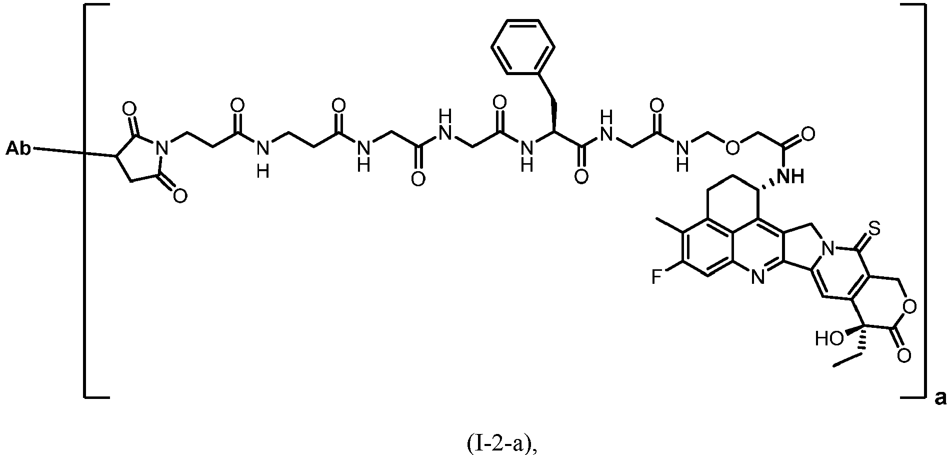 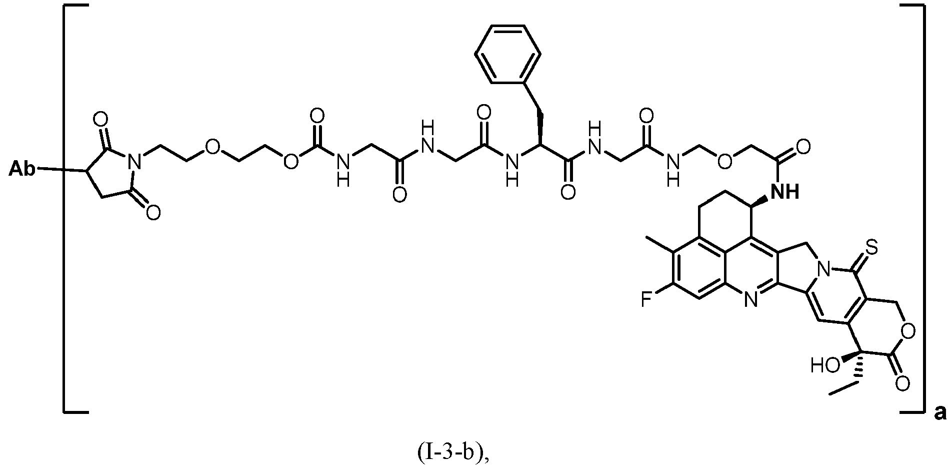 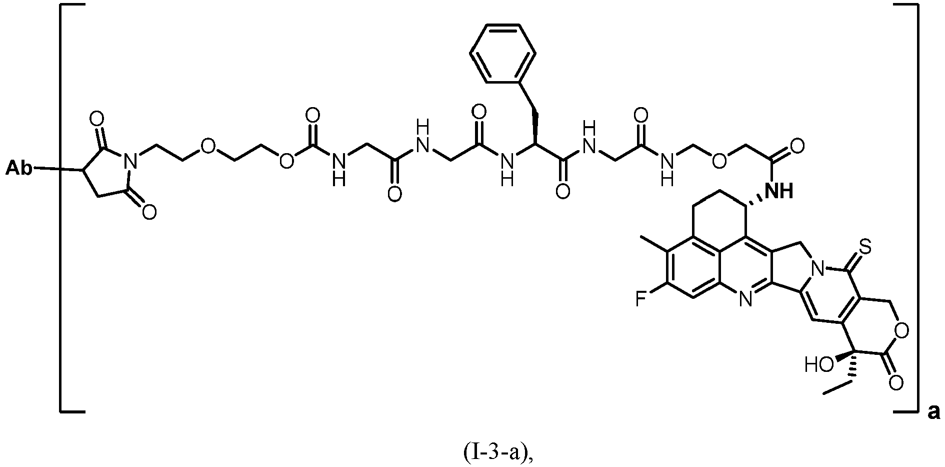 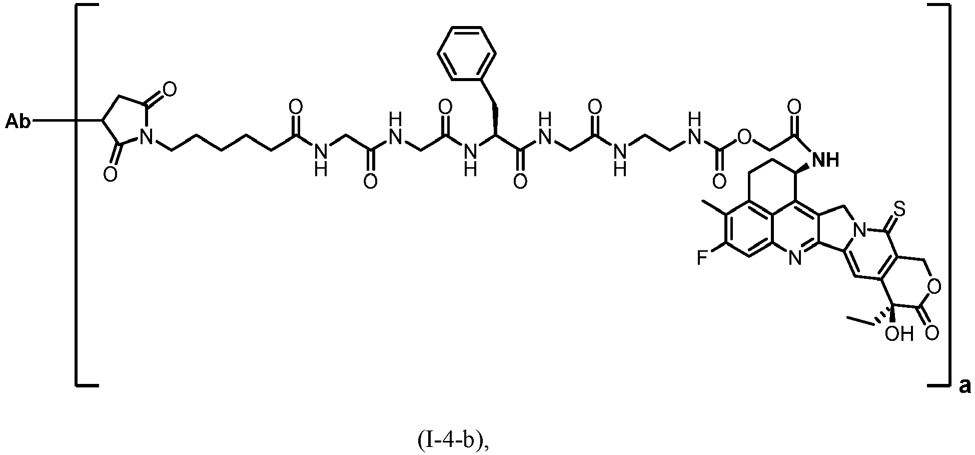 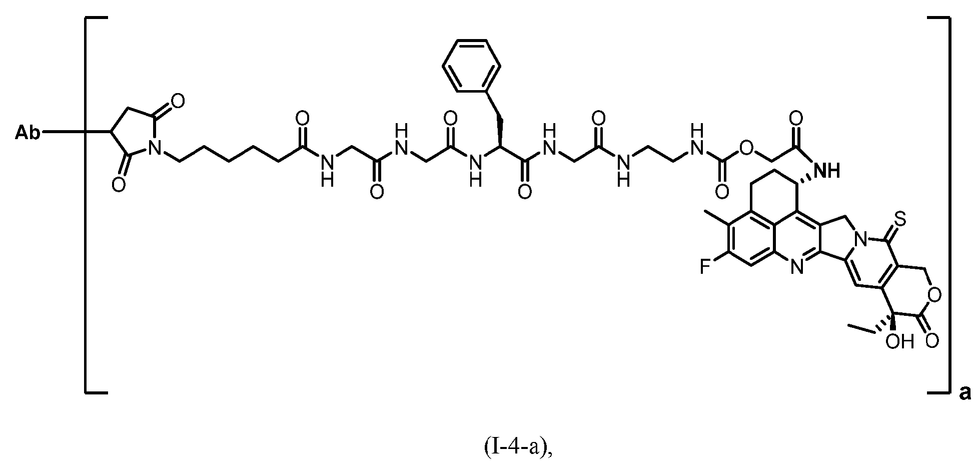 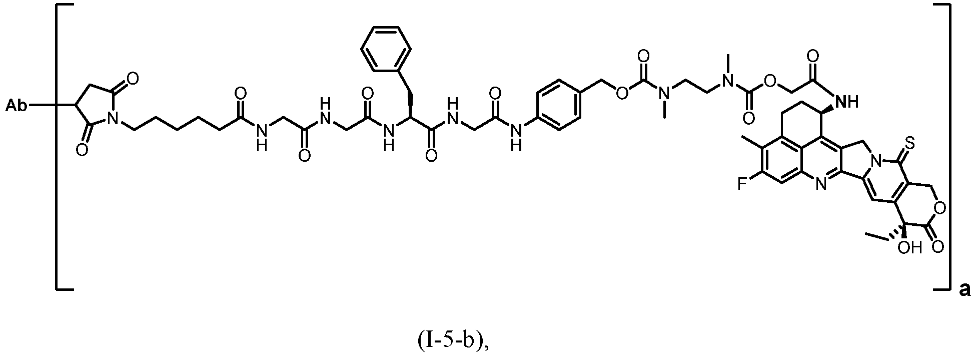 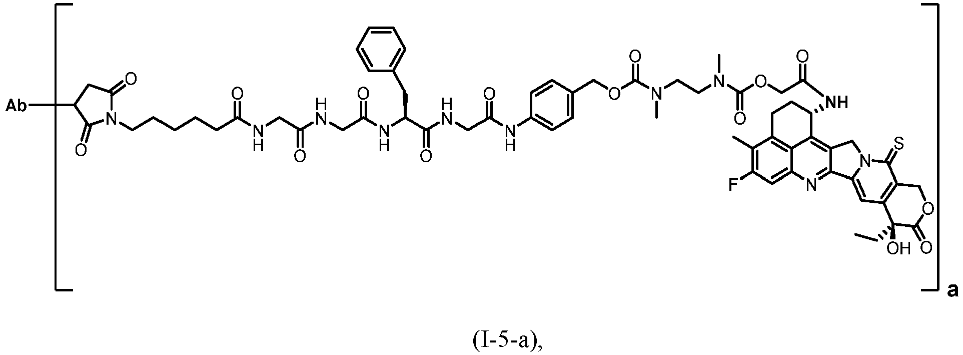 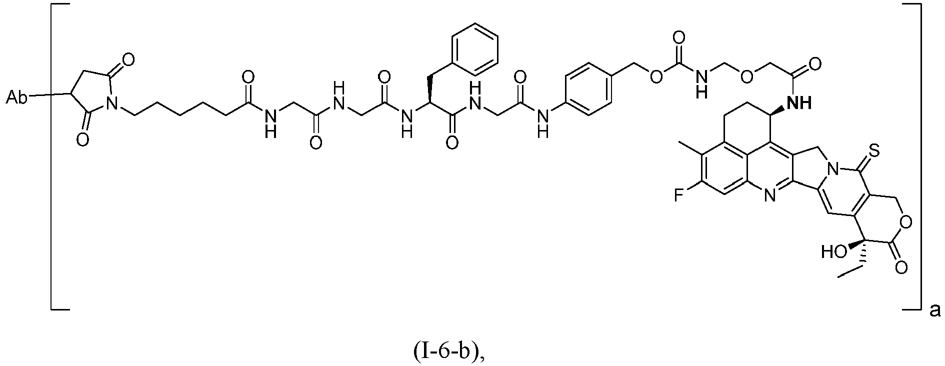 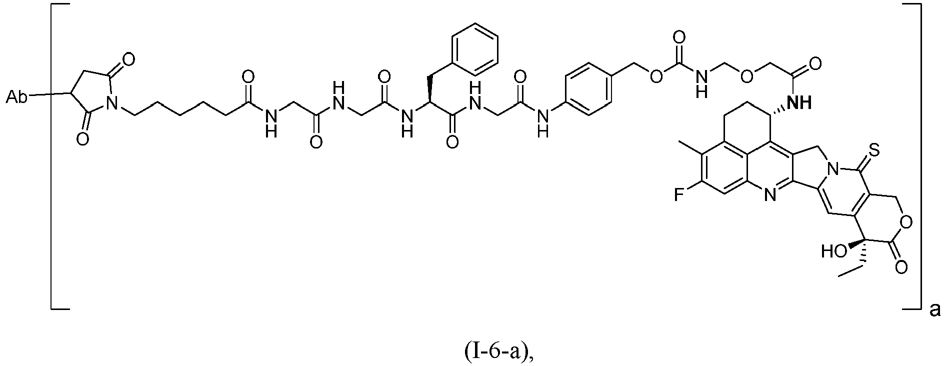 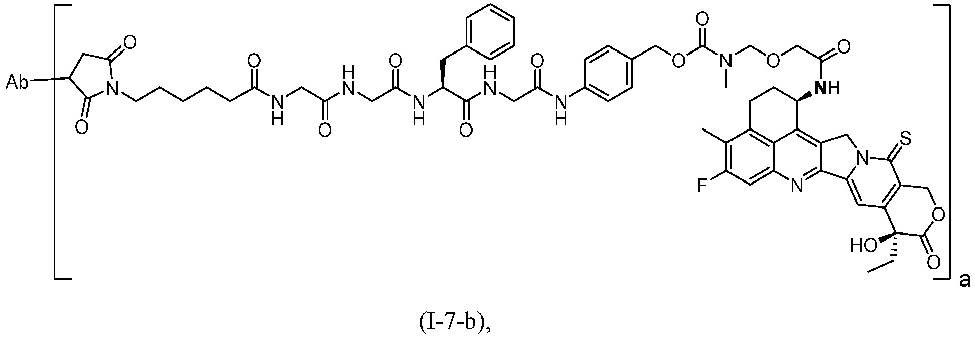 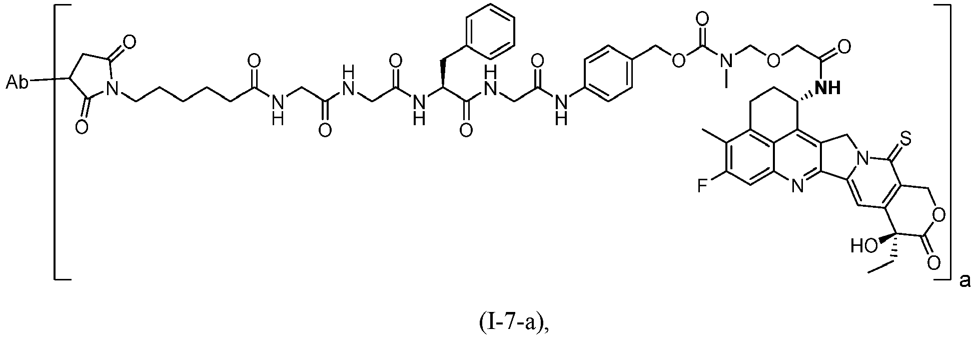 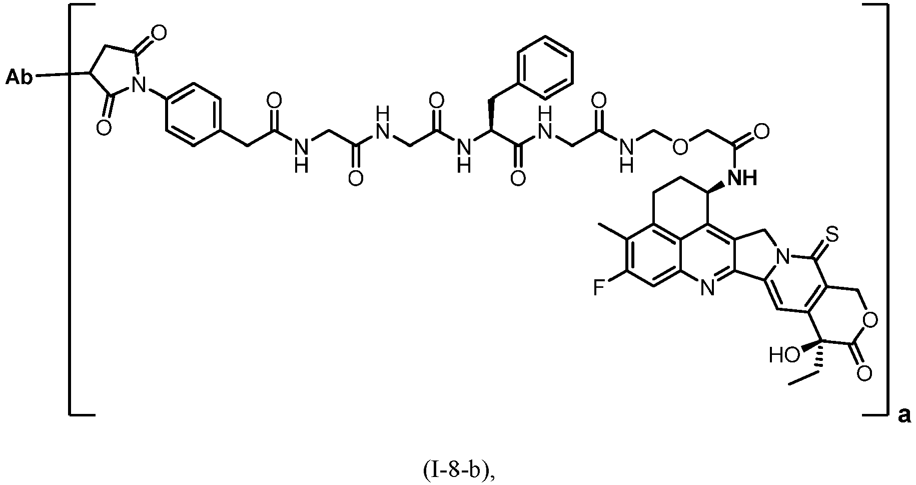 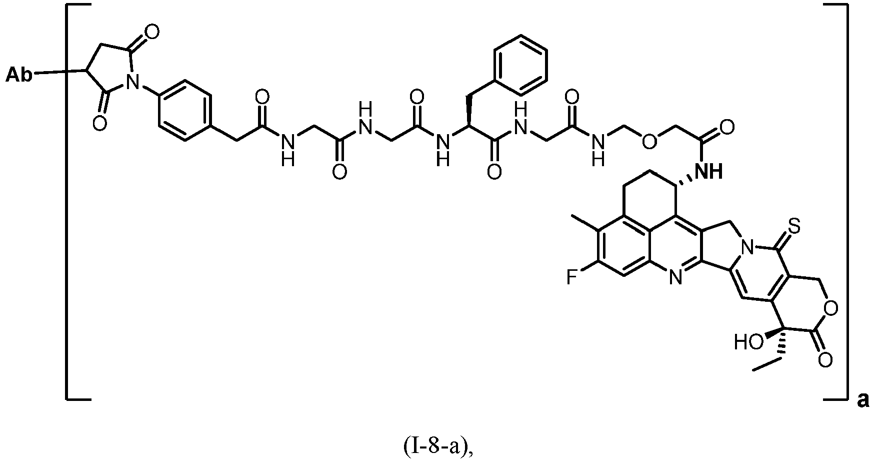 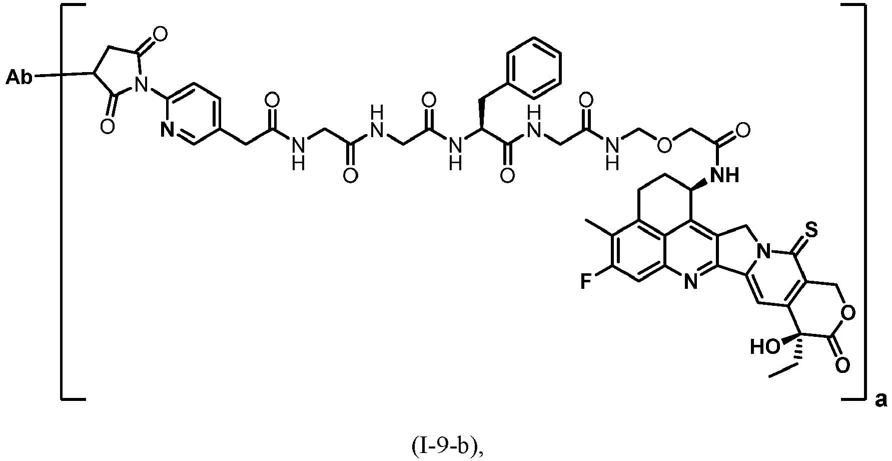 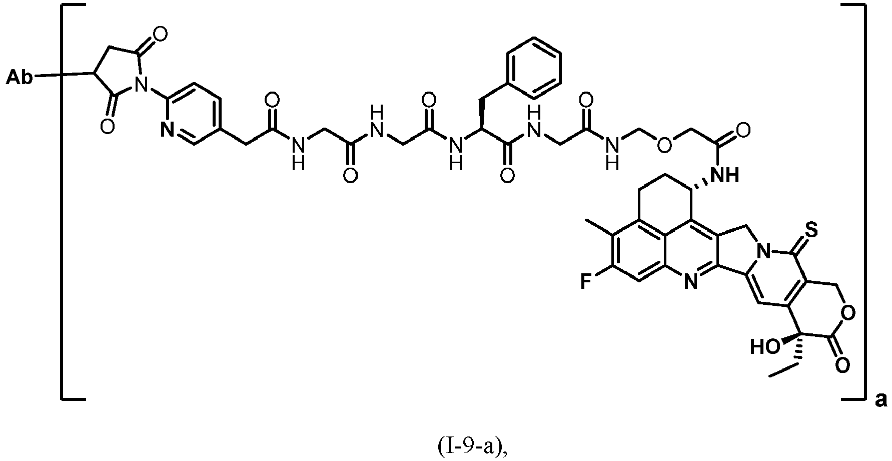 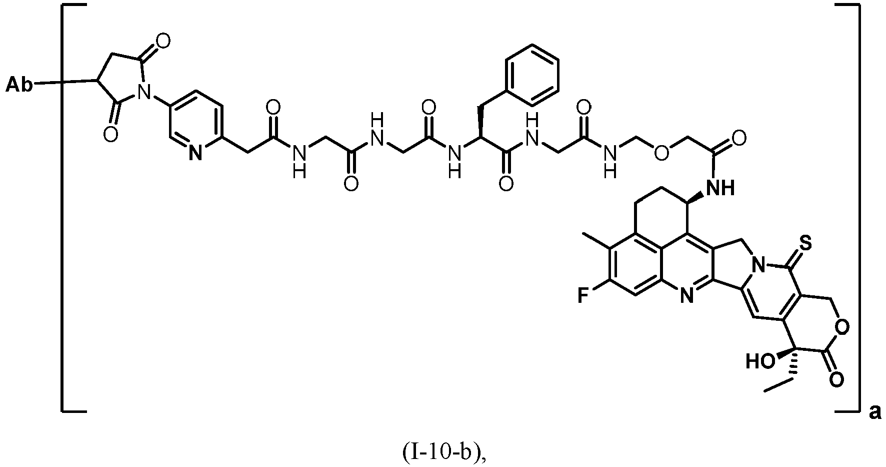 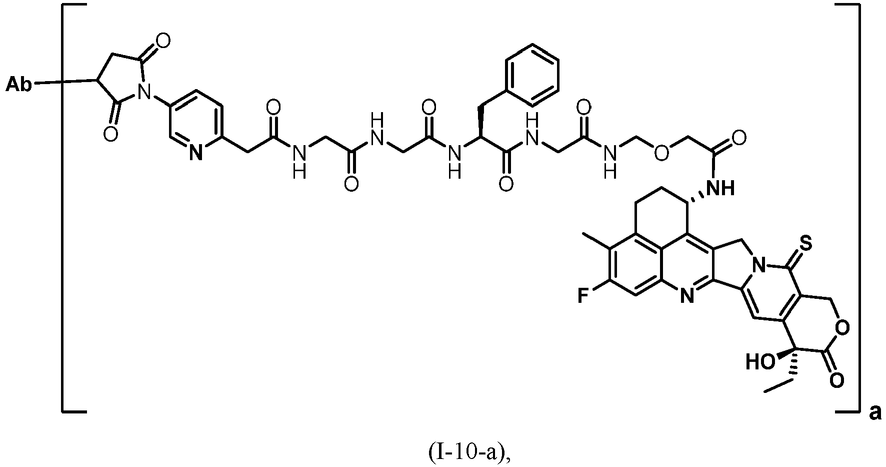 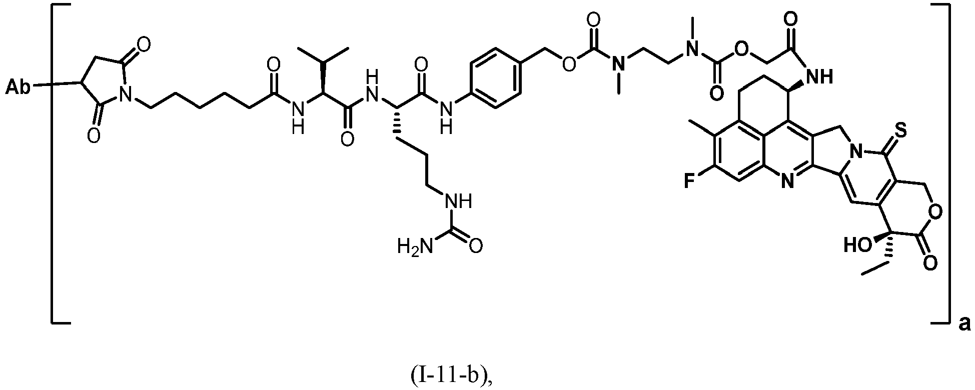 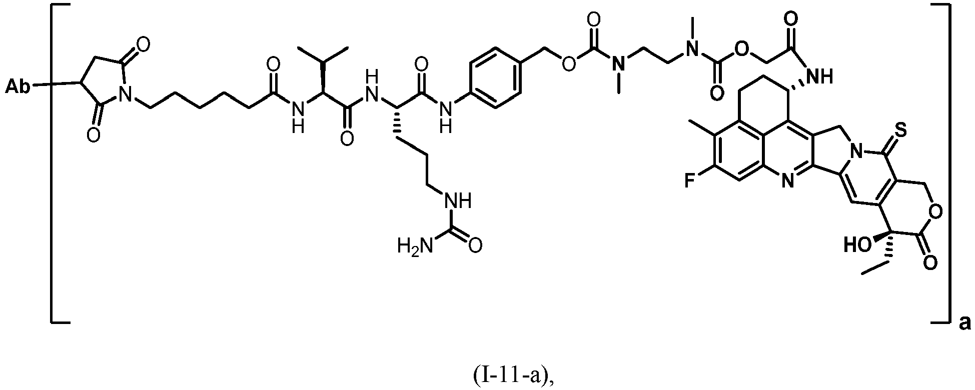 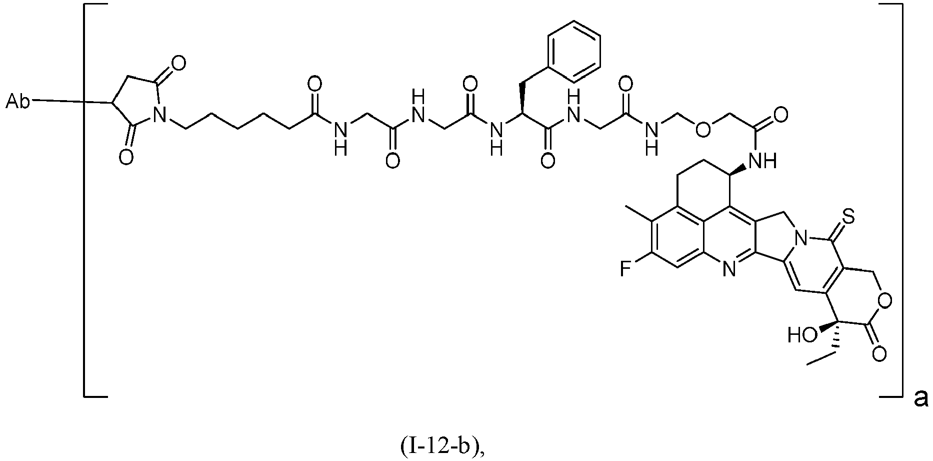 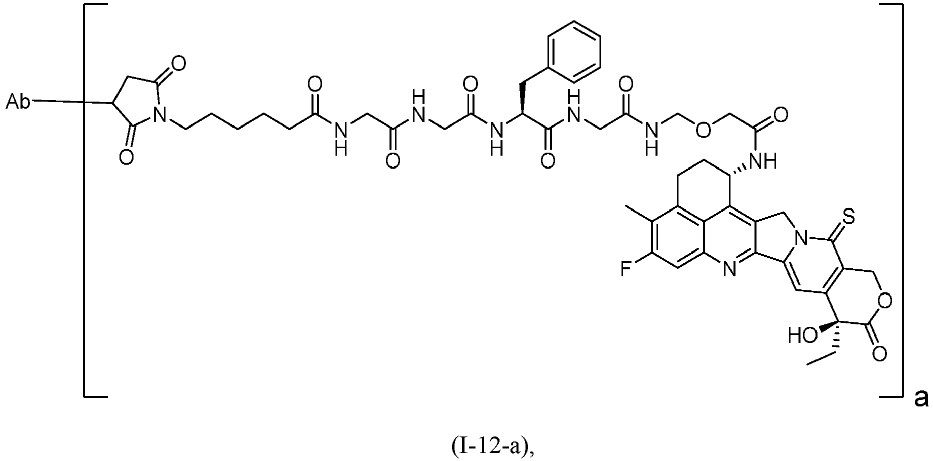 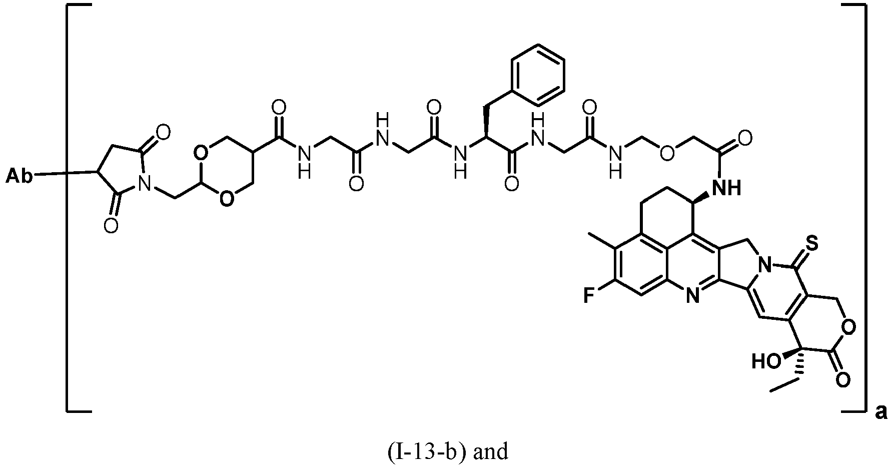 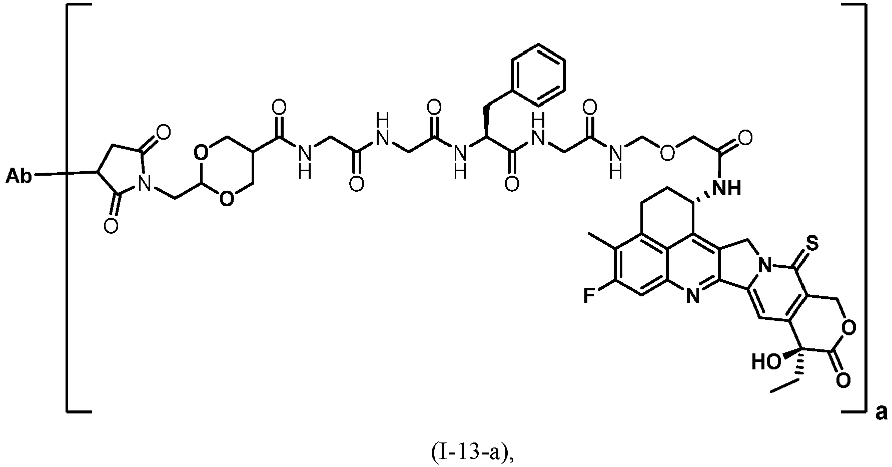 where Ab is the ligand, "a" is a number greater than 0, and a is a decimal or integer.

In one aspect, the present application provides a compound, or a tautomer, a mesomer, a racemate, an enantiomer, a diastereomer, or a mixture thereof, or a pharmaceutically acceptable salt or hydrate thereof, wherein the ligand conjugate comprises the structure shown in formula (II): 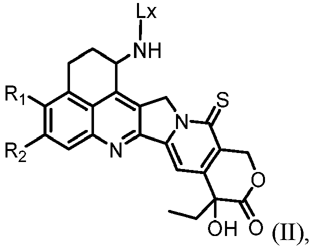 for example it can be 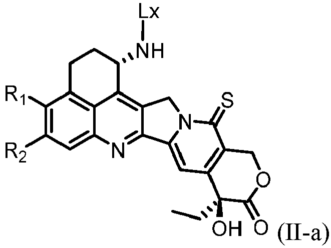 and/or 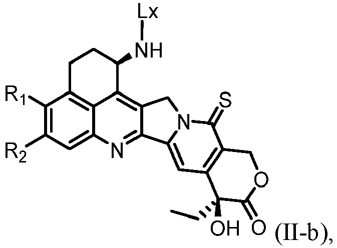 wherein,
R₁ and R₂ may each independently be selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl,
Lx is a linking group.

For example, in the structure shown in formula (II), said Lx may comprise a linker L₁ₓ, said L₁ₓ may be directly or indirectly linked to a ligand. For example, said L₁ₓ may be directly or indirectly linked to the thiol group of the ligand. For example, said L₁ₓ may comprise optionally substituted 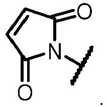 For example, L₁ₓ in the structure shown in formula (II) can exist in a ring-opened form as shown in the following equation.

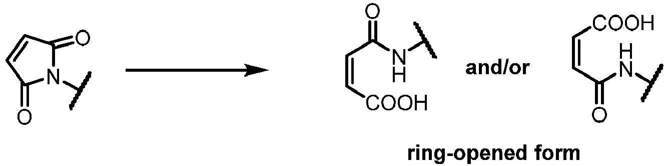

For example, in the structure shown in formula (II), said Lx may comprise a linker L₂. For example, said L₂ may be directly or indirectly linked to L₁ₓ. For example, said L₂ may comprise a group selected from the group consisting of optionally substituted -(CH₂) ₘ₁-X₁-(CH₂)ₘ₂-C(O)-, optionally substituted -(CH₂CH₂O)ₙ-C(O)-, and optionally substituted -(CH₂)ₚ-C(O)-, X₁ may be selected from the group consisting of -O-, optionally substituted -C(O)-NH-, optionally substituted aryl, optionally substituted heteroaryl, optionally substituted aliphatic heterocyclyl, and optionally substituted aliphatic cyclyl, wherein, m1, m2, and n are each independently selected from numbers of at least 0.

For example, in the structure shown in formula (II), said L₂ may comprise optionally substituted -(CH₂CH₂O)ₙ-C(O)-. For example, wherein, n can be 2.

For example, in the structure shown in formula (II), said L₂ may comprise optionally substituted -(CH₂)ₘ₁-X₁-(CH₂)ₘ₂-C(O)-. For example, X₁ may be selected from the group consisting of -O-, optionally substituted -C(O)-NH-. For example, m1 can be 2. For example, m2 can be 2.

For example, in the structure shown in formula (II), X₁ may comprise a group selected from the group consisting of optionally substituted aryl and optionally substituted heteroaryl. For example, X₁ may comprise optionally substituted phenyl. For example, X₁ may comprise optionally substituted pyridyl. For example, m1 can be 0. For example, m2 can be 1.

For example, in the structure shown in formula (II), X₁ may comprise an optionally substituted aliphatic heterocyclyl including two oxygen atoms. For example, X₁ may comprise optionally substituted 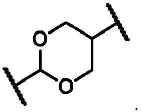 For example, X₁ may comprise an optionally substituted cyclohexyl group. For example, m1 can be 1. For example, m2 can be 0.

For example, in the structure shown in formula (II), said L₂ may comprise optionally substituted -(CH₂)ₚ-C(O)-. For example, p can be 5.

For example, in the structure shown in formula (II), said L₂ may comprise a structure selected from the group consisting of optionally substituted 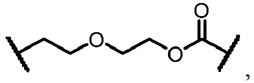 optionally substituted 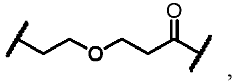 optionally substituted 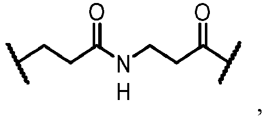 optionally substituted 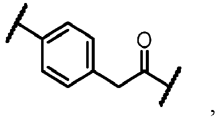 optionally substituted 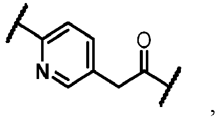 optionally substituted 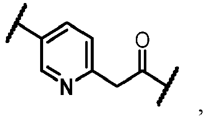 optionally substituted 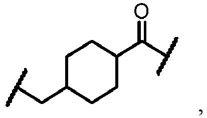 and optionally substituted

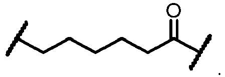

For example, in the structure shown in formula (II), said Lx may also comprise a linker L₃. For example, said L₃ may be directly or indirectly linked to said L₂. For example, said L₃ may comprise a peptide residue.

For example, said L₃ may comprise peptide residues composed of amino acids selected from the group consisting of phenylalanine, isoleucine, leucine, tryptophan, valine, methionine, tyrosine, alanine, threonine, histidine, serine, glutamine, arginine, lysine, asparagine, glutamate, proline, citrulline, aspartate, and glycine. For example, said L₃ may comprise peptide residues composed of amino acids selected from the group consisting of glycine, phenylalanine, valine, and citrulline. For example, said L₃ may comprise peptide residues selected from the group consisting of -glycine-phenylalanine-glycine- (-Gly-Phe-Gly-), -glycine-glycine-phenylalanine-glycine- (-Gly-Gly-Phe-Gly-), and -valine-citrulline- (-Val-Cit-).

For example, in the structure shown in formula (II), said L₃ may comprise a structure selected from the group consisting of 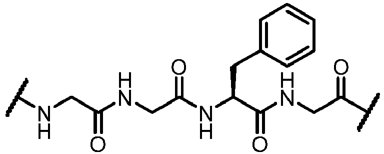 and

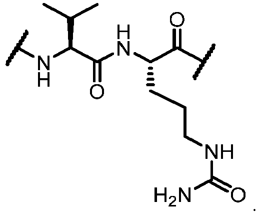

For example, in the structure shown in formula (II), said Lx may also comprise a linker L₄. For example, said L₄ may be directly or indirectly linked to said L₃.

For example, in the structure shown in formula (II), said L₄ may comprise optionally substituted -L₄ₐ-NR₄-CH₂-L_{4b}-,
L₄ₐ may be absent or L₄ₐ may comprise optionally substituted 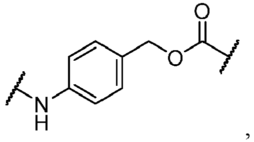
L_{4b} may be absent or L_{4b} may comprise optionally substituted 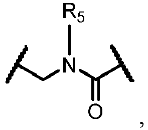
R₄ and R₅ may each independently be selected from the group consisting of hydrogen and optionally substituted alkyl.

For example, in the structure shown in formula (II), said L₄ may comprise optionally substituted 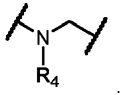 For example, said L₄ may comprise optionally substituted 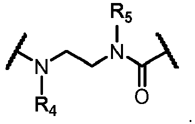 For example, said L₄ may comprise optionally substituted 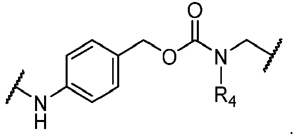 For example, said L₄ may comprise optionally substituted 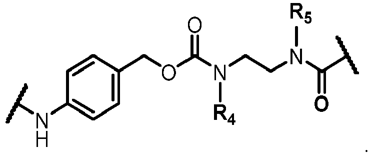 For example, said R₄ may be selected from the group consisting of hydrogen and optionally substituted methyl. For example, said R₅ may be selected from the group consisting of hydrogen and optionally substituted methyl.

For example, in the structure shown in formula (II), said L₄ may comprise a structure selected from the group consisting of optionally substituted 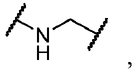 optionally substituted 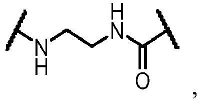 optionally substituted 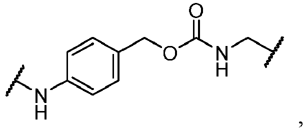 optionally substituted 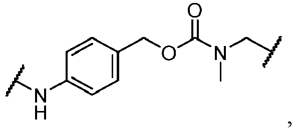 optionally substituted

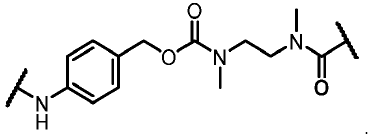

For example, in the structure shown in formula (II), said Lx may also comprise a linker L₅. For example, said L₅ may be directly or indirectly linked to said L₄.

For example, in the structure shown in formula (II), said L₅ may comprise optionally substituted 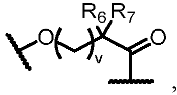 R₆ and R₇ may each be independently selected from the group consisting of hydrogen, deuterium, optionally substituted alkyl, optionally substituted haloalkyl, optionally substituted cycloalkyl, and optionally substituted cycloalkylalkyl, or R₆ and R₇ may be joined with the atom to which they attached to form an optionally substituted cycloalkyl, wherein v may be selected from a number at least 0.

For example, in the structure shown in formula (II), said R₆ may be hydrogen. For example, said R₆ may be optionally substituted methyl. For example, said R₆ may be optionally substituted halomethyl. For example, said R₆ may be fluorine substituted methyl. For example, said R₆ may be trifluoromethyl. For example, said R₆ may be optionally substituted cycloalkyl. For example, said R₆ may be optionally substituted cyclopropyl. For example, in the structure shown in formula (II), said R₇ may be hydrogen.

For example, in the structure shown in formula (II), R₆ and R₇ may be joined with the atom to which they attached to form an optionally substituted cyclopropyl. For example, R₆ and R₇ may be joined with the atom to which they attached to form an optionally substituted cyclobutyl. For example, v may be 0. For example, v may be 1.

For example, in the structure shown in formula (II), said L₅ may comprise a structure selected from the group consisting of optionally substituted 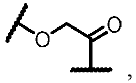 optionally substituted 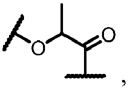 optionally substituted 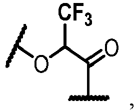 optionally substituted 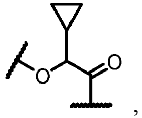 optionally substituted 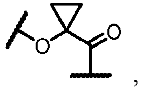 optionally substituted 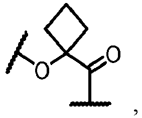 optionally substituted 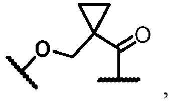 and optionally substituted

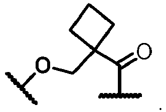

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (II): 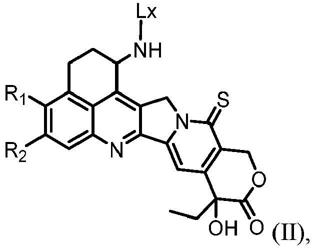 for example it can be 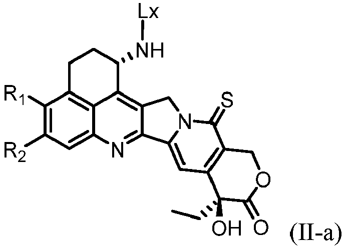 and/or 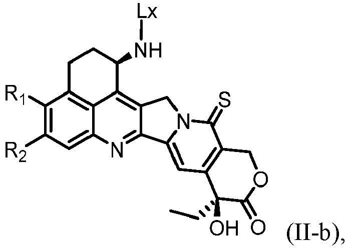
wherein R₁ and R₂ may each be independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached may form a structure selected from the group consisting of optionally substituted aliphatic cyclyl, optionally substituted aliphatic heterocyclyl, optionally substituted aryl, and optionally substituted heteroaryl;
Lx may be an optionally substituted linker group, said Lx may comprise L₁ₓ-L₂-L₃-L₄-L₅-.

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (II): 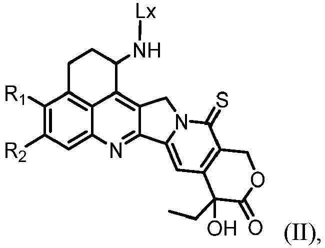 for example it can be 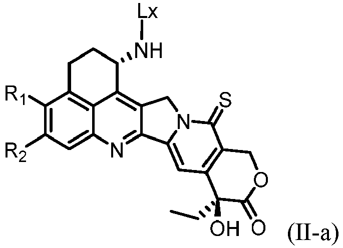 and/or 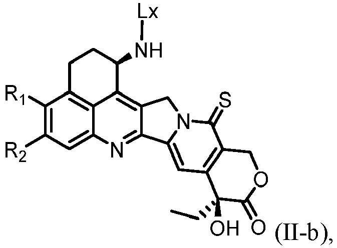
wherein, R₁ and R₂ may each be independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached may form a structure selected from the group consisting of optionally substituted aliphatic cyclyl, optionally substituted aliphatic heterocyclyl, optionally substituted aryl, and optionally substituted heteroaryl;
Lx may be an optionally substituted linker, said Lx may comprise L₁ₓ-L₂-L₃-L₄-L₅-,
wherein, said L₁ₓ may comprise a linking group that can be linked to a thiol group,
said L₂ may comprise a group selected from the group consisting of optionally substituted-(CH₂)ₘ₁-X₁-(CH₂)ₘ₂-C(O)-, optionally substituted -(CH₂CH₂O)ₙ-C(O)-, and optionally substituted-(CH₂)ₚ-C(O)-, X₁ may be selected from the group consisting of -O-, optionally substituted -C(O)-NH-, optionally substituted phenyl, optionally substituted pyridyl, optionally substituted aliphatic heterocyclyl containing two oxygen atoms and four carbon atoms, and optionally substituted cyclohexyl, wherein m1, m2 and n may each be independently selected from a number of at least 0;
said L₃ may comprise peptide residues,
said L₄ may comprise optionally substituted 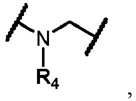 optionally substituted 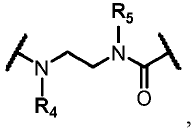 optionally substituted 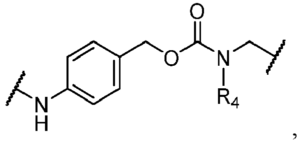 or optionally substituted 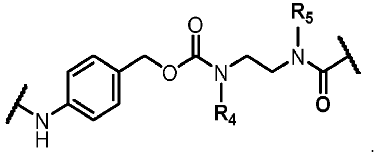 For example, said R₄ may be selected from the group consisting of hydrogen and optionally substituted C₁-C₆ alkyl. For example, said R₅ may be selected from the group consisting of hydrogen and optionally substituted C₁-C₆ alkyl.
said L₅ may comprise optionally substituted 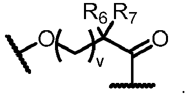 R₆ and R₇ may each be independently selected from the group consisting of hydrogen, deuterium, optionally substituted alkyl, optionally substituted haloalkyl, optionally substituted cycloalkyl, and optionally substituted cycloalkylalkyl, or R₆ and R₇ may be joined with the atom to which they attached to form an optionally substituted cycloalkyl, wherein v may be selected from a number at least 0. For example, cycloalkylalkyl in the present application may refer to an alkyl group substituted with cycloalkyl, wherein the cycloalkylalkyl group may also be optionally substituted with an additional group.

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (II): 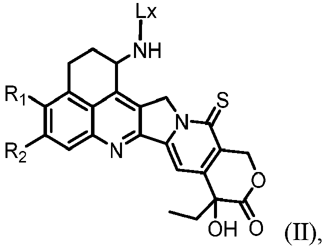 for example it can be 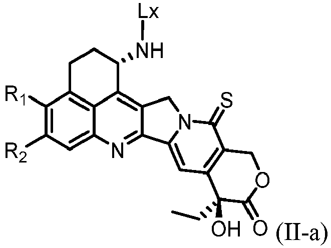 and/or 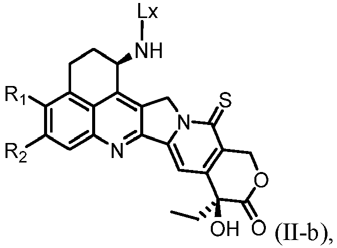
wherein R₁ and R₂ may each be independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached may form a structure selected from the group consisting of optionally substituted aliphatic cyclyl, optionally substituted aliphatic heterocyclyl, optionally substituted aryl, and optionally substituted heteroaryl;
Lx may be an optionally substituted linking group, said Lx may comprise L₁ₓ-L₂-L₃-L₄-L₅-.
wherein, said L₁ₓ may comprise optionally substituted 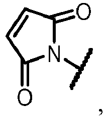
said L₂ may comprise a structure selected from the group consisting of optionally substituted 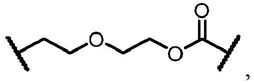 optionally substituted 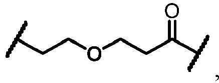 optionally substituted 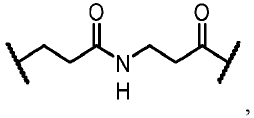 optionally substituted 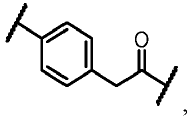 optionally substituted 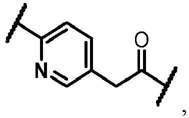 optionally substituted 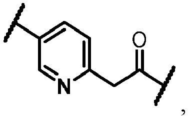 optionally substituted 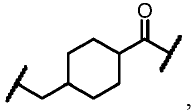 and optionally substituted 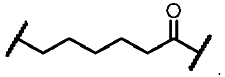
said L₃ may comprise a structure selected from the group consisting of 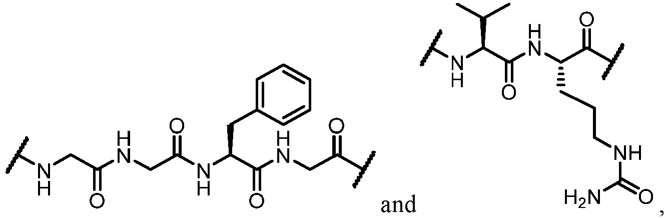
said L₄ may comprise a structure selected from the group consisting of optionally substituted 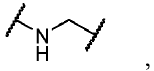 optionally substituted 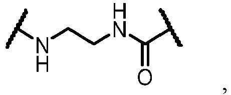 optionally substituted 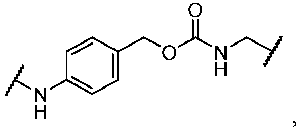 optionally substituted 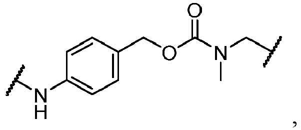 and optionally substituted 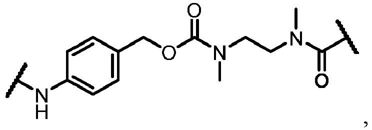
said L₅ may comprise a structure selected from the group consisting of optionally substituted 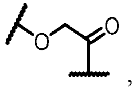 optionally substituted 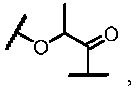 optionally substituted 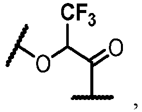 optionally substituted 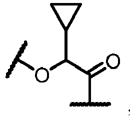 optionally substituted 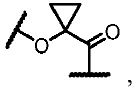 optionally substituted 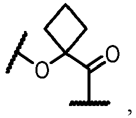 optionally substituted 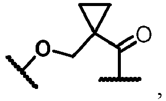 and optionally substituted

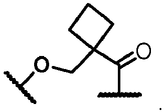

In another aspect, the present application also provides a compound, or a tautomer, a mesomer, a racemate, an enantiomer, a diastereomer, or a mixture thereof, or a pharmaceutically acceptable salt or hydrate thereof, wherein said the compound may comprise a structure selected from the group consisting of

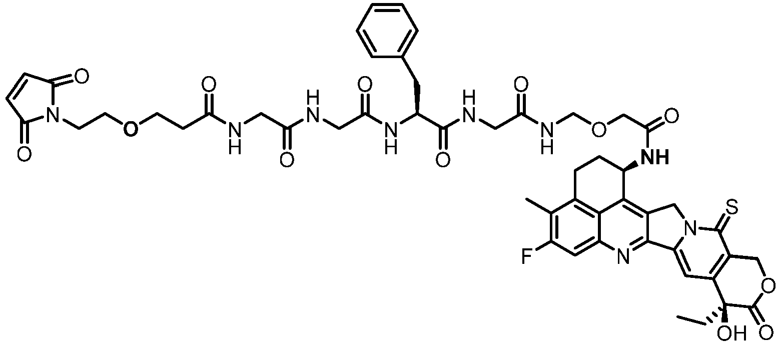

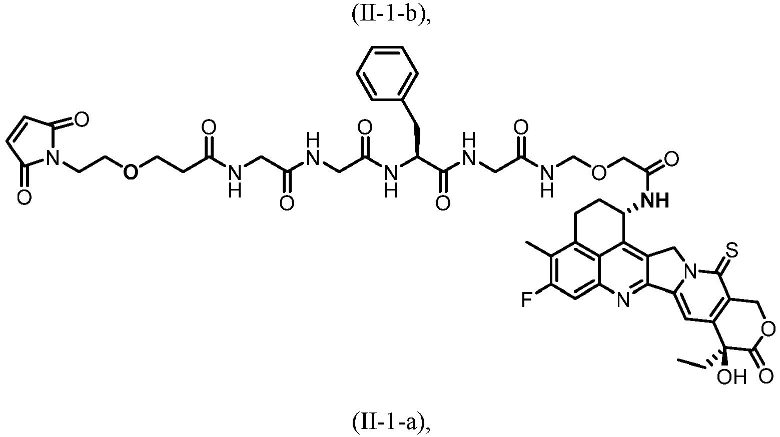

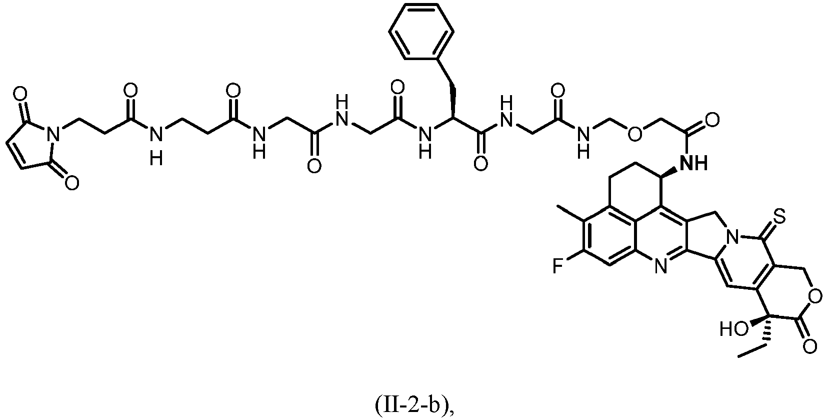

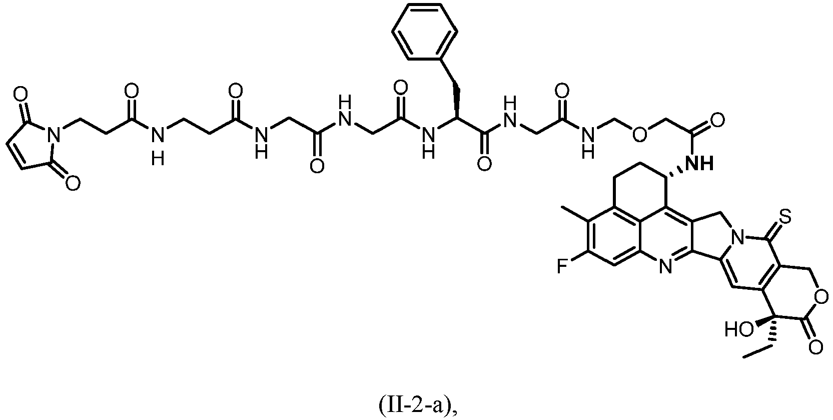

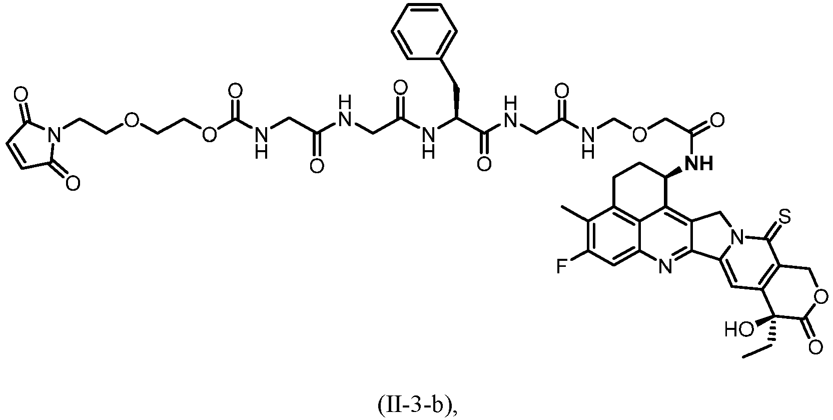

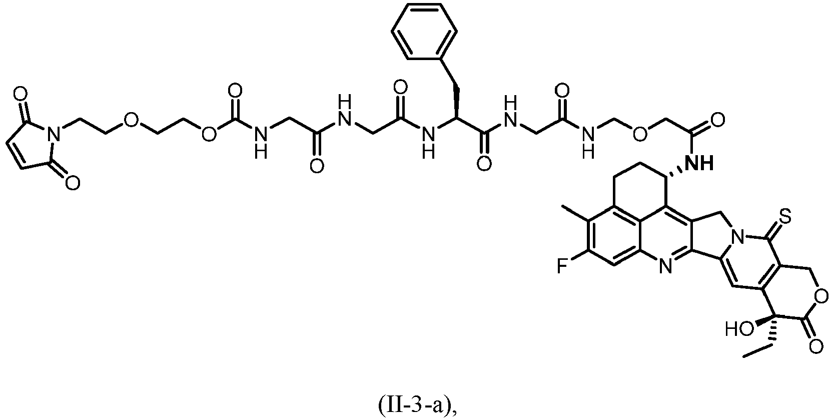

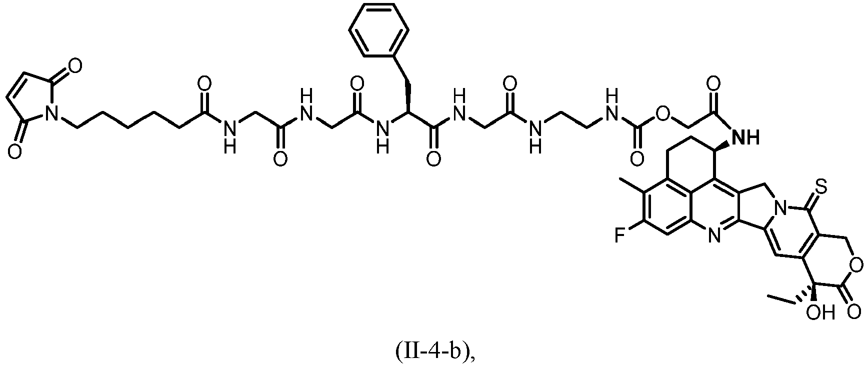

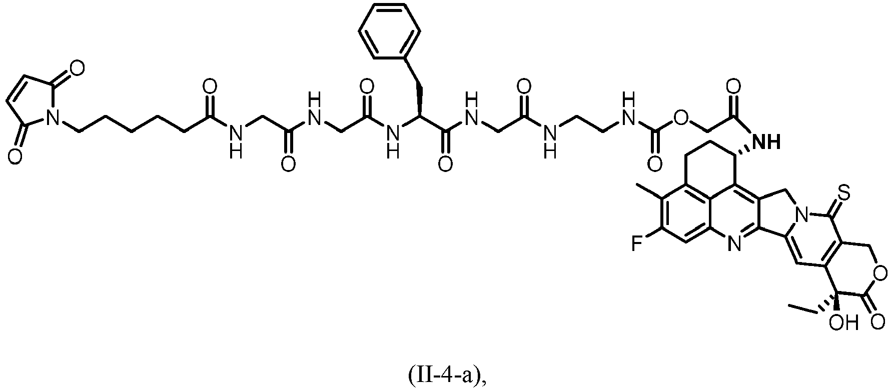

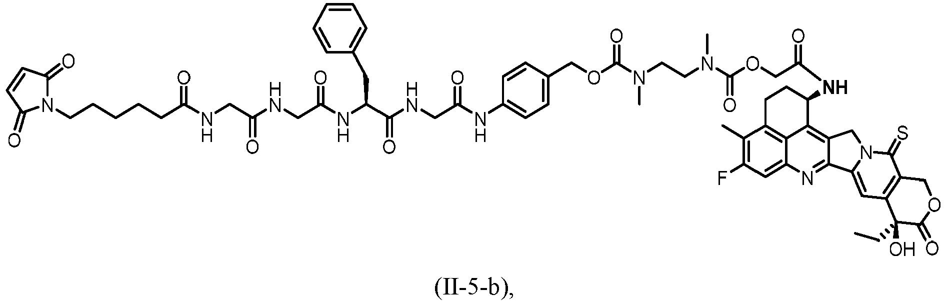

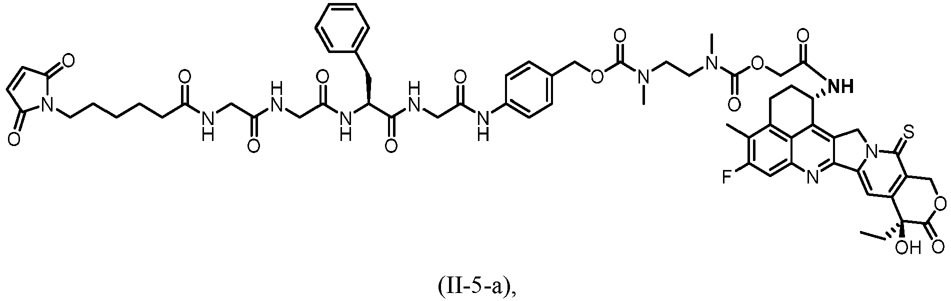

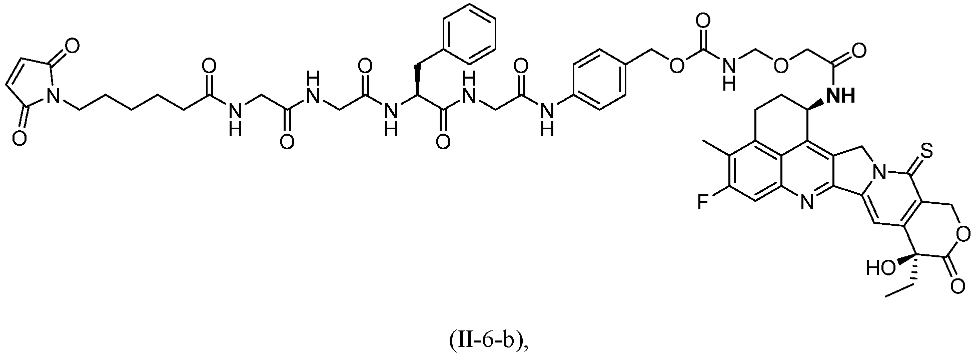

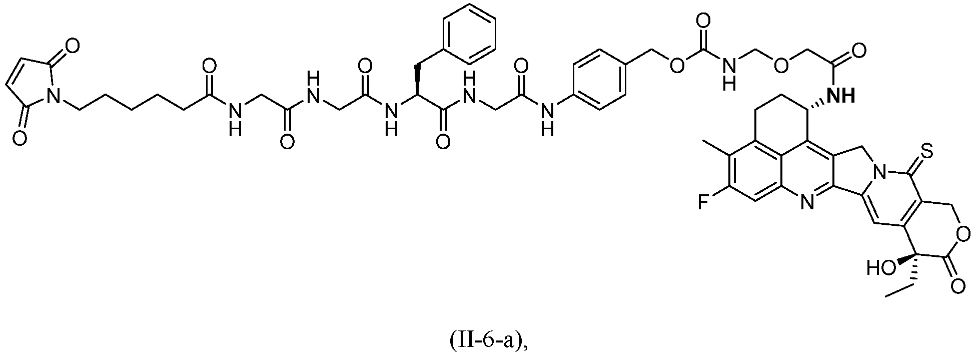

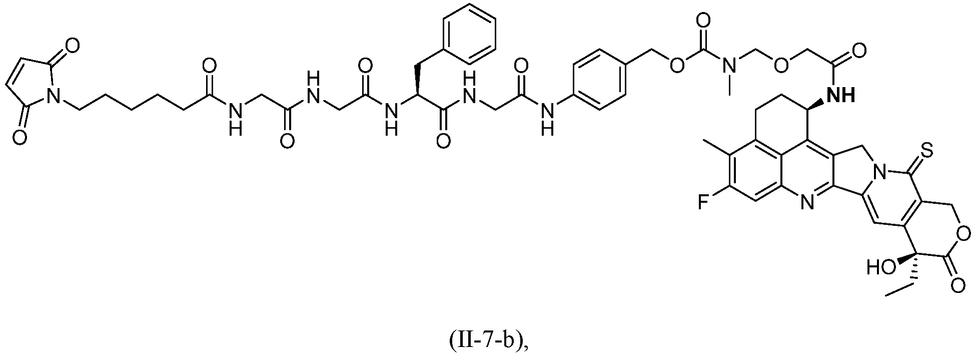

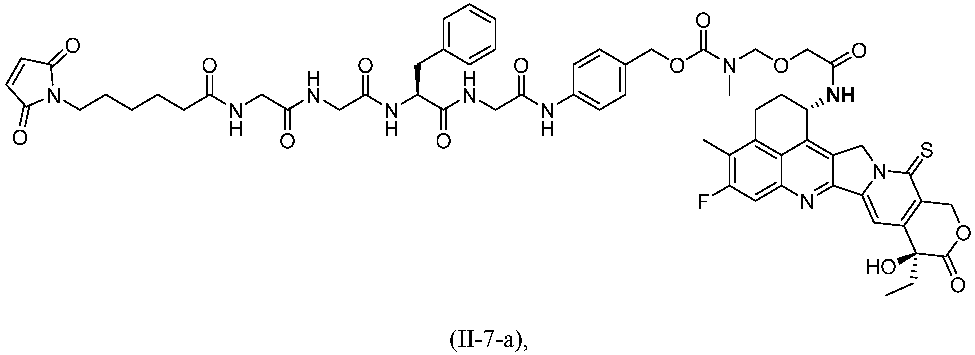

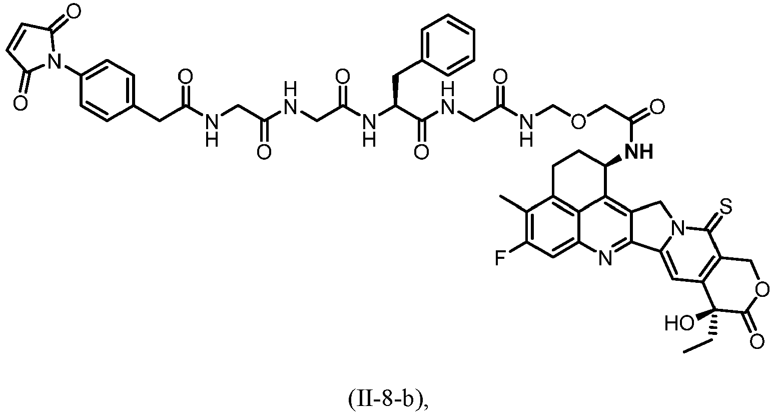

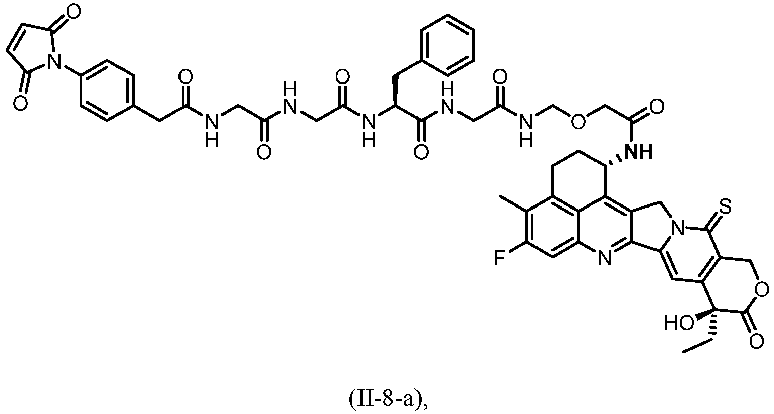

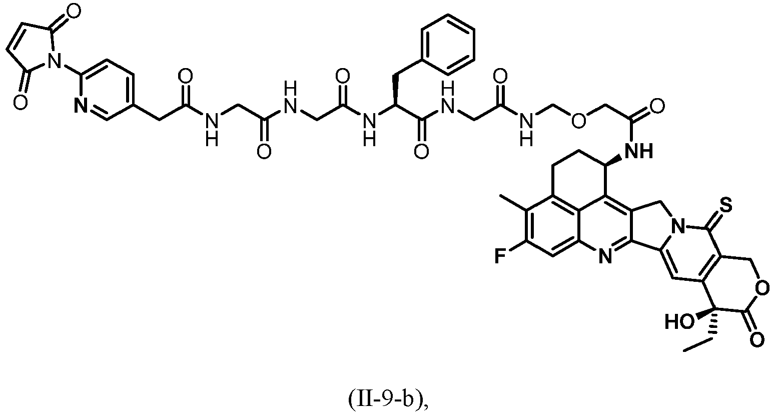

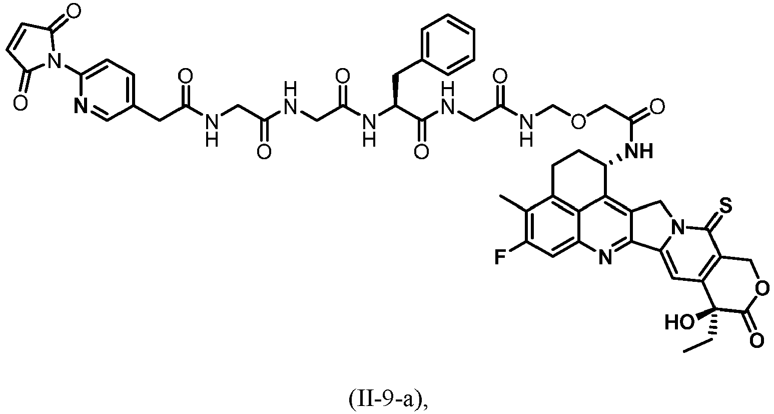

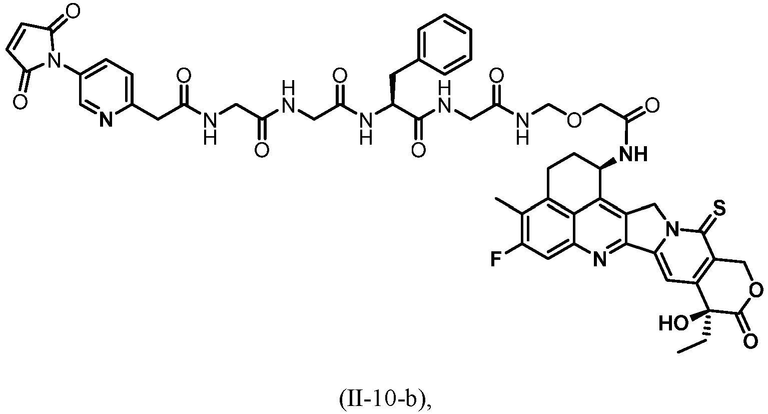

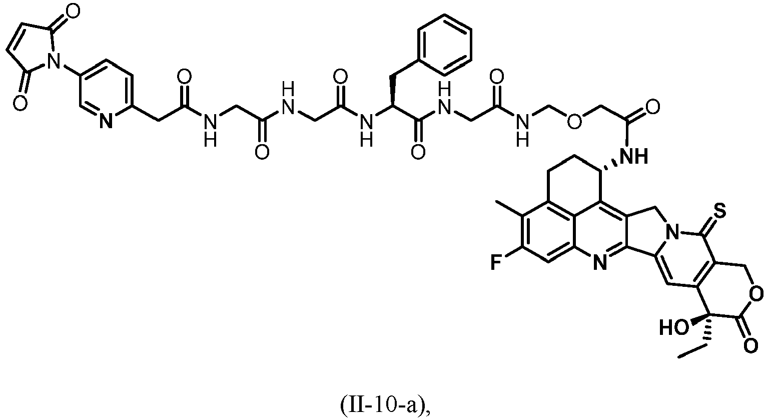

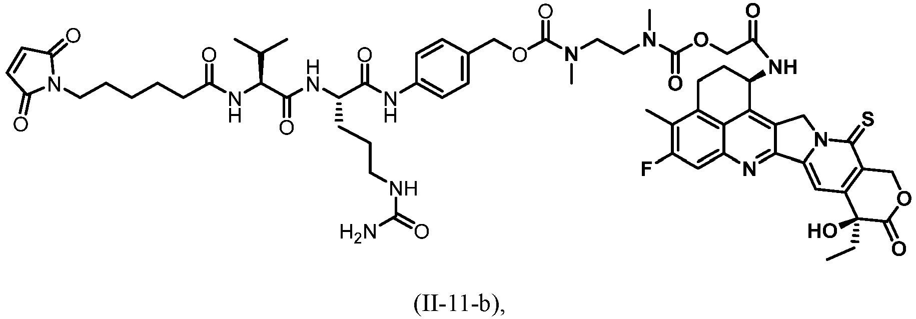

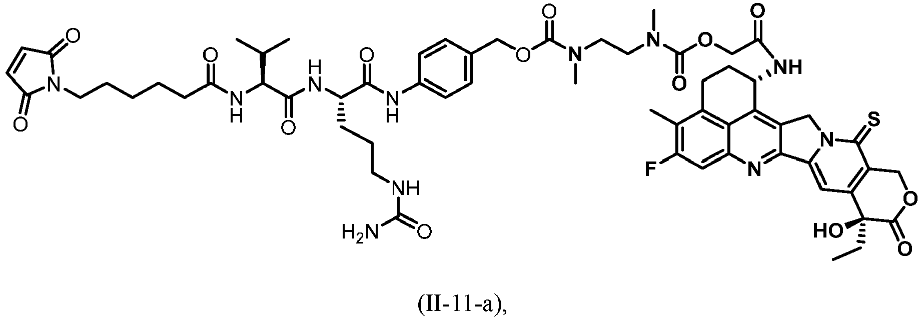

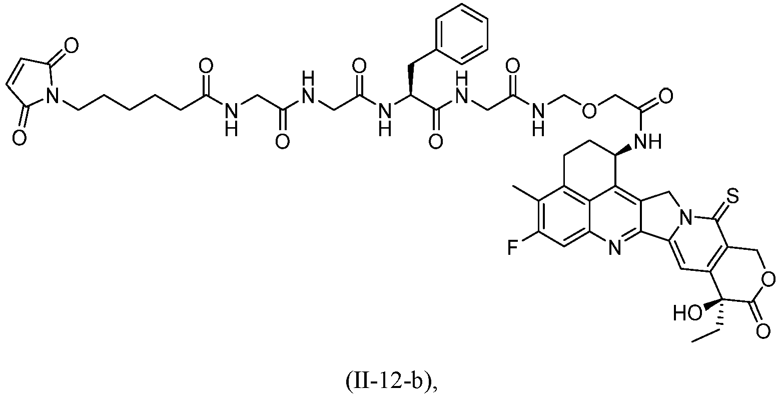

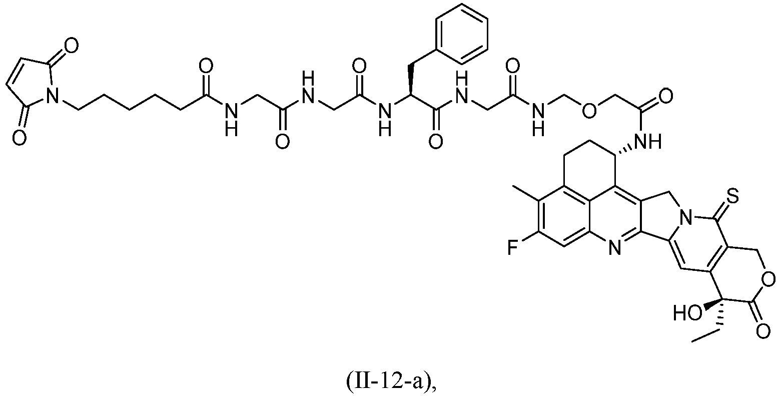

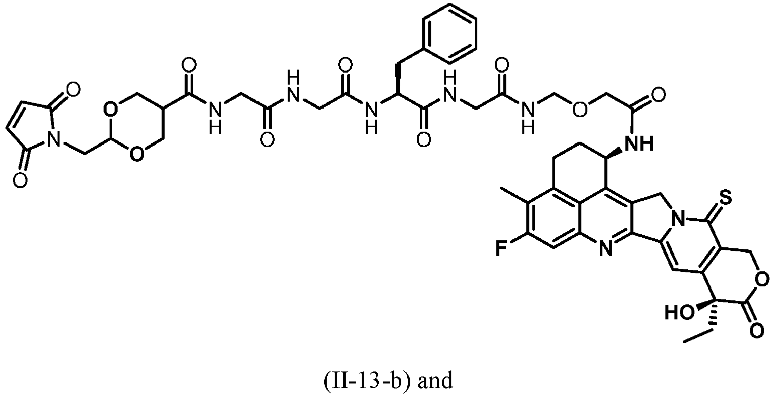

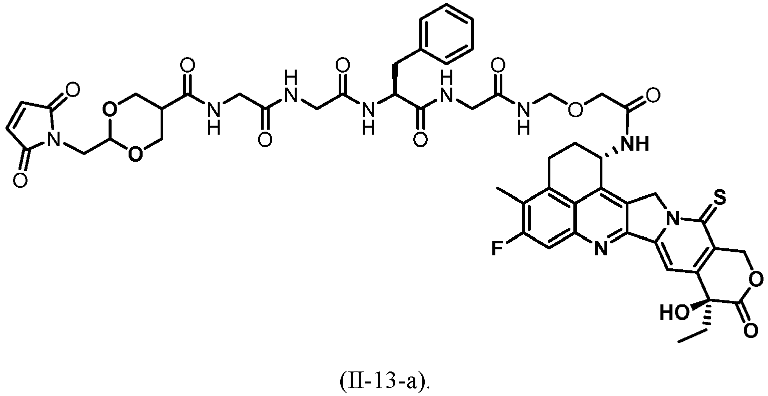

In another aspect, the present application provides a compound, or a tautomer, a mesomer, a racemate, an enantiomer, a diastereomer, or a mixture thereof, or a pharmaceutically acceptable salt or hydrate thereof, wherein said the compound may comprise the structure shown in formula (III): 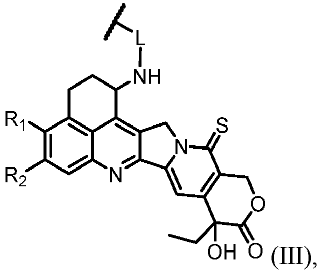 for example it can be 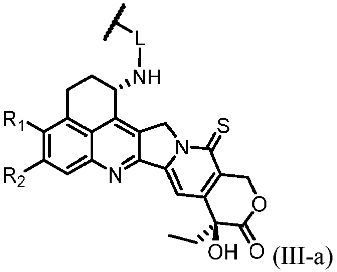 and/or 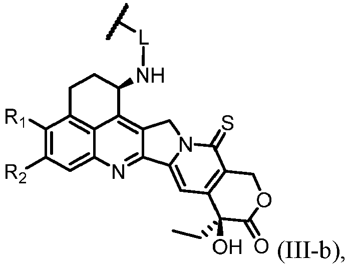 wherein,
R₁ and R₂ may each independently be selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached may form a structure selected from the group consisting of optionally substituted aliphatic cyclyl, optionally substituted aliphatic heterocyclyl, optionally substituted aryl, and optionally substituted heteroaryl;
L may be an optionally substituted linker,

For example, in the structure shown in formula (III), said L may comprise a linker L₁, said L₁ may be directly or indirectly linked to a ligand. For example, in the structure shown in formula (III), said L₁ may comprise optionally substituted 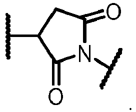 For example, in the structure shown in formula (III), said L₁ can exist in a ring-opened form as shown in the following equation.

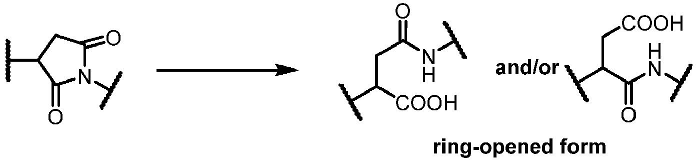

For example, in the structure shown in formula (III), said L may also comprise a linker L₂.

For example, in the structure shown in formula (III), said L₂ may be directly or indirectly linked to said L₁.

For example, in the structure shown in formula (III), said L₂ may comprise a group selected from the group consisting of optionally substituted -(CH₂)ₘ₁-X₁-(CH₂)ₘ₂-C(O)-, optionally substituted -(CH₂CH₂O)ₙ-C(O)-, and optionally substituted -(CH₂)ₚ-C(O)-, X₁ may be selected from the group consisting of -O-, optionally substituted -C(O)-NH-, optionally substituted aryl, optionally substituted heteroaryl, optionally substituted aliphatic heterocyclyl, and optionally substituted aliphatic cyclyl, wherein m1, m2 and n may each be independently selected from a number of at least 0;

For example, in the structure shown in formula (III), said L₂ may comprise optionally substituted -(CH₂CH₂O)ₙ-C(O)-.

For example, in the structure shown in formula (III), wherein, n can be 2.

For example, in the structure shown in formula (III), said L₂ may comprise optionally substituted -(CH₂)ₘ₁-X₁-(CH₂)ₘ₂-C(O)-.

For example, in the structure shown in formula (III), X₁ may be selected from the group consisting of -O- and optionally substituted -C(O)-NH-.

For example, in the structure shown in formula (III), wherein, m1 can be 2.

For example, in the structure shown in formula (III), wherein, m2 can be 2.

For example, in the structure shown in formula (III), X₁ may comprise a group selected from the group consisting of an optionally substituted aryl and an optionally substituted heteroaryl.

For example, in the structure shown in formula (III), X₁ may comprise optionally substituted phenyl.

For example, in the structure shown in formula (III), X₁ may comprise optionally substituted pyridyl.

For example, in the structure shown in formula (III), wherein, m1 can be 0.

For example, in the structure shown in formula (III), wherein, m2 can be 1.

For example, in the structure shown in formula (III), X₁ may comprise an optionally aliphatic heterocyclyl including two oxygen atoms.

For example, in the structure shown in formula (III), X₁ may comprise optionally substituted

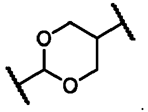

For example, in the structure shown in formula (III), X₁ may comprise an optionally substituted cyclohexyl.

For example, in the structure shown in formula (III), m1 can be 1.

For example, in the structure shown in formula (III), m2 can be 0.

For example, in the structure shown in formula (III), said L₂ may comprise optionally substituted -(CH₂)ₚ-C(O)-.

For example, in the structure shown in formula (III), wherein, p can be 5.

For example, in the structure shown in formula (III), said L₂ may comprise a structure selected from the group consisting of optionally substituted 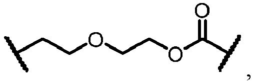 optionally substituted 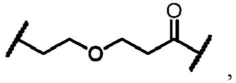 optionally substituted 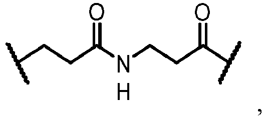 optionally substituted 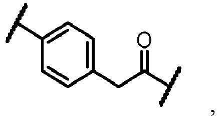 optionally substituted 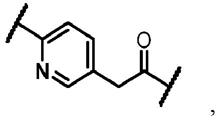 optionally substituted 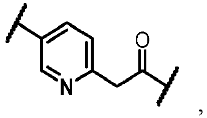 optionally substituted 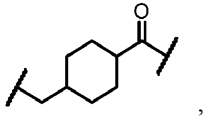 and optionally substituted

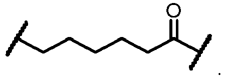

For example, in the structure shown in formula (III), said L may also comprise a linker L₃.

For example, in the structure shown in formula (III), said L₃ may be directly or indirectly linked to said L₂.

For example, in the structure shown in formula (III), said L₃ may comprise a peptide residue.

For example, in the structure shown in formula (III), said L₃ may comprise peptide residues composed of amino acids selected from the group consisting of phenylalanine, isoleucine, leucine, tryptophan, valine, methionine, tyrosine, alanine, threonine, histidine, serine, glutamine, arginine, lysine, asparagine, glutamate, proline, citrulline, aspartate, and glycine.

For example, in the structure shown in formula (III), said L₃ may comprise peptide residues composed of amino acids selected from the group consisting of glycine, phenylalanine, valine, and citrulline.

For example, in the structure shown in formula (III), said L₃ may comprise peptide residues selected from the group consisting of -glycine-phenylalanine-glycine- (-Gly-Phe-Gly-), -glycine-glycine-phenylalanine-glycine- (-Gly-Gly-Phe-Gly-), and -valine-citrulline- (-Val-Cit-).

For example, in the structure shown in formula (III), said L₃ may comprise a structure selected from the group consisting of

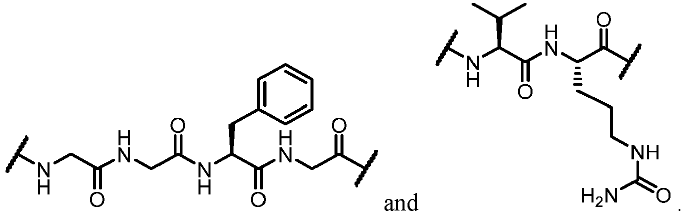

For example, in the structure shown in formula (III), said L may also comprise a linker L₄.

For example, in the structure shown in formula (III), said L₄ may be directly or indirectly linked to said L₃.

For example, in the structure shown in formula (III), said L₄ may comprise optionally substituted -L₄ₐ-NR₄-CH₂-L_{4b}-,
L₄ₐ may be absent or L₄ₐ may comprise optionally substituted 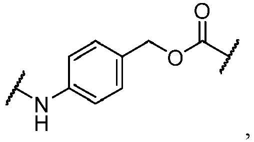
L_{4b} may be absent or L_{4b} may comprise optionally substituted 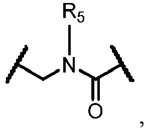
R₄ and R₅ may each independently be selected from the group consisting of hydrogen and optionally substituted alkyl.

For example, in the structure shown in formula (III), said L₄ may comprise optionally substituted

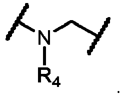

For example, in the structure shown in formula (III), said L₄ may comprise optionally substituted

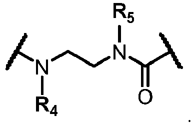

For example, in the structure shown in formula (III), said L₄ may comprise optionally substituted

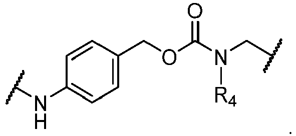

For example, in the structure shown in formula (III), said L₄ may comprise optionally substituted

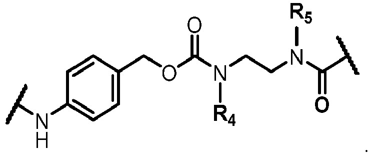

For example, in the structure shown in formula (III), said R₄ may be selected from the group consisting of hydrogen and optionally substituted methyl.

For example, in the structure shown in formula (III), said R₅ may be selected from the group consisting of hydrogen and optionally substituted methyl.

For example, in the structure shown in formula (III), said L₄ may comprise a structure selected from the group consisting of optionally substituted 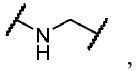 optionally substituted 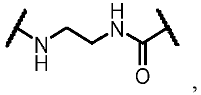 optionally substituted 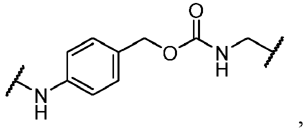 optionally substituted 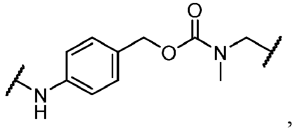 optionally substituted

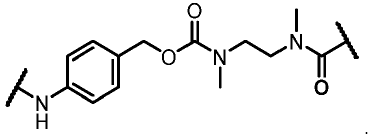

For example, in the structure shown in formula (III), said L may also comprise a linker L₅.

For example, in the structure shown in formula (III), said L₅ may be directly or indirectly linked to said L₄.

For example, in the structure shown in formula (III), said L₅ may comprise optionally substituted 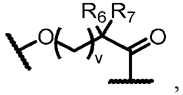 R₆ and R₇ may each be independently selected from the group consisting of hydrogen, deuterium, optionally substituted alkyl, optionally substituted haloalkyl, optionally substituted cycloalkyl, and optionally substituted cycloalkylalkyl, or R₆ and R₇ may be joined with the atom to which they attached to form an optionally substituted cycloalkyl, wherein v may be selected from a number at least 0.

For example, in the structure shown in formula (III), said R₆ may be hydrogen.

For example, in the structure shown in formula (III), said R₆ may be optionally substituted methyl.

For example, in the structure shown in formula (III), said R₆ may be optionally substituted halomethyl.

For example, in the structure shown in formula (III), said R₆ may be fluorine substituted methyl.

For example, in the structure shown in formula (III), said R₆ may be trifluoromethyl.

For example, in the structure shown in formula (III), said R₆ may be optionally substituted cycloalkyl.

For example, in the structure shown in formula (III), said R₆ may be optionally substituted cyclopropyl.

For example, in the structure shown in formula (III), said R₇ may be hydrogen.

For example, in the structure shown in formula (III), R₆ and R₇ may be joined with the atom to which they attached to form an optionally substituted cyclopropyl.

For example, in the structure shown in formula (III), R₆ and R₇ may be joined with the atom to which they attached to form an optionally substituted cyclobutyl.

For example, in the structure shown in formula (III), wherein, v can be 0.

For example, in the structure shown in formula (III), wherein, v can be 1.

For example, in the structure shown in formula (III), said L₅ may comprise a structure selected from the group consisting of optionally substituted 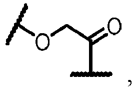 optionally substituted 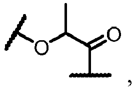 optionally substituted 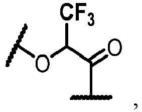 optionally substituted 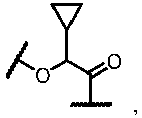 optionally substituted 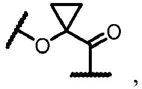 optionally substituted 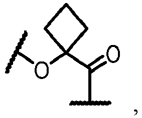 optionally substituted 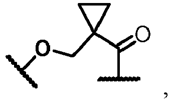 and optionally substituted

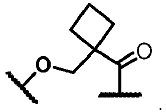

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise a structure selected from the group consisting of

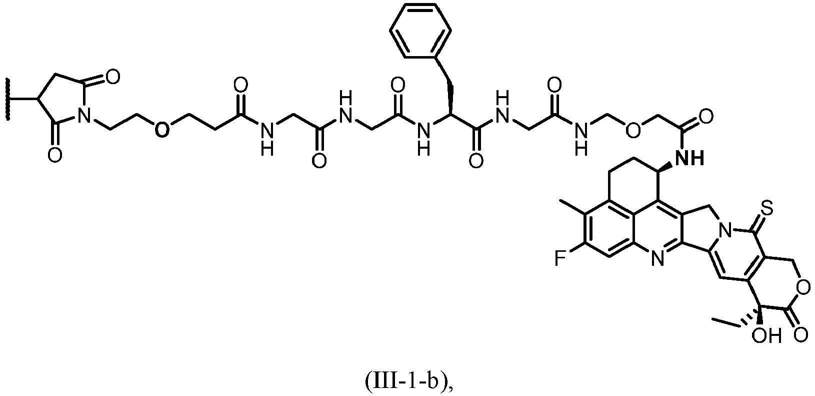

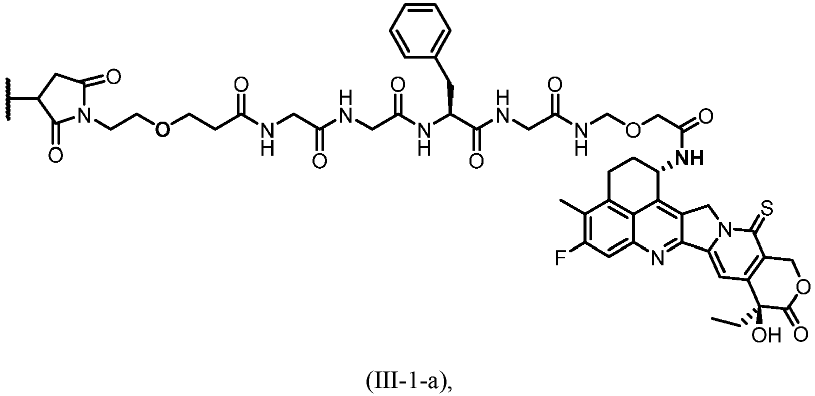

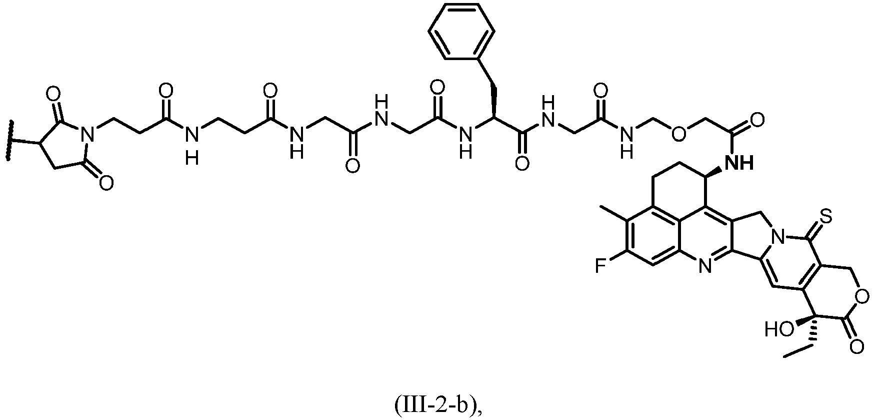

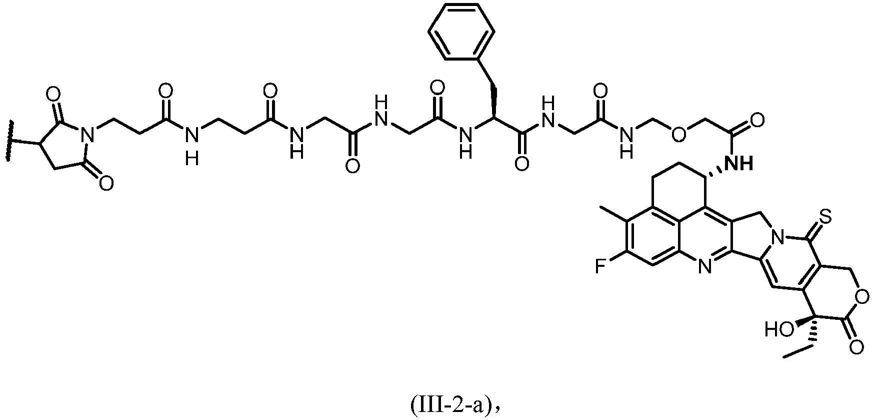

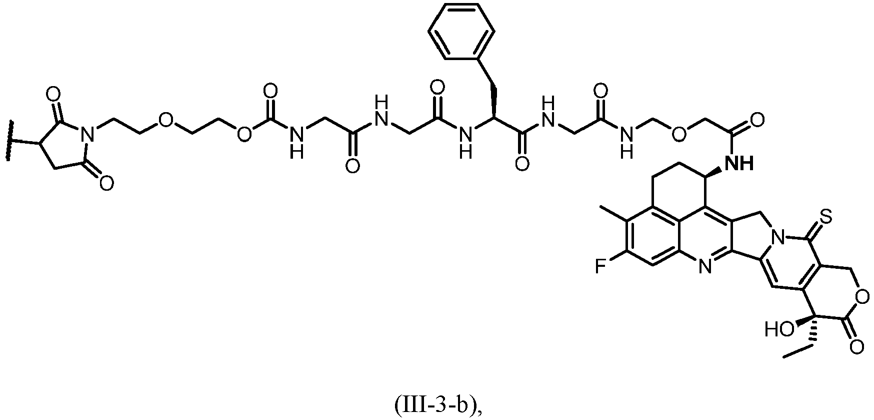

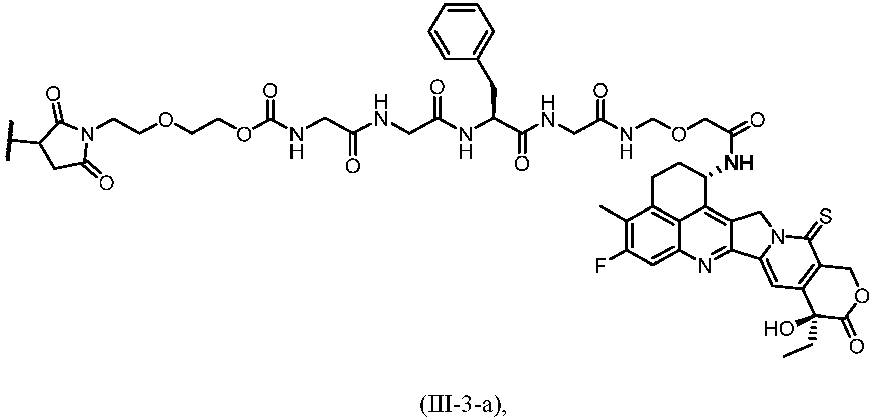

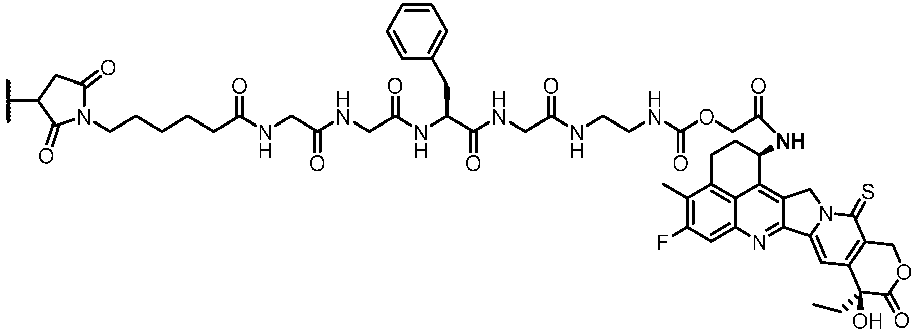

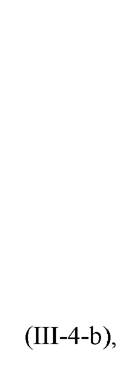

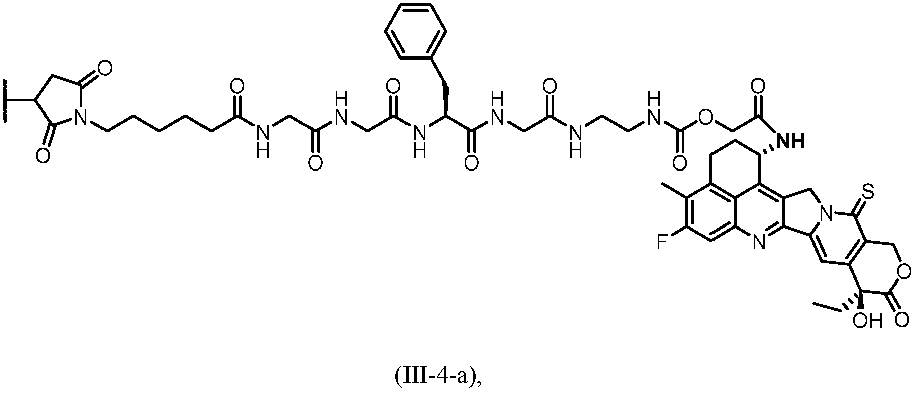

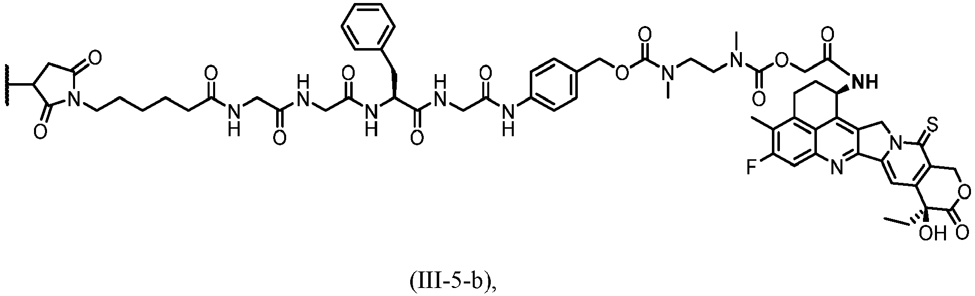

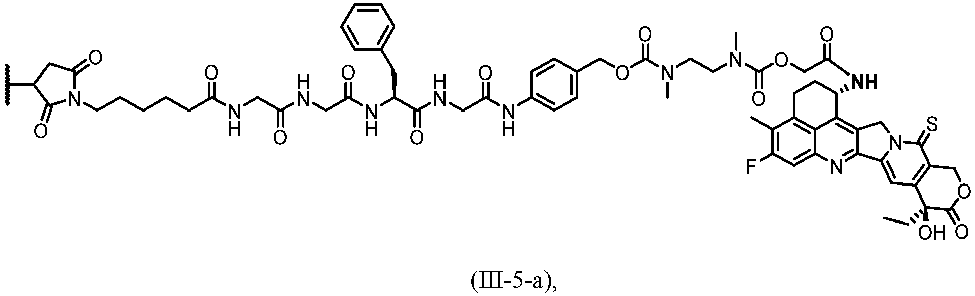

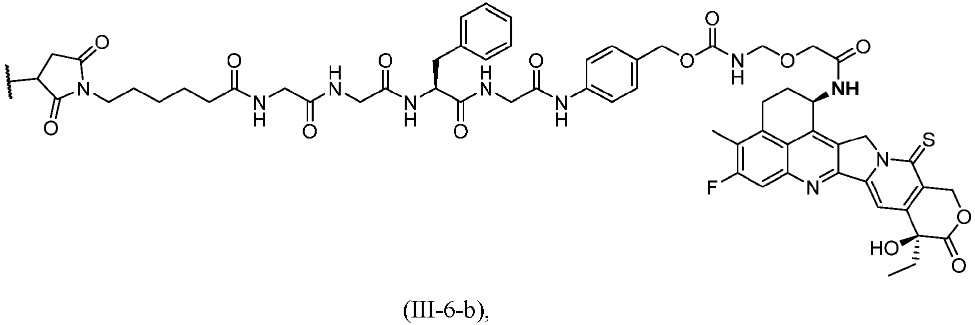

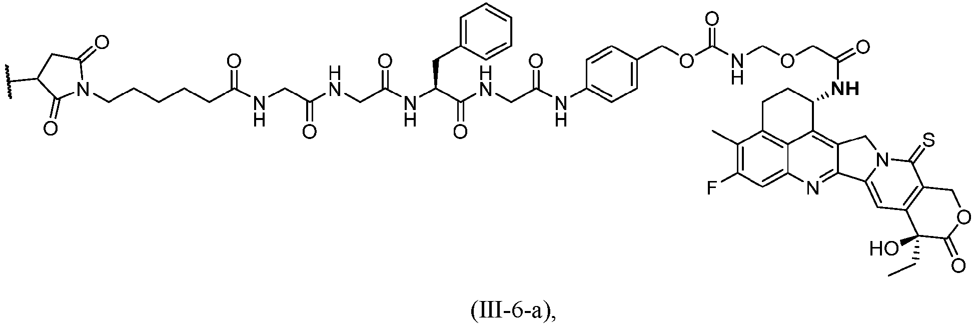

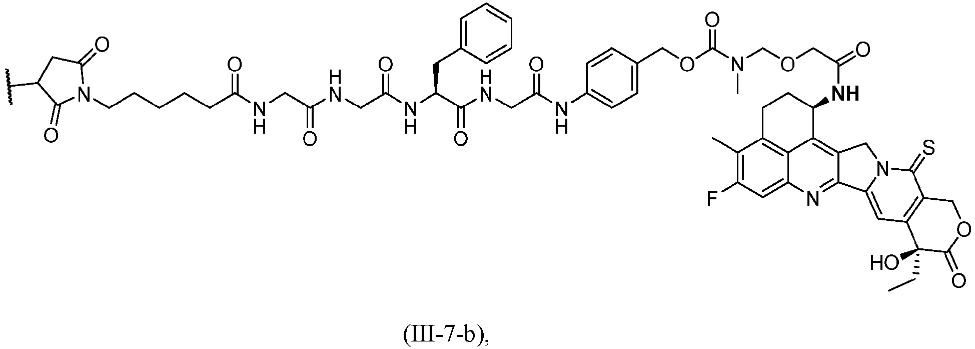

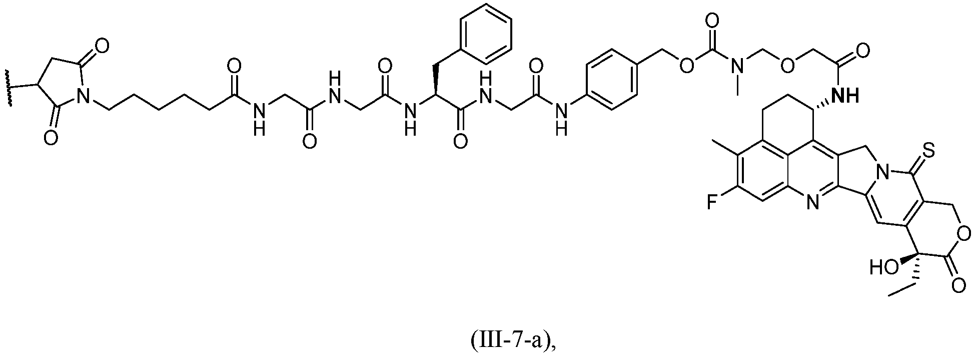

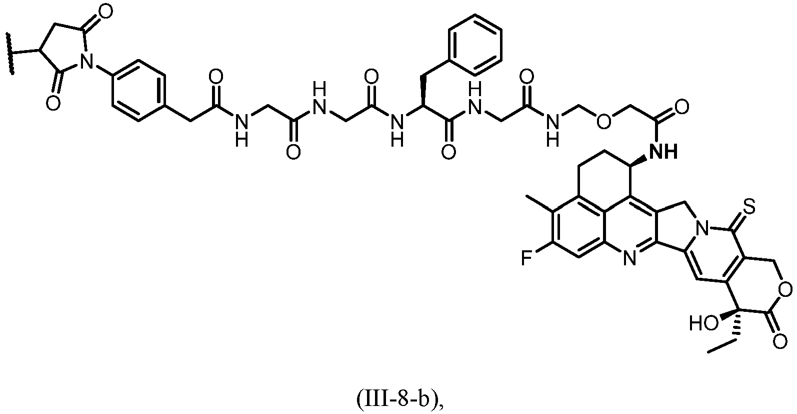

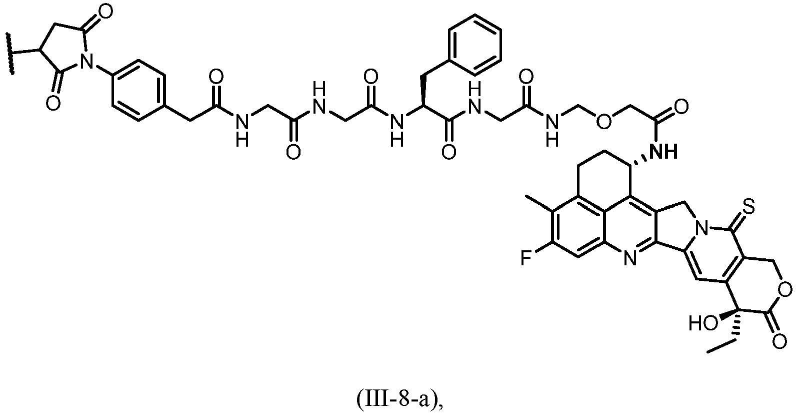

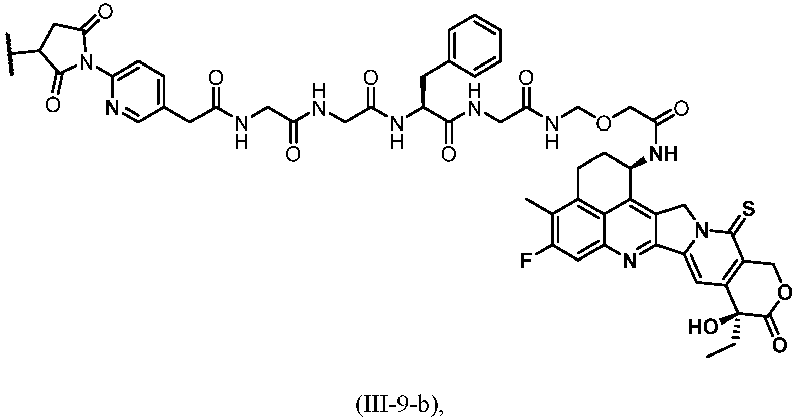

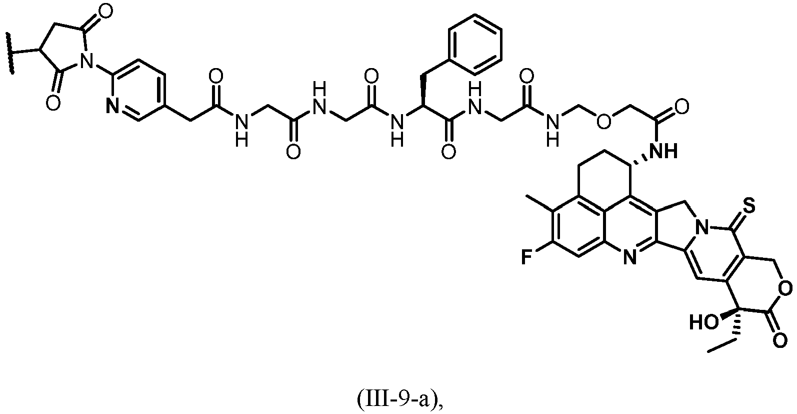

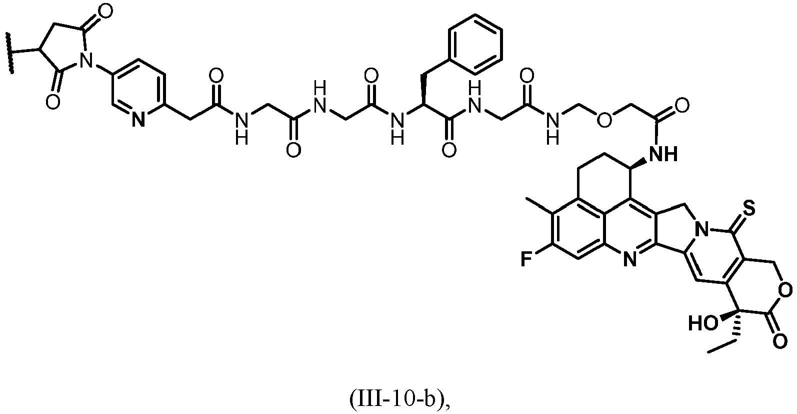

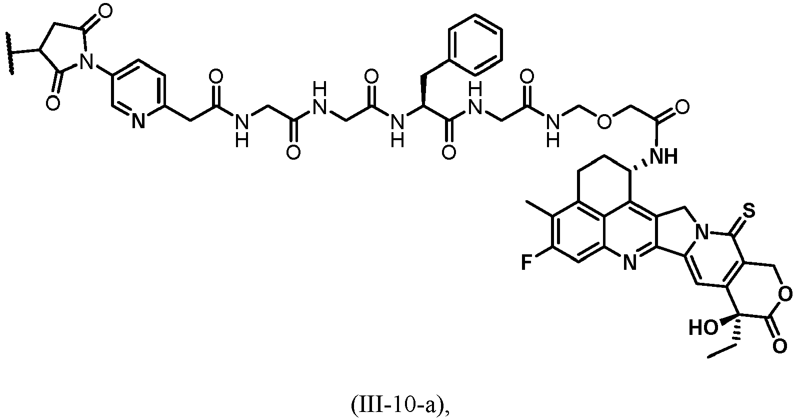

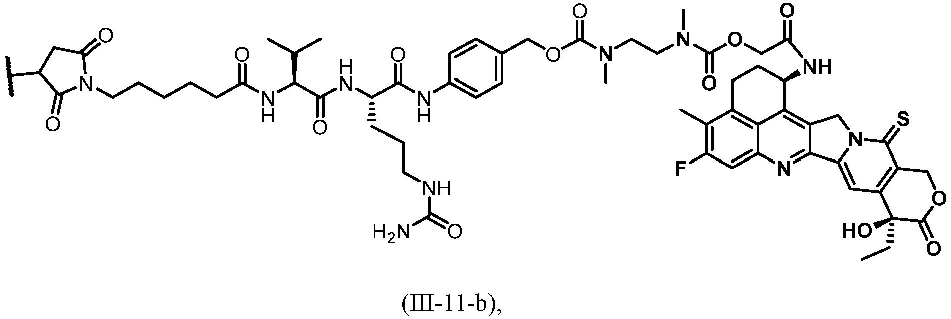

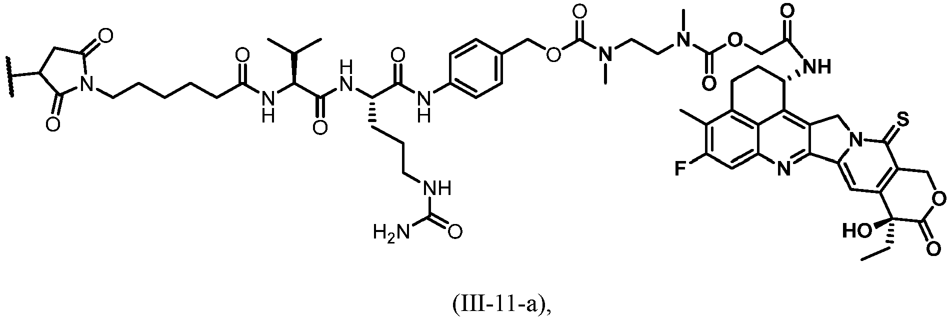

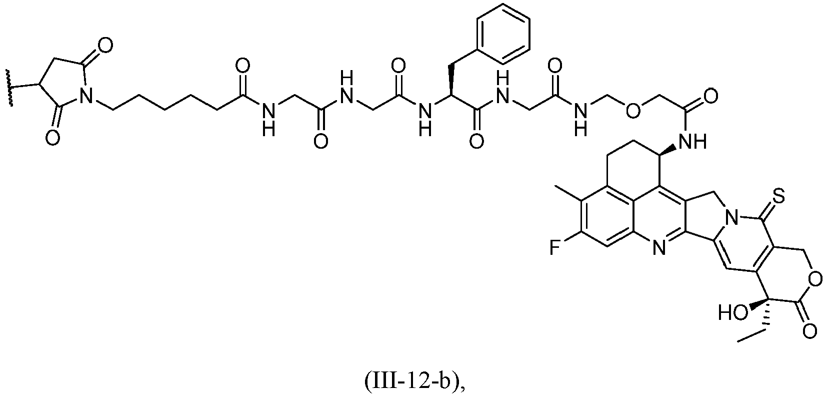

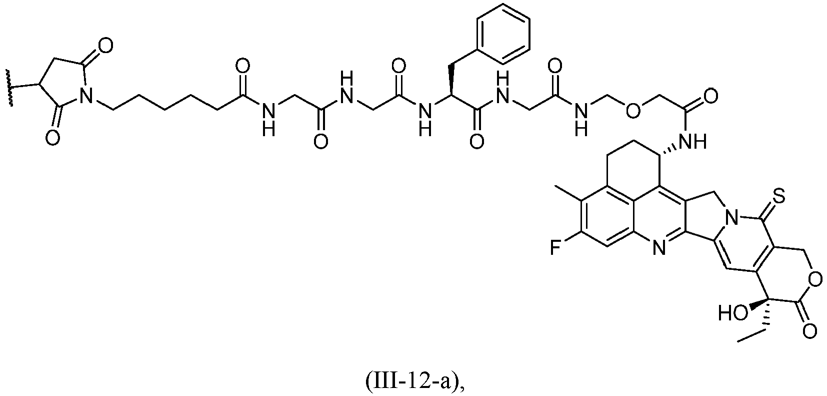

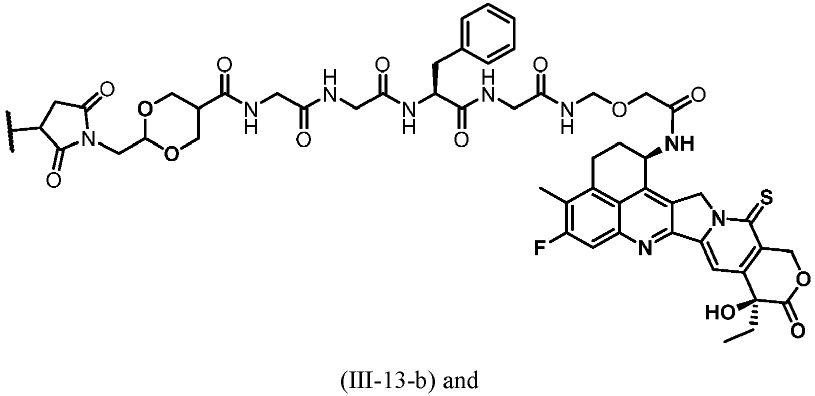

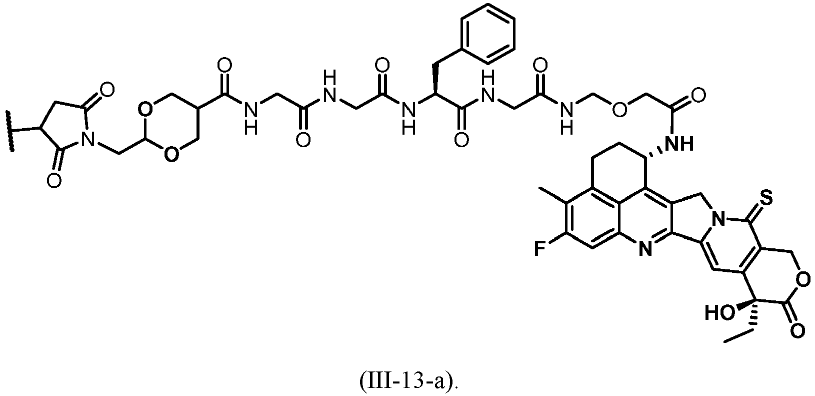

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (L-1):

-L₁-L₂-L₃-L₄-L₅- (L-1),

wherein,
L₁, L₂, L₃, L₄ and L₅ can each be independent as a linker,
wherein, said L₂ may comprise a group selected from the group consisting of optionally substituted -(CH₂)ₘ₁-X₁-(CH₂)ₘ₂-C(O)- and optionally substituted -(CH₂CH₂O)ₙ-C(O)-.
X₁ may be selected from the group consisting of -O-, optionally substituted -C(O)-NH-, optionally substituted aryl, optionally substituted heteroaryl, optionally aliphatic heterocyclyl and optionally substituted aliphatic cyclyl,
wherein, m1, m2, and n are each independently selected from numbers of at least 0.

For example, in the structure shown in formula (L-1), said L₂ may comprise optionally substituted -(CH₂CH₂O)ₙ-C(O)-. For example, wherein, n can be 2.

For example, in the structure shown in formula (L-1), said L₂ may comprise optionally substituted -(CH₂)ₘ₁-X₁-(CH₂)ₘ₂-C(O)-. For example, X₁ is selected from the group consisting of - O-, optionally substituted -C(O)-NH-. For example, m1 can be 2. For example, m2 can be 2.

For example, in the structure shown in formula (L-1), X₁ may comprise optionally substituted aryl and optionally substituted heteroaryl. For example, X₁ may comprise optionally substituted phenyl. For example, X₁ may comprise optionally substituted pyridyl. For example, m1 can be 0. For example, m2 can be 1.

For example, in the structure shown in formula (L-1), X₁ may comprise an optionally substituted aliphatic heterocyclyl including two oxygen atoms. For example, X₁ may comprise optionally substituted 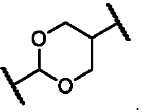 For example, X₁ may comprise an optionally substituted cyclohexyl group. For example, m1 can be 1. For example, m2 can be 0.

For example, in the structure shown in formula (L-1), said L₂ may comprise a structure selected from the group consisting of optionally substituted 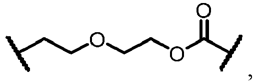 optionally substituted 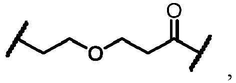 optionally substituted 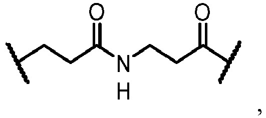 optionally substituted 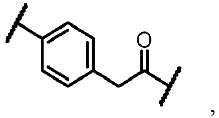 optionally substituted 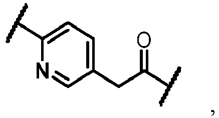 optionally substituted 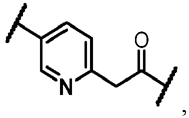 and optionally substituted

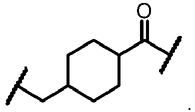

For example, in the structure shown in formula (L-1), said L₁ may comprise optionally substituted 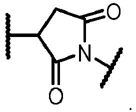 For example, L₁ in the structure shown in formula (L-1) can exist in a ring-opened form as shown in the following equation.

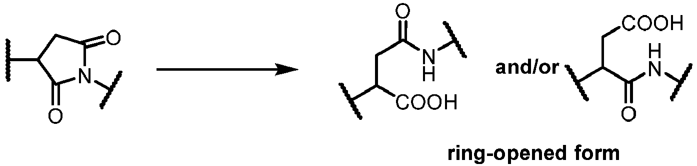

For example, in the structure shown in formula (L-1), said L₃ may comprise peptide residues composed of amino acids selected from the group consisting of phenylalanine, isoleucine, leucine, tryptophan, valine, methionine, tyrosine, alanine, threonine, histidine, serine, glutamine, arginine, lysine, asparagine, glutamate, proline, citrulline, aspartate, and glycine. For example, said L₃ may comprise peptide residues composed of amino acids selected from the group consisting of glycine, phenylalanine, valine, and citrulline. For example, said L₃ may comprise peptide residues selected from the group consisting of -glycine-phenylalanine-glycine- (-Gly-Phe-Gly-), -glycine-glycine-phenylalanine-glycine- (-Gly-Gly-Phe-Gly-), and -valine-citrulline- (-Val-Cit-).

For example, in the structure shown in formula (L-1), said L₃ may comprise a structure selected from the group consisting of 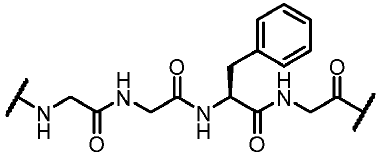 and

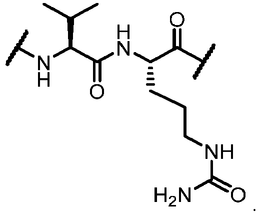

For example, in the structure shown in formula (L-1), said L₄ may comprise optionally substituted -L₄ₐ-NR₄-CH₂-L_{4b}-,
L₄ₐ may be absent or L₄ₐ may comprise optionally substituted 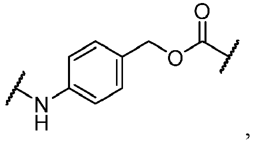
L_{4b} may be absent or L_{4b} may comprise optionally substituted 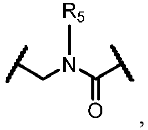
R₄ and R₅ may each independently be selected from the group consisting of hydrogen and optionally substituted alkyl.

For example, in the structure shown in formula (L-1), said L₄ may comprise optionally substituted 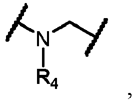 optionally substituted 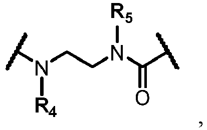 optionally substituted 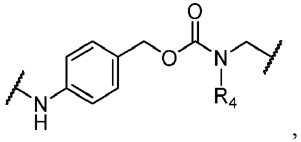 or optionally substituted 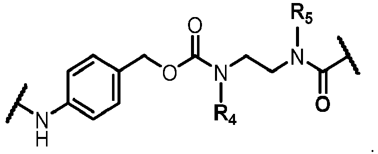 For example, said R₄ may be selected from the group consisting of hydrogen and optionally substituted methyl. For example, said R₅ may be selected from the group consisting of hydrogen and optionally substituted methyl.

For example, in the structure shown in formula (L-1), said L₄ may comprise a structure selected from the group consisting of optionally substituted 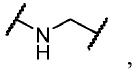 optionally substituted 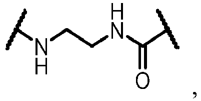 optionally substituted 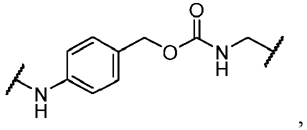 optionally substituted 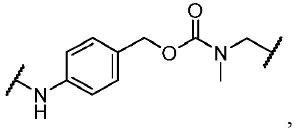 optionally substituted

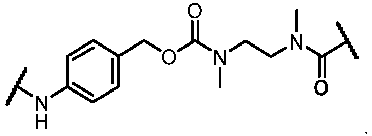

For example, in the structure shown in formula (L-1), said L₅ may comprise optionally substituted 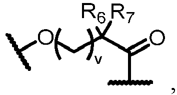 said L₅ may be directly linked to the following structure: 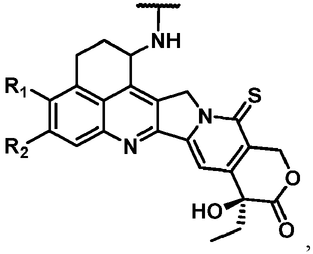 for example, 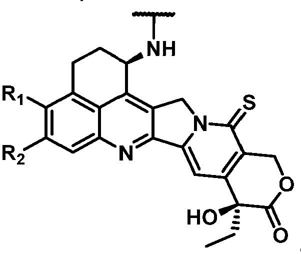 and/or 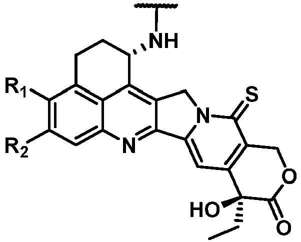
wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl,
R₆ and R₇ may each be independently selected from the group consisting of hydrogen, deuterium, optionally substituted alkyl, optionally substituted haloalkyl, optionally substituted cycloalkyl, and optionally substituted cycloalkylalkyl, or R₆ and R₇ may be joined with the atom to which they attached to form an optionally substituted cycloalkyl, wherein v may be selected from a number at least 0.

For example, in the structure shown in formula (L-1), said R₆ may be hydrogen. For example, said R₆ may be optionally substituted methyl. For example, said R₆ may be optionally substituted halomethyl. For example, said R₆ may be fluorine substituted methyl. For example, said R₆ may be trifluoromethyl. For example, said R₆ may be optionally substituted cycloalkyl. For example, said R₆ may be optionally substituted cyclopropyl. For example, said R₇ may be hydrogen. For example, R₆ and R₇ may be joined with the atom to which they attached to form an optionally substituted cyclopropyl. For example, R₆ and R₇ may be joined with the atom to which they attached to form an optionally substituted cyclobutyl. For example, v may be 0. For example, v may be 1.

For example, in the structure shown in formula (L-1), said L₅ may comprise a structure selected from the group consisting of optionally substituted 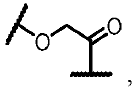 optionally substituted 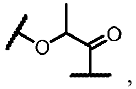 optionally substituted 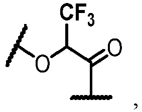 optionally substituted 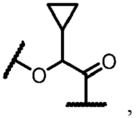 optionally substituted 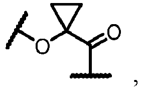 optionally substituted 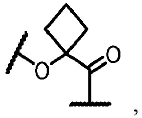 optionally substituted 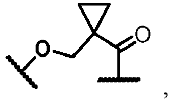 and optionally substituted

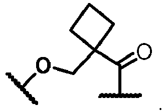

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (L-1):

-L₁-L₂-L₃-L₄-L₅- (L-1),

wherein,
wherein, said L₁ may comprise a linker after attachment to a thiol group,
said L₂ may comprise a group selected from the group consisting of optionally substituted - (CH₂)ₘ₁-X₁-(CH₂)ₘ₂-C(O)- and optionally substituted -(CH₂CH₂O)ₙ-C(O)-, X₁ may be selected from the group consisting of -O-, optionally substituted -C(O)-NH-, optionally substituted phenyl, optionally substituted pyridyl, optionally substituted aliphatic heterocyclyl containing two oxygen atoms and four carbon atoms, and optionally substituted cyclohexyl, wherein m1, m2 and n may each be independently selected from a number of at least 0;
said L₃ may comprise peptide residues.
said L₄ may comprise optionally substituted 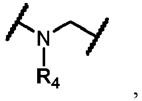 optionally substituted 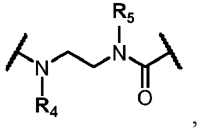 optionally substituted 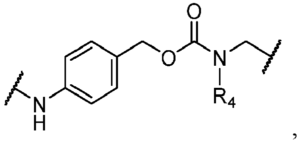 or optionally substituted 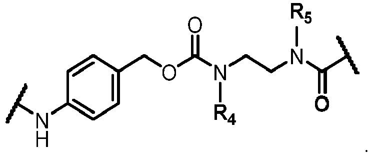 For example, said R₄ may be selected from the group consisting of hydrogen and optionally substituted C₁-C₆ alkyl. For example, said R₅ may be selected from the group consisting of hydrogen and optionally substituted C₁-C₆ alkyl.
said L₅ may comprise optionally substituted 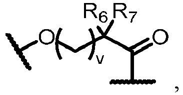 said L₅ may be directly linked to the following structure: 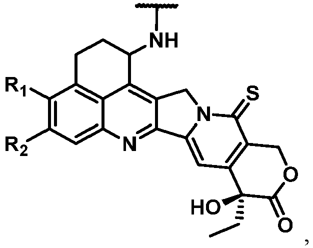 for example, 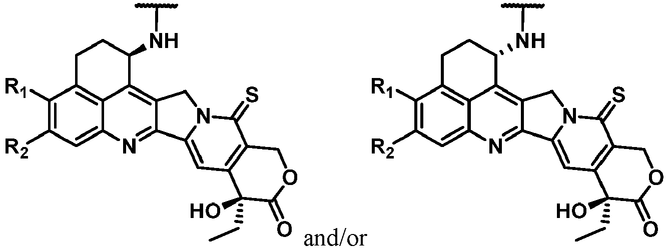
wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl,
R₆ and R₇ may each be independently selected from the group consisting of hydrogen, deuterium, optionally substituted alkyl, optionally substituted haloalkyl, optionally substituted cycloalkyl, and optionally substituted cycloalkylalkyl, or R₆ and R₇ may be joined with the atom to which they attached to form an optionally substituted cycloalkyl, wherein v may be selected from a number at least 0. For example, cycloalkylalkyl in the present application may refer to an alkyl group substituted with cycloalkyl, wherein the cycloalkylalkyl group may also be optionally substituted with an additional group.

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (L-1):

-L₁-L₂-L₃-L₄-L₅- (L-1),

wherein, said L₁ may comprise optionally substituted 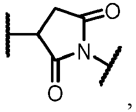
said L₂ may comprise a structure selected from the group consisting of optionally substituted 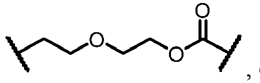 optionally substituted 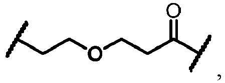 optionally substituted 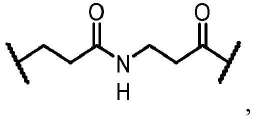 optionally substituted 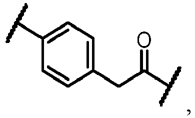 optionally substituted 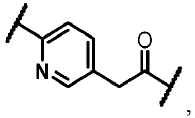 optionally substituted 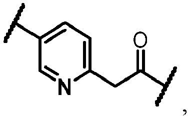 and optionally substituted 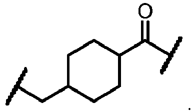
said L₃ may comprise a structure selected from the group consisting of 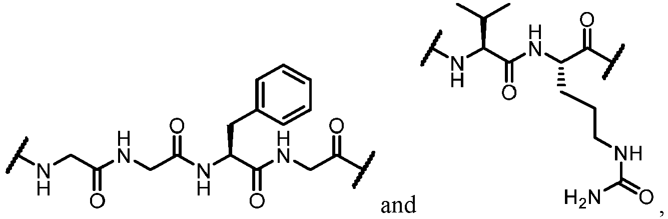
said L₄ may comprise a structure selected from the group consisting of optionally substituted 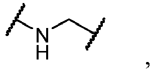 optionally substituted 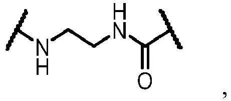 optionally substituted 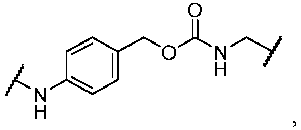 optionally substituted 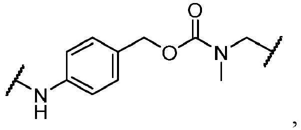 and optionally substituted 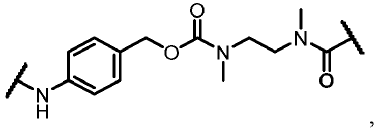
said L₅ may comprise a structure selected from the group consisting of optionally substituted 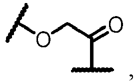 optionally substituted 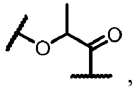 optionally substituted 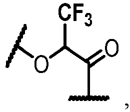 optionally substituted 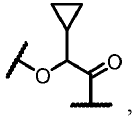 optionally substituted 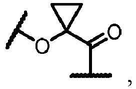 optionally substituted 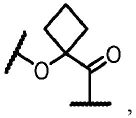 optionally substituted 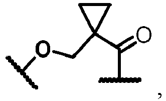 and optionally substituted 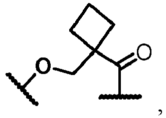 said L₅ may be directly linked to the following structure: 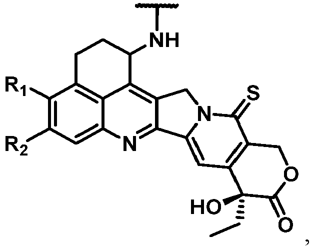 for example, 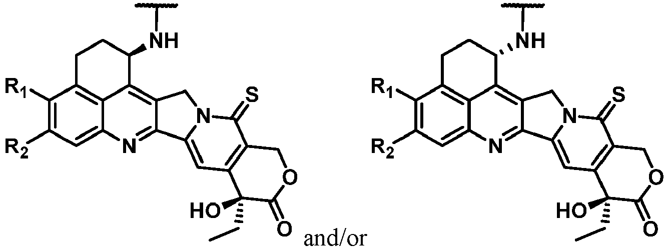
wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl.

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise a structure selected from the group consisting of

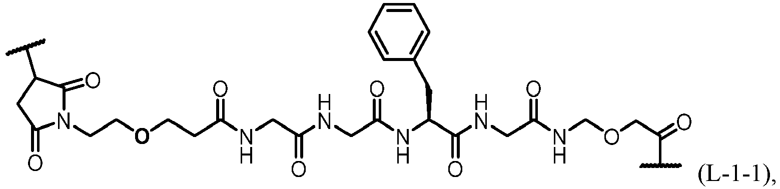

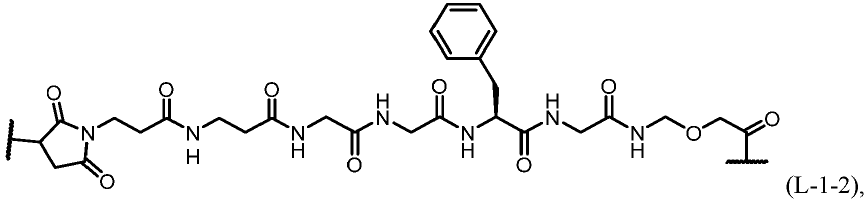

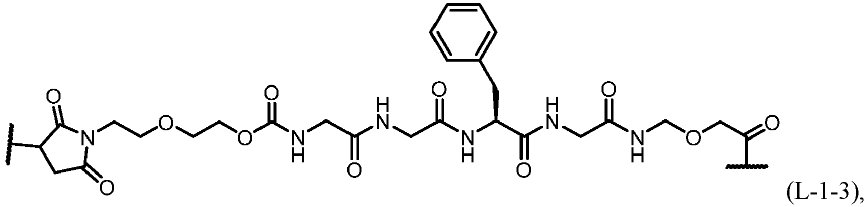

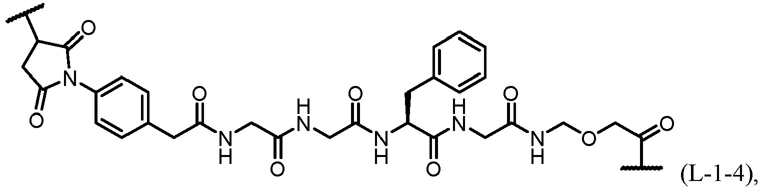

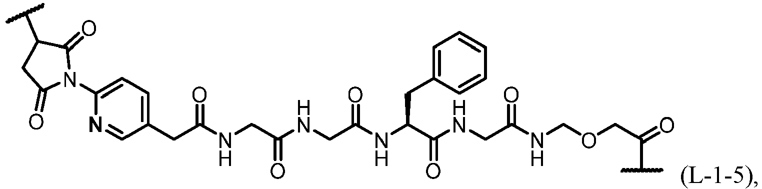

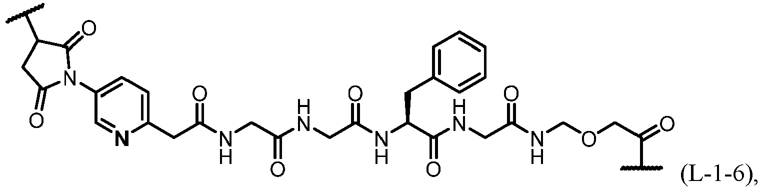

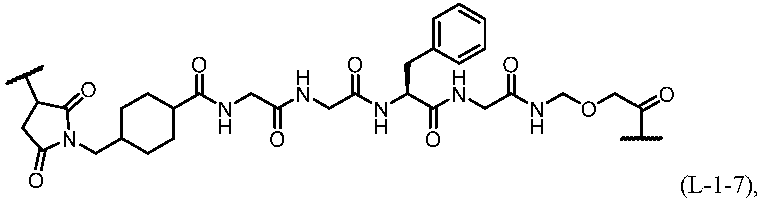

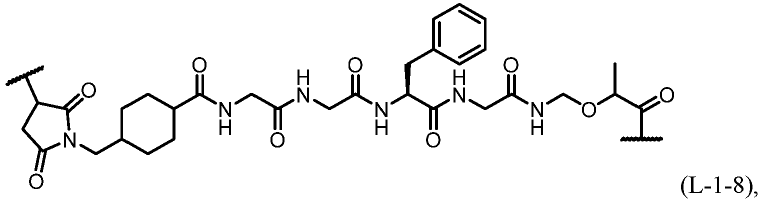

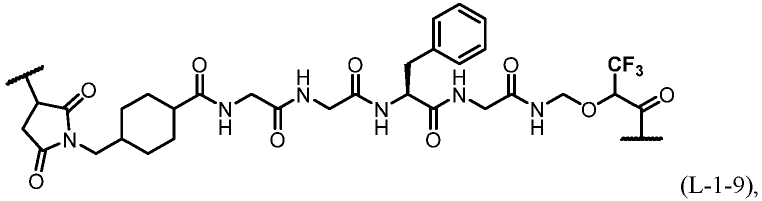

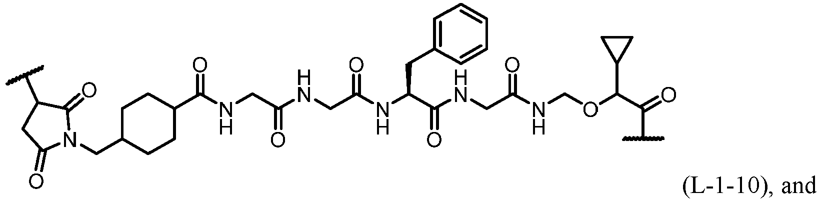

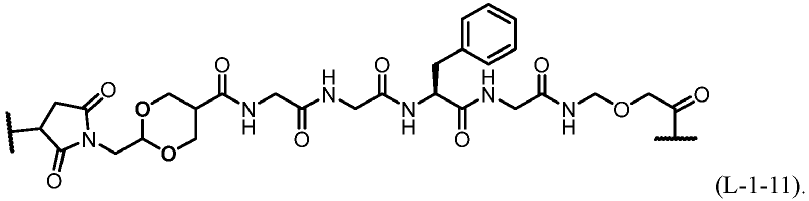

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (L-2):

-L₁-L₂-L₃-L₄-L₅- (L-2),

wherein, L₁, L₂, L₃, L₄ and L₅ can each be independent as a linker,
L₄ may comprise optionally substituted -L₄ₐ-NR₄-CH₂-L_{4b}-,
L₄ₐ may be absent or L₄ₐ may comprise optionally substituted 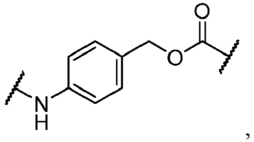
L_{4b} may be absent or L_{4b} may comprise optionally substituted 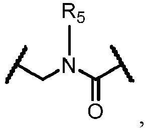 L₄ₐ and L_{4b} can not be absent at the same time,
R₄ and R₅ may each independently be selected from the group consisting of hydrogen and optionally substituted alkyl.

For example, in the structure shown in formula (L-2), said L₄ may comprise optionally substituted 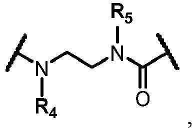 optionally substituted 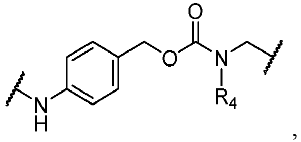 or optionally substituted 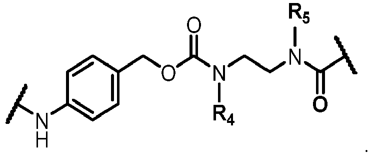 For example, said R₄ may be selected from the group consisting of hydrogen and optionally substituted methyl. For example, said R₅ may be selected from the group consisting of hydrogen and optionally substituted methyl.

For example, in the structure shown in formula (L-2), said L₄ may comprise a structure selected from the group consisting of optionally substituted 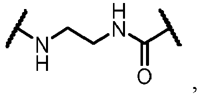 optionally substituted 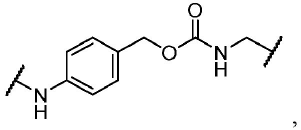 optionally substituted 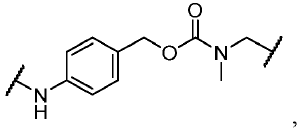 and optionally substituted

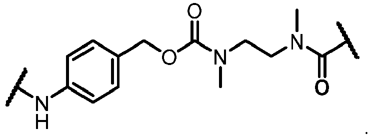

For example, in the structure shown in formula (L-2), said L₁ may comprise optionally substituted 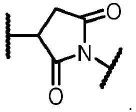 For example, in the structure shown in formula (L-2), said L₁ can exist in a ring-opened form as shown in the following equation.

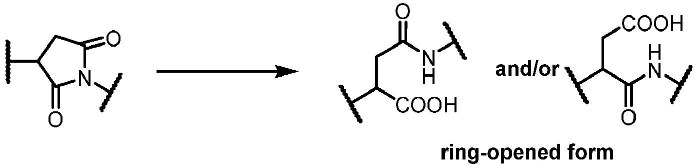

For example, in the structure shown in formula (L-2), said L₂ may comprise a group selected from the group consisting of optionally substituted -(CH₂)ₘ₁-X₁-(CH₂)ₘ₂-C(O)- and optionally substituted -(CH₂CH₂O)ₙ-C(O)-, and optionally substituted -(CH₂)ₚ-C(O)-. X₁ may be selected from the group consisting of -O-, optionally substituted -C(O)-NH-, optionally substituted aryl, optionally substituted heteroaryl, optionally substituted aliphatic heterocyclyl and optionally substituted aliphatic cyclyl,
wherein, m1, m2, and n are each independently selected from numbers of at least 0.

For example, in the structure shown in formula (L-2), said L₂ may comprise optionally substituted -(CH₂CH₂O)ₙ-C(O)-. For example, wherein, n can be 2.

For example, in the structure shown in formula (L-2), said L₂ may comprise optionally substituted -(CH₂)ₘ₁-X₁-(CH₂)ₘ₂-C(O)-. For example, X₁ is selected from the group consisting of - O-, optionally substituted -C(O)-NH-. For example, m1 can be 2. For example, m2 can be 2. For example, X₁ may comprise a group selected from the group consisting of optionally substituted aryl and optionally substituted heteroaryl. For example, X₁ may comprise optionally substituted phenyl. For example, X₁ may comprise optionally substituted pyridyl. For example, m1 can be 0. For example, m2 can be 1. For example, X₁ may comprise an optionally substituted aliphatic heterocyclyl containing two oxygen atoms. For example, X₁ may comprise optionally substituted 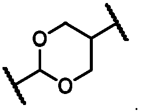 For example, X₁ may comprise an optionally substituted cyclohexyl group. For example, m1 can be 1. For example, m2 can be 0. For example, said L₂ may comprise optionally substituted -(CH₂)ₚ-C(O)-. For example, p can be 5.

For example, in the structure shown in formula (L-2), said L₂ may comprise a structure selected from the group consisting of optionally substituted 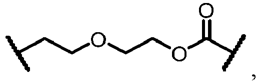 optionally substituted 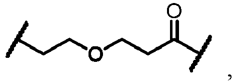 optionally substituted 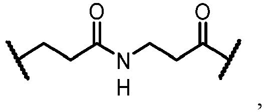 optionally substituted 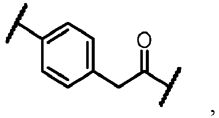 optionally substituted 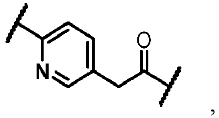 optionally substituted 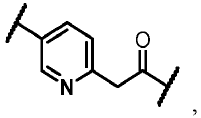 and optionally substituted 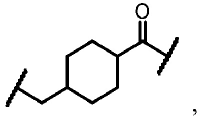 and optionally substituted

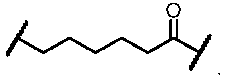

For example, in the structure shown in formula (L-2), said L₃ may comprise peptide residues. For example, said L₃ may comprise peptide residues composed of amino acids selected from the group consisting of phenylalanine, isoleucine, leucine, tryptophan, valine, methionine, tyrosine, alanine, threonine, histidine, serine, glutamine, arginine, lysine, asparagine, glutamate, proline, citrulline, aspartate, and glycine. For example, said L₃ may comprise peptide residues composed of amino acids selected from the group consisting of glycine, phenylalanine, valine, and citrulline. For example, said L₃ may comprise peptide residues selected from the group consisting of -glycine-phenylalanine-glycine- (-Gly-Phe-Gly-), -glycine-glycine-phenylalanine-glycine- (-Gly-Gly-Phe-Gly-), and -valine-citrulline- (-Val-Cit-).

For example, in the structure shown in formula (L-2), said L₃ may comprise a structure selected from the group consisting of

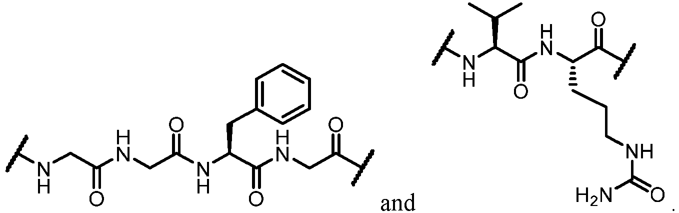

For example, in the structure shown in formula (L-2), said L₅ may comprise optionally substituted 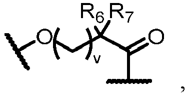 said L₅ may be directly linked to the following structure: 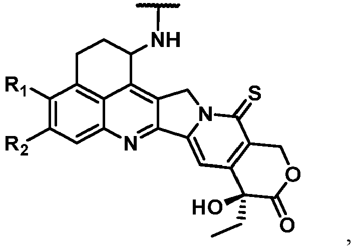 for example, 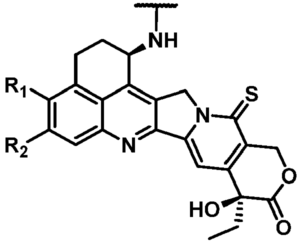 and/or 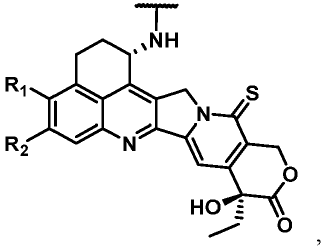 wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl, R₆ and R₇ may each be independently selected from the group consisting of hydrogen, deuterium, optionally substituted alkyl, optionally substituted haloalkyl, optionally substituted cycloalkyl, and optionally substituted cycloalkylalkyl, or R₆ and R₇ may be joined with the atom to which they attached to form an optionally substituted cycloalkyl, wherein v may be selected from a number at least 0.

For example, in the structure shown in formula (L-2), said R₆ may be hydrogen. For example, said R₆ may be optionally substituted methyl. For example, said R₆ may be optionally substituted halomethyl. For example, said R₆ may be fluorine substituted methyl. For example, said R₆ may be trifluoromethyl. For example, said R₆ may be optionally substituted cycloalkyl. For example, said R₆ may be optionally substituted cyclopropyl. For example, said R₇ may be hydrogen. For example, R₆ and R₇ may be joined with the atom to which they attached to form an optionally substituted cyclopropyl. For example, R₆ and R₇ may be joined with the atom to which they attached to form an optionally substituted cyclobutyl. For example, v may be 0. For example, v may be 1.

For example, in the structure shown in formula (L-2), said L₅ may comprise a structure selected from the group consisting of optionally substituted 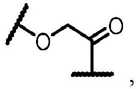 optionally substituted 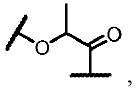 optionally substituted 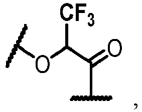 optionally substituted 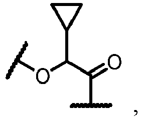 optionally substituted 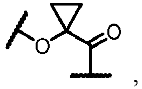 optionally substituted 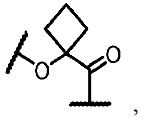 optionally substituted 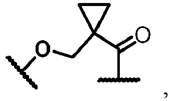 and optionally substituted

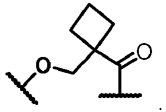

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (L-2):

-L₁-L₂-L₃-L₄-L₅- (L-2),

wherein, said L₁ may comprise a linker after attachment to a thiol group,
said L₂ may comprise a group selected from the group consisting of optionally substituted - (CH₂)ₘ₁-X₁-(CH₂)ₘ₂-C(O)-, optionally substituted -(CH₂CH₂O)ₙ-C(O)-, and optionally substituted - (CH₂)ₚ-C(O)-, X₁ may be selected from the group consisting of -O-, optionally substituted -C(O)-NH-, optionally substituted phenyl, optionally substituted pyridyl, optionally substituted aliphatic heterocyclyl containing two oxygen atoms and four carbon atoms, and optionally substituted cyclohexyl, wherein m1, m2 and n may each be independently selected from a number of at least 0;
Said L₃ may comprise peptide residues.
Said L₄ may comprise optionally substituted 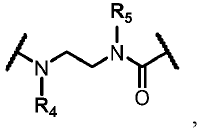 optionally substituted 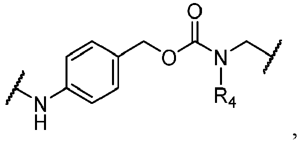 or optionally substituted 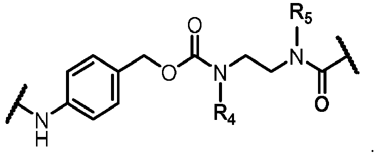 For example, said R₄ may be selected from the group consisting of hydrogen and optionally substituted C₁-C₆ alkyl. For example, said R₅ may be selected from the group consisting of hydrogen and optionally substituted C₁-C₆ alkyl.
said L₅ may comprise optionally substituted 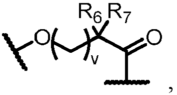 said L₅ may be directly linked to the following structure: 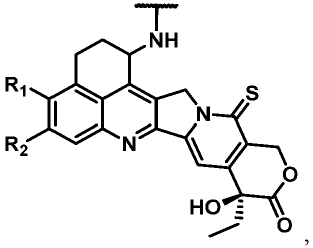 for example, 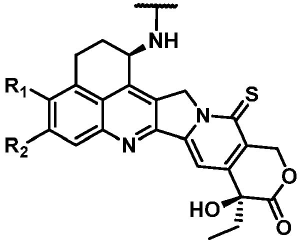 and/or 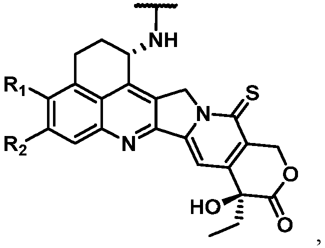 wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl,
R₆ and R₇ may each be independently selected from the group consisting of hydrogen, deuterium, optionally substituted alkyl, optionally substituted haloalkyl, optionally substituted cycloalkyl, and optionally substituted cycloalkylalkyl, or R₆ and R₇ may be joined with the atom to which they attached to form an optionally substituted cycloalkyl, wherein v may be selected from a number at least 0. For example, cycloalkylalkyl in the present application may refer to an alkyl group substituted with cycloalkyl, wherein the cycloalkylalkyl group may also be optionally substituted with an additional group.

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (L-2):

-L₁-L₂-L₃-L₄-L₅- (L-2),

wherein, said L₁ may comprise optionally substituted 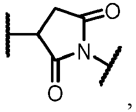
said L₂ may comprise a structure selected from the group consisting of optionally substituted 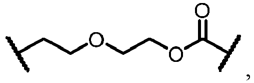 optionally substituted 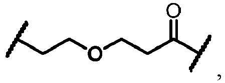 optionally substituted 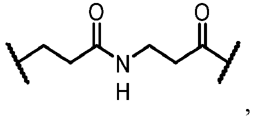 optionally substituted 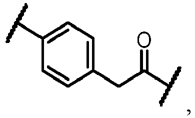 optionally substituted 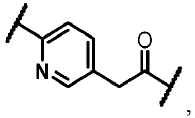 optionally substituted 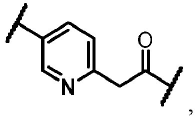 optionally substituted 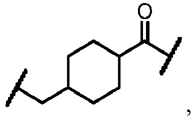 and optionally substituted 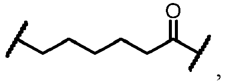
said L₃ may comprise a structure selected from the group consisting of 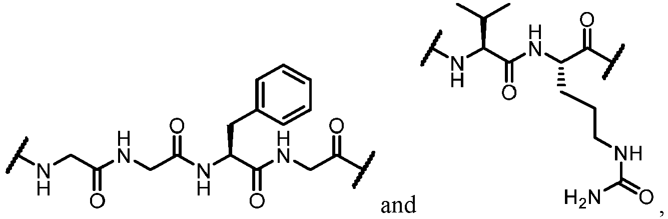
said L₄ may comprise a structure selected from the group consisting of optionally substituted 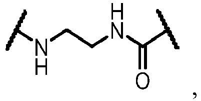 optionally substituted 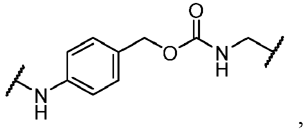 optionally substituted 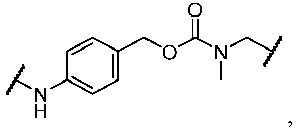 and optionally substituted 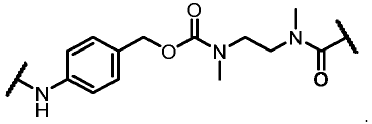
said L₅ may comprise a structure selected from the group consisting of optionally substituted 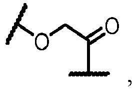 optionally substituted 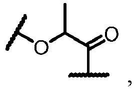 optionally substituted 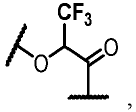 optionally substituted 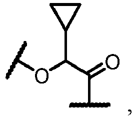 optionally substituted 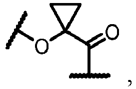 optionally substituted 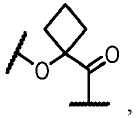 optionally substituted 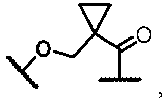 and optionally substituted 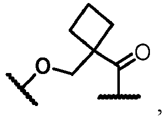 said L₅ may be directly linked to the following structure: 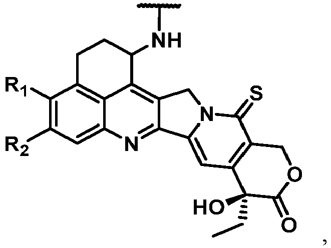 for example, 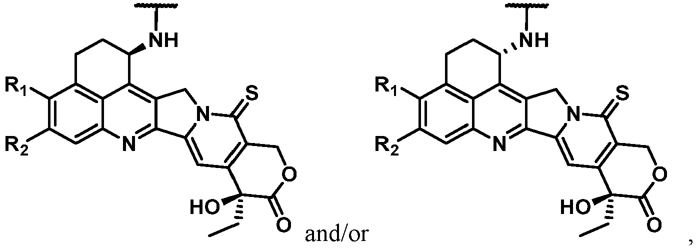 wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl.

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise a structure selected from the group consisting of

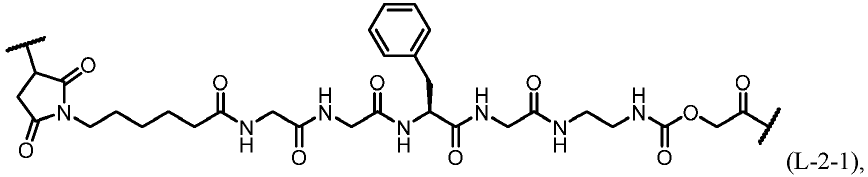

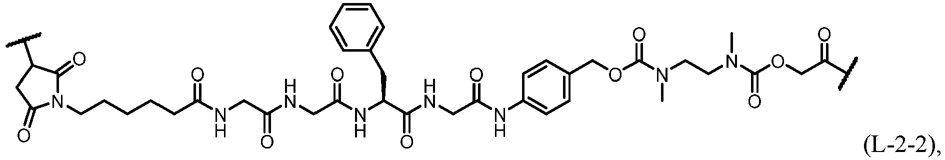

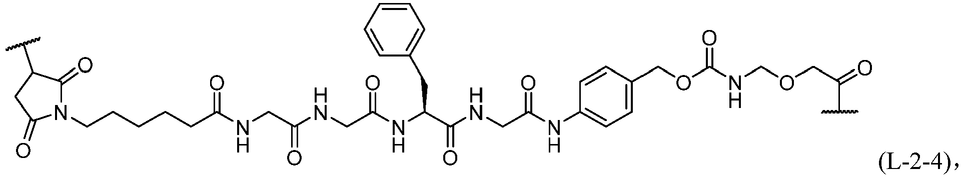

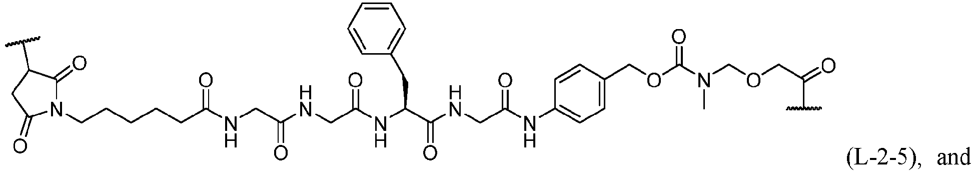

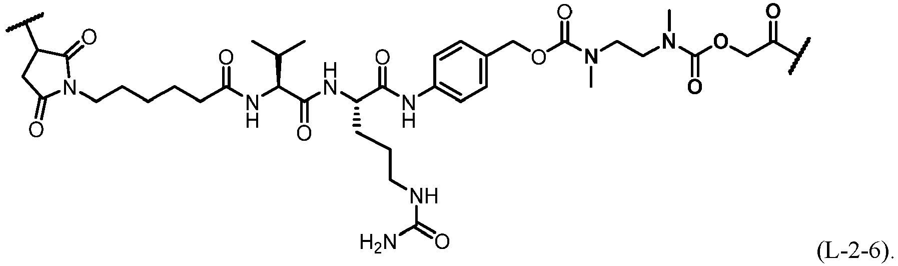

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (Lx-1):

L₁ₓ-L₂-L₃-L₄-L₅- (Lx-1),

wherein.
L₁ₓ may comprise the linking group, L₂, L₃, L₄ and L₅ can each be independent as a linker,
wherein, said L₂ may comprise a group selected from the group consisting of optionally substituted -(CH₂)ₘ₁-X₁-(CH₂)ₘ₂-C(O)- and optionally substituted -(CH₂CH₂O)ₙ-C(O)-,
X₁ may be selected from the group consisting of -O-, optionally substituted -C(O)-NH-, optionally substituted aryl, optionally substituted heteroaryl, optionally substituted aliphatic heterocyclyl and optionally substituted aliphatic cyclyl,
wherein, m1, m2, and n are each independently selected from numbers of at least 0.

For example, in the structure shown in formula (Lx-1), said L₂ may comprise optionally substituted -(CH₂CH₂O)ₙ-C(O)-.

For example, in the structure shown in formula (Lx-1), wherein , n can be 2.

For example, in the structure shown in formula (Lx-1), said L₂ may comprise optionally substituted -(CH₂)ₘ₁-X₁-(CH₂)ₘ₂-C(O)-.

For example, in the structure shown in formula (Lx-1), X₁ may be selected from the group consisting of -O- and optionally substituted -C(O)-NH-.

For example, in the structure shown in formula (Lx-1), wherein, m1 can be 2.

For example, in the structure shown in formula (Lx-1), wherein, m2 can be 2.

For example, in the structure shown in formula (Lx-1), X₁ may comprise a group selected from the group consisting of an optionally substituted aryl and an optionally substituted heteroaryl.

For example, in the structure shown in formula (Lx-1), X₁ may comprise optionally substituted phenyl.

For example, in the structure shown in formula (Lx-1), X₁ may comprise optionally substituted pyridyl.

For example, in the structure shown in formula (Lx-1), wherein, m1 can be 0.

For example, in the structure shown in formula (Lx-1), wherein, m2 can be 1.

For example, in the structure shown in formula (Lx-1), X₁ may comprise an optionally substituted aliphatic heterocyclyl containing two oxygen atoms.

For example, in the structure shown in formula (Lx-1), X₁ may comprise optionally substituted

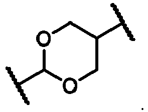

For example, in the structure shown in formula (Lx-1), X₁ may comprise an optionally substituted cyclohexyl.

For example, in the structure shown in formula (Lx-1), m1 can be 1.

For example, in the structure shown in formula (Lx-1), m2 can be 0.

For example, in the structure shown in formula (Lx-1), said L₂ may comprise a structure selected from the group consisting of optionally substituted 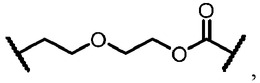 optionally substituted 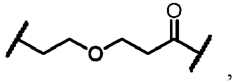 optionally substituted 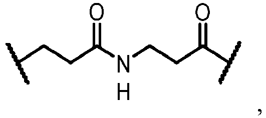 optionally substituted 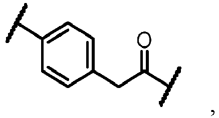 optionally substituted 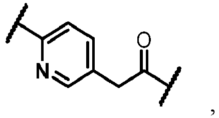 optionally substituted 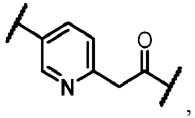 and optionally substituted

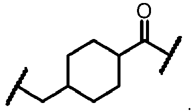

For example, in the structure shown in formula (Lx-1), said L₁ₓ may comprise optionally substituted 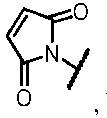 For example, in the structure shown in formula (Lx-1), said L₁ₓ can exist in a ring-opened form as shown in the following equation.

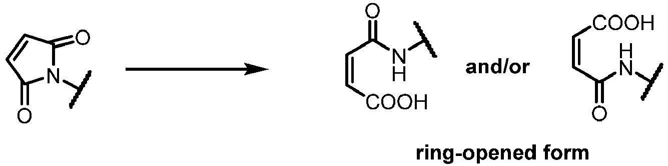

For example, in the structure shown in formula (Lx-1), said L₃ may comprise peptide residues.

For example, in the structure shown in formula (Lx-1), said L₃ may comprise peptide residues composed of amino acids selected from the group consisting of phenylalanine, isoleucine, leucine, tryptophan, valine, methionine, tyrosine, alanine, threonine, histidine, serine, glutamine, arginine, lysine, asparagine, glutamate, proline, citrulline, aspartate, and glycine.

For example, in the structure shown in formula (Lx-1), said L₃ may comprise peptide residues composed of amino acids selected from the group consisting of glycine, phenylalanine, valine, and citrulline.

For example, in the structure shown in formula (Lx-1), said L₃ may comprise peptide residues selected from the group consisting of -glycine-phenylalanine-glycine- (-Gly-Phe-Gly-), -glycine-glycine-phenylalanine-glycine- (-Gly-Gly-Phe-Gly-), and -valine-citrulline- (-Val-Cit-).

For example, in the structure shown in formula (Lx-1), said L₃ may comprise a structure selected from the group consisting of

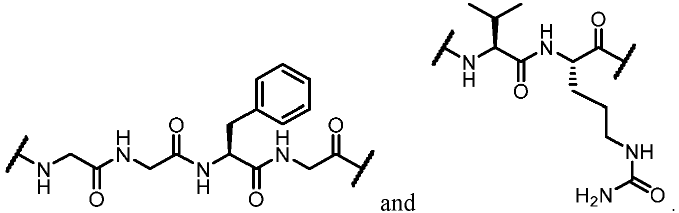

For example, in the structure shown in formula (Lx-1), said L₄ may comprise optionally substituted -L₄ₐ-NR₄-CH₂-L_{4b}-,
L₄ₐ may be absent or L₄ₐ may comprise optionally substituted 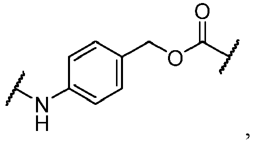
L_{4b} may be absent or L_{4b} may comprise optionally substituted 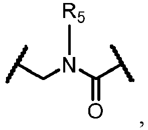
R₄ and R₅ may each independently be selected from the group consisting of hydrogen and optionally substituted alkyl.

For example, in the structure shown in formula (Lx-1), said L₄ may comprise optionally substituted

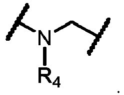

For example, in the structure shown in formula (Lx-1), said L₄ may comprise optionally substituted

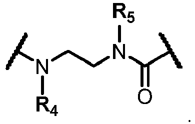

For example, in the structure shown in formula (Lx-1), said L₄ may comprise optionally substituted

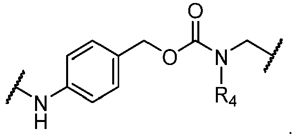

For example, in the structure shown in formula (Lx-1), said L₄ may comprise optionally substituted

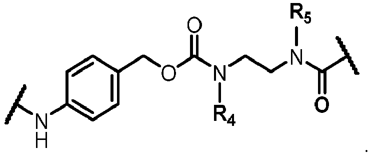

For example, in the structure shown in formula (Lx-1), said R₄ may be selected from the group consisting of hydrogen and optionally substituted methyl.

For example, in the structure shown in formula (Lx-1), said R₅ may be selected from the group consisting of hydrogen and optionally substituted methyl.

For example, in the structure shown in formula (Lx-1), said L₄ may comprise a structure selected from the group consisting of optionally substituted 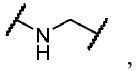 optionally substituted 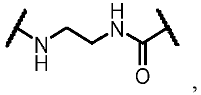 optionally substituted 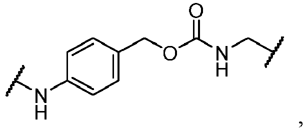 optionally substituted 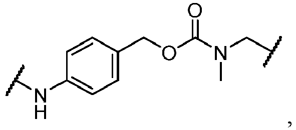 and optionally substituted

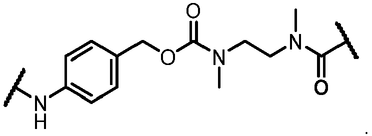

For example, in the structure shown in formula (Lx-1), said L₅ may comprise optionally substituted 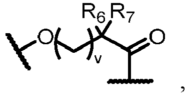 said L₅ may be directly linked to the following structure: 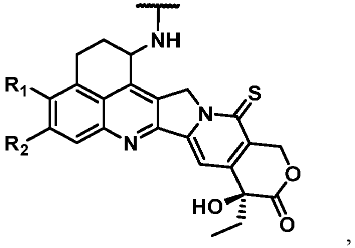 for example, 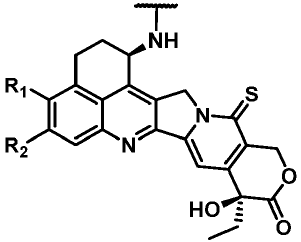 and/or 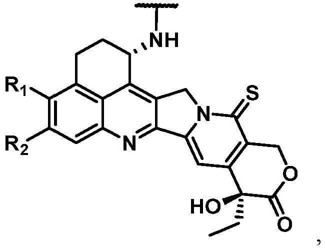 wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl.
R₆ and R₇ may each be independently selected from the group consisting of hydrogen, deuterium, optionally substituted alkyl, optionally substituted haloalkyl, optionally substituted cycloalkyl, and optionally substituted cycloalkylalkyl, or R₆ and R₇ may be joined with the atom to which they attached to form an optionally substituted cycloalkyl, wherein v may be selected from a number at least 0.

For example, in the structure shown in formula (Lx-1), said R₆ may be hydrogen.

For example, in the structure shown in formula (Lx-1), said R₆ may be optionally substituted methyl.

For example, in the structure shown in formula (Lx-1), said R₆ may be optionally substituted halomethyl.

For example, in the structure shown in formula (Lx-1), said R₆ may be fluorine substituted methyl.

For example, in the structure shown in formula (Lx-1), said R₆ may be trifluoromethyl.

For example, in the structure shown in formula (Lx-1), said R₆ may be optionally substituted cycloalkyl.

For example, in the structure shown in formula (Lx-1), said R₆ may be optionally substituted cyclopropyl.

For example, in the structure shown in formula (Lx-1), said R₇ may be hydrogen.

For example, in the structure shown in formula (Lx-1), R₆ and R₇ may be joined with the atom to which they attached to form an optionally substituted cyclopropyl.

For example, in the structure shown in formula (Lx-1), R₆ and R₇ may be joined with the atom to which they attached to form an optionally substituted cyclobutyl.

For example, in the structure shown in formula (Lx-1), wherein, v can be 0.

For example, in the structure shown in formula (Lx-1), wherein, v can be 1.

For example, in the structure shown in formula (Lx-1), said L₅ may comprise a structure selected from the group consisting of optionally substituted 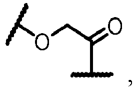 optionally substituted 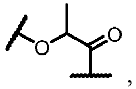 optionally substituted 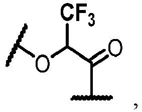 optionally substituted 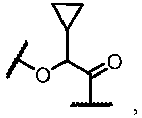 optionally substituted 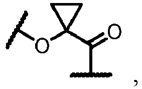 optionally substituted 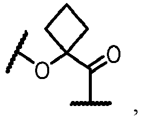 optionally substituted 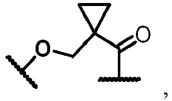 and optionally substituted

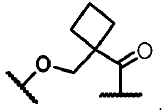

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise a structure selected from the group consisting of

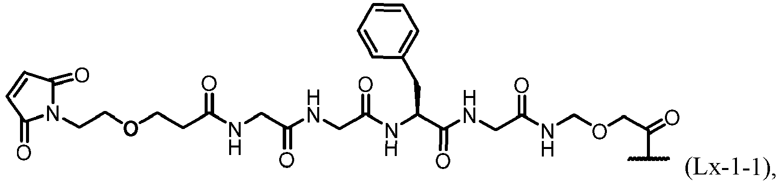

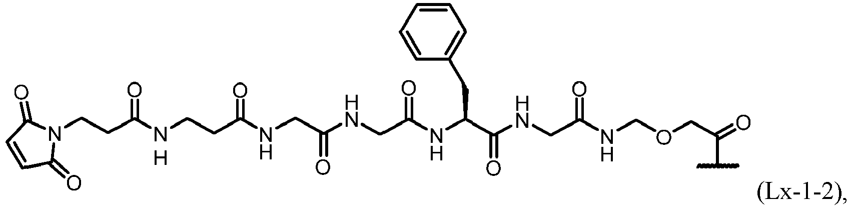

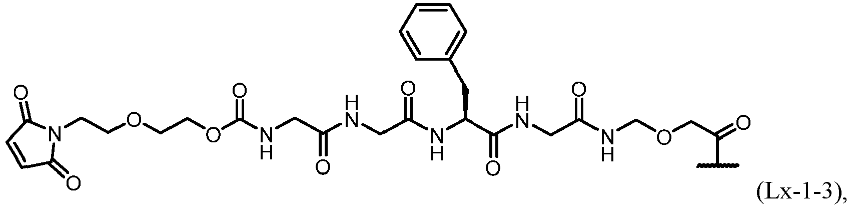

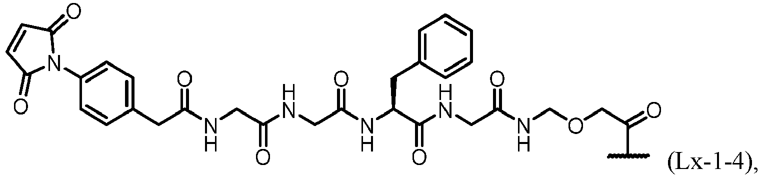

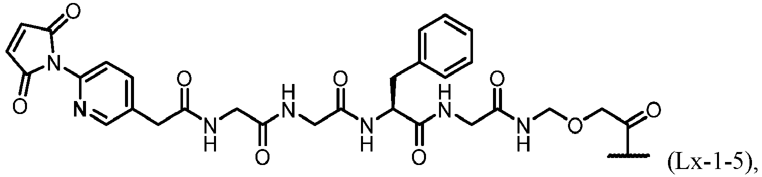

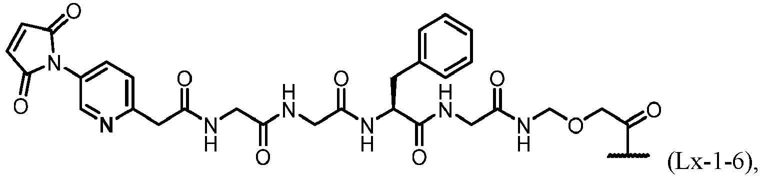

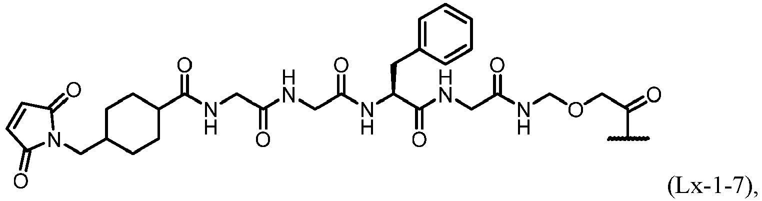

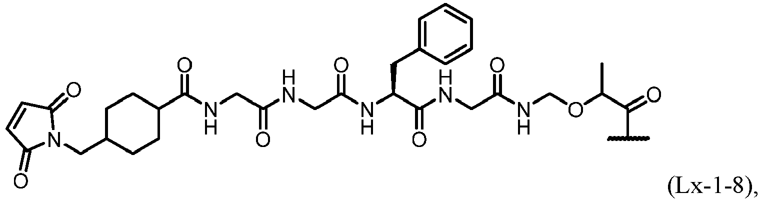

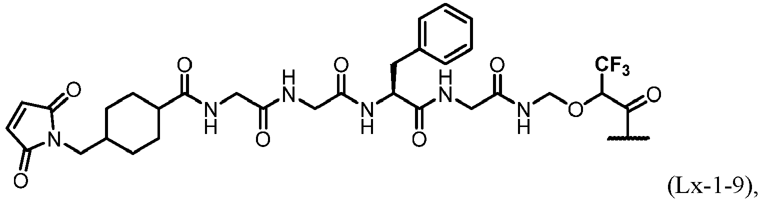

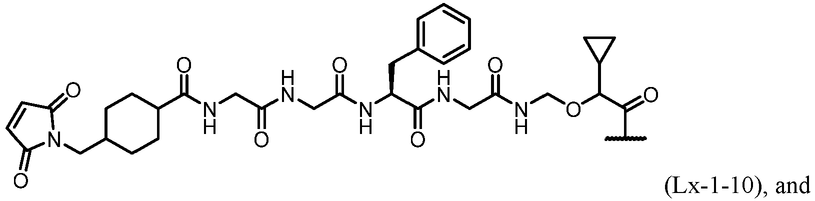

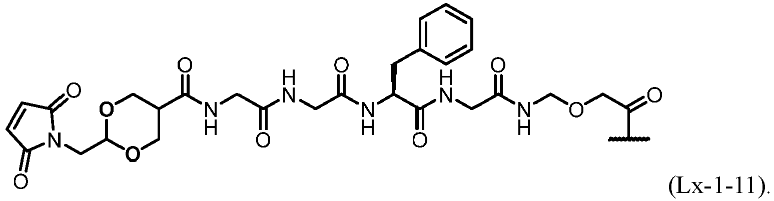

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise the structure shown in formula (Lx-2):

L₁ₓ-L₂-L₃-L₄-L₅- (Lx-2),

wherein.
L₁ₓ may comprise the linking group, L₂, L₃, L₄ and L₅ can each be independent as a linker,
L₄ may comprise optionally substituted -L₄ₐ-NR₄-CH₂-L_{4b}-,
L₄ₐ may be absent or L₄ₐ may comprise optionally substituted 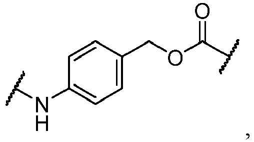
L_{4b} may be absent or L_{4b} may comprise optionally substituted 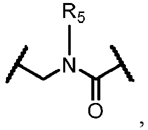 L₄ₐ and L_{4b} can not be absent at the same time,
R₄ and R₅ may each independently be selected from the group consisting of hydrogen and optionally substituted alkyl.

For example, in the structure shown in formula (Lx-2), said L₄ may comprise optionally substituted

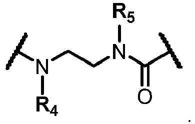

For example, in the structure shown in formula (Lx-2), said L₄ may comprise optionally substituted

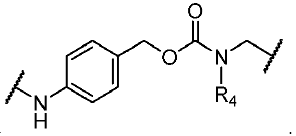

For example, in the structure shown in formula (Lx-2), said L₄ may comprise optionally substituted

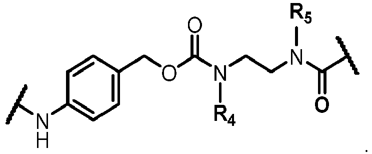

For example, in the structure shown in formula (Lx-2), said R₄ may be selected from the group consisting of hydrogen and optionally substituted methyl.

For example, in the structure shown in formula (Lx-2), said R₅ may be selected from the group consisting of hydrogen and optionally substituted methyl.

For example, in the structure shown in formula (Lx-2), said L₄ may comprise a structure selected from the group consisting of optionally substituted 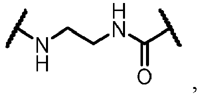 optionally substituted 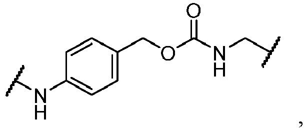 optionally substituted 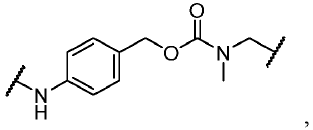 optionally substituted

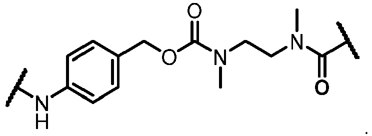

For example, in the structure shown in formula (Lx-2), said L₁ₓ may comprise optionally substituted 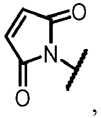 For example, in the structure shown in formula (Lx-1), said L₁ₓ can exist in a ring-opened form as shown in the following equation.

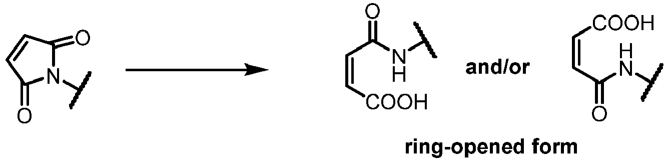

For example, in the structure shown in formula (Lx-2), said L₂ may comprise a group selected from the group consisting of optionally substituted -(CH₂)ₘ₁-X₁-(CH₂)ₘ₂-C(O)-, optionally substituted -(CH₂CH₂O)ₙ-C(O)-, and optionally substituted -(CH₂)ₚ-C(O)-.

X₁ may be selected from the group consisting of -O-, optionally substituted -C(O)-NH-, optionally substituted aryl, optionally substituted heteroaryl, optionally substituted aliphatic heterocyclyl and optionally substituted aliphatic cyclyl,
wherein, m1, m2, and n are each independently selected from numbers of at least 0.

For example, in the structure shown in formula (Lx-2), said L₂ may comprise optionally substituted -(CH₂CH₂O)ₙ-C(O)-.

For example, in the structure shown in formula (Lx-2), wherein, n can be 2.

For example, in the structure shown in formula (Lx-2), said L₂ may comprise optionally substituted -(CH₂)ₘ₁-X₁-(CH₂)ₘ₂-C(O)-.

For example, in the structure shown in formula (Lx-2), X₁ may be selected from the group consisting of -O- and optionally substituted -C(O)-NH-.

For example, in the structure shown in formula (Lx-2), wherein, m1 can be 2.

For example, in the structure shown in formula (Lx-2), wherein, m2 can be 2.

For example, in the structure shown in formula (Lx-2), X₁ may comprise a group selected from the group consisting of an optionally substituted aryl and an optionally substituted heteroaryl.

For example, in the structure shown in formula (Lx-2), X₁ may comprise optionally substituted phenyl.

For example, in the structure shown in formula (Lx-2), X₁ may comprise optionally substituted pyridyl.

For example, in the structure shown in formula (Lx-2), wherein, m1 can be 0.

For example, in the structure shown in formula (Lx-2), wherein, m1 can be 1.

For example, in the structure shown in formula (Lx-2), X₁ may comprise an optionally substituted aliphatic heterocyclyl containing two oxygen atoms.

For example, in the structure shown in formula (Lx-2), X₁ may comprise optionally substituted

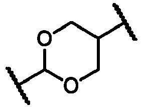

For example, in the structure shown in formula (Lx-2), X₁ may comprise an optionally substituted cyclohexyl.

For example, in the structure shown in formula (Lx-2), m1 can be 1.

For example, in the structure shown in formula (Lx-2), m2 can be 0.

For example, in the structure shown in formula (Lx-2), said L₂ may comprise optionally substituted -(CH₂)ₚ-C(O)-.

For example, in the structure shown in formula (Lx-2), wherein, p can be 5.

For example, in the structure shown in formula (Lx-2), said L₂ may comprise a structure selected from the group consisting of optionally substituted 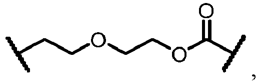 optionally substituted 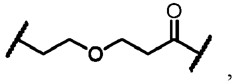 optionally substituted 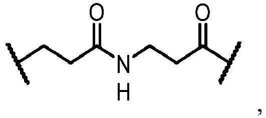 optionally substituted 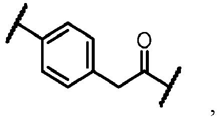 optionally substituted 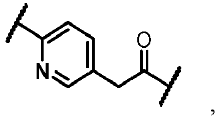 optionally substituted 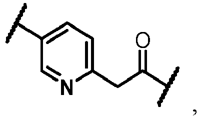 and optionally substituted 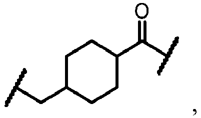 and optionally substituted

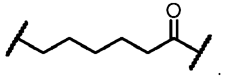

For example, in the structure shown in formula (Lx-2), said L₃ may comprise peptide residues.

For example, in the structure shown in formula (Lx-2), said L₃ may comprise peptide residues composed of amino acids selected from the group consisting of phenylalanine, isoleucine, leucine, tryptophan, valine, methionine, tyrosine, alanine, threonine, histidine, serine, glutamine, arginine, lysine, asparagine, glutamate, proline, citrulline, aspartate, and glycine.

For example, in the structure shown in formula (Lx-2), said L₃ may comprise peptide residues composed of amino acids selected from the group consisting of glycine, phenylalanine, valine, and citrulline.

For example, in the structure shown in formula (Lx-2), said L₃ may comprise peptide residues selected from the group consisting of -glycine-phenylalanine-glycine- (-Gly-Phe-Gly-), -glycine-glycine-phenylalanine-glycine- (-Gly-Gly-Phe-Gly-), and -valine-citrulline- (-Val-Cit-).

For example, in the structure shown in formula (Lx-2), said L₃ may comprise a structure selected from the group consisting of

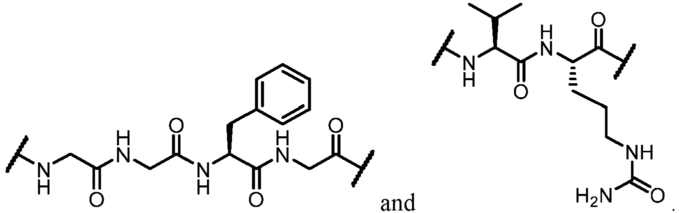

For example, in the structure shown in formula (Lx-2), said L₅ may comprise optionally substituted 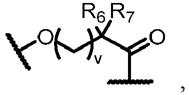 said L₅ may be directly linked to the following structure: 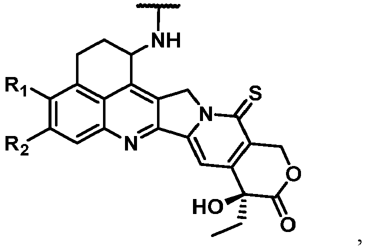 for example, 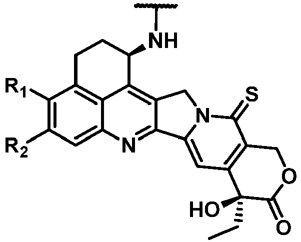 and/or 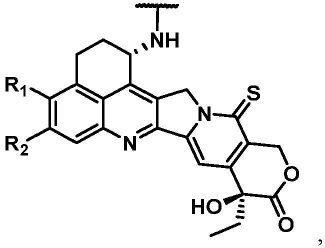 wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl,
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl.
R₆ and R₇ may each be independently selected from the group consisting of hydrogen, deuterium, optionally substituted alkyl, optionally substituted haloalkyl, optionally substituted cycloalkyl, and optionally substituted cycloalkylalkyl, or R₆ and R₇ may be joined with the atom to which they attached to form an optionally substituted cycloalkyl, wherein v may be selected from a number at least 0.

For example, in the structure shown in formula (Lx-2), said R₆ may be hydrogen.

For example, in the structure shown in formula (Lx-2), said R₆ may be optionally substituted methyl.

For example, in the structure shown in formula (Lx-2), said R₆ may be optionally substituted halomethyl.

For example, in the structure shown in formula (Lx-2), said R₆ may be fluorine substituted methyl.

For example, in the structure shown in formula (Lx-2), said R₆ may be trifluoromethyl.

For example, in the structure shown in formula (Lx-2), said R₆ may be optionally substituted cycloalkyl.

For example, in the structure shown in formula (Lx-2), said R₆ may be optionally substituted cyclopropyl.

For example, in the structure shown in formula (Lx-2), said R₇ may be hydrogen.

For example, in the structure shown in formula (Lx-2), R₆ and R₇ may be joined with the atom to which they attached to form an optionally substituted cyclopropyl.

For example, in the structure shown in formula (Lx-2), R₆ and R₇ may be joined with the atom to which they attached to form an optionally substituted cyclobutyl.

For example, in the structure shown in formula (Lx-2), wherein, v can be 0.

For example, in the structure shown in formula (Lx-2), wherein, v can be 1.

For example, in the structure shown in formula (Lx-2), said L₅ may comprise a structure selected from the group consisting of optionally substituted 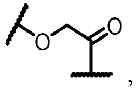 optionally substituted 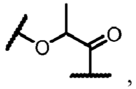 optionally substituted 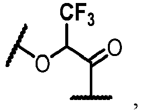 optionally substituted 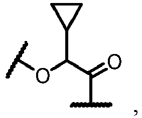 optionally substituted 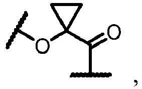 optionally substituted 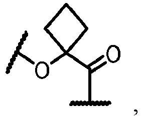 optionally substituted 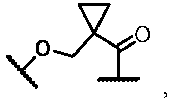 and optionally substituted

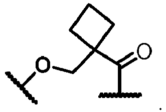

In another aspect, the present application also provides a compound, or a tautomer, a mesomer, a racemate, an enantiomer, a diastereomer, or a mixture thereof, or a pharmaceutically acceptable salt or hydrate thereof, wherein said compound may comprise a structure selected from the group consisting of

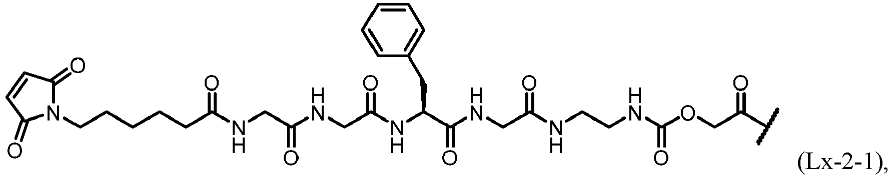

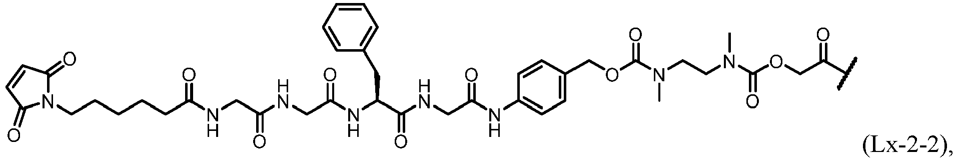

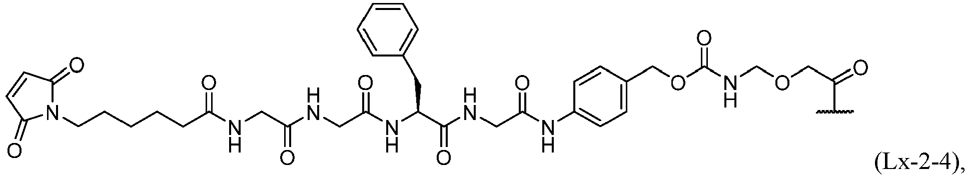

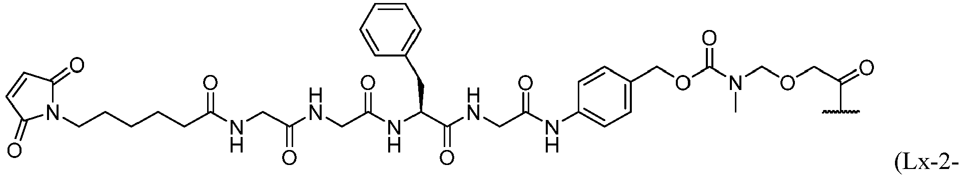

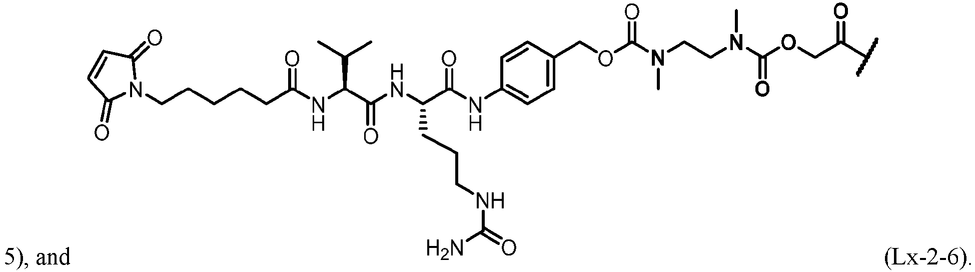

### Pharmaceutical composition

The pharmaceutical composition described in the present application may contain one or more excipients in addition to active compounds, and the excipients may be selected from the group consisting of fillers (diluents), adhesives, wetting agents, disintegrating agents, and excipients. Depending on the method of administration, the composition may contain 0.1wt % to 99 wt % of the active compound.

The pharmaceutical composition containing the active ingredient may be in a form suitable for oral administration, such as tablets, dragees, lozenges, aqueous or oily suspensions, dispersible powders or granules, emulsions, hard or soft capsules, or syrups. Pharmaceutical compositions intended for oral use can be prepared according to any method known to the art for the manufacture of pharmaceutical compositions. The composition may contain adhesives, fillers, lubricants, disintegrating agents, or pharmaceutically acceptable wetting agents, etc. The composition may also contain one or more components selected from the group consisting of sweeting agents, flavoring agents, coloring agents, and preservatives.

Aqueous suspensions may contain the active ingredient and excipients suitable for the preparation of aqueous suspensions which can be used for mixing. An aqueous suspension may also contain one or more preservatives, such as one or more colouring agents, one or more flavoring agents, and one or more sweetening agents. An oily suspension may be formulated by suspending active ingredients in vegetable oil. An oily suspension may contain thickening agents. The above-mentioned sweetening agents and flavoring agents may also be added.

The pharmaceutical composition can also be dispersible powders and granules providing the active ingredients, which were used for preparing aqueous suspensions by adding water and mixing with one or more of dispersing agents, wetting agents, suspending agents or preservatives. Other excipients such as sweetening, flavouring and colouring agents may also be added. These compositions are preserved by adding antioxidants, e.g., ascorbic acid. The pharmaceutical composition of the present application may also be in the form of oil-in-water emulsion.

The pharmaceutical composition may be in the form of sterile injectable aqueous solution. Available and acceptable vehicle or solvents that can be used include water, Ringer's solution, and isotonic sodium chloride solution. A sterile injectable formulation may be a sterile injectable oil-in-water microemulsion in which the active ingredient is dissolved in an oily phase. For example, dissolving the active ingredient in a mixture of soybean oil and lecithin. Then, the oil solution can be added to a mixture of water and glycerol to form a microemulsion. The injection or microemulsion may be locally injected into the patient's bloodstream in large quantities. Alternatively, the solution and microemulsion may be administered in such a way that a constant circulating concentration of the compound in the present application can be maintained. To maintain the constant concentration, a continuous intravenous drug delivery device may be used. For example, the device may be an intravenous injection pump.

The pharmaceutical composition may be in the form of a sterile injectable aqueous or oily suspension for intramuscular and subcutaneous administration. The suspension may be formulated using suitable dispersing or wetting agent and suspending agent as described in the present application according to known art. A sterile injectable formulation may also be a sterile injectable solution or suspension prepared in a non-toxic parenterally acceptable diluent or solvent. Alternatively, sterile fixed oil can be conveniently used as solvents or suspension medium.

The compound of the present application may be administered in the form of a suppository for rectal administration. These pharmaceutical compositions may be prepared by mixing the drug with suitable non-irritating excipients that are solid at ordinary temperature but liquid in the rectum and will therefore melt in the rectum to release the drug. These substances include cocoa butter, glycerol gelatin, hydrogenated vegetable oil, mixtures of polyethylene glycol and polyethylene glycol fatty esters of various molecular weights.

As is well known to those skilled in the art, the dosage of a drug depends on various factors, including but not limited to: the activity of the particular compound used, the age of the patient, the weight of the patient, the health of the patient, the behavior of the patient, the diet of the patient, the administration time, the administration mode, the excretion rate, the combination of drugs, etc. In addition, the optimal treatment modality such as the mode of treatment, the compound described in this application or a tautomer, a mesomer, a racemate, an enantiomer, and a diastereomer, or a mixture thereof, or a pharmaceutically acceptable salt thereof, and/or the daily dosage of the compound or a tautomer, a mesomer, a racemate, an enantiomer, and a diastereomer, or a mixture thereof, or a pharmaceutically acceptable salt thereof, or the type of pharmaceutically acceptable salts, can be verified according to conventional therapeutic regimens.

### Methods of prevention and/or treatment

The present application provides a use of the compound of the present application, or a tautomer, a mesomer, a racemate, an enantiomer, a diastereomer, or a mixture thereof, or a pharmaceutically acceptable salt or hydrate thereof in the preparation of drugs, the drugs of the present application can be used to treat and/or prevent tumors. For example, the tumor as described in the present application can be selected from tumors associated with the expression of the target consisting of HER2 and TROP2. For example, the tumor associated with the target expression as described in the present application can comprises a tumor with high target expression and/or a tumor with positive target expression. For example, the tumor as described in the present application may include solid tumors and/or hematologic malignancies. For example, the tumor as described in the present application can be selected from the group consisting of breast cancer, gastric cancer, ovarian cancer, bladder cancer, colorectal cancer, endometrial cancer, kidney cancer, lung cancer, pancreatic cancer, prostate cancer, and thyroid cancer. For example, the tumor as described in the present application can be selected from the group consisting of breast cancer, ovarian cancer, cervical cancer, endometrial cancer, urothelial cancer, lung cancer, prostate cancer, colorectal cancer, gastric cancer, esophageal cancer, bladder cancer, kidney cancer, pancreatic cancer, thyroid cancer, and head and neck cancer.

The present application provides a compound of the application, or a tautomer, a mesomer, a racemate, an enantiomer, and a diastereomer, or a mixture thereof, or a pharmaceutically acceptable salt or hydrate thereof for the treatment and/or prevention of tumors. For example, the tumor can be selected from tumors associated with the expression of the target consisting of HER2 and TROP2. For example, the tumor associated with the target expression can comprises a tumor with high target expression and/or a tumor with positive target expression. For example, the tumor as described in the present application may include solid tumors and/or hematologic malignancies. For example, the tumor as described in the present application can be selected from the group consisting of breast cancer, gastric cancer, ovarian cancer, bladder cancer, colorectal cancer, endometrial cancer, kidney cancer, lung cancer, pancreatic cancer, prostate cancer, and thyroid cancer. For example, the tumor as described in the present application can be selected from the group consisting of breast cancer, ovarian cancer, cervical cancer, endometrial cancer, urothelial cancer, lung cancer, prostate cancer, colorectal cancer, gastric cancer, esophageal cancer, bladder cancer, kidney cancer, pancreatic cancer, thyroid cancer, and head and neck cancer.

The present application provides a method for treating and/or preventing tumors, which may comprises administering the compound of the present application, or a tautomer, a mesomer, a racemate, an enantiomer, and a diastereomer, or a mixture thereof, or a pharmaceutically acceptable salt and or hydrate thereof, and/or the pharmaceutical composition of the present application to a subject in need thereof. For example, the tumor can be selected from tumors associated with the expression of the target consisting of HER2 and TROP2. For example, the tumor associated with the target expression can comprises a tumor with high target expression and/or a tumor with positive target expression. For example, the tumor as described in the present application may include solid tumors and/or hematologic malignancies. For example, the tumor as described in the present application can be selected from the group consisting of breast cancer, gastric cancer, ovarian cancer, bladder cancer, colorectal cancer, endometrial cancer, kidney cancer, lung cancer, pancreatic cancer, prostate cancer, and thyroid cancer. For example, the tumor as described in the present application can be selected from the group consisting of breast cancer, ovarian cancer, cervical cancer, endometrial cancer, urothelial cancer, lung cancer, prostate cancer, colorectal cancer, gastric cancer, esophageal cancer, bladder cancer, kidney cancer, pancreatic cancer, thyroid cancer, and head and neck cancer.

Not wanting to be limited by any theory, the examples in the following content are only intended to illustrate the compounds, preparation methods, and uses of the present application, and are not intended to limit the scope of the present application.

### Examples

### Example 1

### Example 1.1

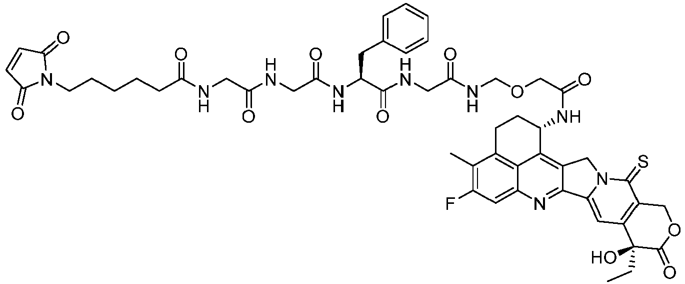

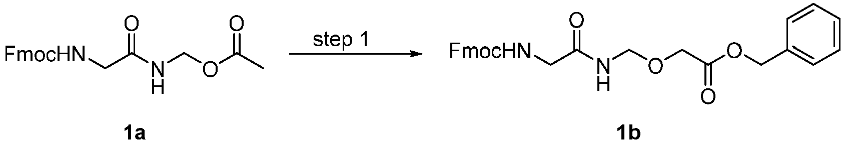

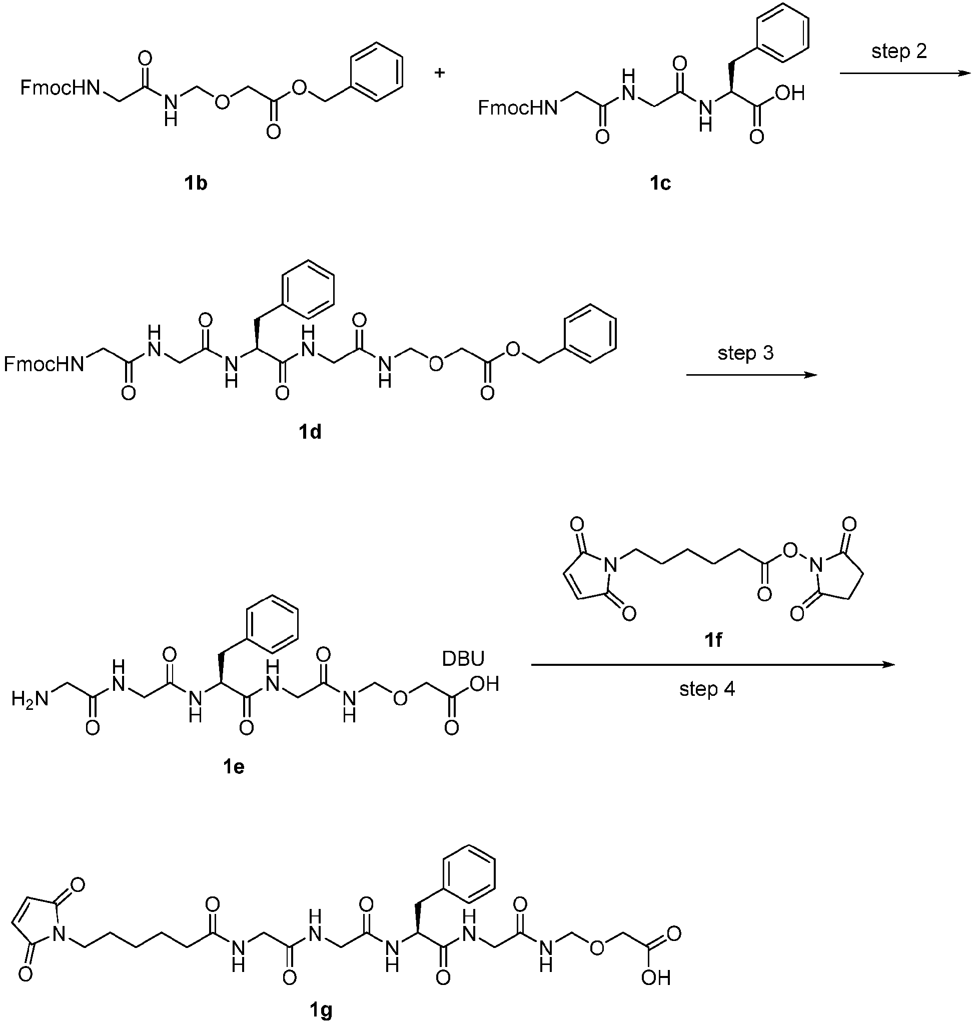

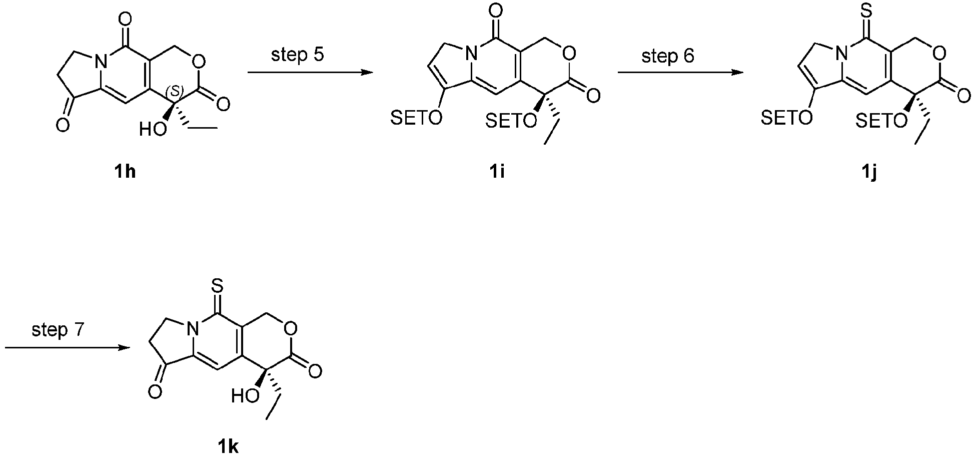

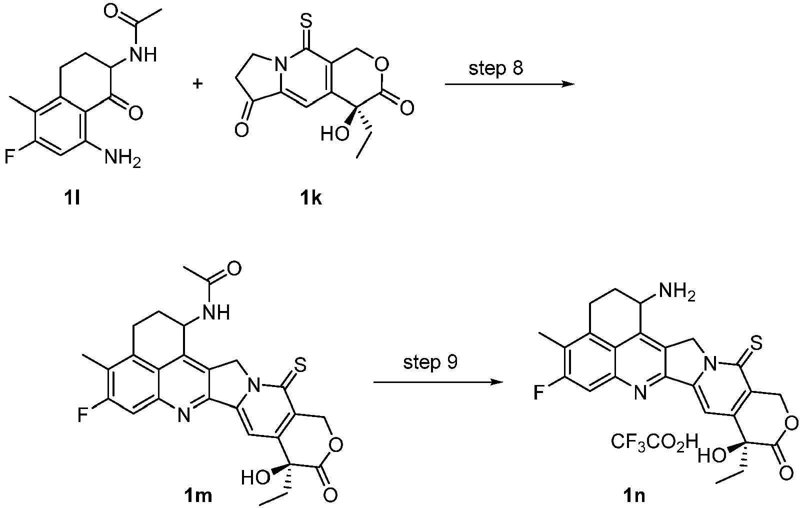

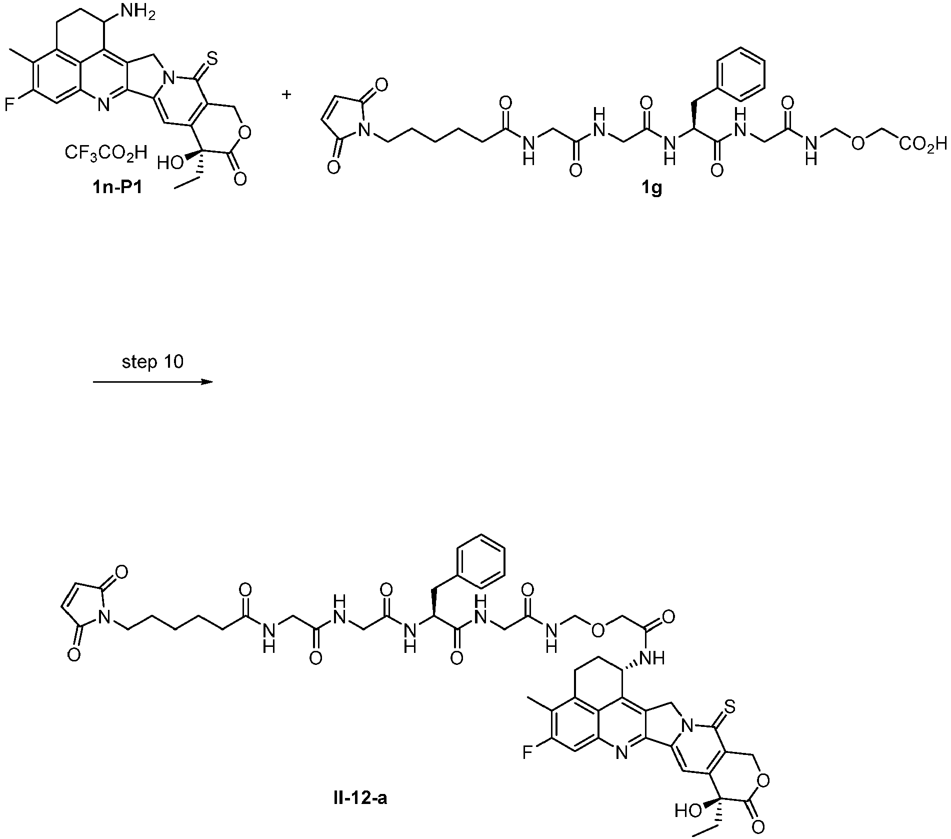

### Step 1

**1a** (50.00 g, 135.90 mmol) and benzyl glycolate (45.00 g, 271.70 mmol) were added to a three-neck flask (1 L) successively. After evacuating the flask and back-filling with nitrogen, anhydrous tetrahydrofuran (500 mL) was added. The flask was then placed in an ice bath followed by the addition of anhydrous *p*-toluenesulfonic acid (2.30 g, 13.40 mmol) and the resultant reaction mixture was stirred for 2 h. Water (1 L) and ethyl acetate (1 L) were added to the reaction, and the layers were separated. The organic phase was washed by aq. sodium bicarbonate, water and brine successively. The organic phase was dried over anhydrous sodium sulfate and then filtrated. The filtrate was concentrated *in vacuo* and the residue was purified by flash column chromatography on silica gel (methanol: dichloromethane = 0-100%) to give **1b** (27.00 g) with 42% yield.

MS-ESI calc. for [M+Na]⁺ 497, found: 497.

### Step 2

1,8-Diazabicyclo[5.4.0]undecane-7-ene (2.78 g, 18.29 mmol) was added to a solution of **1b** (17.00 g, 35.86 mmol) in *N*,*N*-dimethylacetamide (170 mL), the reaction mixture was stirred at room temperature for 1 h under N₂ atmosphere. Pyridinium *p*-toluenesulfonate (4.59 g, 18.29 mmol), 1-hydroxybenzotriazole (4.59 g, 34.00 mmol), **1c** (16.66 g, 33.18 mmol) and 1-(3-dimethylaminopropyl)-3-ethylcarbodiimide hydrochloride (6.37 g, 33.18 mmol) were added to the above reaction solution, and the resulting mixture was stirred at room temperature for an additional 2.5 h. 2-Methyltetrahydrofuran (175 mL) and saturated brine (260 mL) were added to the reaction, and the layers were separated. The aqueous phase was extracted with 2-methyltetrahydrofuran (100 mL x 2). The combined organic phases were washed with 10% citric acid (90 mL), aq. sodium bicarbonate (175 mL×3) and saturated brine (100 mL), dried over anhydrous sodium sulfate and then filtrated. The filtrate was concentrated *in vacuo* to approximately 50 mL, isopropanol (120 mL) was added. The solution was reconcentrated *in vacuo* to approximately 50 mL followed by addition of isopropanol (300 mL). the solution was stirred at 60°C for 1 h, and subsequently cooled down to 0-5°C. The suspension was stirred at 0-5°C for 2 h, and filtered. The solid was washed with isopropanol (5°C) to afford **1d** (19.00 g) with 78% yield.

### Step 3

1,8-Diazabicyclo[5.4.0]undecane-7-ene (10.70 g, 70.39 mmol) was added to a solution of **1d** (11.50 g, 15.65 mmol) in tetrahydrofuran (230 mL) and H₂O (115 mL), the mixture was stirred at room temperature for 4 h under N₂ atmosphere. The reaction mixture was washed with tert-butyl methyl ether (100 mL x 5). The aqueous layer was concentrated *in vacuo* to give yellow oily residue followed by redissolvation in isopropanol (70 mL). The obtained solution was slowly added to *tert-*butyl methyl ether (700 mL) with the formation of white solid. The suspension was then filtered, and the collected white solid was redissolved with EtOH. The solution was concentrated *in vacuo* to afford white foamy solid **1e** (9.50 g) with 93% yield.

### Step 4

**1f** (0.54 g, 1.75 mmol) was added to a solution of **1e** (1.00 g, 1.53 mmol) in acetonitrile (10 mL) and H₂O (20 mL), the mixture was stirred at room temperature for 5 h under N₂ atmosphere. Water (20 mL) was added followed by 0.5N HCl_{(aq)} to reach pH = 3~4. The mixture was extraction with isopropanol/dichloromethane (V : V= 4:1, 50 mL x 4). The combined organic layers were dried over anhydrous sodium sulfate and then filtrated. The filtrate was concentrated *in vacuo* to give yellow oily residue followed by redissolvation in dichloromethane/methanol (v:v = 4:1, 5 mL). The obtained solution was slowly added to tert-butyl methyl ether (100 mL) with the formation of white solid. The solid was then filtered to afford **1g** (850 mg) with 90% yield.

MS-ESI calc. for [M+Na]⁺ 639, found: 639.

### Step 5

**1h** (12.20 g, 46.39 mmol) was added to a three-neck flask (500 mL). After evacuating the flask and back-filling with nitrogen, the flask was then placed in an ice bath. *N*,*N-*dimethylformamide (120 mL) was added followed by the addition of imidazole (15.77 g, 232.00 mmol) and chlorotriethylsilane (27.97 g, 185.56 mmol). After stirred for 10 min, 4-dimethylaminopyridine (5.66 g, 46.39 mmol) was added. The resulting mixture was stirred at 0°C for 3 h. the reaction was diluted with methyl *tert*-butyl ether (1.20 L) followed by water (1.2 L), The biphasic mixture was separated. The aqueous layer was extracted with methyl tert-butyl ether (1.2 L). the combined organic phases were washed by water (1.20 L), dried over anhydrous sodium sulfate and then filtrated. The filtrate was concentrated and the residue was purified by flash column chromatography on silica gel (ethyl acetate: hexanes = 0-100%) to give crude **1i** (16.00 g).

### Step 6

To a three-neck flask (1 L) was added crude **1i** (16.00 g, 32.59 mmol) and Lawesson's reagent (13.18 g, 32.59 mmol) successively followed by anhydrous toluene (600 mL). After evacuating the flask and back-filling with nitrogen, the reaction mixture was refluxed at 125°C for 5 h. The reaction mixture was concentrated *in vacuo* and the residue was purified by flash column chromatography on silica gel (ethyl acetate: hexanes = 0-100%) to give crude **1j** (16.00 g).

### Step 7

**1j** (16 g crude) was dissolved in anhydrous tetrahydrofuran (640 mL), and cooled under an ice bath. Triethylamine trihydrofluoride (11.93 g, 74.00 mmol) was added dropwise. The resulting mixture was warmed naturally to room temperature and stirred overnight. The reaction mixture was then diluted with ethyl acetate (1 L) and the aqueous phase was discarded. The organic phase was washed by saturated brine (500 mL x 2), dried over anhydrous sodium sulfate and then filtrated. The filtrate was concentrated *in vacuo* and the residue was purified by flash column chromatography on silica gel (ethyl acetate: hexanes = 0-100%) to give **1k** (6.38 g) with 49% yield over three steps.

### Step 8

To a three-neck flask (500 mL) was added **11** (3.00 g, 12.00 mmol), **1k** (4.33 g, 15.52 mmol), pyridinium *p*-toluenesulfonate (3.90 g, 15.50 mmol) and o-cresol (15 mL), followed by anhydrous toluene (300 mL). It was evacuated and backfilled with nitrogen, and the reaction mixture was stirred for 32 h at 120 °C. The reaction was cooled to room temperature followed by filtration, and the obtained crude product was triturated with methyl *tert-*butyl ether (60 mL) for 1 h. The suspension was then filtered, and the wet cake was dried to afford **1m** (3.0 g) with 51% yield.

### Step 9

To a single-neck flask (500 mL) was added **1m** (2.30 g, 4.67 mmol) followed the addition of 6N HCl_{(aq)} (230 mL). The resulting mixture was refluxed at 110 °C for 12 h. The reaction mixture was thermally filtered to remove insoluble material. The filtrate was concentrated *in vacuo* to get crude product **1n** (diastereomers). The crude **1n** was purified by prep-HPLC to afford **1n-P1** (412 mg, isomer with lower polarity in TLC) and **1n-P2** (495 mg, isomer with higher polarity in TLC) with 34% yield.

### Step 10

To a single-neck flask (10 mL) was added **1n-P1** (50 mg, 0.09 mmol), **1g** (76 mg, 0.12 mmol), 1-(3-dimethylaminopropyl)-3-ethylcarbodiimide hydrochloride (31 mg, 0.16 mmol) and *N*,*N-*dimethylformamide (2 mL), followed by dropwise addition of 2,4,6-trimethylpyridine (60 mg, 0.49 mmol). The resulting mixture was stirred at room temperature for 3 h. The reaction solution was added dropwise to methyl *tert*-butyl ether (60 mL), during which a precipitate was formed. The suspension was then filtered, and the collected solid was redissolved in dichloromethane/methanol (V:V = 6:1, 30 mL). The obtained solution was concentrated *in vacuo* and the residue was purified by prep-HPLC to afford **II-12-a** (33 mg) with 35% yield.

MS-ESI calc. for [M+H]⁺ 1050, found: 1050.

¹H NMR (400 MHz, DMSO-*d₆*) δ 8.65 (t, *J* = 6.4 Hz, 1H), 8.60 (d, *J* = 8.8 Hz, 1H), 8.28 (t, *J* = 5.6 Hz, 1H), 8.10 (d, *J* = 8.0 Hz, 1H), 8.06 (t, *J* = 6.0 Hz, 1H), 7.99 (t, *J* = 5.6 Hz, 1H), 7.79 (d, *J* = 10.8 Hz, 1H), 7.78 (s, 1H), 7.28-7.14 (m, 5H), 6.97 (s, 1H), 5.90 (d, *J=* 16.4 Hz, 1H), 5.70-5,60 (m, 1H), 5.51 (s, 1H), 5.46 (d, *J* = 2.8 Hz, 1H), 5.30 (d, *J*= 19.6 Hz, 1H), 4.67 (d, *J* = 6.4 Hz, 1H), 4.51-4.41 (m, 1H), 4.18-4.04 (m, 2H), 3.77-3.73 (m, 1H), 3.73-3.68 (m, 2H), 3.68-3.62 (m, 2H), 3.62-3.58 (m, 1H), 3.58-3.54 (m, 1H), 3.38-3.31 (m, 2H), 3.30-3.20 (m, 1H), 3.20-3.08 (m, 1H), 3.01 (dd, *J* =14.0, 4.8 Hz, 1H), 2.80-2.71 (m, 1H), 2.37 (s, 3H), 2.26-2.15 (m, 2H), 2.08 (t, *J* = 7.6 Hz, 2H), 1.94-1.82 (m, 2H), 1.54-1.37 (m, 4H), 1.25-1.10 (m, 2H), 0.85 (t, J=7.6 Hz, 3H).

### Example 1.2

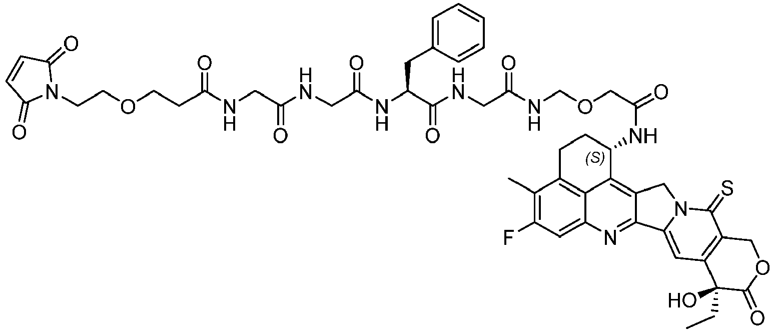

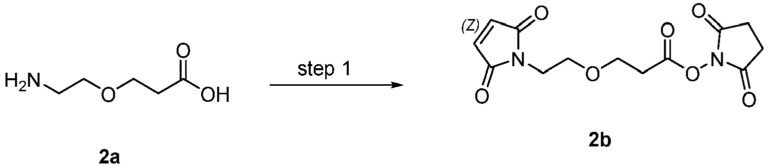

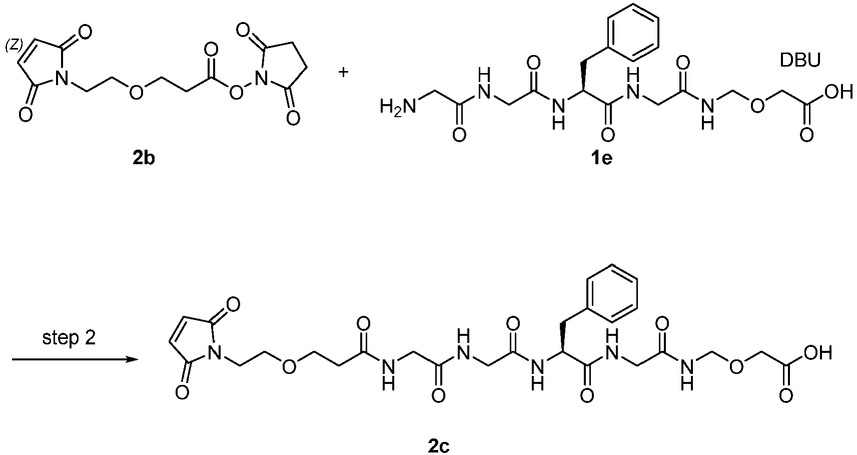

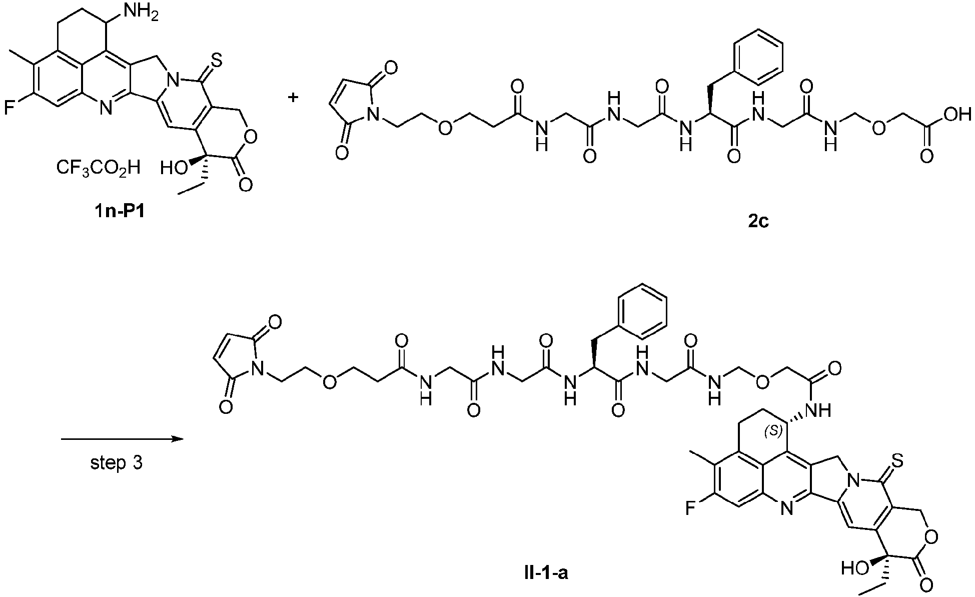

### Step 1

Maleic anhydride (0.74 g, 7.52 mmol) was added to a solution of **2a** (1.00 g, 7.52 mmol) in *N*,*N-*dimethylformamide (10 mL), and the reaction mixture was stirred at room temperature for 16 h under N₂ atmosphere. The resulting reaction solution was recorded as solution A and set aside.

To a three-neck flask (50 mL) was added N hydroxysuccinimide (3.46 g, 30.08 mmol) and *N,N-*dimethylformamide (10 mL), and the solution was cooled to 0°C followed by dropwise addition of trifluoroacetic anhydride (6.31 g, 30.08 mmol). The resultant reaction mixture was stirred for 0.5 h, and then 2,4,6-trimethylpyridine (5.46 g, 45.10 mmol) was added. After stirring for additional 0.5 h, the reaction solution was added dropwise to the above solution A. The reaction mixture was stirred at room temperature for 16 h. 1N HCl_{(aq)} was added to the reaction mixture to adjust the pH to 2~3, the aqueous layer was extracted with dichloromethane (50 mL). The organic phase was washed with water for three times, dried over anhydrous sodium sulfate and then filtrated. The filtrate was concentrated and the residue was purified by flash column chromatography on silica gel (ethyl acetate: hexanes = 0-100%) to give **2b** (1.20 g) with 51% yield.

### Step 2

**1e** (0.54 g, 0.80 mmol) was added to a solution of **2b** (0.40 g, 1.29 mmol) in acetonitrile (8 mL) and water (16 mL), and the reaction mixture was stirred at room temperature for 16 h under N₂ atmosphere. The reaction mixture was concentrated and the residue was purified by prep-HPLC to give **2c** (0.30 g) with 61% yield.

### Step 3

To a single-neck flask (10 mL) was added **1n-P1** (50 mg, 0.09 mmol), **2c** (76 mg, 0.12 mmol), 1-(3-dimethylaminopropyl)-3-ethylcarbodiimide hydrochloride (31 mg, 0.16 mmol) and *N*,*N-*dimethylformamide (2 mL), followed by dropwise addition of 2,4,6-trimethylpyridine (60 mg, 0.49 mmol). The resulting mixture was stirred at room temperature for 3 h. The reaction solution was added dropwise to methyl *tert*-butyl ether (60 mL), during which a precipitate was formed. The suspension was then filtered, and the collected solid was redissolved in dichloromethane/methanol (V:V = 6:1, 30 mL). The obtained solution was concentrated *in vacuo* and the residue was purified by prep-HPLC to afford **II-1-a** (16 mg) with 17% yield.

MS-ESI calc. for [M+H]⁺ 1052, found: 1052.

¹H NMR (400 MHz, DMSO-*d*₆) δ 8.65 (t, *J=* 6.8 Hz, 1H), 8.61 (d, *J=* 9.2 Hz, 1H), 8.28 (t, *J* = 4.4 Hz, 1H), 8.14-8.06 (m, 2H), 8.06 (t, *J* = 5.6 Hz, 1H), 7.82 (d, *J* = 11.2 Hz, 1H), 7.79 (s, 1H), 7.28-7.13 (m, 5H), 6.99 (s, 2H), 5.92 (d, *J* = 16.8 Hz, 1H), 5.70-5,60 (m, 1H), 5.53 (d, *J* = 8.0 Hz, 1H), 5.48 (d, *J* = 4.8 Hz, 1H), 5.37 (d, *J* = 19.6 Hz, 1H), 4.68 (d, *J* = 6.4 Hz, 1H), 4.51-4.41 (m, 1H), 4.17-4.04 (m, 2H), 3.72-3.68 (m, 2H), 3.68-3.63 (m, 2H), 3.62-3.58 (m, 1H), 3.58-3.54 (m, 3H), 3.54-3.39 (m, 3H), 3.48-3.43 (m, 2H), 3.19-3.08 (m, 1H), 3.06-2.97 (m, 1H), 2.80-2.70 (m, 1H), 2.70-2.65 (m, 1H), 2.39 (s, 3H), 2.35-2.28 (m, 3H), 2.27-2.18 (m, 2H), 1.94-1.82 (m, 2H), 0.85 (t, *J* = 7.2 Hz, 3H).

### Example 1.3

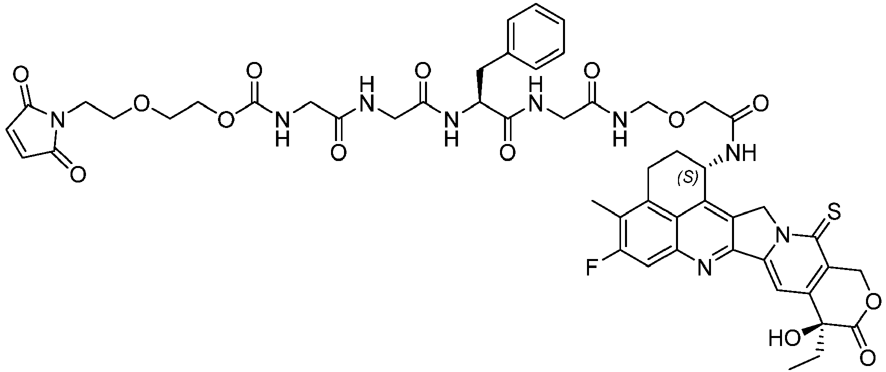

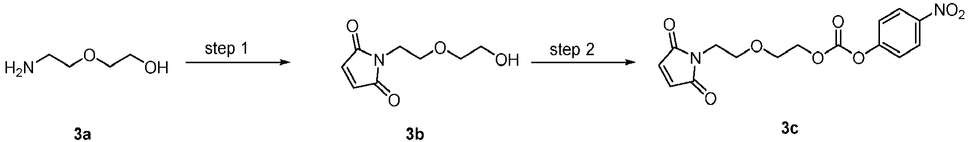

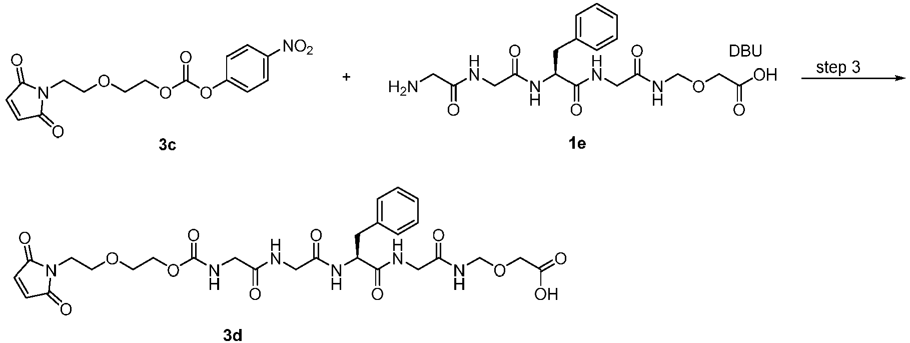

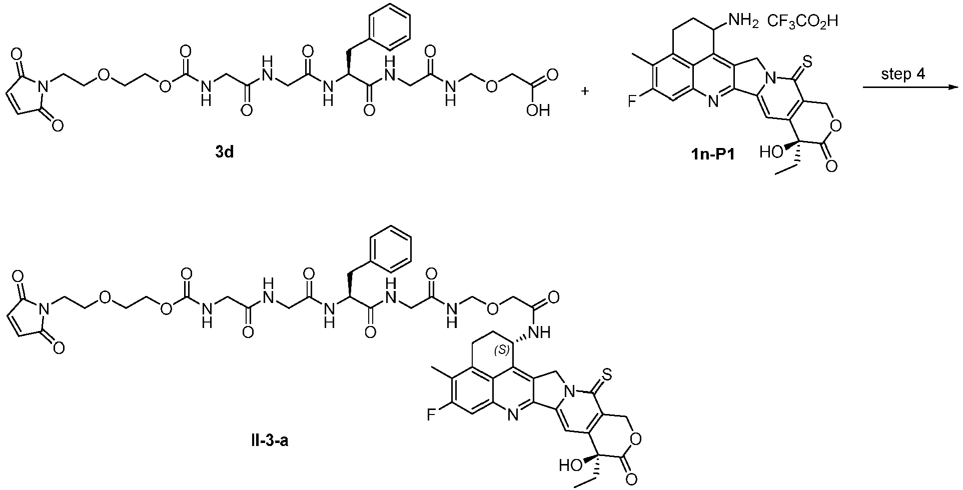

### Step 1

Maleic anhydride (9.32 g, 95.11 mmol) was added to a solution of **3a** (10.00 g, 95.11 mmol) in *N*,*N*-dimethylformamide (100 mL), and the reaction mixture was stirred at room temperature for 16 h under N₂ atmosphere. Water (250 mL) was added to the reaction mixture, and the aqueous phase was extracted with dichloromethane/isopropanol (v:v = 4:1, 200 mL x 3). The combined organic phases were concentrated *in vacuo* to give crude **3b** (12.00 g).

### Step 2

4,4'-Dinitrodiphenyl carbonate (16.40 g, 53.95 mmol) was added to a solution of crude **3b** (4.00 g, 31.70 mmol) in *N*,*N*-dimethylformamide (60 mL), the mixture was heated up to 30°C followed by the addition of *N*,*N*-diisopropylethylamine (4.18 g, 32.40 mmol). The reaction was then stirred for 2 h. The reaction was cooled to room temperature followed by the addition of water (600 mL), and the aqueous phase was extracted with dichloromethane (300 mL x 2). The combined organic phases were dried over anhydrous sodium sulfate and then filtrated. The filtrate was concentrated and the residue was purified by flash column chromatography on silica gel (ethyl acetate: hexanes = 0-100%) to give **3c** (2.40 g) with 13% yield over 2 steps.

### Step 3

**1e** (760 mg, 1.13 mmol) was added to a solution of **3c** (400 mg, 1.14 mmol) in acetonitrile (8 mL) and water (16 mL), and the reaction mixture was stirred at room temperature for 16 h under N₂ atmosphere. The reaction mixture was directly purified by prep-HPLC to give **3d** (109 mg) with 15% yield.

### Step 4

To a single-neck flask (10 mL) was added **1n-P1** (69 mg, 0.12 mmol), **3d** (110 mg, 0.17 mmol), 1-(3-dimethylaminopropyl)-3-ethylcarbodiimide hydrochloride (44 mg, 0.23 mmol) and *N*,*N-*dimethylformamide (2.5 mL), followed by dropwise addition of 2,4,6-trimethylpyridine (84 mg, 0.69 mmol). The resulting mixture was stirred at room temperature for 3 h. The reaction solution was added dropwise to methyl tert-butyl ether (75 mL), during which a precipitate was formed. The suspension was then filtered, and the collected solid was redissolved in dichloromethane/methanol (V:V = 6:1, 30 mL). The obtained solution was concentrated *in vacuo* and the residue was purified by prep-HPLC to afford **II-3-a** (45 mg) with 35% yield.

MS-ESI calc. for [M+H]⁺ 1068.4, found: 1068.9.

¹H NMR (400 MHz, DMSO-*d*₆) δ 8.68 (t, *J=* 6.8 Hz, 1H), 8.62 (d, *J=* 9.2 Hz, 1H), 8.31 (t, *J* = 5.6 Hz, 1H), 8.12 (d, *J* = 8.0 Hz, 1H), 7.99 (t, *J* = 5.6 Hz, 1H), 7.78 (s, 1H), 7.77 (d, *J=* 10.8 Hz, 1H), 7.38 (t, *J=* 6.0 Hz, 1H), 7.27-7.13 (m, 5H), 6.99 (s, 2H), 5.90 (d, *J*= 16.4 Hz, 1H), 5.70-5,61 (m, 1H), 5.51 (s, 1H), 5.46 (d, *J=* 4.4 Hz, 1H), 5.29 (d, *J=* 19.6 Hz, 1H), 4.67 (d, *J=* 6.4 Hz, 1H), 4.51-4.41 (m, 1H), 4.17-4.04 (m, 2H), 4.02-3.94 (m, 2H), 3.79-3.67 (m, 3H), 3.35-3.20 (m, 1H), 3.20-3.07 (m, 1H), 3.00 (dd, *J* = 14.0, 4.4 Hz, 1H), 2.78-2.65 (m, 1H), 2.37 (s, 3H), 2.25-2.15 (m, 2H), 1.92-1.82 (m, 2H), 0.85 (t, *J* = 7.2 Hz, 3H).

### Example 1.4

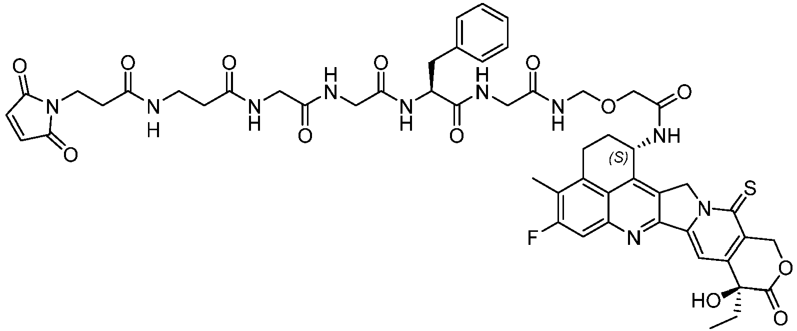

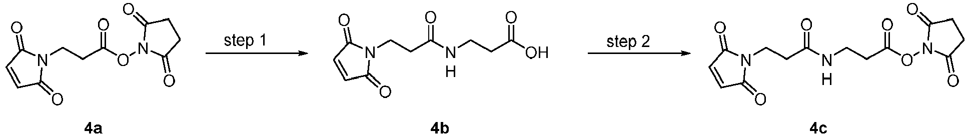

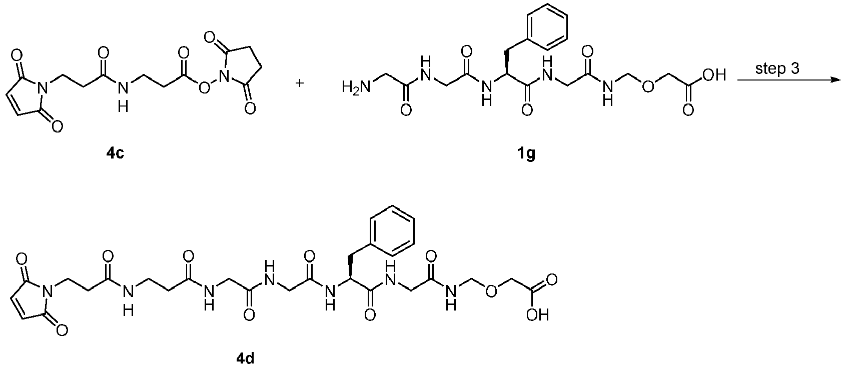

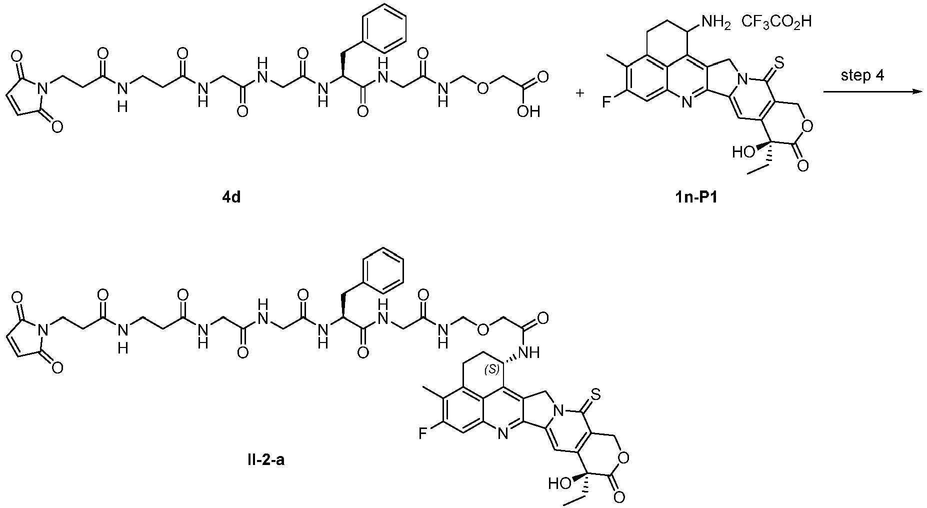

### Step 1

**4a** (2.00 g, 7.52 mmol) and *β*-alanine (0.67 g, 7.52 mmol) were dissolved in acetonitrile (20 mL), the mixture was cooled to 0°C followed by the addition of *N*,*N*-diisopropylethylamine (1.94 g, 15.03 mmol). The reaction solution was recovered to room temperature and was continuously stirred for 16 h. Water (100 mL) was added to the reaction mixture, and the aqueous phase was extracted dichloromethane/isopropanol (v:v = 4:1, 250 mL x 10). The combined organic phases were concentrated and the residue was purified by flash column chromatography on silica gel (methanol: dichloromethane = 0-100%) to give **4b** (2.00 g).

### Step 2

Dicyclohexylcarbodiimide (1.85 g, 9.00 mmol) was added to a solution of **4b** (1.80 g, 7.50 mmol) in *N*,*N*-diisopropylethylamine (8 mL), the reaction mixture was stirred for 30 min followed by the addition of *N*-hydroxysuccinimide (0.95 g, 8.25 mmol). The resulting mixture was stirred at room temperature for 16 h. The reaction mixture was filtered, and the filtrate was purified by prep-HPLC to afford **4c** (530 mg) with 21% yield.

### Step 3

**1e** (0.98 g, 1.50 mmol) was added to a solution of **4c** (0.50 g, 1.48 mmol) in acetonitrile (8 mL) and water (16 mL), and the reaction mixture was stirred at room temperature for 16 h under N₂ atmosphere. The reaction mixture was directly purified by prep-HPLC to give **4d** (42 mg) with 4% yield.

### Step 4

To a single-neck flask (10 mL) was added **1n-P1** (26 mg, 0.046 mmol), **4d** (42 mg, 0.065 mmol), 1-(3-dimethylaminopropyl)-3-ethylcarbodiimide hydrochloride (16 mg, 0.083 mmol) and *N*,*N-*dimethylformamide (1 mL), followed by dropwise addition of 2,4,6-trimethylpyridine (32 mg, 0.26 mmol), and the resulting mixture was stirred at room temperature for 3 h. The reaction solution was added dropwise to methyl tert-butyl ether (30 mL), during which a precipitate was formed. The suspension was then filtered, and the collected solid was redissolved in dichloromethane/methanol (V:V = 6:1, 30 mL). The obtained solution was concentrated *in vacuo* and the residue was purified by prep-HPLC to afford **II-2-a** (13 mg) with 26% yield.

MS-ESI calc. for [M+H]⁺ 1079.4, found: 1079.9.

¹H NMR (400 MHz, DMSO-*d*₆) δ 8.68 (t, *J* = 6.8 Hz, 1H), 8.63 (d, *J* = 8.8 Hz, 1H), 8.31 (t, *J* = 6.0 Hz, 1H), 8.18 (t, *J* = 7.2 Hz, 1H), 8.10 (d, *J* = 7.2 Hz, 1H), 8.03 (t, *J* = 5.6 Hz, 1H), 7.98 (t, *J* = 5.6 Hz, 1H), 7.82 (d, *J* = 10.8 Hz, 1H), 7.79 (s, 1H), 7.28-7.13 (m, 5H), 6.98 (s, 2H), 6.70 (s, 1H), 5.90 (d, *J=* 16.8 Hz, 1H), 5.70-5,60 (m, 1H), 5.56-5.45 (m, 2H), 5.36 (d, *J=* 19.6 Hz, 1H), 4.68 (d, *J=* 6.8 Hz, 1H), 4.53-4.42 (m, 1H), 4.18-4.04 (m, 2H), 3.78-3.64 (m, 5H), 3.62-3.52 (m, 4H), 3.22-3.14 (m, 1H), 3.06-2.97 (m, 1H), 2.80-2.70 (m, 1H), 2.68-2.65 (m, 1H), 2.39 (s, 3H), 2.34-2.16 (m, 6H), 1.92-1.82 (m, 2H), 0.85 (t, *J=* 7.2 Hz, 3H).

### Example 1.5

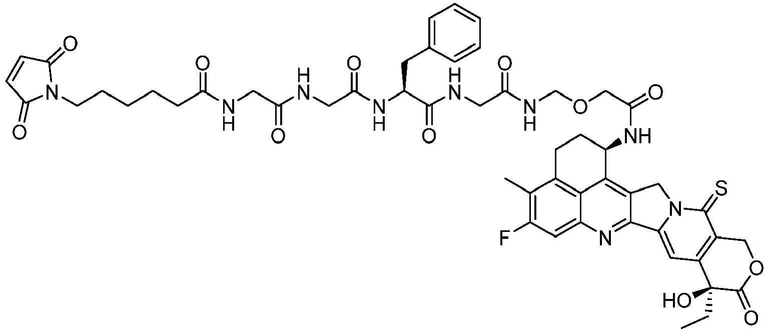

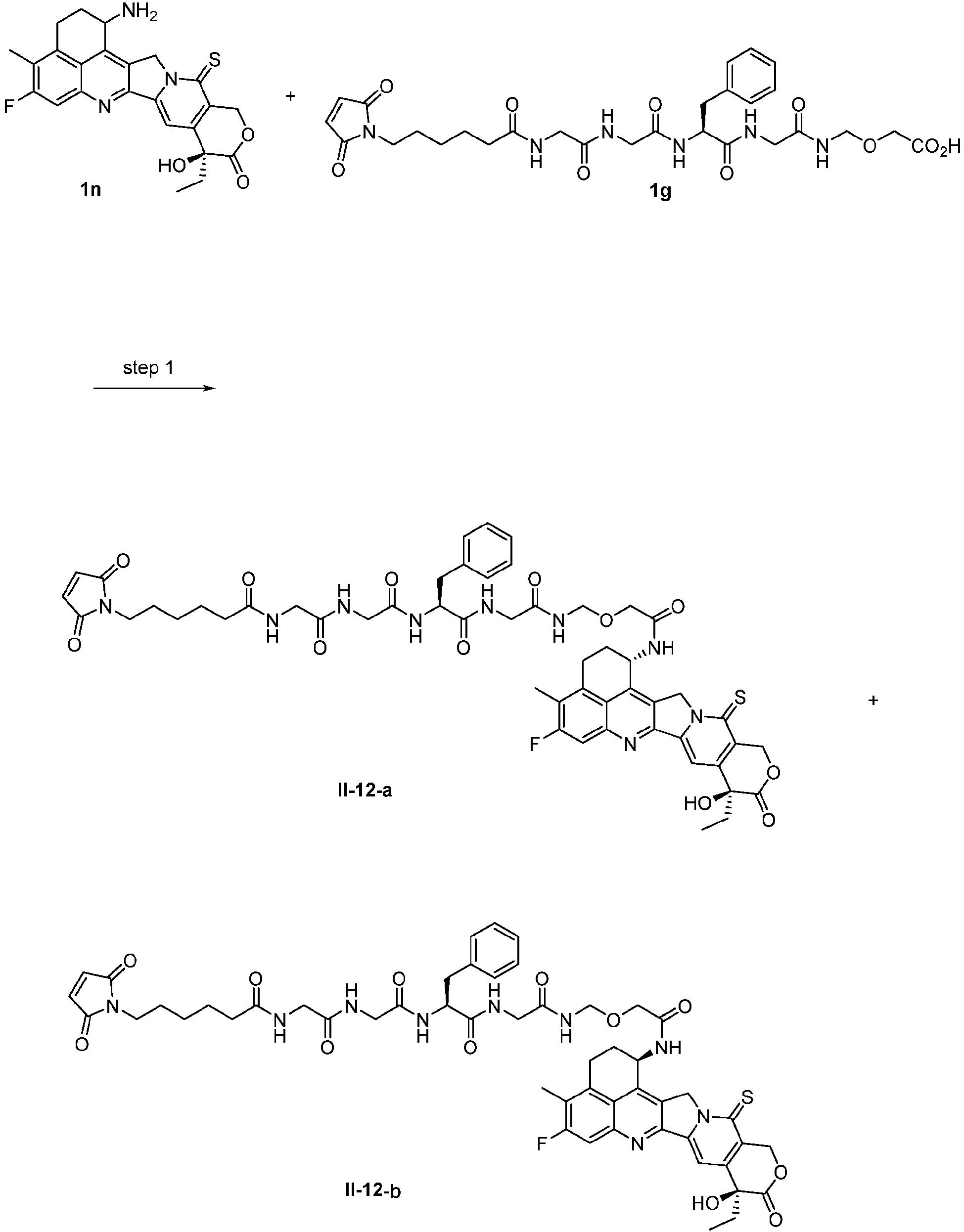

### Step 1

Under nitrogen protection and at 0°C, **1n** (24.00 mg, 0.053 mmol) and **1g** (32.78 mg, 0.053 mmol were dissolved in *N*,*N*-dimethylformamide (1.5 mL) followed by the addition of a solution of *N*,*N*-diisopropylethylamine (17.10 mg, 0.0133 mmol) in *N,N*-dimethylformamide (0.4 mL) and a solution of O-(7-Azabenzotriazol-1-yl)-*N*,*N*,*N*',*N*'-tetramethyluronium hexafluorophosphate (24.20 mg, 0.064 mmol) in *N,N*-dimethylformamide (0.6 mL), and the resulting reaction mixture was kept at 0°C and stirred for 1 h. The reaction mixture was purified by prep-HPLC to afford **II-12-a** (16 mg) and **II-12-b** (19 mg) with 64% combined yield.

### 11-12-b:

MS-ESI calc. for [M+H]⁺ 1050.4, found: 1050.3.

¹H NMR (400 MHz, DMSO-*d₆*) δ 8.71-8.61 (m, 2H), 8.31 (t, *J=* 4.8 Hz, 1H), 8.12 (d, *J* = 7.2 Hz, 1H), 8.07 (t, *J=* 6.0 Hz, 1H), 8.00 (t, *J=* 5.2 Hz, 1H), 7.82 (dd, *J* = 10.8, 2.0 Hz, 1H), 7.80 (s, 1H), 7.28-7.13 (m, 6H), 6.99 (s, 2H), 6.70 (br s, 1H), 5.92 (d, *J* = 16.8 Hz, 1H), 5.70-5,61 (m, 1H), 5.52 (d, *J* = 17.2 Hz, 2H), 5.37 (d, *J* = 19.6 Hz, 1H), 4.68 (d, *J* = 6.8 Hz, 2H), 4.51-4.41 (m, 1H), 4.18-4.04 (m, 2H), 3.77-3.50 (m, 7H), 3.32-3.20 (m, 1H), 3.20-3.08 (m, 1H), 3.07-2.96 (m, 1H), 2.82-2.71 (m, 1H), 2.39 (s, 3H), 2.26-2.15 (m, 2H), 2.09 (t, *J=* 7.6 Hz, 2H), 1.96-1.82 (m, 2H), 1.52-1.39 (m, 4H), 1.25-1.10 (m, 2H), 0.85 (t, *J=* 7.6 Hz, 3H).

### Example 1.6

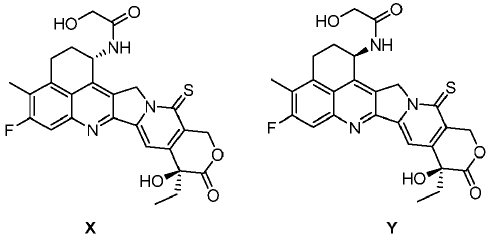

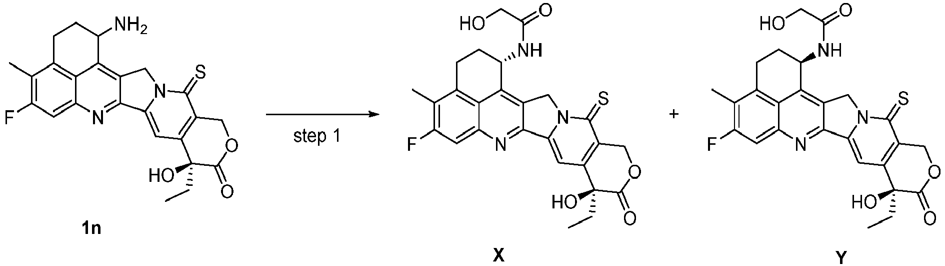

### Step 1

To a single-neck flask (25 mL) were added **1n** (20 mg, 0.04 mmol) and a solution of glycolic acid in *N*,*N*-dimethylformamide (2.00 mL, 0.032 mmol, 1.2 mg/mL), the mixture was cooled down to 0°C followed by addition of NN-dii sopropyl ethyl amine (11 mg, 0.08 mmol) and O-(7-azabenzotriazol-1-yl)-*N*,*N*,*N*',*N*'-tetramethyluronium hexafluorophosphate (16 mg, 0.04 mmol), the reaction mixture was stirred for 0.5 h. Another batch of solution of glycolic acid in *N*,*N-*dimethylformamide (1.2 mg/mL, 0.20 mL, 0.003 mmol) and O-(7-azabenzotriazol-1-yl)-N,N,N,N'-tetramethyluroniumhexafluorophosphate (1 mg) were added, the reaction mixture was stirred for additional 1 h. Ethyl acetate (80 mL) was added to the reaction mixture. The organic phase was washed by 0.5 N HCl_{(aq)} (5 mL x 1), saturated aq. sodium bicarbonate (10 mL x 2) and saturated brine (10 mL x 5) successively. The organic phase was dried over anhydrous sodium sulfate and filtered. The filtrate was concentrated *in vacuo* and the residue was purified by Prep-TLC (methanol: dichloromethane) to give **X** (2 mg) and **Y** (5 mg) with 34% overall yield.

### X:

MS-ESI calc. for [M+H]⁺ 510, found: 510.

¹H NMR (400 MHz, DMSO-*d*₆) δ 8.54 (d, *J =* 8.8 Hz, 1H), 7.82 (d, *J =* 10.8 Hz, 1H), 7.80 (s, 1H), 5.92 (d, *J =* 16.8 Hz, 1H), 5.69-5.60 (m, 1H), 5.53 (d, *J =* 20.4 Hz, 1H), 5.52 (d, *J=* 16.4 Hz, 1H), 5.35 (d, *J=* 20.0 Hz, 1H), 4.15-3.98 (m, 2H), 3.30-3.22 (m, 1H), 3.19-3.09 (m, 1H), 2.40 (s, 3H), 2.26-2.14 (m, 2H), 1.96-1.84 (m, 2H), 0.86 (t, *J* = 7.2 Hz, 3H).

### Y:

MS-ESI calc. for [M+H]⁺ 510, found: 510.

¹H NMR (400 MHz, DMSO-*d*₆) δ 8.54 (d, *J =* 9.2 Hz, 1H), 7.81 (d, *J =* 11.2 Hz, 1H), 7.80 (s, 1H), 6.69 (s, 1H), 5.92 (d, *J=* 16.8 Hz, 1H), 5.69-5.62 (m, 1H), 5.62-5.56 (m, 1H), 5.55-5.47 (m, 2H), 5.33 (d, *J=* 20.0 Hz, 1H), 4.16-3.98 (m, 2H), 3.30-3.20 (m, 1H), 3.16-3.07 (m, 1H), 2.39 (s, 3H), 2.26-2.15 (m, 2H), 1.95-1.85 (m, 2H), 0.86 (t, *J* = 7.2 Hz, 3H).

### Example 2

### Preparation method for antibody-drug conjugates

The antibody was dialyzed into 50 mM PB (pH 7.4±0.1) buffer to obtain the antibody intermediate. Take 20 mg of the antibody intermediate, add 0.2 mL of 10 mM diethylenetriaminepentaacetic acid (DTPA) mother liquor to a final concentration of DTPA of 1 mM; add 10 mM tris(2-carboxyethyl) phosphine hydrochloride (TCEP) mother liquor, with the molar ratio of reducing agent TCEP to antibody at (2.4-2.6)/1.0; add 50 mM PB (pH 7.4±0.1) buffer into the reaction system to make the final volume of 2 mL. After mixing thoroughly, it was placed in a constant temperature mixer with the reduction reaction temperature being set at 25±2°C and the rotation speed being set at 400 rpm, and the reduction reaction time is 120-150 minutes. After the reduction, an appropriate amount of 5 mM linker-payload mother liquor was sequentially added to each reaction system in an ice water bath with the molar ratio of linker-payload to antibody being (8.0-10.0)/1.0. After mixing thoroughly, it was placed in a constant temperature mixer with the coupling reaction temperature being set at 25±2°C and the rotating speed being set at 400rpm, and the coupling reaction time is 60-90 minutes. After the coupling, the ADC sample was dialyzed into the dialysate (20 mM His/His-HCl, pH 6.0±0.1) using an ultrafiltration centrifuge tube to obtain the ADC stock solution, which was aliquoted and stored at -80°C.

| Linker-payload compounds | | antibody | Antibody-drug conjugate (ADC) | |
|---|---|---|---|---|
| | II-1-a (example 1.2) | Trastuzumab | | Trastuzumab ADC I-1-a |
| | II-2-a (example 1.4) | Trastuzumab | | Trastuzumab ADC I-2-a |
| | II-3-a (example 1.3) | Trastuzumab | | Trastuzumab ADC I-3-a |
| | II-12-a (example 1.1) | Trastuzumab | | Trastuzumab ADC I-12-a |
| | II-12-b (example 1.5) | Trastuzumab | | Trastuzumab ADC I-12-b |
| | II-1-a (example 1.2) | TROP2 antibody | | TROP2 antibody ADC I-I-a |

Exemplary TROP2 antibody sequences may comprise LCDR1-3 in the light chain variable region VL and HCDR1-3 in the heavy chain variable region VH, wherein said VH may comprise the amino acid sequence shown in SEQ ID NO: 17 and said VL may comprise the amino acid sequence shown in SEQ ID NO: 18.

### Example 3

### In Vitro Anti-Proliferation Test in Human-derived Tumor Cells

Human breast cancer cells SK-BR-3 or gastric cancer cells NCI-N87 in logarithmic growth phase were taken, resuspended using complete medium and adjusted to the appropriate concentration, and added into 96-well cell culture plates at 100 µL/well. The cell culture plates were placed in a CO₂ incubator (37°C, 5% CO₂) overnight. Cell suspension was added to 96 wells of one of the plates and tested by CTG (CellTiter Glo assay kit, Promega, G7558) before adding samples as G₀ value.

A linker-payload of the present application is obtained with reference to the method of example 1 of the present application, and an antibody-drug coupling compound (ADC) of the present application is obtained with reference to the method of example 2 of the present application. For example, the linker-payload compound II-1 can be obtained with reference to the method of example 1 of the present application, and coupled with any antibody (e.g., a HER2 antibody) with reference to the method of example 2 of the present application to obtain the ADC I-1. Samples of the reference group can be prepared with reference to the coupling with any antibody (e.g., a HER2 antibody) with reference to example 2 of the present application.

The gradient-diluted working solution of the tested ADC was added to cell culture plates using HP D300 in duplicate, starting at a final ADC concentration of 1 µM with 4-fold gradient dilution for a total of 9 concentrations. After incubated in a CO₂ incubator (37°C, 5% CO₂) for 120 h, the cell culture plates were removed and equilibrated to room temperature, and the luminescence readings were detected using a CellTiter Glo assay kit (Promega, G7558), a multifunctional microplate reader (PerkinElmer, EnVision), and the inhibition curves were plotted and IC₅₀ and GI₅₀ values were calculated using XLFit.

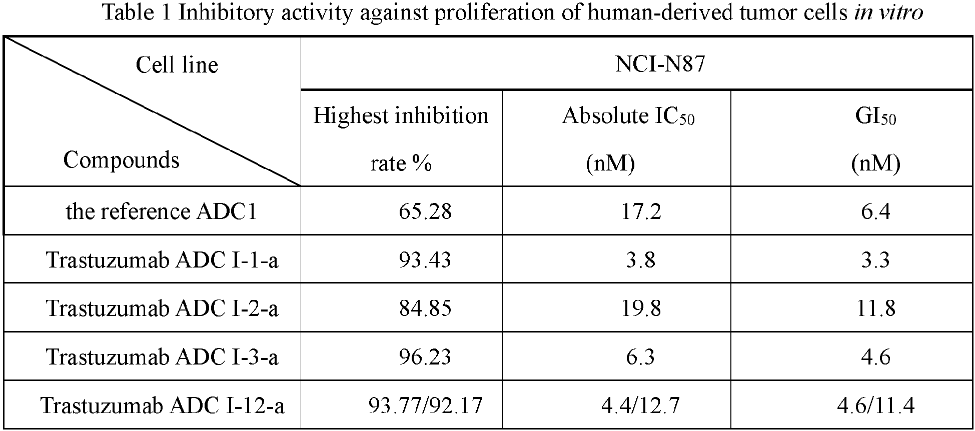

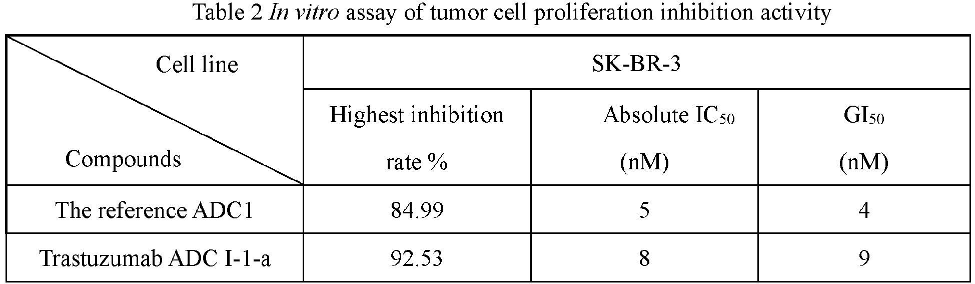

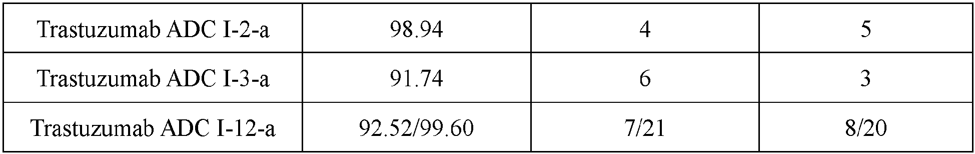

As shown in Tables 1 and 2, according to the results of the activity tests, the ADCs prepared from the compounds of the present application all exhibit certain anti-tumor activity and are superior to the reference ADC1. The structure of the reference ADC1 can be shown in the following formula:

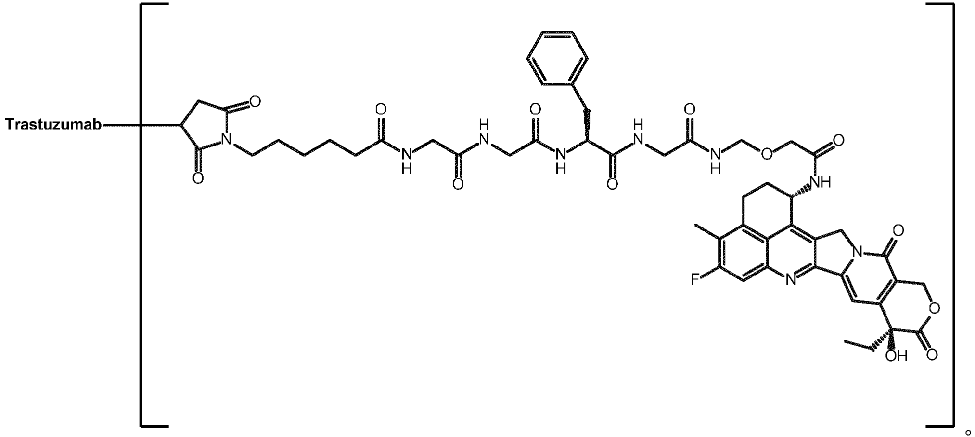

### In vitro anti-proliferation assay in human-derived tumor cells

Human-derived tumor cells in logarithmic growth phase were harvested, digested, resuspended using fresh complete medium and adjusted them to the appropriate concentration, and then were added to 96-well cell culture plates at 100 µL/well. The cell culture plates were placed in a CO₂ incubator (37°C, 5% CO₂) overnight. In the next day, different concentrations of ADC samples to be tested (highest final concentration 1 µM, 4-fold gradient dilution) or buffer control were added to the corresponding wells of the cell culture plates, and continued to be incubated in a CO₂ incubator for 120 h. The test plates were equilibrated to room temperature, and the luminescence readings were detected by using a CellTiter Glo assay kit (Promega, G7558), and multifunctional microplate reader (Enspire 2300, PerkinElmer). The cell inhibitory rate was calculated according to the following formula: inhibition rate (%) = (1-(RLU_{ADC} - RLU_{blank})/(RLU_{buffer} - RLU_{blank})) x 100%. XLFit was utilized to plot the inhibition curve and calculate the IC₅₀ value (see Table 3). The experimental results showed that the inhibitory activity of TROP2 antibody ADC I-I-a was significantly better than that of reference ADC2 against several tumor cell lines with different target expression levels (see Table 3).

**Table 3. Inhibitory activity against proliferation of human-derived tumor cells in vitro**

| Cell line | TROP2 antibody ADC I-I-a | The reference ADC2 |
|---|---|---|
| | IC₅₀ (nM) | IC₅₀ (nM) |
| Fadu | 0.3 | 2.4 |
| BxPC-3 | 0.8 | 2.0 |
| Calu-3 | 2.3 | 27.5 |
| MDA-MB-468 | 3.9 | 82.0 |
| COLO205 | 6.2 | 651.4 |
| NCI-N87 | 8.9 | 806.8 |
| Calu-6 | 10.8 | 675.1 |
| MDA-MB-231 | 43.7 | >1000 |
| MCF-7 | 107.1 | >1000 |

As shown in Table 3, based on the results of the activity test, the ADCs prepared from the compounds of the present application exhibited excellent anti-tumor activities and were superior to the reference ADC2.The structure of the reference ADC2 can be shown in the following formula:

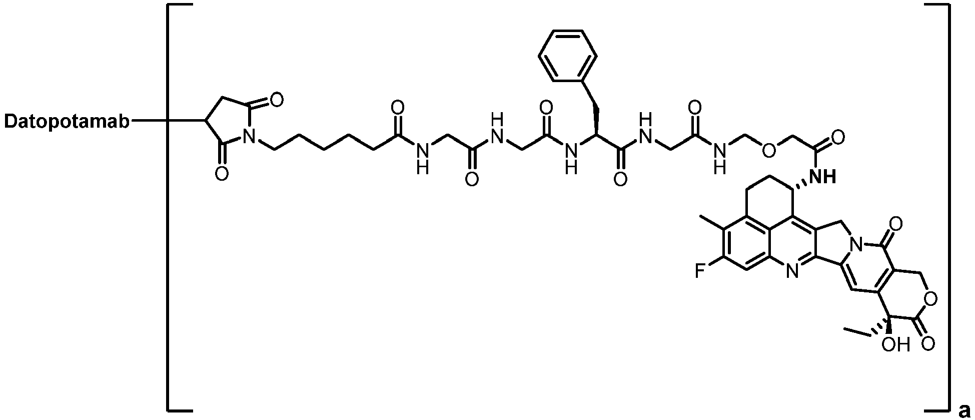

### Example 4

### In vivo anti-tumor efficacy assay

The gastric cancer cells NCI-N87 with high target expression level in logarithmic growth phase were harvested, counted, adjusted to the appropriate concentration, resuspended in a 1:1 mixture of serum-free medium and Matrigel (Corning 356234) on ice, and inoculated to BALB/c nude mice subcutaneously on the right back of the mouse, with 200 µL each. After the tumors had grown to a measurable range, the long and short diameters of each tumor were measured using vernier calipers, and the tumor volume was calculated according to the following formula: V = (a × b²)/2, where "a' represents the long diameter of the tumor and "b" represents the short diameter of the tumor.

When the average tumor volume grew to 100-200 mm³, the animals were grouped according to tumor volume by the random block method. The tumor-bearing nude mice were injected with a solvent control (normal saline) or different doses of ADC (3 mg/kg or 10 mL/kg) *via* the tail vein for once. The tumor length and short diameter were measured twice a week, and the animal weight was recorded. The tumor volume of each group was calculated, and the tumor growth inhibition rate TGI was calculated according to the following formula: TGI=100%×[1-(TVt_{T}-TV0_{T})/(TVt_{C}-TV0_{C})]. Among them (TVt_{T} represents the tumor volume of the administration group on the measurement day, TV0_{T} represents the tumor volume of the drug administration group at the time of grouping; TVtc represents the tumor volume of the solvent control group on the measurement day, and TV0_{C} represents the tumor volume of the solvent control group at the time of grouping (see Table 4).

**Table 4. Growth inhibition rate of NCI-N87 subcutaneous xenograft nude mice model**

| Compounds | Dosage | Tumor Growth Inhibition |
|---|---|---|
| | | TGI (%) |
| The reference ADC1 | 3 mg/kg | 56% |
| The reference ADC1 | 10 mg/kg | 108% |
| Trastuzumab ADC I-1-a | 3 mg/kg | 71% |
| Trastuzumab ADC I-1-a | 10 mg/kg | 111% |
| Trastuzumab ADC I-3-a | 3 mg/kg | 67% |
| Trastuzumab ADC I-2-a | 3 mg/kg | 80% |

From the results of the tests, all ADCs prepared from the compounds of the present application showed a certain *in vivo* anti-tumor activity, and could show significantly stronger anti-tumor activity compared to the reference ADC1. The tumor-bearing nude mice tolerated the above drugs well and no symptoms such as weight loss occurred.

### In vivo anti-tumor efficacy assay

Human lung cancer cells Calu-6 with low expression of the target in the logarithmic growth phase were taken, and the cells were counted and resuspended in EMEM culture medium, and the cell concentration was adjusted to 5×10⁷ cells/mL and placed on ice before use. Cells were injected subcutaneously into female BALB/c nude mice using a syringe, and each animal was inoculated with 100 µL (5×10⁶ cells/animal) to establish a Calu-6 nude mouse xenograft tumor model. After the tumors had grown to a measurable range, the long and short diameters of each tumor were measured using vernier calipers, and the tumor volume was calculated according to the following formula: V = (a × b²)/2, where a represents the long diameter of the tumor and b represents the short diameter of the tumor.

When the average tumor volume grew to about 150 mm³, the animals are randomized into different groups according to tumor volume and weight. The tumor-bearing nude mice were injected with a solvent control (normal saline) or different doses of ADC (3 mg/kg or 10 mL/kg) *via* the tail vein, once a week for total 3 times. Tumor long and short diameters were measured twice a week and animal weights were recorded. The tumor volume of each group was calculated and the tumor growth inhibition rate TGI was calculated according to the following formula: TGI = 100%×[1-(TVt_{T}-TV0_{T})/(TVt_{C}-TV0_{C})]. Among them (TVt_{T} represents the tumor volume of the administration group on the measurement day, TV0_{T} represents the tumor volume of the drug administration group at the time of grouping; TVtc represents the tumor volume of the solvent control group on the measurement day, and TV0_{C} represents the tumor volume of the solvent control group at the time of grouping. The tumor growth inhibition rate at the end of the experiment is shown in Table 5.

The experiment results showed that the TROP2 antibody ADC I-I-a inhibited the growth of Calu-6 subcutaneous xenograft tumor at both 3 mg/kg and 10 mg/kg doses, and all the inhibitory effects were superior to the reference ADC2 dosed at 10 mg/kg.

**Table 5. Growth inhibition rate of Calu-6 subcutaneous xenograft nude mice model**

| Compounds | TROP2 antibody ADC I-I-a | | The reference ADC2 |
|---|---|---|---|
| dosage | (3 mg/kg) | (10 mg/kg) | (10 mg/kg) |
| Tumor Growth Inhibition TGI (%) | 33.3% | 78.0% | 21.8% |

### Example 5

### Cell proliferation inhibition assays

KPL-4 Cells in the logarithmic phase of growth were collected and re-suspended with fresh RPMI1640 complete cell culture medium, and adjusted to 2×10⁴ cells/mL after cell counting. Cells were seeded into 96-well cell culture plates at 100 µL/well, and placed in a CO₂ incubator (37°C, 5% CO₂) overnight. In the next day, one of the cell culture plates were taken out, balanced to room temperature, and CellTiter-Glo reagent (Promega, USA), which was pre-balanced to room temperature and evenly mixed, was added into the plate at 100 µl/well. The plate was kept in dark for 30 min, and then luminescence value was read with a microplate reader (denoted as G0 value). The other cell culture plates were taken out and added with different concentrations of test compounds or DMSO (final concentration 0.5%) in corresponding wells. After incubated for 72 h in a CO₂ incubator, the cell culture plates were balanced to room temperature and tested for cell viabilities using CellTiter-Glo reagent as described above (denoted as G3 value).

The cell proliferation rates were calculated using the following formula: Cell proliferation rate (%) = (mean of G_{3compound} - mean of G₀) / (mean of G_{3DMSO} - mean of G₀) × 100. Inhibition curves were fitted with GraphPad Prism and GI₅₀ values were obtained (shown in the table below).

| Compounds | GI₅₀ (nM) |
|---|---|
| X (example 1.6) | 13.9 |
| Y (example 1.6) | 10.3 |
| DXd | 72 |

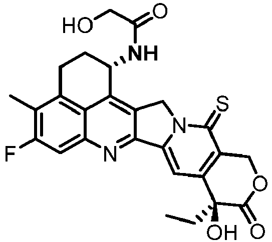

Compound X (example 1.6); 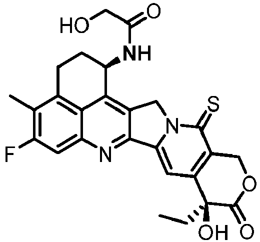 Compound Y (example 1.6);

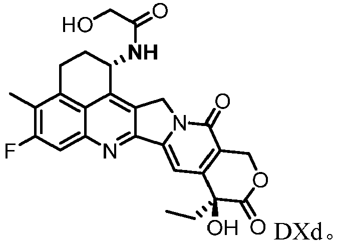

The foregoing detailed description is provided by way of explanation and example and is not intended to limit the scope of the appended claims. Multiple variations of the embodiments presently enumerated in this application will be apparent to one of ordinary skill in the art and are retained within the scope of the appended claims and equivalent embodiments thereof.

## Claims

1. A ligand conjugate, or a tautomer, a mesomer, a racemate, an enantiomer, a diastereomer, or a mixture thereof, or a pharmaceutically acceptable salt or hydrate thereof, wherein the ligand conjugate comprises the structure shown in formula (I): 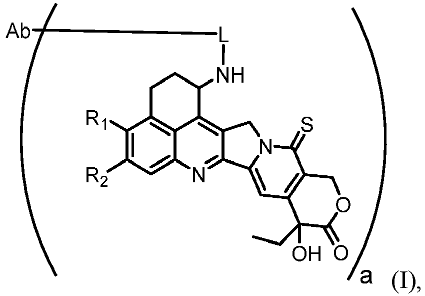
wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
L is an optionally substituted linker,
Ab is a ligand, "a" is a number greater than 0, and "a" is a decimal or integer.

2. A ligand conjugate, or a tautomer, a mesomer, a racemate, an enantiomer, a diastereomer, or a mixture thereof, or a pharmaceutically acceptable salt or hydrate thereof, wherein the ligand conjugate comprises the structure shown in formula (I-a): 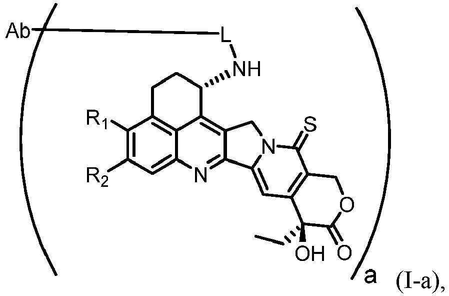
wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
L is an optionally substituted linker,
Ab is a ligand, "a" is a number greater than 0, and "a" is a decimal or integer.

3. A ligand conjugate, or a tautomer, a mesomer, a racemate, an enantiomer, a diastereomer, or a mixture thereof, or a pharmaceutically acceptable salt or hydrate thereof, wherein the ligand conjugate comprises the structure shown in formula (I-b): 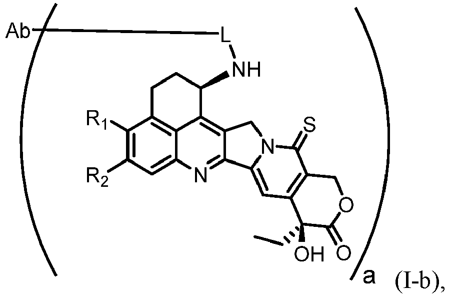
wherein, R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
L is an optionally substituted linker,
Ab is a ligand, "a" is a number greater than 0, and "a" is a decimal or integer.

4. The ligand conjugate according to any one of claims 1-3, or a tautomer, a mesomer, a racemate, an enantiomer, a diastereomer, or a mixture thereof, or a pharmaceutically acceptable salt or hydrate thereof,
wherein L is linker - L₁-L₂-L₃-L₄-L₅-.

5. The ligand conjugate according to claim 4, or a tautomer, a mesomer, a racemate, an enantiomer, a diastereomer, or a mixture thereof, or a pharmaceutically acceptable salt or hydrate thereof,
wherein L₁ is optionally substituted 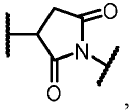 and L₁ is directly linked to Ab.

6. The ligand conjugate according to any one of claims 4-5, or a tautomer, a mesomer, a racemate, an enantiomer, a diastereomer, or a mixture thereof, or a pharmaceutically acceptable salt or hydrate thereof,
wherein L₂ is selected from the group consisting of optionally substituted -(CH₂) ₘ₁-X₁-(CH₂)ₘ₂-C(O)-, optionally substituted -(CH₂CH₂O)ₙ-C(O)-, and optionally substituted -(CH₂)ₚ-C(O)-,
X₁ is selected from the group consisting of -O-, optionally substituted -C(O)-NH-, optionally substituted aryl, optionally substituted heteroaryl, optionally substituted aliphatic heterocyclyl, and optionally substituted aliphatic cyclyl,
wherein, m1, m2, and n are each independently selected from integers of at least 0, and p is an integer selected from 2 to 8.

7. The ligand conjugate according to claim 6, or a tautomer, a mesomer, a racemate, an enantiomer, a diastereomer, or a mixture thereof, or a pharmaceutically acceptable salt or hydrate thereof,
wherein L₂ is optionally substituted -(CH₂CH₂O)ₙ-C(O)-.

8. The ligand conjugate according to any one of claims 6-7, or a tautomer, a mesomer, a racemate, an enantiomer, a diastereomer, or a mixture thereof, or a pharmaceutically acceptable salt or hydrate thereof,
wherein, n is 2.

9. The ligand conjugate according to claim 6, or a tautomer, a mesomer, a racemate, an enantiomer, and a diastereomer, or a mixture thereof, or a pharmaceutically acceptable salt or hydrate thereof,
wherein L₂ is optionally substituted -(CH₂)ₘ₁-X₁-(CH₂) ₘ₂-C(O)-.

10. The ligand conjugate according to claim 9, or a tautomer, a mesomer, a racemate, an enantiomer, a diastereomer, or a mixture thereof, or a pharmaceutically acceptable salt or hydrate thereof,
wherein X₁ is selected from the group consisting of -O- and optionally substituted -C(O)-NH-.

11. The ligand conjugate according to any one of claims 9-10, or a tautomer, a mesomer, a racemate, an enantiomer, a diastereomer, or a mixture thereof, or a pharmaceutically acceptable salt or hydrate thereof,
wherein, m1 is 2.

12. The ligand conjugate according to any one of claims 9-11, or a tautomer, a mesomer, a racemate, an enantiomer, a diastereomer, or a mixture thereof, or a pharmaceutically acceptable salt or hydrate thereof,
wherein, m2 is 2.

13. The ligand conjugate according to claim 9, or a tautomer, a mesomer, a racemate, an enantiomer, a diastereomer, or a mixture thereof, or a pharmaceutically acceptable salt or hydrate thereof,
wherein X₁ is selected from the group consisting of optionally substituted aryl and optionally substituted heteroaryl.

14. The ligand conjugate according to claim 13, or a tautomer, a mesomer, a racemate, an enantiomer, a diastereomer, or a mixture thereof, or a pharmaceutically acceptable salt or hydrate thereof,
wherein X₁ is optionally substituted phenyl.

15. The ligand conjugate according to claim 13, or a tautomer, a mesomer, a racemate, an enantiomer, a diastereomer, or a mixture thereof, or a pharmaceutically acceptable salt or hydrate thereof,
wherein X₁ is optionally substituted pyridinyl.

16. The ligand conjugate according to any one of claims 13-15, or a tautomer, a mesomer, a racemate, an enantiomer, a diastereomer, or a mixture thereof, or a pharmaceutically acceptable salt or hydrate thereof,
wherein, m1 is 0.

17. The ligand conjugate according to any one of claims 13-16, or a tautomer, a mesomer, a racemate, an enantiomer, a diastereomer, or a mixture thereof, or a pharmaceutically acceptable salt or hydrate thereof,
wherein, m2 is 1.

18. The ligand conjugate according to claim 9, or a tautomer, a mesomer, a racemate, an enantiomer, a diastereomer, or a mixture thereof, or a pharmaceutically acceptable salt or hydrate thereof,
wherein X₁ is optionally substituted

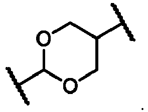

19. The ligand conjugate according to claim 9, or a tautomer, a mesomer, a racemate, an enantiomer, a diastereomer, or a mixture thereof, or a pharmaceutically acceptable salt or hydrate thereof,
wherein X₁ is optionally substituted cyclohexyl.

20. The ligand conjugate according to any one of claims 18-19, or a tautomer, a mesomer, a racemate, an enantiomer, a diastereomer, or a mixture thereof, or a pharmaceutically acceptable salt or hydrate thereof,
wherein, m1 is 1.

21. The ligand conjugate according to any one of claims 18-20, or a tautomer, a mesomer, a racemate, an enantiomer, a diastereomer, or a mixture thereof, or a pharmaceutically acceptable salt or hydrate thereof,
wherein, m2 is 0.

22. The ligand conjugate according to claim 6, or a tautomer, a mesomer, a racemate, an enantiomer, a diastereomer, or a mixture thereof, or a pharmaceutically acceptable salt or hydrate thereof,
wherein L₂ is optionally substituted -(CH₂)ₚ-C(O)-.

23. The ligand conjugate according to claim 22, or a tautomer, a mesomer, a racemate, an enantiomer, a diastereomer, or a mixture thereof, or a pharmaceutically acceptable salt or hydrate thereof,
wherein, p is 5.

24. The ligand conjugate according to any one of claims 4-23, or a tautomer, a mesomer, a racemate, an enantiomer, a diastereomer, or a mixture thereof, or a pharmaceutically acceptable salt or hydrate thereof,
wherein L₂ is a structure selected from the group consisting of optionally substituted 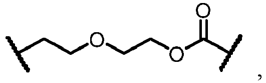 optionally substituted 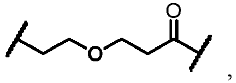 optionally substituted 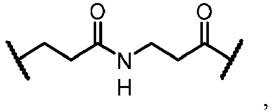 optionally substituted 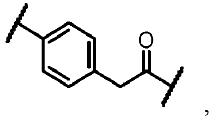 optionally substituted 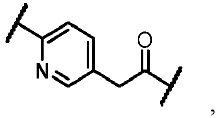 optionally substituted 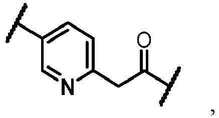 optionally substituted 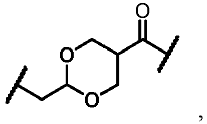 optionally substituted 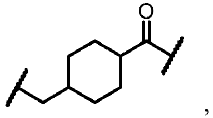 and optionally substituted

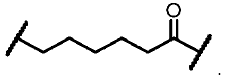

25. The ligand conjugate according to any one of claims 4-24, or a tautomer, a mesomer, a racemate, an enantiomer, a diastereomer, or a mixture thereof, or a pharmaceutically acceptable salt or hydrate thereof,
wherein L₃ is a peptide residue.

26. The ligand conjugate according to any one of claims 4-25, or a tautomer, a mesomer, a racemate, an enantiomer, a diastereomer, or a mixture thereof, or a pharmaceutically acceptable salt or hydrate thereof,
wherein L₃ is a peptide residue is composed of amino acids selected from the group consisting of phenylalanine, isoleucine, leucine, tryptophan, valine, methionine, tyrosine, alanine, threonine, histidine, serine, glutamine, arginine, lysine, asparagine, glutamate, proline, citrulline, aspartate, and glycine.

27. The ligand conjugate according to any one of claims 4-26, or a tautomer, a mesomer, a racemate, an enantiomer, a diastereomer, or a mixture thereof, or a pharmaceutically acceptable salt or hydrate thereof,
wherein L₃ is a peptide residue is composed of amino acids selected from the group consisting of glycine, phenylalanine, valine, and citrulline.

28. The ligand conjugate according to any one of claims 4-27, or a tautomer, a mesomer, a racemate, an enantiomer, a diastereomer, or a mixture thereof, or a pharmaceutically acceptable salt or hydrate thereof,
wherein L₃ is a peptide residue selected from the group consisting of -glycine-phenylalanine-glycine- (-Gly-Phe-Gly-), -glycine-glycine-phenylalanine-glycine- (-Gly-Gly-Phe-Gly-), and - valine-citrulline- (-Val-Cit-).

29. The ligand conjugate according to any one of claims 4-28, or a tautomer, a mesomer, a racemate, an enantiomer, a diastereomer, or a mixture thereof, or a pharmaceutically acceptable salt or hydrate thereof,
wherein L₃ is a structure selected from the group consisting of

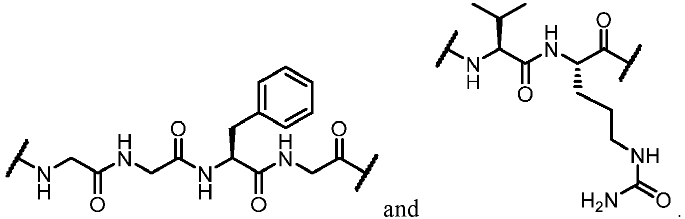

30. The ligand conjugate according to any one of claims 4-29, or a tautomer, a mesomer, a racemate, an enantiomer, a diastereomer, or a mixture thereof, or a pharmaceutically acceptable salt or hydrate thereof,
wherein L₄ is optionally substituted -L₄ₐ-NR₄-CH₂-L_{4b}-,
L₄ₐ is absent or L₄ₐ is optionally substituted 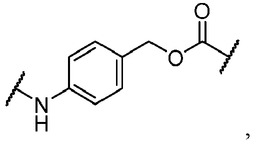
L_{4b} is absent or L_{4b} is optionally substituted 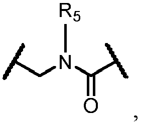
R₄ and R₅ are each independently selected from the group consisting of hydrogen and optionally substituted alkyl.

31. The ligand conjugate according to claim 30, or a tautomer, a mesomer, a racemate, an enantiomer, a diastereomer, or a mixture thereof, or a pharmaceutically acceptable salt or hydrate thereof,
L₄ is optionally substituted

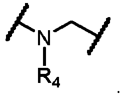

32. The ligand conjugate according to claim 30, or a tautomer, a mesomer, a racemate, an enantiomer, a diastereomer, or a mixture thereof, or a pharmaceutically acceptable salt or hydrate thereof,
wherein L₄ is optionally substituted

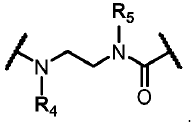

33. The ligand conjugate according to claim 30, or a tautomer, a mesomer, a racemate, an enantiomer, a diastereomer, or a mixture thereof, or a pharmaceutically acceptable salt or hydrate thereof,
wherein L₄ is optionally substituted

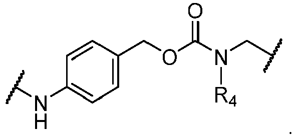

34. The ligand conjugate according to claim 30, or a tautomer, a mesomer, a racemate, an enantiomer, a diastereomer, or a mixture thereof, or a pharmaceutically acceptable salt or hydrate thereof,
wherein L₄ is optionally substituted

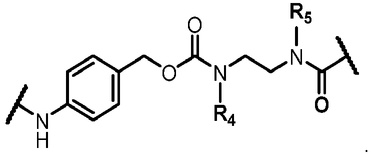

35. The ligand conjugate according to any one of claims 30-34, or a tautomer, a mesomer, a racemate, an enantiomer, a diastereomer, or a mixture thereof, or a pharmaceutically acceptable salt or hydrate thereof,
wherein R₄ is selected from the group consisting of hydrogen and optionally substituted methyl.

36. The ligand conjugate according to any one of claims 30-35, or a tautomer, a mesomer, a racemate, an enantiomer, a diastereomer, or a mixture thereof, or a pharmaceutically acceptable salt or hydrate thereof,
wherein R₅ is selected from the group consisting of hydrogen and optionally substituted methyl.

37. The ligand conjugate according to any one of claims 4-36, or a tautomer, a mesomer, a racemate, an enantiomer, a diastereomer, or a mixture thereof, or a pharmaceutically acceptable salt or hydrate thereof,
wherein L₄ is a structure selected from the group consisting of optionally substituted 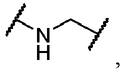 optionally substituted 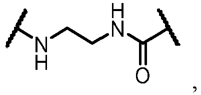 optionally substituted 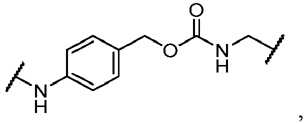 optionally substituted 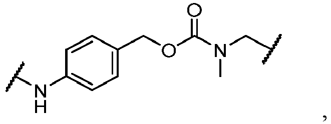 and optionally substituted

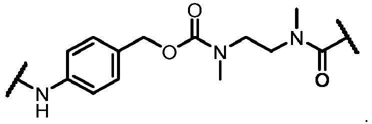

38. The ligand conjugate according to any one of claims 4-37, or a tautomer, a mesomer, a racemate, an enantiomer, a diastereomer, or a mixture thereof, or a pharmaceutically acceptable salt or hydrate thereof,
wherein L₅ is optionally substituted 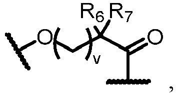 and L₅ is directly linked to the following structure: 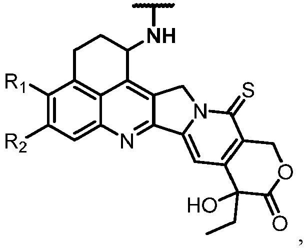
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl, or R₆ and R ₇ together with the atom to which they are attached form an optionally substituted cycloalkyl,
wherein, v is a number that is at least 0.

39. The ligand conjugate according to any one of claims 4-38, or a tautomer, a mesomer, a racemate, an enantiomer, a diastereomer, or a mixture thereof, or a pharmaceutically acceptable salt or hydrate thereof,
wherein L₅ is optionally substituted 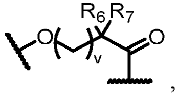 and L₅ is directly linked to the following structure: 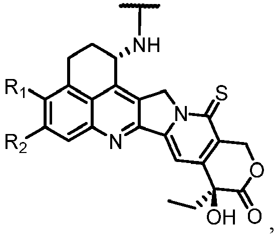
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl, or R₆ and R₇ together with the atom to which they are attached form an optionally substituted cycloalkyl,
wherein, v is a number that is at least 0.

40. The ligand conjugate according to any one of claims 4-39, or a tautomer, a mesomer, a racemate, an enantiomer, a diastereomer, or a mixture thereof, or a pharmaceutically acceptable salt or hydrate thereof,
wherein L₅ is optionally substituted 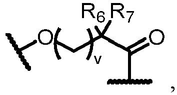 and L₅ is directly linked to the following structure: 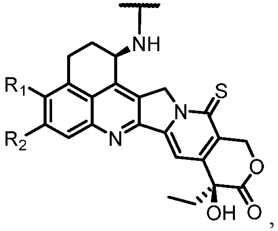
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl, or R₆ and R₇ together with the atom to which they are attached form an optionally substituted cycloalkyl,
wherein, v is a number that is at least 0.

41. The ligand conjugate according to any one of claims 38-40, or a tautomer, a mesomer, a racemate, an enantiomer, a diastereomer, or a mixture thereof, or a pharmaceutically acceptable salt or hydrate thereof,
wherein R₆ is hydrogen.

42. The ligand conjugate according to any one of claims 38-40, or a tautomer, a mesomer, a racemate, an enantiomer, a diastereomer, or a mixture thereof, or a pharmaceutically acceptable salt or hydrate thereof,
wherein R₆ is optionally substituted methyl.

43. The ligand conjugate according to claim 42, or a tautomer, a mesomer, a racemate, an enantiomer, a diastereomer, or a mixture thereof, or a pharmaceutically acceptable salt or hydrate thereof,
wherein R₆ is fluorine substituted methyl.

44. The ligand conjugate according to any one of claims 42-43, or a tautomer, a mesomer, a racemate, an enantiomer, a diastereomer, or a mixture thereof, or a pharmaceutically acceptable salt or hydrate thereof,
wherein R₆ is trifluoromethyl.

45. The ligand conjugate according to any one of claims 38-40, or a tautomer, a mesomer, a racemate, an enantiomer, a diastereomer, or a mixture thereof, or a pharmaceutically acceptable salt or hydrate thereof,
wherein R₆ is optionally substituted cycloalkyl.

46. The ligand conjugate according to claim 45, or a tautomer, a mesomer, a racemate, an enantiomer, a diastereomer, or a mixture thereof, or a pharmaceutically acceptable salt or hydrate thereof,
wherein R₆ is optionally substituted cyclopropyl.

47. The ligand conjugate according to any one of claims 38-46, or a tautomer, a mesomer, a racemate, an enantiomer, a diastereomer, or a mixture thereof, or a pharmaceutically acceptable salt or hydrate thereof,
wherein R₇ is hydrogen.

48. The ligand conjugate according to any one of claims 38-40, or a tautomer, a mesomer, a racemate, an enantiomer, a diastereomer, or a mixture thereof, or a pharmaceutically acceptable salt or hydrate thereof,
wherein R₆ and R₇ together with the atom to which they are attached form an optionally substituted cyclopropyl.

49. The ligand conjugate according to claim 48, or a tautomer, a mesomer, a racemate, an enantiomer, a diastereomer, or a mixture thereof, or a pharmaceutically acceptable salt or hydrate thereof,
wherein R₆ and R₇ together with the atom to which they are attached form an optionally substituted cyclobutyl.

50. The ligand conjugate according to any one of claims 38-49, or a tautomer, a mesomer, a racemate, an enantiomer, and a diastereomer, or a mixture thereof, or a pharmaceutically acceptable salt or hydrate thereof,
wherein, v is 0.

51. The ligand conjugate according to any one of claims 38-49, or a tautomer, a mesomer, a racemate, an enantiomer, and a diastereomer, or a mixture thereof, or a pharmaceutically acceptable salt or hydrate thereof,
wherein, v is 1.

52. The ligand conjugate according to any one of claims 4-51, or a tautomer, a mesomer, a racemate, an enantiomer, a diastereomer, or a mixture thereof, or a pharmaceutically acceptable salt or hydrate thereof,
wherein L₅ is a structure selected from the group consisting of optionally substituted 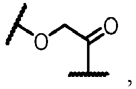 optionally substituted 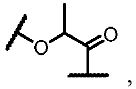 optionally substituted 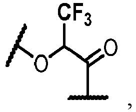 optionally substituted 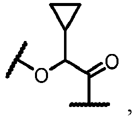 optionally substituted 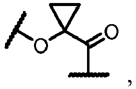 optionally substituted 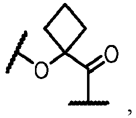 optionally substituted 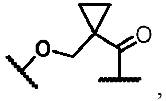 and optionally substituted

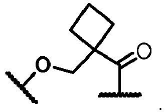

53. The ligand conjugate according to any one of claims 1-52, or a tautomer, a mesomer, a racemate, an enantiomer, a diastereomer, or a mixture thereof, or a pharmaceutically acceptable salt or hydrate thereof,
wherein, Ab is an antibody or antigen-binding fragment thereof.

54. The ligand conjugate according to claim 53, or a tautomer, a mesomer, a racemate, an enantiomer, a diastereomer, or a mixture thereof, or a pharmaceutically acceptable salt or hydrate thereof,
wherein, the antibody is selected from the group consisting of a murine antibody, a chimeric antibody, a humanized antibody, and a fully human antibody.

55. The ligand conjugate according to any one of claims 53-54, or a tautomer, a mesomer, a racemate, an enantiomer, a diastereomer, or a mixture thereof, or a pharmaceutically acceptable salt or hydrate thereof,
wherein, the antibody is a monoclonal antibody.

56. The ligand conjugate according to claim 53, or a tautomer, a mesomer, a racemate, an enantiomer, a diastereomer, or a mixture thereof, or a pharmaceutically acceptable salt or hydrate thereof,
wherein, the antigen-binding fragment is selected from the group consisting of Fab, Fab', Fv fragment, F(ab')₂, F(ab)₂, scFv, di-scFv, VHH and dAb.

57. The ligand conjugate according to any one of claims 1-56, or a tautomer, a mesomer, a racemate, an enantiomer, a diastereomer, or a mixture thereof, or a pharmaceutically acceptable salt or hydrate thereof,
wherein, the Ab comprises HER2 antibodies and/or TROP2 antibodies.

58. The ligand conjugate according to any one of claims 1-57, or a tautomer, a mesomer, a racemate, an enantiomer, a diastereomer, or a mixture thereof, or a pharmaceutically acceptable salt or hydrate thereof,
wherein, the Ab comprises HCDR1, HCDR2, and HCDR3 of heavy chain variable regions, the amino acid sequence of HCDR1 is shown in SEQ ID NO: 1, the amino acid sequence of HCDR2 is shown in SEQ ID NO: 2, and the amino acid sequence of HCDR3 is shown in SEQ ID NO: 3.

59. The ligand conjugate according to any one of claims 1-58, or a tautomer, a mesomer, a racemate, an enantiomer, a diastereomer, or a mixture thereof, or a pharmaceutically acceptable salt or hydrate thereof,
wherein, the Ab comprises LCDR1, LCDR2, and LCDR3 of light chain variable regions, the amino acid sequence of LCDR1 is shown in SEQ ID NO: 4, the amino acid sequence of LCDR2 is shown in SEQ ID NO: 5, and the amino acid sequence of LCDR3 is shown in SEQ ID NO: 6.

60. The ligand conjugate according to any one of claims 1-59, or a tautomer, a mesomer, a racemate, an enantiomer, a diastereomer, or a mixture thereof, or a pharmaceutically acceptable salt or hydrate thereof,
wherein, the Ab comprises a heavy chain variable region, and the amino acid sequence of the heavy chain variable region is shown in SEQ ID NO: 7.

61. The ligand conjugate according to any one of claims 1-60, or a tautomer, a mesomer, a racemate, an enantiomer, a diastereomer, or a mixture thereof, or a pharmaceutically acceptable salt or hydrate thereof,
wherein, the Ab comprises a light chain variable region, and the amino acid sequence of the light chain variable region is shown in SEQ ID NO: 8.

62. The ligand conjugate according to any one of claims 1-61, or a tautomer, a mesomer, a racemate, an enantiomer, a diastereomer, or a mixture thereof, or a pharmaceutically acceptable salt or hydrate thereof,
wherein, the Ab comprises a heavy chain, and the amino acid sequence of the heavy chain is shown in SEQ ID NO: 9.

63. The ligand conjugate according to any one of claims 1-62, or a tautomer, a mesomer, a racemate, an enantiomer, a diastereomer, or a mixture thereof, or a pharmaceutically acceptable salt or hydrate thereof,
wherein, the Ab comprises a light chain, and the amino acid sequence of the light chain is shown in SEQ ID NO: 10.

64. The ligand conjugate according to any one of claims 1-57, or a tautomer, a mesomer, a racemate, an enantiomer, a diastereomer, or a mixture thereof, or a pharmaceutically acceptable salt or hydrate thereof,
wherein, the Ab comprises HCDR1, HCDR2, and HCDR3 of heavy chain variable region, the amino acid sequence of HCDR1 is shown in SEQ ID NO: 11, the amino acid sequence of HCDR2 is shown in SEQ ID NO: 12, and the amino acid sequence of HCDR3 is shown in SEQ ID NO: 13.

65. The ligand conjugate according to claim 64, or a tautomer, a mesomer, a racemate, an enantiomer, a diastereomer, or a mixture thereof, or a pharmaceutically acceptable salt or hydrate thereof,
wherein, the Ab comprises LCDR1, LCDR2, and LCDR3 of light chain variable region, the amino acid sequence of LCDR1 is shown in SEQ ID NO: 14, the amino acid sequence of LCDR2 is shown in SEQ ID NO: 15, and the amino acid sequence of LCDR3 is shown in SEQ ID NO: 16.

66. The ligand conjugate according to any one of claims 64-65, or a tautomer, a mesomer, a racemate, an enantiomer, a diastereomer, or a mixture thereof, or a pharmaceutically acceptable salt or hydrate thereof,
wherein, the Ab comprises a heavy chain variable region, and the amino acid sequence of the heavy chain variable region is shown in SEQ ID NO: 17.

67. The ligand conjugate according to any one of claims 64-66, or a tautomer, a mesomer, a racemate, an enantiomer, a diastereomer, or a mixture thereof, or a pharmaceutically acceptable salt or hydrate thereof,
wherein, the Ab comprises a light chain variable region, and the amino acid sequence of the light chain variable region is shown in SEQ ID NO: 18.

68. The ligand conjugate according to any one of claims 64-67, or a tautomer, a mesomer, a racemate, an enantiomer, a diastereomer, or a mixture thereof, or a pharmaceutically acceptable salt or hydrate thereof,
wherein, the Ab comprises a heavy chain, and the amino acid sequence of the heavy chain is shown in SEQ ID NO: 19.

69. The ligand conjugate according to any one of claims 64-68, or a tautomer, a mesomer, a racemate, an enantiomer, a diastereomer, or a mixture thereof, or a pharmaceutically acceptable salt or hydrate thereof,
wherein, the Ab comprises a light chain, and the amino acid sequence of the light chain is shown in SEQ ID NO: 20.

70. A ligand conjugate, or a tautomer, a mesomer, a racemate, an enantiomer, a diastereomer, or a mixture thereof, or a pharmaceutically acceptable salt or hydrate thereof, wherein the ligand conjugate is a structure selected from the group consisting of 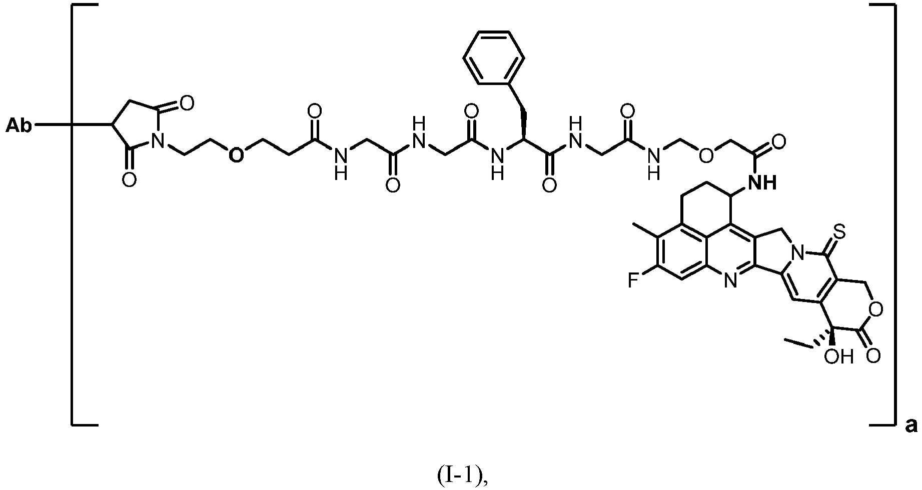 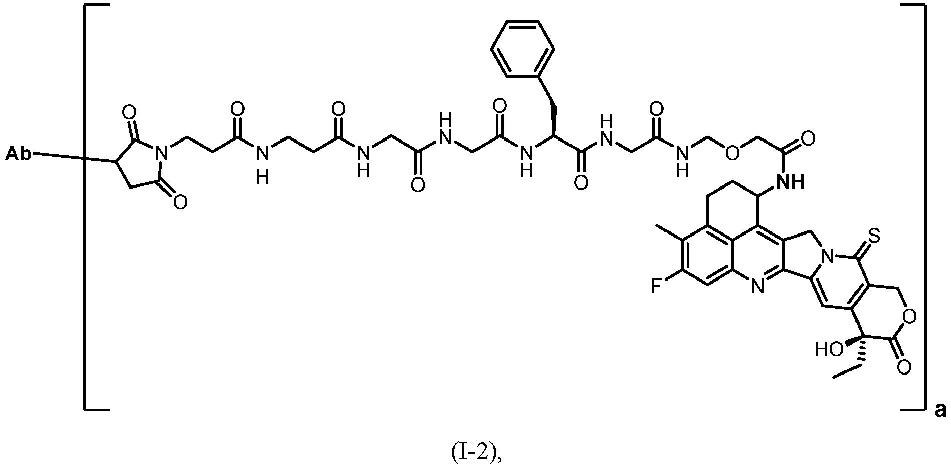 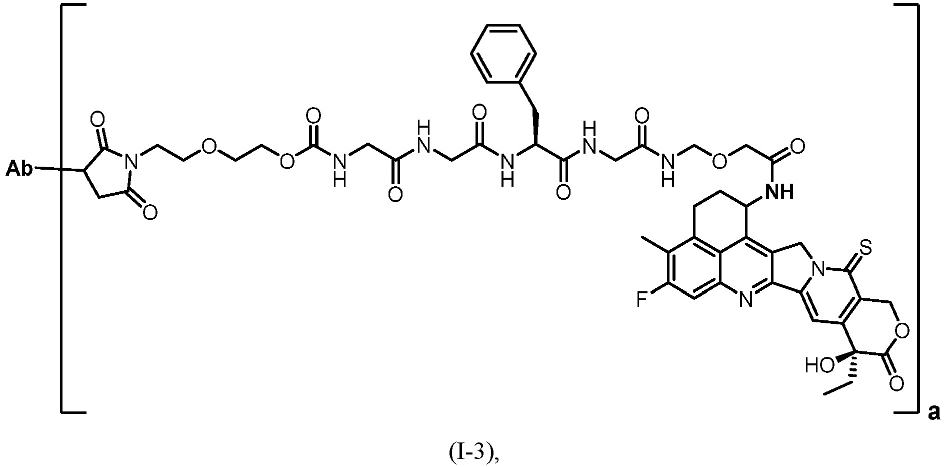 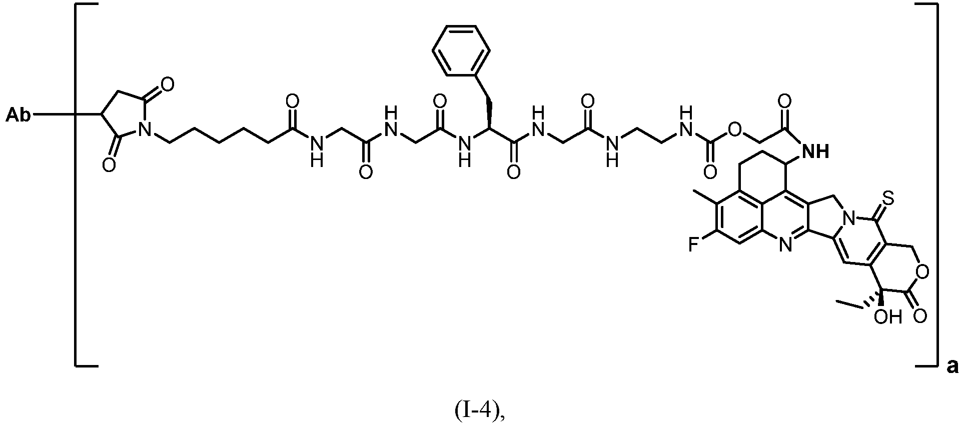 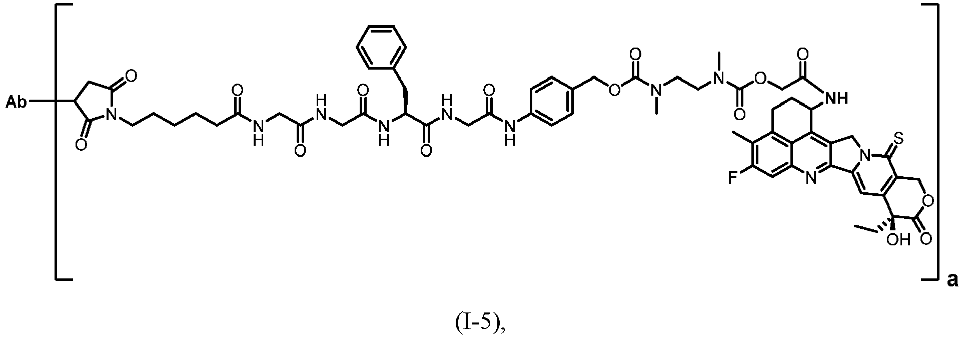 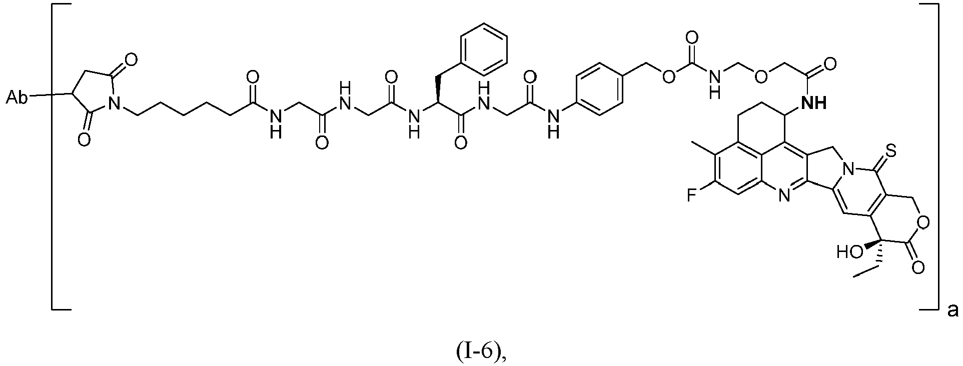 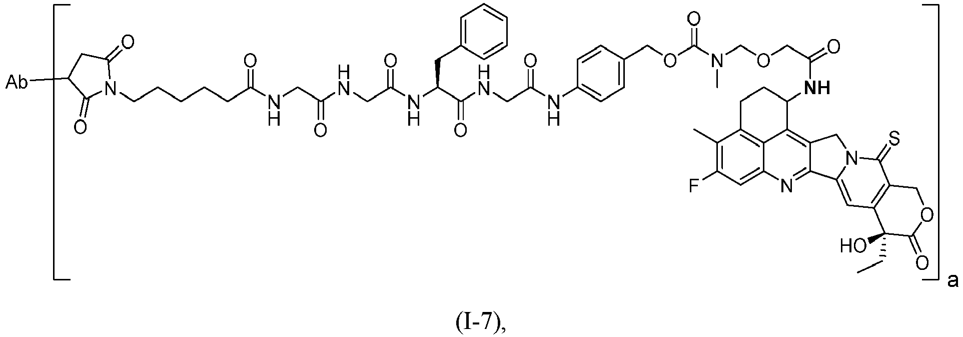 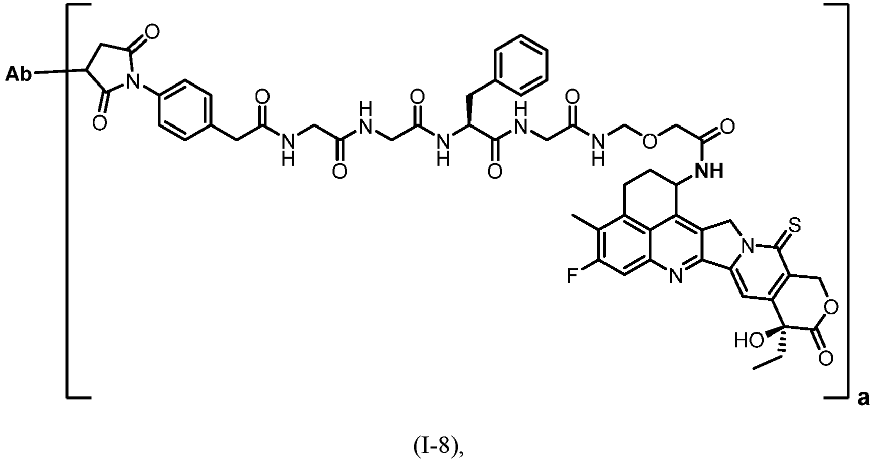 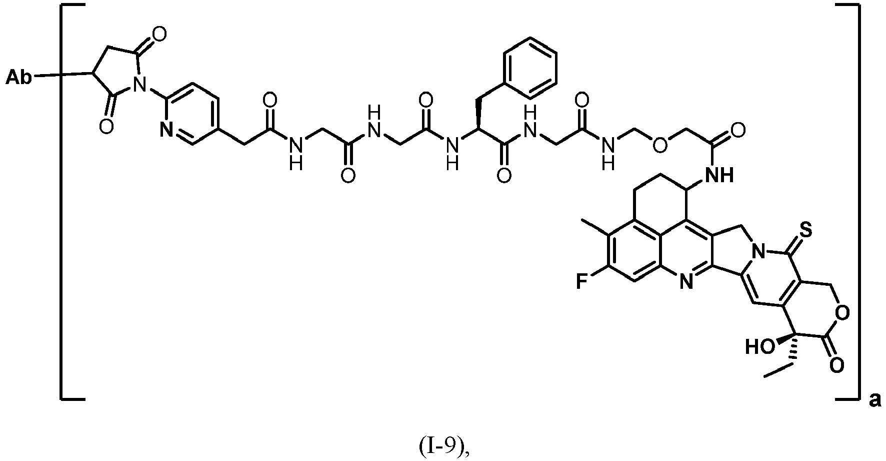 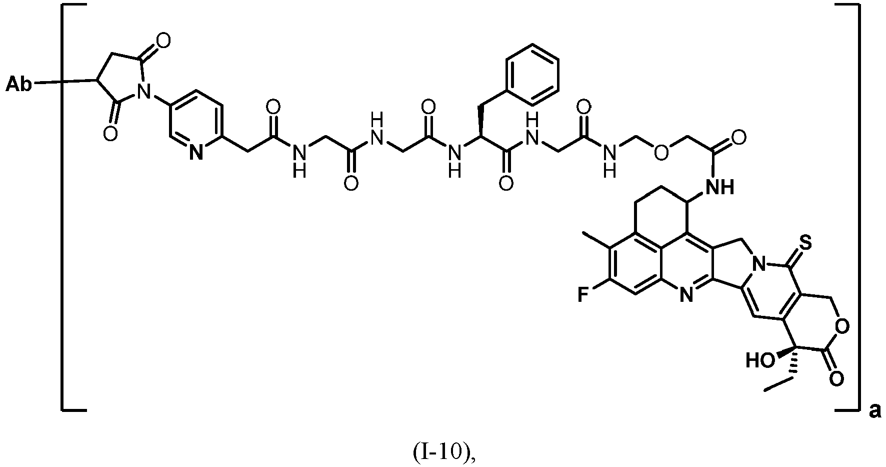 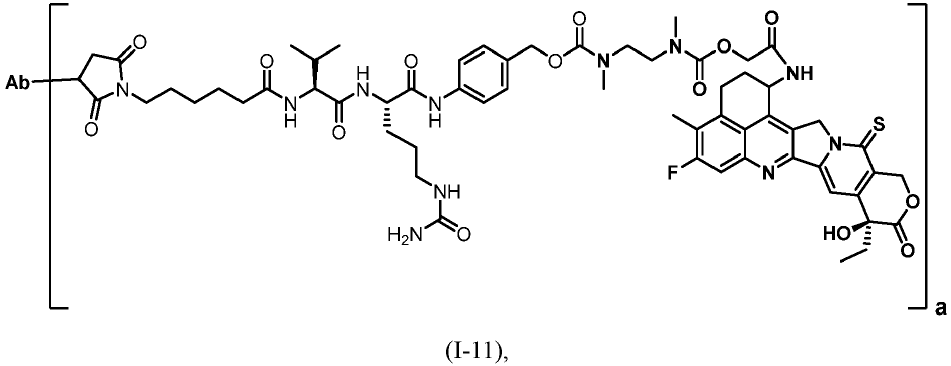 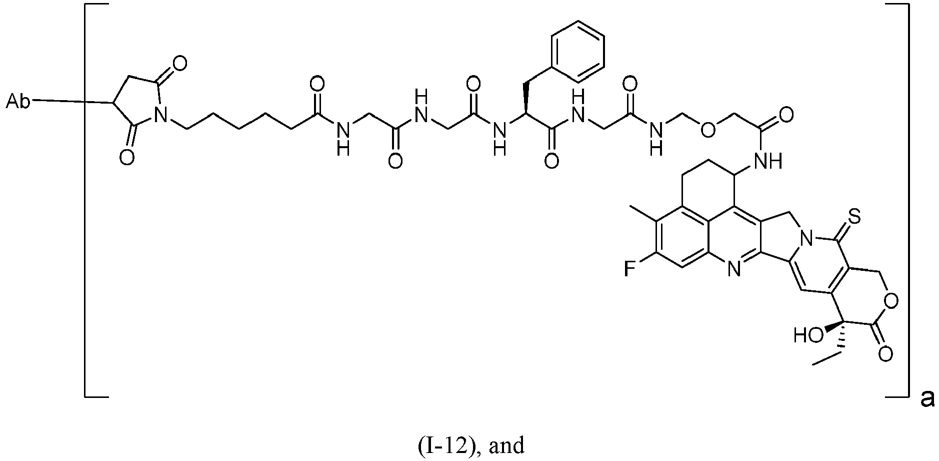 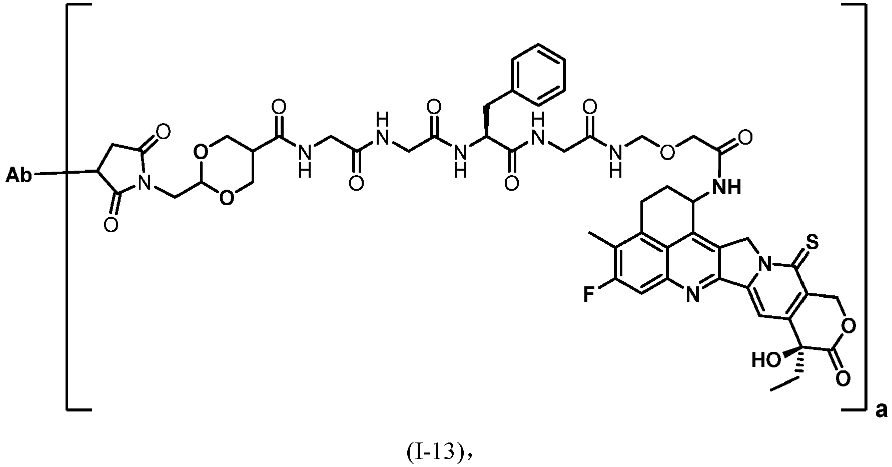 wherein, Ab is a ligand, "a" is a number greater than 0, and "a" is a decimal or integer.

71. A compound, or a tautomer, a mesomer, a racemate, an enantiomer, a diastereomer, or a mixture thereof, or a pharmaceutically acceptable salt or hydrate thereof, wherein the compound comprises the structure shown in formula (II): 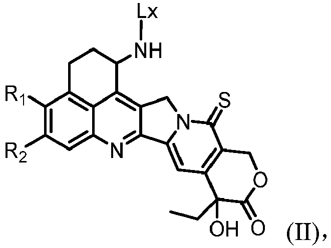 wherein,
R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
Lx is a linking group, and Lx is L₁ₓ-L₂-L₃-L₄-L₅-, and L₁ₓ can be directly linked to the ligand.

72. A compound, or a tautomer, a mesomer, a racemate, an enantiomer, a diastereomer, or a mixture thereof, or a pharmaceutically acceptable salt or hydrate thereof, wherein the compound comprises the structure shown in formula (II-a): 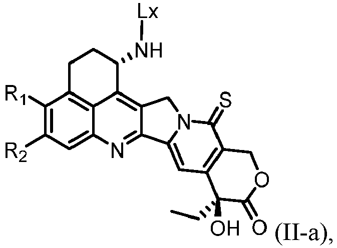 wherein,
R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
Lx is a linking group, Lx is L₁ₓ-L₂-L₃-L₄-L₅-, and L₁ₓ can be directly linked to the ligand.

73. A compound, or a tautomer, a mesomer, a racemate, an enantiomer, a diastereomer, or a mixture thereof, or a pharmaceutically acceptable salt or hydrate thereof, wherein the compound comprises the structure shown in formula (II-b): 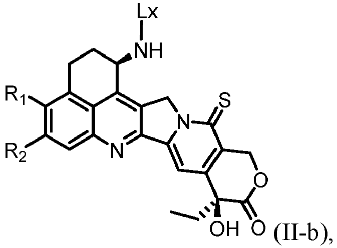 wherein,
R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
Lx is a linking group, Lx is L₁ₓ-L₂-L₃-L₄-L₅-, and L₁ₓ can be directly linked to the ligand.

74. The compound according to any one of claims 71-73, or a tautomer, a mesomer, a racemate, an enantiomer, a diastereomer, or a mixture thereof, or a pharmaceutically acceptable salt or hydrate thereof,
L₁ₓ is capable of being linked directly to the thiol group of the ligand.

75. The compound according to any one of claims 71-74, or a tautomer, a mesomer, a racemate, an enantiomer, and a diastereomer, or a mixture thereof, or a pharmaceutically acceptable salt or hydrate thereof, wherein L₁ₓ is optionally substituted

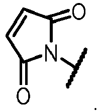

76. The compound according to any one of claims 71-75, or a tautomer, a mesomer, a racemate, an enantiomer, a diastereomer, or a mixture thereof, or a pharmaceutically acceptable salt or hydrate thereof,
wherein L₂ is selected from the group consisting of optionally substituted -(CH₂)ₘ₁-X₁-(CH₂)ₘ₂-C(O)-, optionally substituted -(CH₂CH₂O)ₙ-C(O)-, and optionally substituted -(CH₂)ₚ-C(O)-,
X₁ is selected from the group consisting of -O-, optionally substituted -C(O)-NH-, optionally substituted aryl, optionally substituted heteroaryl, optionally substituted aliphatic heterocyclyl, and optionally substituted aliphatic cyclyl,
wherein, m1, m2, and n are each independently selected from integers at least 0, and p is an integer selected from 2 to 8.

77. The compound according to claim 76, or a tautomer, a mesomer, a racemate, an enantiomer, a diastereomer, or a mixture thereof, or a pharmaceutically acceptable salt or hydrate thereof,
wherein L₂ is optionally substituted -(CH₂CH₂O)ₙ-C(O)-.

78. The compound according to claim 77, or a tautomer, a mesomer, a racemate, an enantiomer, a diastereomer, or a mixture thereof, or a pharmaceutically acceptable salt or hydrate thereof,
wherein, n is 2.

79. The compound according to claim 76, or a tautomer, a mesomer, a racemate, an enantiomer, a diastereomer, or a mixture thereof, or a pharmaceutically acceptable salt or hydrate thereof,
wherein L₂ comprises optionally substituted -(CH₂)ₘ₁-X₁-(CH₂)ₘ₂-C(O)-.

80. The compound according to any of claim 79, or a tautomer, a mesomer, a racemate, an enantiomer, a diastereomer, or a mixture thereof, or a pharmaceutically acceptable salt or hydrate thereof,
wherein X₁ is selected from the group consisting of -O- and optionally substituted -C(O)-NH-.

81. The compound according to any one of claims 79-80, or a tautomer, a mesomer, a racemate, an enantiomer, a diastereomer, or a mixture thereof, or a pharmaceutically acceptable salt or hydrate thereof,
wherein, m1 is 2.

82. The compound according to any one of claims 79-81, or a tautomer, a mesomer, a racemate, an enantiomer, a diastereomer, or a mixture thereof, or a pharmaceutically acceptable salt or hydrate thereof,
wherein, m2 is 2.

83. The compound according to claim 79, or a tautomer, a mesomer, a racemate, an enantiomer, a diastereomer, or a mixture thereof, or a pharmaceutically acceptable salt or hydrate thereof,
wherein X₁ is selected from the group consisting of optionally substituted aryl and optionally substituted heteroaryl.

84. The compound according to claim 83, or a tautomer, a mesomer, a racemate, an enantiomer, a diastereomer, or a mixture thereof, or a pharmaceutically acceptable salt or hydrate thereof,
wherein X₁ is optionally substituted phenyl.

85. The compound according to claim 83, or a tautomer, a mesomer, a racemate, an enantiomer, a diastereomer, or a mixture thereof, or a pharmaceutically acceptable salt or hydrate thereof,
wherein X₁ is optionally substituted pyridinyl.

86. The compound according to any one of claims 83-85, or a tautomer, a mesomer, a racemate, an enantiomer, a diastereomer, or a mixture thereof, or a pharmaceutically acceptable salt or hydrate thereof,
wherein, m1 is 0.

87. The compound according to any one of claims 83-86, or a tautomer, a mesomer, a racemate, an enantiomer, a diastereomer, or a mixture thereof, or a pharmaceutically acceptable salt or hydrate thereof,
wherein, m2 is 1.

88. The compound according to claim 79, or a tautomer, a mesomer, a racemate, an enantiomer, a diastereomer, or a mixture thereof, or a pharmaceutically acceptable salt or hydrate thereof,
wherein X₁ is optionally substituted

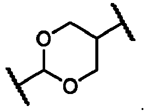

89. The compound according to claim 79, or a tautomer, a mesomer, a racemate, an enantiomer, a diastereomer, or a mixture thereof, or a pharmaceutically acceptable salt or hydrate thereof,
wherein X₁ is optionally substituted cyclohexyl.

90. The compound according to any one of claims 88-89, or a tautomer, a mesomer, a racemate, an enantiomer, a diastereomer, or a mixture thereof, or a pharmaceutically acceptable salt or hydrate thereof,
wherein, m1 is 1.

91. The compound according to any one of claims 88-90, or a tautomer, a mesomer, a racemate, an enantiomer, and a diastereomer, or a mixture thereof, or a pharmaceutically acceptable salt or hydrate thereof,
wherein, m2 is 0.

92. The compound according to claim 76, or a tautomer, a mesomer, a racemate, an enantiomer, a diastereomer, or a mixture thereof, or a pharmaceutically acceptable salt or hydrate thereof,
wherein L₂ is optionally substituted -(CH₂)ₚ-C(O)-.

93. The compound according to claim 92, or a tautomer, a mesomer, a racemate, an enantiomer, a diastereomer, or a mixture thereof, or a pharmaceutically acceptable salt or hydrate thereof,
wherein, p is 5.

94. The compound according to any one of claims 71-93, or a tautomer, a mesomer, a racemate, an enantiomer, a diastereomer, or a mixture thereof, or a pharmaceutically acceptable salt or hydrate thereof,
wherein L₂ is a structure selected from the group consisting of optionally substituted 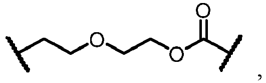 optionally substituted 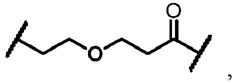 optionally substituted 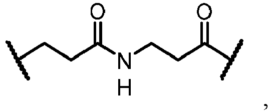 optionally substituted 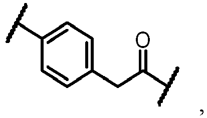 optionally substituted 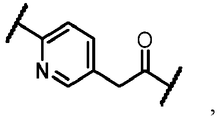 optionally substituted 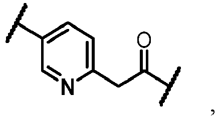 optionally substituted 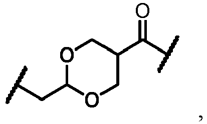 optionally substituted 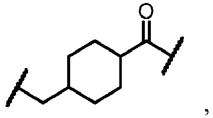 and optionally substituted

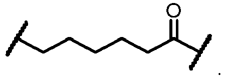

95. The compound according to any one of claims 71-94, or a tautomer, a mesomer, a racemate, an enantiomer, a diastereomer, or a mixture thereof, or a pharmaceutically acceptable salt or hydrate thereof,
wherein L₃ is a peptide residue.

96. The compound according to any one of claims 71-95, or a tautomer, a mesomer, a racemate, an enantiomer, a diastereomer, or a mixture thereof, or a pharmaceutically acceptable salt or hydrate thereof,
wherein L₃ is a peptide residue is composed of amino acids selected from the group consisting of phenylalanine, isoleucine, leucine, tryptophan, valine, methionine, tyrosine, alanine, threonine, histidine, serine, glutamine, arginine, lysine, asparagine, glutamate, proline, citrulline, aspartate, and glycine.

97. The compound according to any one of claims 71-96, or a tautomer, a mesomer, a racemate, an enantiomer, a diastereomer, or a mixture thereof, or a pharmaceutically acceptable salt or hydrate thereof,
wherein L₃ is a peptide residue is composed of amino acids selected from the group consisting of glycine, phenylalanine, valine, and citrulline.

98. The compound according to any one of claims 71-97, or a tautomer, a mesomer, a racemate, an enantiomer, a diastereomer, or a mixture thereof, or a pharmaceutically acceptable salt or hydrate thereof,
wherein L₃ is a peptide residue selected from the group consisting of -glycine-phenylalanine-glycine- (-Gly-Phe-Gly-), -glycine-glycine-phenylalanine-glycine- (-Gly-Gly-Phe-Gly-), and - valine-citrulline- (-Val-Cit-).

99. The compound according to any one of claims 71-98, or a tautomer, a mesomer, a racemate, an enantiomer, a diastereomer, or a mixture thereof, or a pharmaceutically acceptable salt or hydrate thereof,
wherein L₃ is a structure selected from the group consisting of

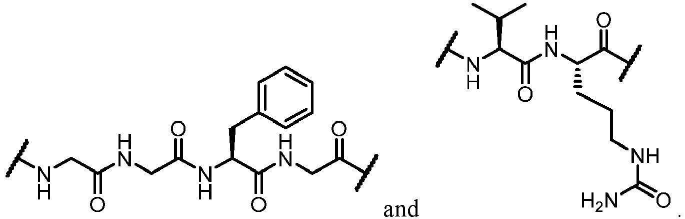

100. The compound according to any one of claims 71-99, or a tautomer, a mesomer, a racemate, an enantiomer, a diastereomer, or a mixture thereof, or a pharmaceutically acceptable salt or hydrate thereof,
wherein L₄ is optionally substituted -L₄ₐ-NR₄-CH₂-L_{4b}-,
L₄ₐ is absent or L₄ₐ is optionally substituted 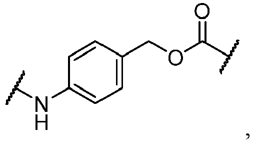
L_{4b} is absent or L_{4b} is optionally substituted 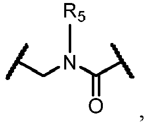
R₄ and R₅ are each independently selected from the group consisting of hydrogen and optionally substituted alkyl.

101. The compound according to claim 100, or a tautomer, a mesomer, a racemate, an enantiomer, a diastereomer, or a mixture thereof, or a pharmaceutically acceptable salt or hydrate thereof,
wherein L₄ is optionally substituted

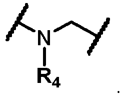

102. The compound according to claim 100, or a tautomer, a mesomer, a racemate, an enantiomer, a diastereomer, or a mixture thereof, or a pharmaceutically acceptable salt or hydrate thereof,
wherein L₄ is optionally substituted

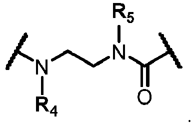

103. The compound according to claim 100, or a tautomer, a mesomer, a racemate, an enantiomer, a diastereomer, or a mixture thereof, or a pharmaceutically acceptable salt or hydrate thereof,
wherein L₄ is optionally substituted

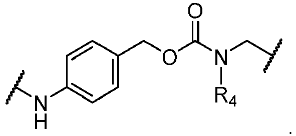

104. The compound according to claim 100, or a tautomer, a mesomer, a racemate, an enantiomer, a diastereomer, or a mixture thereof, or a pharmaceutically acceptable salt or hydrate thereof,
wherein L₄ is optionally substituted

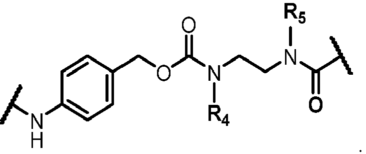

105. The compound according to any one of claims 100-104, or a tautomer, a mesomer, a racemate, an enantiomer, a diastereomer, or a mixture thereof, or a pharmaceutically acceptable salt or hydrate thereof,
wherein R₄ is selected from the group consisting of hydrogen and optionally substituted methyl.

106. The compound according to any one of claims 100-105, or a tautomer, a mesomer, a racemate, an enantiomer, a diastereomer, or a mixture thereof, or a pharmaceutically acceptable salt or hydrate thereof,
wherein R₅ is selected from the group consisting of hydrogen and optionally substituted methyl.

107. The compound according to any one of claims 71-106, or a tautomer, a mesomer, a racemate, an enantiomer, a diastereomer, or a mixture thereof, or a pharmaceutically acceptable salt or hydrate thereof,
wherein L₄ is a structure selected from the group consisting of optionally substituted 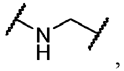 optionally substituted 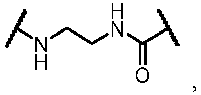 optionally substituted 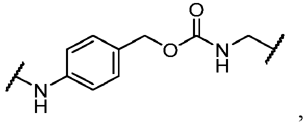 optionally substituted 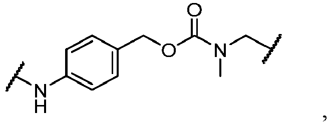 and optionally substituted

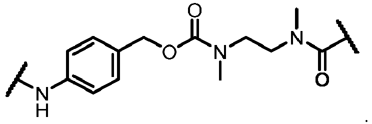

108. The compound according to any one of claims 71-107, or a tautomer, a mesomer, a racemate, an enantiomer, a diastereomer, or a mixture thereof, or a pharmaceutically acceptable salt or hydrate thereof,
wherein L₅ is optionally substituted 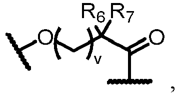 and L₅ is directly linked to the following structure: 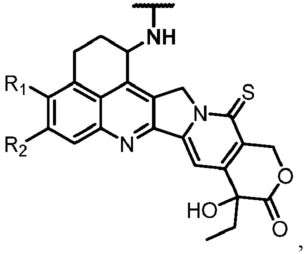
R₆ and R₇ are each independently selected from the group consisting of hydrogen, deuterium, optionally substituted alkyl, optionally substituted haloalkyl, optionally substituted cycloalkyl, and optionally substituted cycloalkyl-alkyl, or R₆ and R₇ together with the atom to which they are attached form an optionally substituted cycloalkyl,
wherein, v is a number that is at least 0.

109. The compound according to any one of claims 71-108, or a tautomer, a mesomer, a racemate, an enantiomer, a diastereomer, or a mixture thereof, or a pharmaceutically acceptable salt or hydrate thereof,
wherein L₅ is optionally substituted 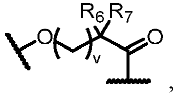 and L₅ is directly linked to the following structure: 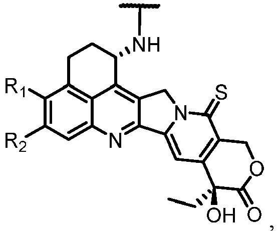
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl, or R₆ and R₇ together with the atom to which they are attached form an optionally substituted cycloalkyl,
wherein, v is a number that is at least 0.

110. The compound according to any one of claims 71-109, or a tautomer, a mesomer, a racemate, an enantiomer, a diastereomer, or a mixture thereof, or a pharmaceutically acceptable salt or hydrate thereof,
wherein L₅ is optionally substituted 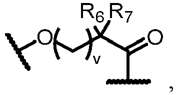 and L₅ is directly linked to the following structure: 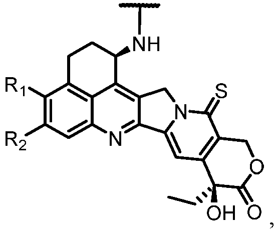
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl, or R₆ and R₇ together with the atom to which they are attached form an optionally substituted cycloalkyl,
wherein, v is a number that is at least 0.

111. The compound according to any one of claims 108-110, or a tautomer, a mesomer, a racemate, an enantiomer, a diastereomer, or a mixture thereof, or a pharmaceutically acceptable salt or hydrate thereof,
wherein R₆ is hydrogen.

112. The compound according to any one of claims 108-110, or a tautomer, a mesomer, a racemate, an enantiomer, and a diastereomer, or a mixture thereof, or a pharmaceutically acceptable salt or hydrate thereof,
R₆ is optionally substituted methyl.

113. The compound according to claim 112, or a tautomer, a mesomer, a racemate, an enantiomer, a diastereomer, or a mixture thereof, or a pharmaceutically acceptable salt or hydrate thereof,
wherein R₆ is fluorine substituted methyl.

114. The compound according to any one of claims 112-113, or a tautomer, a mesomer, a racemate, an enantiomer, a diastereomer, or a mixture thereof, or a pharmaceutically acceptable salt or hydrate thereof,
wherein R₆ is trifluoromethyl.

115. The compound according to any one of claims 108-110, or a tautomer, a mesomer, a racemate, an enantiomer, a diastereomer, or a mixture thereof, or a pharmaceutically acceptable salt or hydrate thereof,
wherein R₆ is optionally substituted cycloalkyl.

116. The compound according to claim 115, or a tautomer, a mesomer, a racemate, an enantiomer, a diastereomer, or a mixture thereof, or a pharmaceutically acceptable salt or hydrate thereof,
wherein R₆ is optionally substituted cyclopropyl.

117. The compound according to any one of claims 108-116, or a tautomer, a mesomer, a racemate, an enantiomer, a diastereomer, or a mixture thereof, or a pharmaceutically acceptable salt or hydrate thereof,
wherein R₇ is hydrogen.

118. The compound according to any one of claims 108-110, or a tautomer, a mesomer, a racemate, an enantiomer, a diastereomer, or a mixture thereof, or a pharmaceutically acceptable salt or hydrate thereof,
wherein R₆ and R₇ together with the atom to which they are attached form an optionally substituted cyclopropyl.

119. The compound according to claim 118, or a tautomer, a mesomer, a racemate, an enantiomer, a diastereomer, or a mixture thereof, or a pharmaceutically acceptable salt or hydrate thereof,
wherein R₆ and R₇ together with the atom to which they are attached form an optionally substituted cyclobutyl.

120. The compound according to any one of claims 108-119, or a tautomer, a mesomer, a racemate, an enantiomer, a diastereomer, or a mixture thereof, or a pharmaceutically acceptable salt or hydrate thereof,
wherein, v is 0.

121. The compound according to any one of claims 108-119, or a tautomer, a mesomer, a racemate, an enantiomer, a diastereomer, or a mixture thereof, or a pharmaceutically acceptable salt or hydrate thereof,
wherein, v is 1.

122. The compound according to any one of claims 71-121, or a tautomer, a mesomer, a racemate, an enantiomer, a diastereomer, or a mixture thereof, or a pharmaceutically acceptable salt or hydrate thereof,
wherein L₅ is a structure selected from the group consisting of optionally substituted 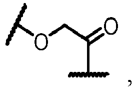 optionally substituted 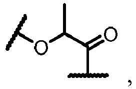 optionally substituted 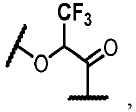 optionally substituted 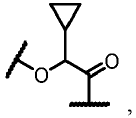 optionally substituted 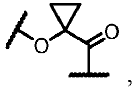 optionally substituted 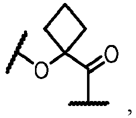 optionally substituted 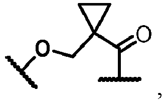 and optionally substituted

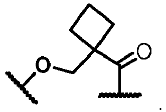

123. A compound, or a tautomer, a mesomer, a racemate, an enantiomer, a diastereomer, or a mixture thereof, or a pharmaceutically acceptable salt or hydrate thereof, wherein the compound is selected from the group consisting of

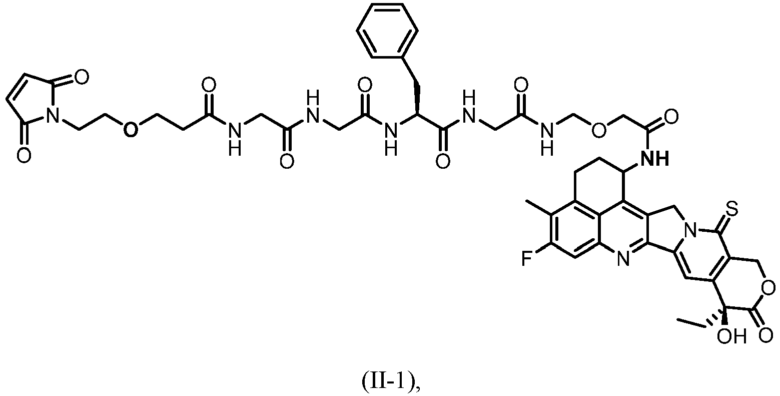

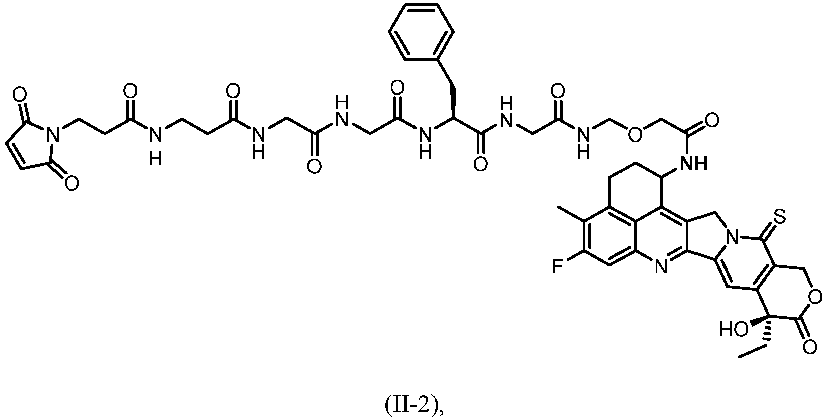

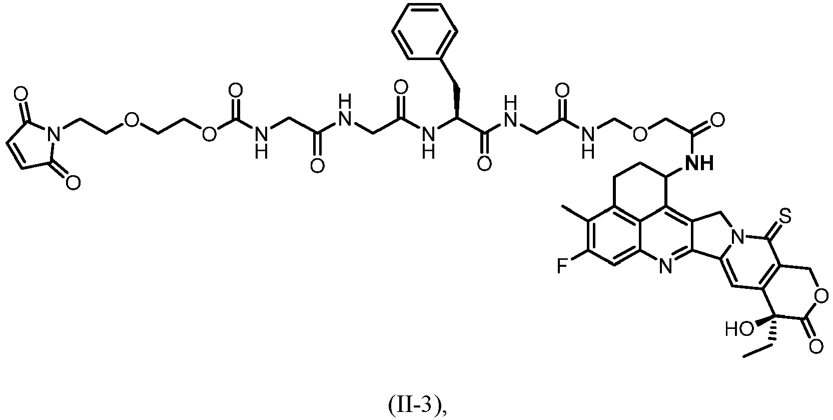

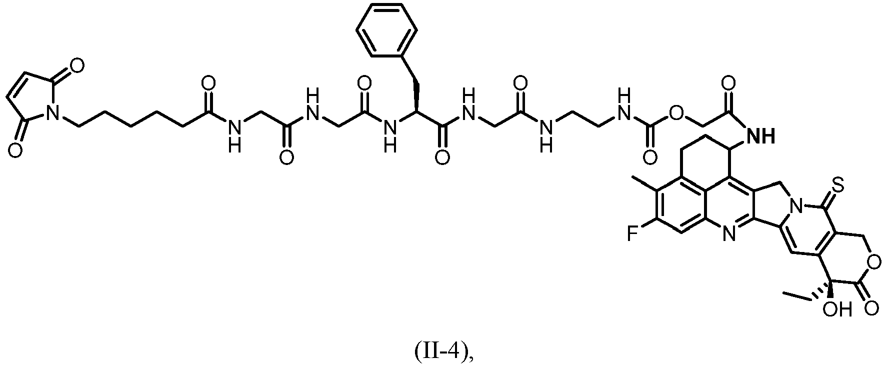

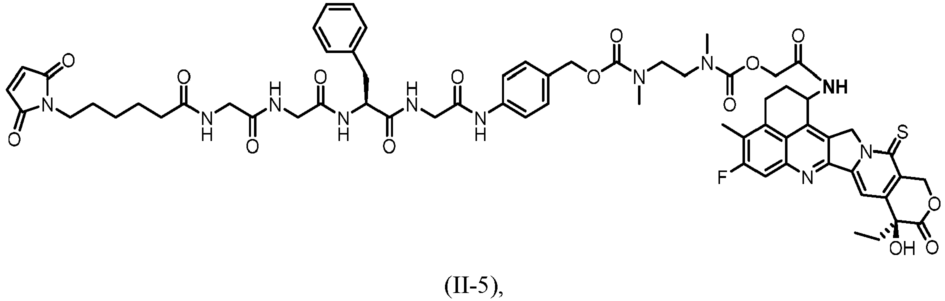

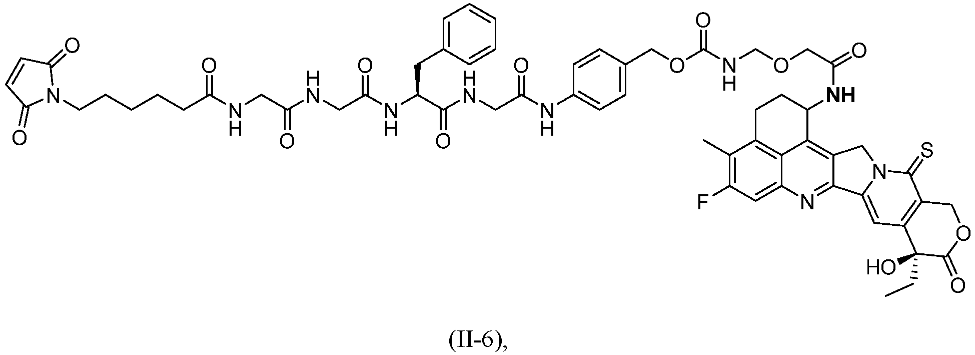

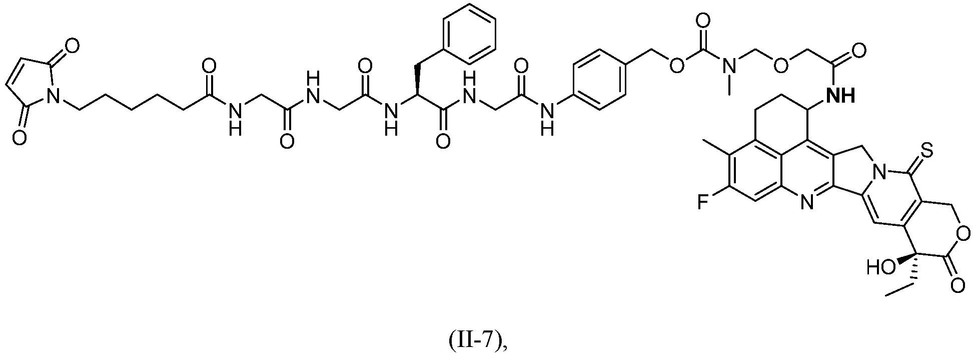

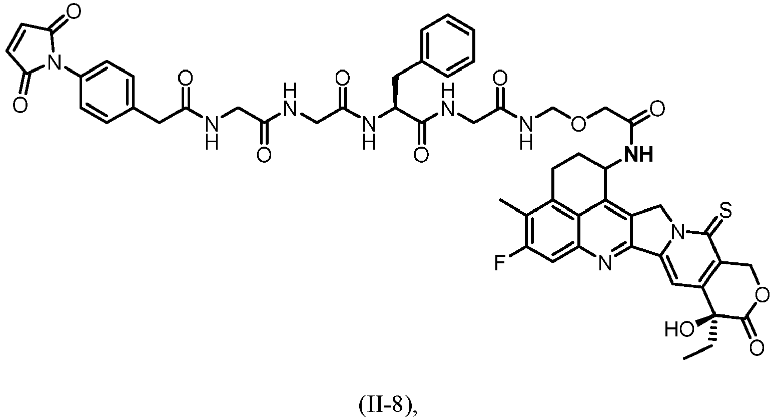

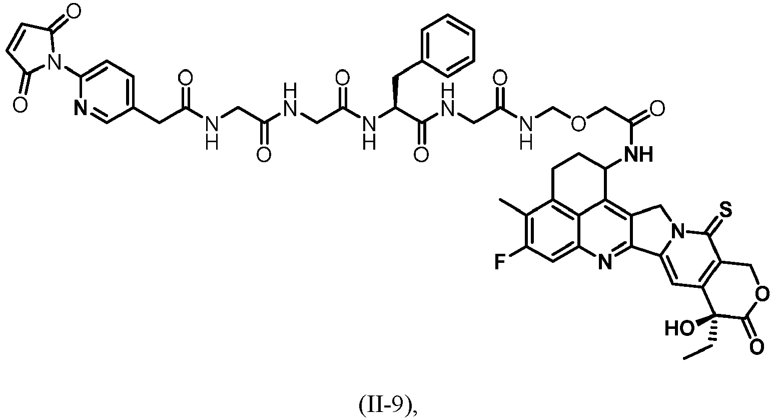

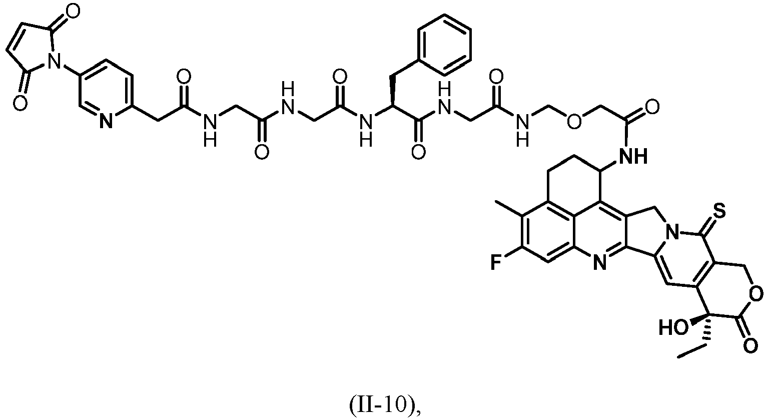

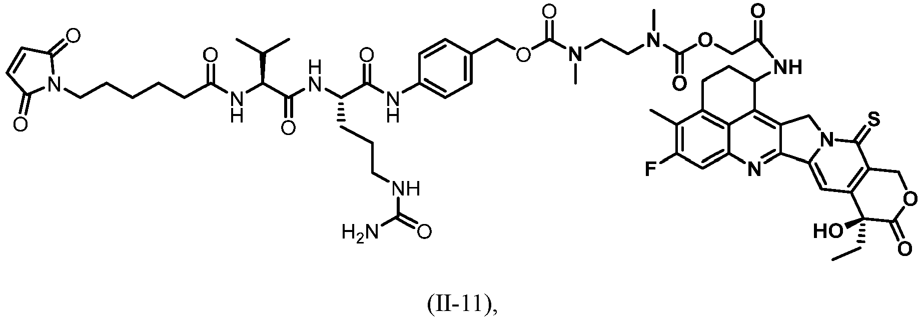

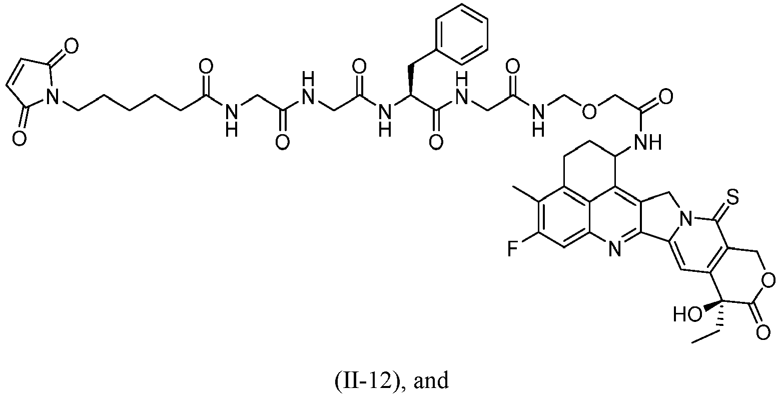

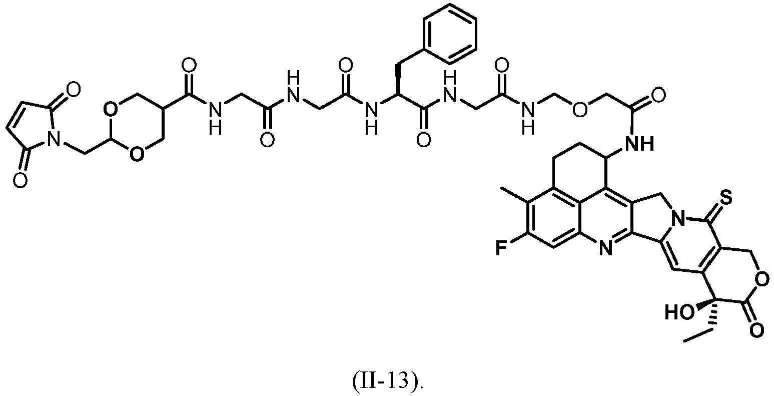

124. A linker as represented in formula (L-1), and the linker can be used to obtain a ligand-drug conjugate formed by linking a drug unit to a ligand via the linker:
-L₁-L₂-L₃-L₄-L₅- (L-1),
wherein L₁ is optionally substituted 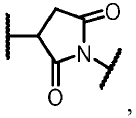
L₂ is selected from the group consisting of optionally substituted -(CH₂)ₘ₁-X₁-(CH₂)ₘ₂-C(O)-, and optionally substituted -(CH₂CH₂O)ₙ-C(O)-,
X₁ is selected from the group consisting of -O-, optionally substituted -C(O)-NH-, optionally substituted aryl, optionally substituted heteroaryl, optionally substituted aliphatic heterocyclyl, and optionally substituted aliphatic cyclyl,
wherein, m1, m2, and n are each independently selected from integers that is at least 0.

125. The linker according to claim 124, or a tautomer, a mesomer, a racemate, an enantiomer, a diastereomer, or a mixture thereof, or a pharmaceutically acceptable salt or hydrate thereof,
wherein, n is 2.

126. The linker according to any one of claims 124-125, or a tautomer, a mesomer, a racemate, an enantiomer, a diastereomer, or a mixture thereof, or a pharmaceutically acceptable salt or hydrate thereof,
wherein X₁ is selected from the group consisting of -O- and optionally substituted -C(O)-NH-.

127. The linker according to any one of claims 124-126, or a tautomer, a mesomer, a racemate, an enantiomer, a diastereomer, or a mixture thereof, or a pharmaceutically acceptable salt or hydrate thereof,
wherein, m1 is 2.

128. The linker according to any one of claims 124-127, or a tautomer, a mesomer, a racemate, an enantiomer, a diastereomer, or a mixture thereof, or a pharmaceutically acceptable salt or hydrate thereof,
wherein, m2 is 2.

129. The linker according to any one of claims 124-125, or a tautomer, a mesomer, a racemate, an enantiomer, a diastereomer, or a mixture thereof, or a pharmaceutically acceptable salt or hydrate thereof,
wherein X₁ is selected from the group consisting of optionally substituted aryl and optionally substituted heteroaryl.

130. The linker according to claim 129, or a tautomer, a mesomer, a racemate, an enantiomer, a diastereomer, or a mixture thereof, or a pharmaceutically acceptable salt or hydrate thereof,
wherein X₁ is optionally substituted phenyl.

131. The linker according to claim 129, or a tautomer, a mesomer, a racemate, an enantiomer, a diastereomer, or a mixture thereof, or a pharmaceutically acceptable salt or hydrate thereof,
wherein X₁ is optionally substituted pyridinyl.

132. The linker according to any one of claims 129-131, or a tautomer, a mesomer, a racemate, an enantiomer, a diastereomer, or a mixture thereof, or a pharmaceutically acceptable salt or hydrate thereof,
wherein, m1 is 0.

133. The linker according to any one of claims 129-132, or a tautomer, a mesomer, a racemate, an enantiomer, a diastereomer, or a mixture thereof, or a pharmaceutically acceptable salt or hydrate thereof,
wherein, m2 is 1.

134. The linker according to any one of claims 124-125, or a tautomer, a mesomer, a racemate, an enantiomer, a diastereomer, or a mixture thereof, or a pharmaceutically acceptable salt or hydrate thereof,
wherein X₁ is optionally substituted

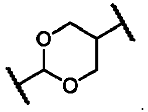

135. The linker according to any one of claims 124-125, or a tautomer, a mesomer, a racemate, an enantiomer, a diastereomer, or a mixture thereof, or a pharmaceutically acceptable salt or hydrate thereof,
wherein X₁ is optionally substituted cyclohexyl.

136. The linker according to any one of claims 134-135, or a tautomer, a mesomer, a racemate, an enantiomer, a diastereomer, or a mixture thereof, or a pharmaceutically acceptable salt or hydrate thereof,
wherein, m1 is 1.

137. The linker according to any one of claims 134-136, or a tautomer, a mesomer, a racemate, an enantiomer, a diastereomer, or a mixture thereof, or a pharmaceutically acceptable salt or hydrate thereof,
wherein, m2 is 0.

138. The linker according to any one of claims 124-137, or a tautomer, a mesomer, a racemate, an enantiomer, a diastereomer, or a mixture thereof, or a pharmaceutically acceptable salt or hydrate thereof,
wherein L₂ is a structure selected from the group consisting of optionally substituted 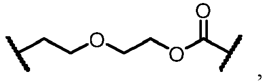 optionally substituted 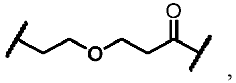 optionally substituted 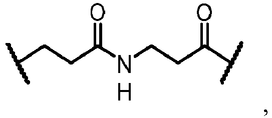 optionally substituted 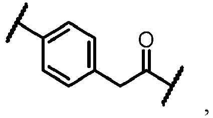 optionally substituted 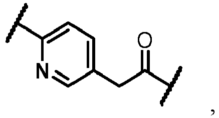 optionally substituted 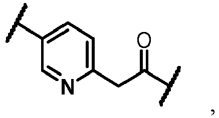 optionally substituted 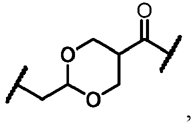 and optionally substituted

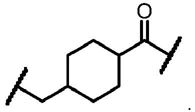

139. The linker according to any one of claims 124-138, or a tautomer, a mesomer, a racemate, an enantiomer, a diastereomer, or a mixture thereof, or a pharmaceutically acceptable salt or hydrate thereof,
wherein L₃ is a peptide residue selected from the group consisting of -glycine-phenylalanine-glycine- (-Gly-Phe-Gly-), -glycine-glycine-phenylalanine-glycine- (-Gly-Gly-Phe-Gly-), and - valine-citrulline- (-Val-Cit-).

140. The linker according to any one of claims 124-139, or a tautomer, a mesomer, a racemate, an enantiomer, a diastereomer, or a mixture thereof, or a pharmaceutically acceptable salt or hydrate thereof,
wherein L₃ is a structure selected from the group consisting of

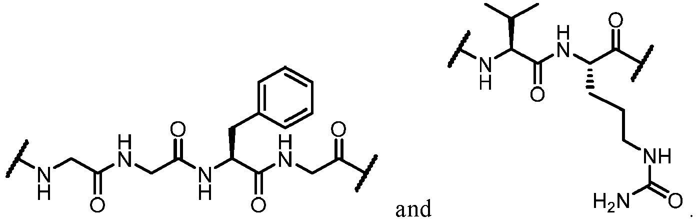

141. The linker according to any one of claims 124-140, or a tautomer, a mesomer, a racemate, an enantiomer, a diastereomer, or a mixture thereof, or a pharmaceutically acceptable salt or hydrate thereof,
wherein L₄ is optionally substituted -L₄ₐ-NR₄-CH₂-L_{4b}-,
L₄ₐ is absent or L₄ₐ is optionally substituted 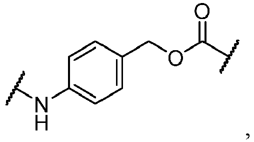
L_{4b} is absent or L_{4b} is optionally substituted 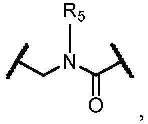
R₄ and R₅ are each independently selected from the group consisting of hydrogen and optionally substituted alkyl.

142. The linker according to claim 141, or a tautomer, a mesomer, a racemate, an enantiomer, a diastereomer, or a mixture thereof, or a pharmaceutically acceptable salt or hydrate thereof,
wherein L₄ is optionally substituted

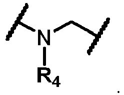

143. The linker according to claim 141, or a tautomer, a mesomer, a racemate, an enantiomer, a diastereomer, or a mixture thereof, or a pharmaceutically acceptable salt or hydrate thereof,
wherein L₄ is a structure selected from the group consisting of optionally substituted 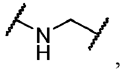 optionally substituted 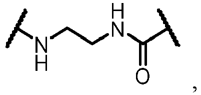 optionally substituted 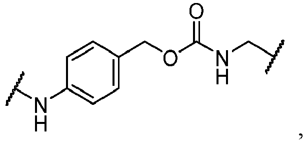 optionally substituted 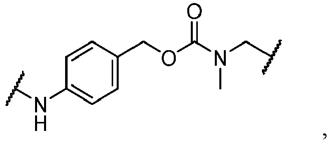 and optionally substituted

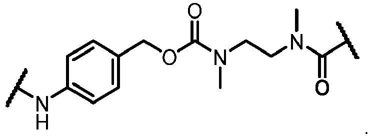

144. The linker according to any one of claims 124-143, or a tautomer, a mesomer, a racemate, an enantiomer, a diastereomer, or a mixture thereof, or a pharmaceutically acceptable salt or hydrate thereof,
wherein L₅ is optionally substituted 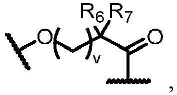 and L₅ is directly linked to the following structure: 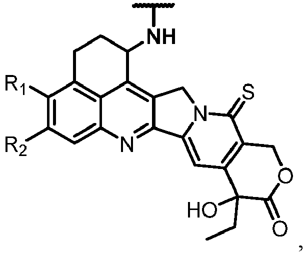
R₆ and R₇ are each independently selected from the group consisting of hydrogen, deuterium, optionally substituted alkyl, optionally substituted haloalkyl, optionally substituted cycloalkyl, and optionally substituted cycloalkyl-alkyl,
or R₆ and R₇ together with the atom to which they are attached form an optionally substituted cycloalkyl,
R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
wherein, v is a number that is at least 0.

145. The linker according to any one of claims 124-144, or a tautomer, a mesomer, a racemate, an enantiomer, a diastereomer, or a mixture thereof, or a pharmaceutically acceptable salt or hydrate thereof,
wherein L₅ is optionally substituted 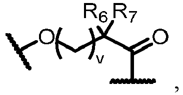 and L₅ is directly linked to the following structure: 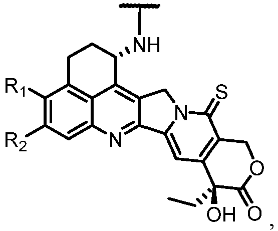
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl,
or R₆ and R₇ together with the atom to which they are attached form an optionally substituted cycloalkyl,
R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
wherein, v is a number that is at least 0.

146. The linker according to any one of claims 124-145, or a tautomer, a mesomer, a racemate, an enantiomer, a diastereomer, or a mixture thereof, or a pharmaceutically acceptable salt or hydrate thereof,
wherein L₅ is optionally substituted 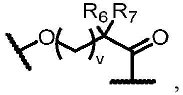 and L₅ is directly linked to the following structure: 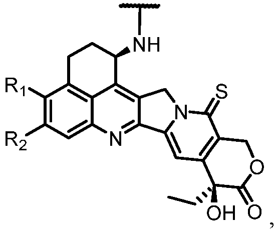
R₆ and R₇ are each independently selected from the group consisting of hydrogen deuterium, optionally substituted alkyl, optionally substituted haloalkyl, optionally substituted cycloalkyl, and optionally substituted cycloalkyl-alkyl,
or R₆ and R₇ together with the atom to which they are attached form an optionally substituted cycloalkyl,
R₁ and R₂ are each independently selected from the group consisting of hydrogen, deuterium, halogen, optionally substituted C₁-C₈ alkyl, optionally substituted C₁-C₈ haloalkyl, and optionally substituted C₁-C₈ deuterated alkyl;
or, R₁ and R₂ together with the carbon atom to which they are attached form a structure selected from the group consisting of optionally substituted aliphatic cyclyl, optionally substituted aliphatic heterocyclyl, optionally substituted aryl, and optionally substituted heteroaryl;
wherein, v is a number that is at least 0.

147. The linker according to any one of claims 144-146, or a tautomer, a mesomer, a racemate, an enantiomer, a diastereomer, or a mixture thereof, or a pharmaceutically acceptable salt or hydrate thereof,
wherein R₆ is hydrogen.

148. The linker according to any one of claims 144-146, or a tautomer, a mesomer, a racemate, an enantiomer, a diastereomer, or a mixture thereof, or a pharmaceutically acceptable salt or hydrate thereof,
wherein R₆ is optionally substituted methyl.

149. The linker according to claim 148, or a tautomer, a mesomer, a racemate, an enantiomer, a diastereomer, or a mixture thereof, or a pharmaceutically acceptable salt or hydrate thereof,
wherein R₆ is fluorine substituted methyl.

150. The linker according to any one of claims 148-149, or a tautomer, a mesomer, a racemate, an enantiomer, a diastereomer, or a mixture thereof, or a pharmaceutically acceptable salt or hydrate thereof,
wherein R₆ is trifluoromethyl.

151. The linker according to any one of claims 144-146, or a tautomer, a mesomer, a racemate, an enantiomer, a diastereomer, or a mixture thereof, or a pharmaceutically acceptable salt or hydrate thereof,
wherein R₆ is optionally substituted cycloalkyl.

152. The linker according to any one of claims 144-151, or a tautomer, a mesomer, a racemate, an enantiomer, a diastereomer, or a mixture thereof, or a pharmaceutically acceptable salt or hydrate thereof,
wherein R₇ is hydrogen.

153. The linker according to any one of claims 144-146, or a tautomer, a mesomer, a racemate, an enantiomer, a diastereomer, or a mixture thereof, or a pharmaceutically acceptable salt or hydrate thereof,
wherein R₆ and R₇ together with the atom to which they are attached form an optionally substituted cyclopropyl.

154. The linker according to claim 153, or a tautomer, a mesomer, a racemate, an enantiomer, a diastereomer, or a mixture thereof, or a pharmaceutically acceptable salt or hydrate thereof,
wherein R₆ and R₇ together with the atom to which they are attached form an optionally substituted cyclobutyl.

155. The linker according to any one of claims 144-154, or a tautomer, a mesomer, a racemate, an enantiomer, a diastereomer, or a mixture thereof, or a pharmaceutically acceptable salt or hydrate thereof,
wherein, v is 0.

156. The linker according to any one of claims 144-154, or a tautomer, a mesomer, a racemate, an enantiomer, and a diastereomer, or a mixture thereof, or a pharmaceutically acceptable salt or hydrate thereof,
wherein, v is 1.

157. The linker according to any one of claims 124-156, or a tautomer, a mesomer, a racemate, an enantiomer, a diastereomer, or a mixture thereof, or a pharmaceutically acceptable salt or hydrate thereof,
wherein L₅ a structure selected from the group consisting of optionally substituted 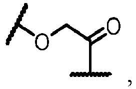 optionally substituted 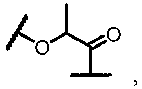 optionally substituted 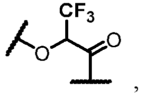 optionally substituted 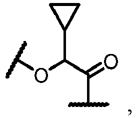 optionally substituted 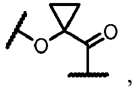 optionally substituted 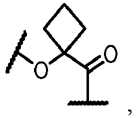 optionally substituted 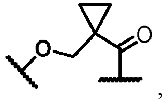 and optionally substituted

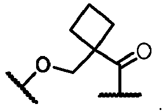

158. A linker, or a tautomer, a mesomer, a racemate, an enantiomer, a diastereomer, or a mixture thereof, or a pharmaceutically acceptable salt or hydrate thereof, wherein the linker comprises structures selected from the group consisting of 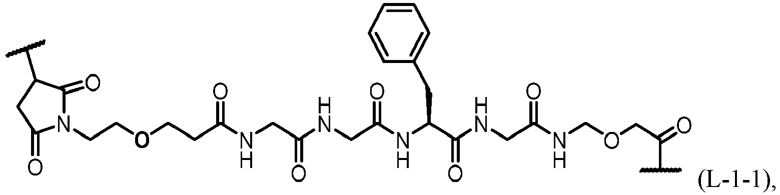 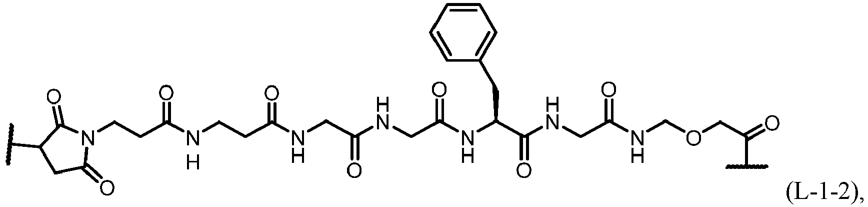 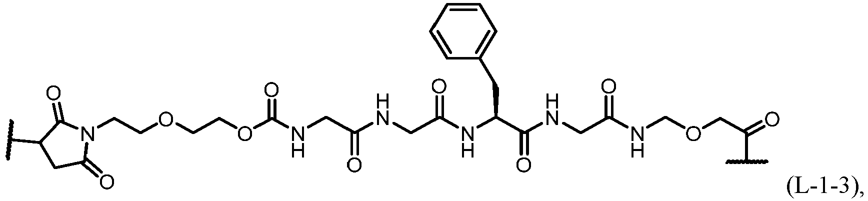 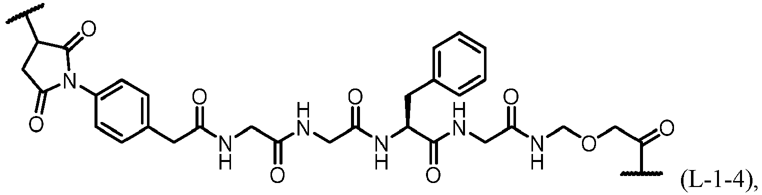 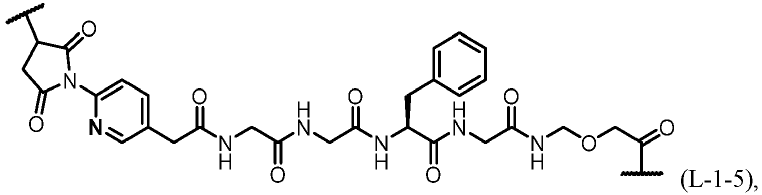 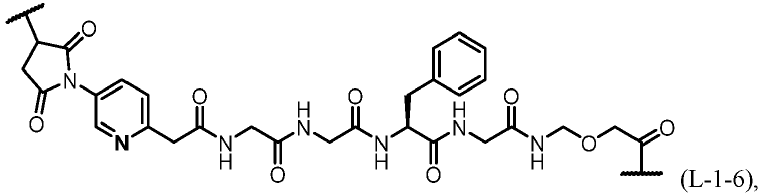 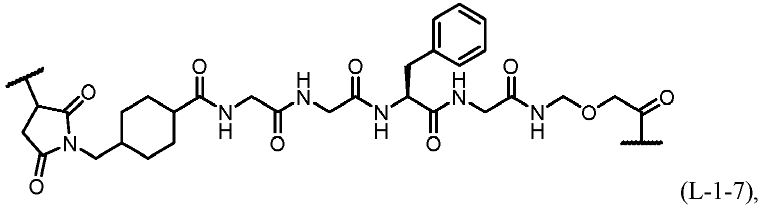 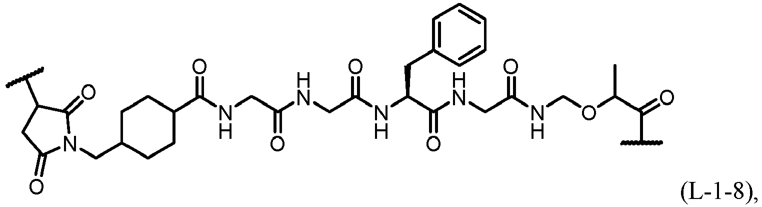 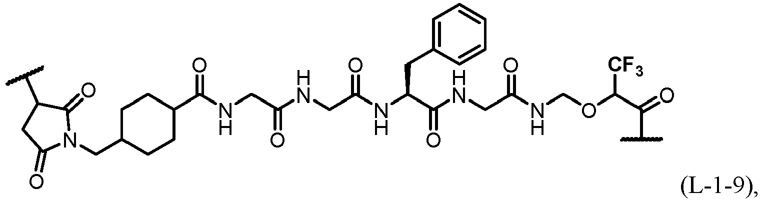 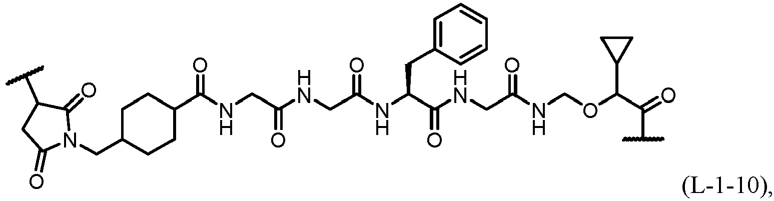 and

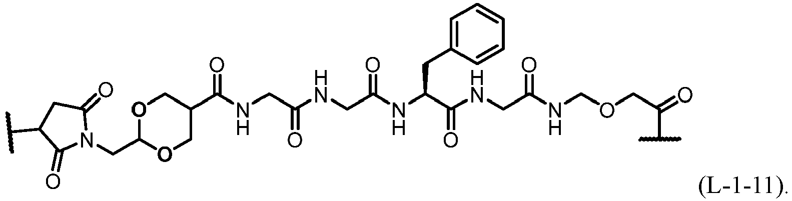

159. A linker as represented in formula (L-2), for obtaining a ligand-drug conjugate formed by linking a drug unit to a ligand via the linker:
-L₁-L₂-L₃-L₄-L₅- (L-2),
wherein L₁ is optionally substituted 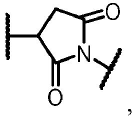
L₂ is a structure selected from the group consisting of optionally substituted 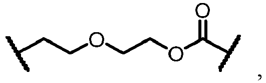 optionally substituted 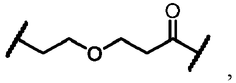 optionally substituted 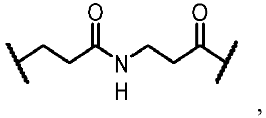 optionally substituted 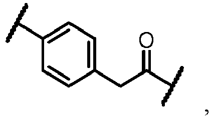 optionally substituted 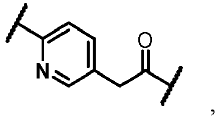 optionally substituted 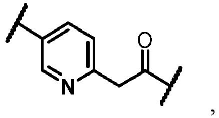 optionally substituted 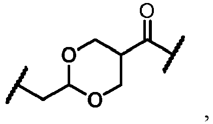 optionally substituted 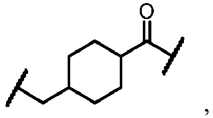 and optionally substituted 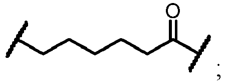
L₃ is a structure selected from the group consisting of 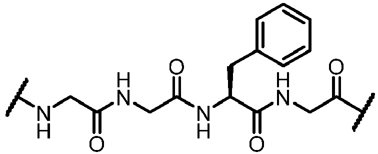 and 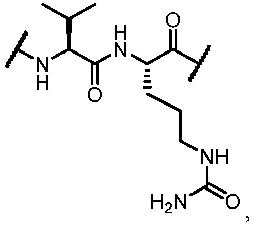
L₄ is optionally substituted -L₄ₐ-NR₄-CH₂-L_{4b}-,
L₄ₐ is absent or L₄ₐ is optionally substituted 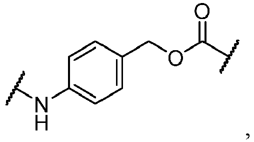
L_{4b} is absent or L_{4b} is optionally substituted 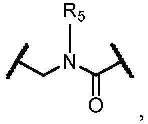 L₄ₐ and L_{4b} are not absent at the same time,
R ₄ and R ₅ are each independently selected from the group consisting of hydrogen and optionally substituted alkyl,
L₅ is a structure selected from the group consisting of optionally substituted 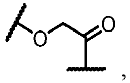 optionally substituted 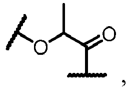 optionally substituted 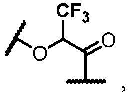 optionally substituted 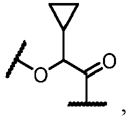 optionally substituted 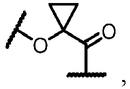 optionally substituted 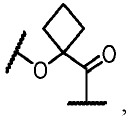 optionally substituted 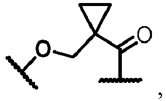 and optionally substituted

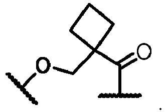

160. The linker according to claim 159, or a tautomer, a mesomer, a racemate, an enantiomer, a diastereomer, or a mixture thereof, or a pharmaceutically acceptable salt or hydrate thereof,
L₄ is optionally substituted

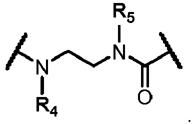

161. The linker according to claim 159, or a tautomer, a mesomer, a racemate, an enantiomer, and a diastereomer, or a mixture thereof, or a pharmaceutically acceptable salt or hydrate thereof,
wherein L₄ is optionally substituted

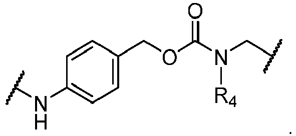

162. The linker according to claim 159, or a tautomer, a mesomer, a racemate, an enantiomer, a diastereomer, or a mixture thereof, or a pharmaceutically acceptable salt or hydrate thereof,
wherein L₄ is optionally substituted

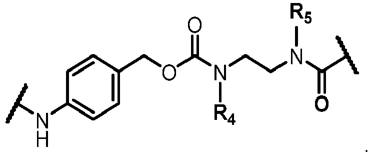

163. The linker according to claim 159, or a tautomer, a mesomer, a racemate, an enantiomer, a diastereomer, or a mixture thereof, or a pharmaceutically acceptable salt or hydrate thereof,
wherein L4 is a structure selected from the group consisting of optionally substituted 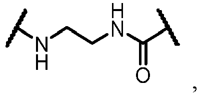 optionally substituted 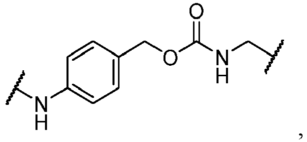 optionally substituted 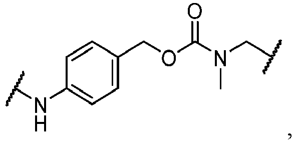 and optionally substituted

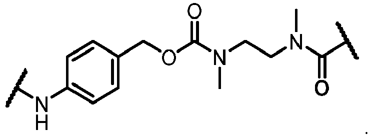

164. A linker, or a tautomer, a mesomer, a racemate, an enantiomer, a diastereomer, or a mixture thereof, or a pharmaceutically acceptable salt or hydrate thereof, wherein the linker comprises structures selected from the group consisting of

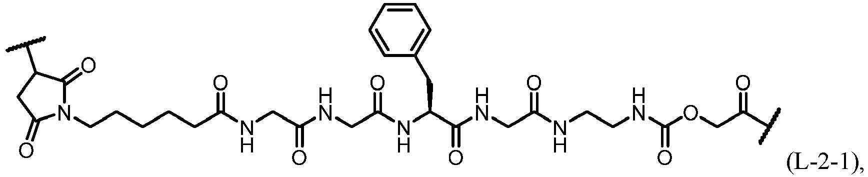

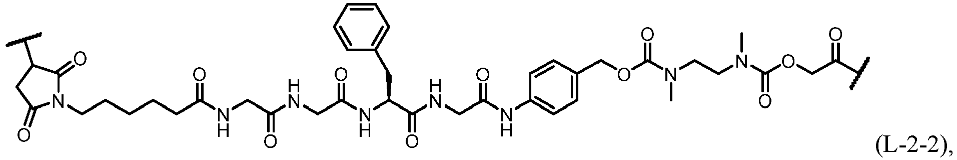

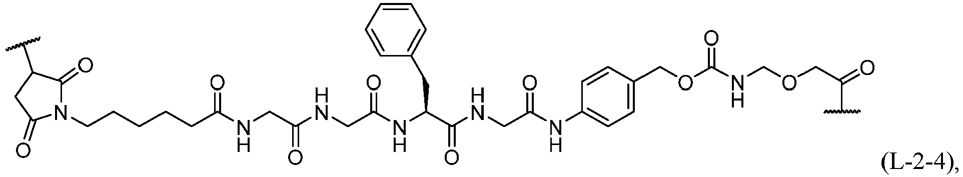

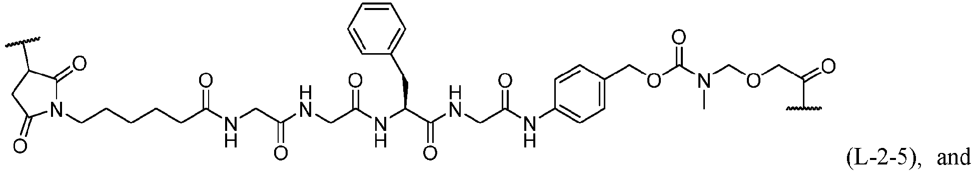

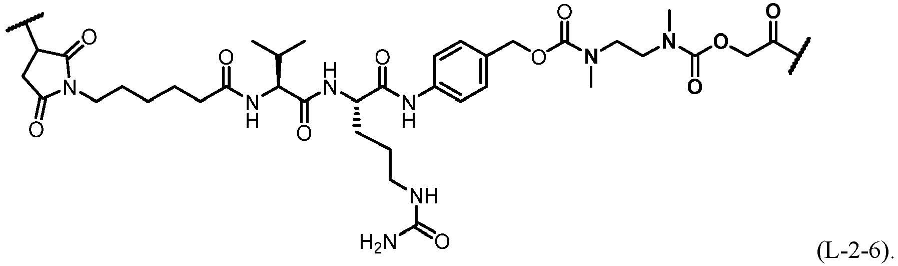

165. A pharmaceutical composition comprising the ligand conjugate according to any one of claims 1-70, or a tautomer, a mesomer, a racemate, an enantiomer, a diastereomer, or a mixture thereof, or a pharmaceutically acceptable salt, a prodrug or a solvate thereof, and/or a compound according to any one of claims 71-123, and optionally pharmaceutically acceptable carriers.

166. The use of the ligand conjugate according to any one of claims 1-70, or a tautomer, a mesomer, a racemate, an enantiomer, a diastereomer, or a mixture thereof, or a pharmaceutically acceptable salt, a prodrug or a solvate thereof, and/or the compound according to any one of claims 71-123, or a tautomer, a mesomer, a racemate, an enantiomer, a diastereomer, or a mixture thereof, or a pharmaceutically acceptable salt, or a hydrate thereof, and/or the pharmaceutical composition according to claim 165 in the preparation a drug for the treatment and/or prevention of tumors.

167. The use according to claim 166, wherein, the tumor is selected from tumors associated with the expression of the target consisting of HER2 and TROP2.

168. The use according to any one of claims 167, wherein, the tumor associated with the target expression comprises a tumor with high target expression and/or a tumor with positive target expression.

169. The use according to any one of claims 166-168, wherein, the tumor comprises solid tumors and/or hematological malignancies.

170. The use according to any one of claims 166-169, wherein, the tumor is selected from the group consisting of breast cancer, ovarian cancer, cervical cancer, endometrial cancer, urothelial cancer, lung cancer, prostate cancer, colorectal cancer, gastric cancer, esophageal cancer, bladder cancer, kidney cancer, pancreatic cancer, thyroid cancer, and head and neck cancer.
